# EUROPÄISCHE PATENTANMELDUNG

(11) **EP 3 424 926 A1**
(43) Veröffentlichungstag der Anmeldung: **09.01.2019**
(21) Anmeldenummer: 18185103.1
(22) Anmeldetag: 18.08.2003
(51) Int. Cl.: C07D 473/04, A61K 31/522, A61P 3/10

(54) **8-[3-AMINO-PIPERIDIN-1-YL]-XANTHINE, DEREN HERSTELLUNG UND DEREN VERWENDUNG ALS ARZNEIMITTEL**

(30) Priorität: 21.08.2002 DE 10238243; 20.03.2003 DE 10312353
(62) Teilanmeldung aus: 08159140.6
(71) Anmelder: Boehringer Ingelheim Pharma GmbH & Co. KG, 55216 Ingelheim am Rhein (DE)
(72) Erfinder: Himmelsbach, Frank, 55216 INGELHEIM AM RHEIN (DE); Langkopf, Elke, 55216 INGELHEIM AM RHEIN (DE); Eckhardt, Matthias, 55216 INGELEHEIM AM RHEIN (DE); Mark, Michael, 55216 INGELHEIM AM RHEIN (DE); Maier, Roland, 55216 INGELHEIM AM RHEIN (DE); Lotz, Ralf, 55216 INGELHEIM AM RHEIN (DE); Tadayyon, Mohammad, 55216 INGELHEIM AM RHEIN (DE)
(74) Vertreter: Simon, Elke Anna Maria

(57) **Zusammenfassung**

Die vorliegende Erfindung betrifft substituierte Xanthine der allgemeinen Formel 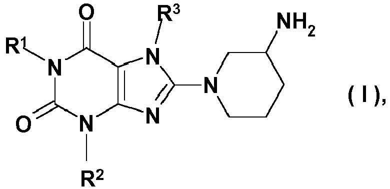 in der R¹ bis R³ wie in den Ansprüchen 1 bis 16 definiert sind, deren Tautomere, deren Stereoisomere, deren Gemische, deren Prodrugs und deren Salze, welche wertvolle pharmakologische Eigenschaften aufweisen, insbesondere eine Hemmwirkung auf die Aktivität des Enzyms Dipeptidylpeptidase-IV (DPP-IV).

## Beschreibung

Gegenstand der vorliegenden Erfindung sind neue substituierte Xanthine der allgemeinen Formel 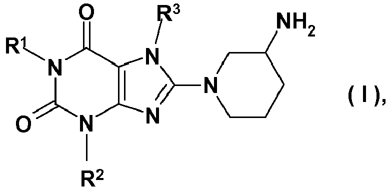 deren Tautomere, Enantiomere, Diastereomere, deren Gemische, deren Prodrugs und deren Salze, insbesonders deren physiologisch verträgliche Salze mit anorganischen oder organischen Säuren oder Basen, welche wertvolle pharmakologische Eigenschaften aufweisen, insbesondere eine Hemmwirkung auf die Aktivität des Enzyms Dipeptidylpeptidase-IV (DPP-IV), deren Herstellung, deren Verwendung zur Prävention oder Behandlung von Krankheiten oder Zuständen, die in Zusammenhang mit einer erhöhten DPP-IV Aktivität stehen oder die durch Reduktion der DPP-IV Aktivität verhindert oder gemildert werden können, insbesondere von Diabetes mellitus Typ I oder Typ II, die eine Verbindung der allgemeinen Formel (I) oder ein physiologisch verträgliches Salz davon enthaltenden Arzneimittel sowie Verfahren zu deren Herstellung.

In der obigen Formel I bedeuten
R¹ eine Methylgruppe,
eine Methylgruppe, die durch eine Dimethylaminocarbonyl-, Pyrrolidin-1-ylcarbonyl-, Piperidin-1-ylcarbonyl-, tert.-Butylcarbonyl- oder eine Cyclohexylcarbonyl-Gruppe substituiert ist,
eine Methylgruppe, die durch eine Naphthyl-, Methylnaphthyl-, Methoxynaphthyl-, Nitronaphthyl- oder Dimethylaminonaphthyl-Gruppe substituiert ist,
eine Methylgruppe, die durch eine 2-Phenylethenyl- oder eine Biphenylyl-Gruppe substituiert ist,
eine Methylgruppe, die durch eine Phenyloxadiazolyl-, 5-Methyl-3-phenyl-isoxazolyl-, Phenylpyridinyl-, Indolyl-, Benzothiophenyl-, Chinolinyl-, Isochinolinyl-, Methylisochinolinyl-, (Methoxycarbonylmethylamino)-isochinolinyl-, Cinnolinyl-, Chinazolinyl-, Methylchinazolinyl-, 1,2-Dihydro-1-methyl-2-oxo-chinolinyl-, 1,2-Dihydro-2-methyl-1-oxo-isochinolinyl-, 3,4-Dihydro-4-oxo-phthalazinyl-, 3,4-Dihydro-3-methyl-4-oxo-phthalazinyl-, 3,4-Dihydro-4-oxo-chinazolinyl-, 3,4-Dihydro-3-methyl-4-oxo-chinazolinyl- oder eine 2-Oxo-2H-chromenyl-Gruppe substituiert ist,
eine 2-Methoxyethyl-, 2-Phenyloxyethyl- oder 2-Cyanethyl-Gruppe,
eine Phenylcarbonylmethyl- oder eine 1-(Phenylcarbonyl)-ethyl-Gruppe,
eine Phenylcarbonylmethylgruppe, in der der Phenylteil durch eine Amino-, Cyanmethylamino-, Methylcarbonylamino-, Ethylcarbonylamino-, Isopropylcarbonylamino-, Methoxycarbonylamino-, (Ethyloxycarbonylamino)-carbonylamino- oder eine 2-Oxo-imidazolidin-1-yl-Gruppe substituiert ist,
eine Phenylcarbonylmethylgruppe, in der der Phenylteil durch eine Carboxy-, Methoxycarbonyl-, Ethyloxycarbonyl-, Aminocarbonyl-, Methylaminocarbonyl-, Dimethylaminocarbonyl- oder Morpholin-4-ylcarbonylgruppe substituiert ist,
eine Phenylcarbonylmethylgruppe, in der der Phenylteil durch eine Methylsulfanyl-, Methylsulfinyl- oder Methylsulfonylgruppe substituiert ist,
eine Phenylcarbonylmethylgruppe, in der der Phenylteil durch eine Carboxymethoxy-, Ethyloxycarbonylmethoxy-, Isopropyloxycarbonylmethoxy-, Aminocarbonylmethoxy-, Methylaminocarbonylmethoxy-, Ethylaminocarbonylmethoxy-, Isopropylaminocarbonylmethoxy-, Dimethylaminocarbonylmethoxy-, Pyrrolidin-1-ylcarbonylmethoxy- oder Morpholin-4-ylcarbonylmethoxy-Gruppe substituiert ist,
eine Phenylcarbonylmethylgruppe, in der der Phenylteil durch eine 1-(Methoxycarbonyl)-ethyloxy- oder eine 1-(Aminocarbonyl)-ethyloxy-Gruppe substituiert ist,
eine Phenylcarbonylmethylgruppe, in der der Phenylteil durch eine Methylsulfinylmethoxy-Gruppe substituiert ist,
eine Phenylcarbonylmethylgruppe, in der der Phenylteil durch zwei Methoxygruppen substituiert ist oder
eine Phenylcarbonylmethylgruppe, in der im Phenylteil zwei benachbarte Wasserstoffatome durch eine -O-CH₂-O-, -O-CH₂-CH₂-O- oder eine -N(CH₃)-CO-O-Gruppe ersetzt sind,
R² ein Wasserstoffatom,
eine Methyl-, Isopropyl-, 2-Propen-1-yl-, 2-Propin-1-yl- oder Phenylgruppe oder
eine Cyanmethyl- oder Methoxycarbonylmethylgruppe und
R³ eine 2-Cyanbenzyl- oder 2,6-Dicyanbenzylgruppe,
eine 2-Methyl-2-propen-1-yl-, 2-Chlor-2-propen-1-yl- oder 3-Brom-2-propen-1-yl-Gruppe
eine 2-Buten-1-yl-, 3-Methyl-2-buten-1-yl- oder 2,3-Dimethyl-2-buten-1-yl-Gruppe,
eine 2-Butin-1-yl-Gruppe,
eine 1-Cyclopenten-1-ylmethyl-Gruppe oder
eine 2-Furanylmethyl-Gruppe.

Die bei der Definition der vorstehend erwähnten Reste erwähnten Carboxygruppen können durch eine in-vivo in eine Carboxygruppe überführbare Gruppe oder durch eine unter physiologischen Bedingungen negativ geladene Gruppe ersetzt sein,
desweiteren können die bei der Definition der vorstehend erwähnten Reste erwähnten Amino- und Iminogruppen durch einen in-vivo abspaltbaren Rest substituiert sein. Derartige Gruppen werden beispielsweise in der WO 98/46576 und von N.M. Nielsen et al. in International Journal of Pharmaceutics 39, 75-85 (1987) beschrieben.

Verbindungen, die eine in-vivo abspaltbare Gruppe enthalten, sind Prodrugs der entsprechenden Verbindungen, bei denen diese in-vivo abspaltbare Gruppe abgespalten ist.

Unter einer in-vivo in eine Carboxygruppe überführbare Gruppe ist beispielsweise eine Hydroxymethylgruppe, eine mit einem Alkohol veresterte Carboxygruppe, in der der alkoholische Teil vorzugsweise ein C₁₋₆-Alkanol, ein Phenyl-C₁₋₃-alkanol, ein C₃₋₉-Cycloalkanol, wobei ein C₅₋₈-Cycloalkanol zusätzlich durch ein oder zwei C₁₋₃-Alkylgruppen substituiert sein kann, ein C₅₋₈-Cycloalkanol, in dem eine Methylengruppe in 3- oder 4-Stellung durch ein Sauerstoffatom oder durch eine gegebenenfalls durch eine C₁₋₃-Alkyl-, Phenyl-C₁₋₃-alkyl-, Phenyl-C₁₋₃-alkyloxy-carbonyl- oder C₂₋₆-Alkanoylgruppe substituierte Iminogruppe ersetzt ist und der Cycloalkanolteil zusätzlich durch ein oder zwei C₁₋₃-Alkylgruppen substituiert sein kann, ein C₄₋₇-Cycloalkenol, ein C₃₋₅-Alkenol, ein Phenyl-C₃₋₅-alkenol, ein C₃₋₅-Alkinol oder Phenyl-C₃₋₅-alkinol mit der Maßgabe, daß keine Bindung an das Sauerstoffatom von einem Kohlenstoffatom ausgeht, welches eine Doppel- oder Dreifachbindung trägt, ein C₃₋₈-CyCloalkyl-C₁₋₃-alkanol, ein Bicycloalkanol mit insgesamt 8 bis 10 Kohlenstoffatomen, das im Bicycloalkylteil zusätzlich durch eine oder zwei C₁₋₃-Alkylgruppen substituiert sein kann, ein 1,3-Dihydro-3-oxo-1-isobenzfuranol oder ein Alkohol der Formel

Rₚ-CO-O-(R_{q}CRᵣ)-OH,

in dem
Rₚ eine C₁₋₈-Alkyl-, C₅₋₇-Cycloalkyl-, C₁₋₈-Alkyloxy-, C₅₋₇-Cycloalkyloxy-, Phenyl- oder Phenyl- C₁₋₃-alkylgruppe,
R_{q} ein Wasserstoffatom, eine C₁₋₃-Alkyl-, C₅₋₇-Cycloalkyl- oder Phenylgruppe und
Rᵣ ein Wasserstoffatom oder eine C₁₋₃-Alkylgruppe darstellen,
unter einer unter physiologischen Bedingungen negativ geladenen Gruppe wie eine Tetrazol-5-yl-, Phenylcarbonylaminocarbonyl-, Trifluormethylcarbonylaminocarbonyl-, C₁₋₆-Alkylsulfonylamino-, Phenylsulfonylamino-, Benzylsulfonylamino-, Trifluormethylsulfonylamino-, C₁₋₆-Alkylsulfonylaminocarbonyl-, Phenylsulfonylamino-carbonyl-, Benzylsulfonylaminocarbonyl- oder Perfluor-C₁₋₆-alkylsulfonylamino-carbonylgruppe
und unter einem von einer Imino- oder Aminogruppe in-vivo abspaltbaren Rest beispielsweise eine Hydroxygruppe, eine Acylgruppe wie eine gegebenenfalls durch Fluor-, Chlor-, Brom- oder Jodatome, durch C₁₋₃-Alkyl- oder C₁₋₃-Alkyloxygruppen mono- oder disubstituierte Phenylcarbonylgruppe, wobei die Substituenten gleich oder verschieden sein können, eine Pyridinoylgruppe oder eine C₁₋₁₆-Alkanoylgruppe wie die Formyl-, Acetyl-, Propionyl-, Butanoyl-, Pentanoyl- oder Hexanoylgruppe, eine 3,3,3-Trichlorpropionyl- oder Allyloxycarbonylgruppe, eine C₁₋₁₆-Alkyloxy-carbonyl- oder C₁₋₁₆-Alkylcarbonyloxygruppe, in denen Wasserstoffatome ganz oder teilweise durch Fluor- oder Chloratome ersetzt sein können, wie die Methoxycarbonyl-, Ethoxycarbonyl-, Propoxycarbonyl-, Isopropoxycarbonyl-, Butoxycarbonyl-, tert.-Butoxycarbonyl-, Pentoxycarbonyl-, Hexoxycarbonyl-, Octyloxycarbonyl-, Nonyloxycarbonyl-, Decyloxycarbonyl-, Undecyloxycarbonyl-, Dodecyloxycarbonyl-, Hexadecyloxycarbonyl-, Methylcarbonyloxy-, Ethylcarbonyloxy-, 2,2,2-Trichlorethylcarbonyloxy-, Propylcarbonyloxy-, Isopropylcarbonyloxy-, Butylcarbonyloxy-, tert.Butylcarbonyloxy-, Pentylcarbonyloxy-, Hexylcarbonyloxy-, Octylcarbonyloxy-, Nonylcarbonyloxy-, Decylcarbonyloxy-, Undecylcarbonyloxy-, Dodecylcarbonyloxy- oder Hexadecylcarbonyloxygruppe, eine Phenyl-C₁₋₆-alkyloxycarbonylgruppe wie die Benzyloxycarbonyl-, Phenylethoxycarbonyl- oder Phenylpropoxycarbonylgruppe, eine 3-Amino-propionylgruppe, in der die Aminogruppe durch C₁₋₆-Alkyl- oder C₃₋₇-Cycloalkylgruppen mono- oder disubstituiert und die Substituenten gleich oder verschieden sein können, eine C₁₋₃-Alkylsulfonyl-C₂₋₄-alkyloxycarbonyl-, C₁₋₃-Alkyloxy-C₂₋₄-alkyloxy-C₂₋₄-alkyloxycarbonyl-, Rₚ-CO-O-(R_{q}CRᵣ)-O-CO-, C₁₋₆-Alkyl-CO-NH-(RₛCRₜ)-O-CO- oder C₁₋₆-Alkyl-CO-O-(RₛCRₜ)-(RₛCRₜ)-O-CO-Gruppe, in denen Rₚ bis Rᵣ wie vorstehend erwähnt definiert sind,
   Rₛ und Rₜ, die gleich oder verschieden sein können, Wasserstoffatome oder C₁₋₃-Alkylgruppen darstellen,
   zu verstehen.

Ein erster Erfindungsgegenstand betrifft Verbindungen der allgemeinen Formel (I),in denen
R¹ eine Methylgruppe, die durch eine Dimethylaminocarbonyl-, Pyrrolidin-1-yl-carbonyl-, Piperidin-1-ylcarbonyl-, tert.-Butylcarbonyl- oder eine Cyclohexylcarbonyl-Gruppe substituiert ist,
eine Methylgruppe, die durch eine Naphthyl-, Methylnaphthyl-, Methoxynaphthyl-, Nitronaphthyl- oder (Dimethylamino)-naphthyl-Gruppe substituiert ist,
eine Methylgruppe, die durch eine 2-Phenylethenyl- oder eine Biphenylyl-Gruppe substituiert ist,
eine Methylgruppe, die durch eine Phenyl-oxadiazolyl-, 5-Methyl-3-phenyl-isoxazolyl-, Phenyl-pyridinyl-, Indolyl-, Benzothiophenyl-, Chinolinyl-, Isochinolinyl-, Methylisochinolinyl-, (Methoxycarbonylmethylamino)-isochinolinyl-, Cinnolinyl-, Chinazolinyl-, Methylchinazolinyl-, 1,2-Dihydro-1-methyl-2-oxo-chinolinyl-, 1,2-Dihydro-2-methyl-1-oxo-isochinolinyl-, 3,4-Dihydro-4-oxo-phthalazinyl-, 3,4-Dihydro-3-methyl-4-oxo-phthalazinyl-, 3,4-Dihydro-4-oxo-chinazolinyl-, 3,4-Dihydro-3-methyl-4-oxo-chinazolinyl- oder eine 2-Oxo-2H-chromenyl-Gruppe substituiert ist,
eine 2-Methoxyethyl-, 2-Phenyloxyethyl- oder 2-Cyanethyl-Gruppe,
eine Phenylcarbonylmethyl- oder eine 1-(Phenylcarbonyl)-ethyl-Gruppe,
eine Phenylcarbonylmethylgruppe, in der der Phenylteil durch eine Amino-, Cyanmethylamino-, Methylcarbonylamino-, Ethylcarbonylamino-, Isopropylcarbonylamino-, Methoxycarbonylamino-, (Ethyloxycarbonylamino)-carbonylamino- oder eine 2-Oxo-imidazolidin-1-yl-Gruppe substituiert ist,
eine Phenylcarbonylmethylgruppe, in der der Phenylteil durch eine Carboxy-, Methoxycarbonyl-, Ethyloxycarbonyl-, Aminocarbonyl-, Methylaminocarbonyl-, Dimethylaminocarbonyl- oder Morpholin-4-ylcarbonylgruppe substituiert ist,
eine Phenylcarbonylmethylgruppe, in der der Phenylteil durch eine Methylsulfanyl-, Methylsulfinyl- oder Methylsulfonylgruppe substituiert ist,
eine Phenylcarbonylmethylgruppe, in der der Phenylteil durch eine Carboxymethoxy-, Ethyloxycarbonylmethoxy-, Isopropyloxycarbonylmethoxy-, Aminocarbonylmethoxy-, Methylaminocarbonylmethoxy-, Ethylaminocarbonylmethoxy-, Isopropylamino-carbonylmethoxy-, Dimethylaminocarbonylmethoxy-, Pyrrolidin-1-ylcarbonylmethoxy- oder Morpholin-4-ylcarbonylmethoxy-Gruppe substituiert ist,
eine Phenylcarbonylmethylgruppe, in der der Phenylteil durch eine 1-(Methoxycarbonyl)-ethyloxy- oder eine 1-(Aminocarbonyl)-ethyloxy-Gruppe substituiert ist,
eine Phenylcarbonylmethylgruppe, in der der Phenylteil durch eine Methylsulfinylmethoxy-Gruppe substituiert ist,
eine Phenylcarbonylmethylgruppe, in der der Phenylteil durch zwei Methoxygruppen substituiert ist oder
eine Phenylcarbonylmethylgruppe, in der im Phenylteil zwei benachbarte Wasserstoffatome durch eine -O-CH₂-O-, -O-CH₂-CH₂-O- oder eine -N(CH₃)-CO-O-Gruppe ersetzt sind,
R² eine Methyl-, Isopropyl- oder Phenylgruppe
und
R³ eine 2-Methyl-2-propen-1-yl-, 2-Chlor-2-propen-1-yl- oder 3-Brom-2-propen-1-yl-Gruppe
eine 2-Buten-1-yl- oder 2,3-Dimethyl-2-buten-1-yl-Gruppe,
eine 2-Butin-1-yl-Gruppe,
eine 1-Cyclopenten-1-ylmethyl-Gruppe oder
eine 2-Furanylmethyl-Gruppe bedeuten,
sowie die Verbindungen
1-(2-Cyano-ethyl)-3-methyl-7-(2-cyano-benzyl)-8-(3-amino-piperidin-1-yl)-xanthin,
1-(2-{2-[(Ethoxycarbonyl)methoxy]-phenyl}-2-oxo-ethyl)-3-methyl-7-(3-methyl-2-buten-1-yl)-8-(3-amino-piperidin-1-yl)-xanthin,
1-(2-{2-[(Aminocarbonyl)methoxy]-phenyl}-2-oxo-ethyl)-3-methyl-7-(3-methyl-2-buten-1-yl)-8-(3-amino-piperidin-1-yl)-xanthin,
1-(2-{3-[(Methansulfinyl)methoxy]-phenyl}-2-oxo-ethyl)-3-methyl-7-(3-methyl-2-buten-1-yl)-8-(3-amino-piperidin-1-yl)-xanthin,
1-(1-Methyl-2-oxo-2-phenyl-ethyl)-3-methyl-7-(3-methyl-2-buten-1-yl)-8-(3-amino-piperidin-1-yl)-xanthin,
1-(2-Phenoxy-ethyl)-3-methyl-7-(2-cyano-benzyl)-8-(3-amino-piperidin-1-yl)-xanthin,
1-(2-Phenyl-2-oxo-ethyl)-7-(3-methyl-2-buten-1-yl)-8-(3-amino-piperidin-1-yl)-xanthin,
1-(2-{3-[(Ethoxycarbonyl)methoxy]-phenyl}-2-oxo-ethyl)-3-methyl-7-(3-methyl-2-buten-1-yl)-8-(3-amino-piperidin-1-yl)-xanthin,
1-(2-{2-[(Methylaminocarbonyl)methoxy]-phenyl}-2-oxo-ethyl)-3-methyl-7-(3-methyl-2-buten-1-yl)-8-(3-amino-piperidin-1-yl)-xanthin,
1-(2-{2-[(Dimethylaminocarbonyl)methoxy]-phenyl}-2-oxo-ethyl)-3-methyl-7-(3-methyl-2-buten-1-yl)-8-(3-amino-piperidin-1-yl)-xanthin,
1-(2-Methoxy-ethyl)-3-methyl-7-(2-cyano-benzyl)-8-(3-amino-piperidin-1-yl)-xanthin,
1-Methyl-3-[(methoxycarbonyl)methyl]-7-(2-cyano-benzyl)-8-(3-amino-piperidin-1-yl)-xanthin,
1-Methyl-3-cyanomethyl-7-(2-cyano-benzyl)-8-(3-amino-piperidin-1-yl)-xanthin,
1-Methyl-3-(2-propin-1-yl)-7-(2-cyano-benzyl)-8-(3-amino-piperidin-1-yl)-xanthin,
1-{2-[3-(2-Oxo-imidazolidin-1-yl)-phenyl]-2-oxo-ethyl}-3-methyl-7-(3-methyl-2-buten-1-yl)-8-(3-amino-piperidin-1-yl)-xanthin,
1-Methyl-3-(2-propen-1-yl)-7-(2-cyano-benzyl)-8-(3-amino-piperidin-1-yl)-xanthin,
1-(2-{2-[(Ethylcarbonyl)amino]-phenyl}-2-oxo-ethyl)-3-methyl-7-(3-methyl-2-buten-1-yl)-8-(3-amino-piperidin-1-yl)-xanthin,
1-Methyl-3-phenyl-7-(2-cyano-benzyl)-8-(3-amino-piperidin-1-yl)-xanthin,
1-[2-(2-Amino-phenyl)-2-oxo-ethyl]-3-methyl-7-(3-methyl-2-buten-1-yl)-8-((S)-3-amino-piperidin-1-yl)-xanthin,
1-(2-Phenyl-2-oxo-ethyl)-3-cyanomethyl-7-(3-methyl-2-buten-1-yl)-8-(3-amino-piperidin-1-yl)-xanthin,
1-[(Chinolin-2-yl)methyl]-3-methyl-7-(3-methyl-2-buten-1-yl)-8-(3-amino-piperidin-1-yl)-xanthin,
1-[(2-Oxo-2*H*-chromen-4-yl)methyl]-3-methyl-7-(3-methyl-2-buten-1-yl)-8-(3-amino-piperidin-1-yl)-xanthin,
1-[(Cinnolin-4-yl)methyl]-3-methyl-7-(3-methyl-2-buten-1-yl)-8-(3-amino-piperidin-1-yl)-xanthin,
1-[(1-Methyl-2-oxo-1,2-dihydro-chinolin-4-yl)methyl]-3-methyl-7-(3-methyl-2-buten-1-yl)-8-(3-amino-piperidin-1-yl)-xanthin,
1-[(4-Oxo-3,4-dihydro-phtha!azin-1-yl)methyl]-3-methyl-7-(3-methyl-2-buten-1-yl)-8-(3-amino-piperidin-1-yl)-xanthin,
1-[(Chinazolin-4-yl)methyl]-3-methyl-7-(3-methyl-2-buten-1-yl)-8-(3-amino-piperidin-1-yl)-xanthin,
1-[(5-Methyl-3-phenyl-isoxazol-4-yl)methyl]-3-methyl-7-(3-methyl-2-buten-1-yl)-8-(3-am ino-piperidin-1-yl)-xanthin,
1-[(Isochinolin-3-yl)methyl]-3-methyl-7-(3-methyl-2-buten-1-yl)-8-(3-amino-piperidin-1-yl)-xanthin,
1-[(3-Phenyl-[1,2,4]oxadiazol-5-yl)methyl]-3-methyl-7-(3-methyl-2-buten-1-yl)-8-(3-am ino-piperidin-1-yl)-xanthin,
1-[(4-Phenyl-pyridin-2-yl)methyl]-3-methyl-7-(3-methyl-2-buten-1-yl)-8-(3-amino-piperidin-1-yl)-xanthin,
1-[(5-Phenyl-pyridin-2-yl)methyl]-3-methyl-7-(3-methyl-2-buten-1-yl)-8-(3-amino-piperidin-1-yl)-xanthin,
1-[(3-Methyl-4-oxo-3,4-dihydro-phthalazin-1-yl)methyl]-3-methyl-7-(3-methyl-2-buten-1-yl)-8-(3-amino-piperidin-1-yl)-xanthin,
1-[2-(3-Methylsulfanyl-phenyl)-2-oxo-ethyl]-3-methyl-7-(3-methyl-2-buten-1-yl)-8-(3-am ino-piperidin-1-yl)-xanthin,
1-[2-(3-Methansulfinyl-phenyl)-2-oxo-ethyl]-3-methyl-7-(3-methyl-2-buten-1-yl)-8-(3-am ino-piperidin-1-yl)-xanthin,
1-[2-(3-Methansulfonyl-phenyl)-2-oxo-ethyl]-3-methyl-7-(3-methyl-2-buten-1-yl)-8-(3-am ino-piperidin-1-yl)-xanthin,
1-[2-(3-Carboxy-phenyl)-2-oxo-ethyl]-3-methyl-7-(3-methyl-2-buten-1-yl)-8-(3-amino-piperidin-1-yl)-xanthin,
1-[2-(3-Methoxycarbonyl-phenyl)-2-oxo-ethyl]-3-methyl-7-(3-methyl-2-buten-1-yl)-8-(3-amino-piperidin-1-yl)-xanthin,
1-{2-[3-(Methylaminocarbonyl)-phenyl]-2-oxo-ethyl}-3-methyl-7-(3-methyl-2-buten-1-yl)-8-(3-amino-piperidin-1-yl)-xanthin,
1-{2-[3-(Dimethylaminocarbonyl)-phenyl]-2-oxo-ethyl}-3-methyl-7-(3-methyl-2-buten-1-yl)-8-(3-amino-piperidin-1-yl)-xanthin,
1-{2-[3-(Morpholin-4-yl-carbonyl)-phenyl]-2-oxo-ethyl}-3-methyl-7-(3-methyl-2-buten-1-yl)-8-(3-amino-piperidin-1-yl)-xanthin,
1-[2-(2-Carboxy-phenyl)-2-oxo-ethyl]-3-methyl-7-(3-methyl-2-buten-1-yl)-8-(3-amino-piperidin-1-yl)-xanthin,
1-[2-(2-Ethoxycarbonyl-phenyl)-2-oxo-ethyl]-3-methyl-7-(3-methyl-2-buten-1-yl)-8-(3-amino-piperidin-1-yl)-xanthin,
1-{2-[2-(Dimethylaminocarbonyl)-phenyl]-2-oxo-ethyl}-3-methyl-7-(3-methyl-2-buten-1-yl)-8-(3-amino-piperidin-1-yl)-xanthin,
1-{2-[2-(Morpholin-4-yl-carbonyl)-phenyl]-2-oxo-ethyl}-3-methyl-7-(3-methyl-2-buten-1-yl)-8-(3-amino-piperidin-1-yl)-xanthin,
1-[2-(2,6-Dimethoxy-phenyl)-2-oxo-ethyl]-3-methyl-7-(3-methyl-2-buten-1-yl)-8-(3-amino-piperidin-1-yl)-xanthin,
1-((E)-3-Phenyl-allyl)-3-methyl-7-(3-methyl-2-buten-1-yl)-8-(3-amino-piperidin-1-yl)-xanthin,
1-[(Benzo[b]thiophen-3-yl)methyl]-3-methyl-7-(3-methyl-2-buten-1-yl)-8-(3-amino-piperidin-1-yl)-xanthin,
1-[(1*H*-Indol-3-yl)methyl]-3-methyl-7-(3-methyl-2-buten-1-yl)-8-(3-amino-piperidin-1-yl)-xanthin,
1-[(Biphenyl-4-yl)methyl]-3-methyl-7-(3-methyl-2-buten-1-yl)-8-(3-amino-piperidin-1-yl)-xanthin,
1-(2-Cyclohexyl-2-oxo-ethyl)-3-methyl-7-(3-methyl-2-buten-1-yl)-8-(3-amino-piperidin-1-yl)-xanthin,
1-(3,3-Dimethyl-2-oxo-butyl)-3-methyl-7-(3-methyl-2-buten-1-yl)-8-(3-amino-piperidin-1-yl)-xanthin,
1-({5-[(Methoxycarbonyl)methylamino]-isochinolin-1-yl}methyl)-3-methyl-7-(3-methyl-2-buten-1-yl)-8-(3-amino-piperidin-1-yl)-xanthin,
1-(2-Dimethylamino-2-oxo-ethyl)-3-methyl-7-(3-methyl-2-buten-1-yl)-8-(3-amino-piperidin-1-yl)-xanthin,
1-[2-(Piperidin-1-yl)-2-oxo-ethyl]-3-methyl-7-(3-methyl-2-buten-1-yl)-8-(3-amino-piperidin-1-yl)-xanthin,
1-[(2-Methyl-1-oxo-1,2-dihydro-isochinolin-4-yl)methyl]-7-(3-methyl-2-buten-1-yl)-8-(3-amino-piperidin-1-yl)-xanthin,
1-[2-(2,3-Dimethoxy-phenyl)-2-oxo-ethyl]-3-methyl-7-(3-methyl-2-buten-1-yl)-8-(3-amino-piperidin-1-yl)-xanthin,
1-[2-(Pyrrolidin-1-yl)-2-oxo-ethyl]-3-methyl-7-(3-methyl-2-buten-1-yl)-8-(3-amino-piperidin-1-yl)-xanthin,
1-[2-(2,3-Dihydro-benzo[1,4]dioxin-5-yl)-2-oxo-ethyl]-3-methyl-7-(3-methyl-2-buten-1-yl)-8-(3-amino-piperidin-1-yl)-xanthin,
1-[2-(3-Methyl-2-oxo-2,3-dihydro-benzooxazol-7-yl)-2-oxo-ethyl]-3-methyl-7-(3-methyl-2-buten-1-yl)-8-(3-amino-piperidin-1-yl)-xanthin,
1-[2-(Benzo[1,3]dioxol-4-yl)-2-oxo-ethyl]-3-methyl-7-(3-methyl-2-buten-1-yl)-8-(3-amino-piperidin-1-yl)-xanthin,
1-Methyl-3-isopropyl-7-(2-cyano-benzyl)-8-(3-amino-piperidin-1-yl)-xanthin,
1-[2-(2-Cyanomethylamino-phenyl)-2-oxo-ethyl]-3-methyl-7-(3-methyl-2-buten-1-yl)-8-(3-amino-piperidin-1-yl)-xanthin,
1-[(Isochinolin-1-yl)methyl]-3-[(methoxycarbonyl)methyl]-7-(3-methyl-2-buten-1-yl)-8-(3-amino-piperidin-1-yl)-xanthin,
1-(2-{2-[(Isopropyloxycarbonyl)methoxy]-phenyl}-2-oxo-ethyl)-3-methyl-7-(3-methyl-2-buten-1-yl)-8-(3-amino-piperidin-1-yl)-xanthin,
1-[2-(2-{[(Ethoxycarbonylamino)carbonyl]amino}-phenyl)-2-oxo-ethyl]-3-methyl-7-(3-methyl-2-buten-1-yl)-8-(3-amino-piperidin-1-yl)-xanthin,
1-[2-(2-Acetylamino-phenyl)-2-oxo-ethyl]-3-methyl-7-(3-methyl-2-buten-1-yl)-8-((S)-3-amino-piperidin-1-yl)-xanthin,
1-(2-{2-[(Methylaminocarbonyl)methoxy]-phenyl}-2-oxo-ethyl)-3-methyl-7-(3-methyl-2-buten-1-yl)-8-((S)-3-amino-piperidin-1-yl)-xanthin,
1-Methyl-7-(2-cyano-benzyl)-8-(3-amino-piperidin-1-yl)-xanthin,
1-(2-{2-[(Isopropylcarbonyl)amino]-phenyl}-2-oxo-ethyl)-3-methyl-7-(3-methyl-2-buten-1-yl)-8-(3-amino-piperidin-1-yl)-xanthin,
1-(2-{2-[(Methoxycarbonyl)amino]-phenyl}-2-oxo-ethyl)-3-methyl-7-(3-methyl-2-buten-1-yl)-8-(3-amino-piperidin-1-yl)-xanthin,
1-[2-(3-Methyl-2-oxo-2,3-dihydro-benzooxazol-4-yl)-2-oxo-ethyl]-3-methyl-7-(3-methyl-2-buten-1-yl)-8-(3-amino-piperidin-1-yl)-xanthin,
1-[2-(2-Nitro-3-methoxy-phenyl)-2-oxo-ethyl]-3-methyl-7-(3-methyl-2-buten-1-yl)-8-(3-amino-piperidin-1-yl)-xanthin,
1-[2-(2-Amino-3-methoxy-phenyl)-2-oxo-ethyl]-3-methyl-7-(3-methyl-2-buten-1-yl)-8-(3-amino-piperidin-1-yl)-xanthin,
1-[2-(2-Oxo-2,3-dihydro-benzooxazol-7-yl)-2-oxo-ethyl]-3-methyl-7-(3-methyl-2-buten-1-yl)-8-(3-amino-piperidin-1-yl)-xanthin,
1-[(3-Methyl-isochinolin-1-yl)methyl]-3-methyl-7-(3-methyl-1-buten-1-yl)-8-((R)-3-amino-piperidin-1-yl)-xanthin,
1-[(3-Methyl-isochinolin-1-yl)methyl]-3-methyl-7-(2-cyano-benzyl)-8-(3-amino-piperidin-1-yl)-xanthin,
1-[2-(3-Carboxymethoxy-phenyl)-2-oxo-ethyl]-3-methyl-7-(3-methyl-2-buten-1-yl)-8-(3-amino-piperidin-1-yl)-xanthin und
1-[2-(2-Carboxymethoxy-phenyl)-2-oxo-ethyl]-3-methyl-7-(3-methyl-2-buten-1-yl)-8-(3-amino-piperidin-1-yl)-xanthin,
deren Tautomere, Enantiomere, Diastereomere, deren Gemische, deren Prodrugs und deren Salze.

Eine erste bevorzugte Untergruppe des ersten Erfindungsgegenstandes sind Verbindungen der allgemeinen Formel I, in denen
R¹ eine 4-Methoxy-1-naphthylmethyl-Gruppe,
eine 2-Chinolinylmethyl-, 4-Chinolinylmethyl- oder eine 6-Chinolinylmethyl-Gruppe,
eine 1-Isochinolinylmethyl-, 3-Methyl-1-isochinolinylmethyl-, 4-Methyl-1-isochinolinylmethyl- oder eine 3-Isochinolinylmethyl-Gruppe oder
eine 2-Chinazolinylmethyl-, 4-Methyl-2-chinazolinylmethyl- oder eine 4-Chinazolinylmethyl-Gruppe,
R² eine Methylgruppe und
R³ eine 2-Buten-1-yl- oder eine 2-Butin-1-yl-Gruppe bedeuten,
deren Tautomere, Enantiomere, Diastereomere, deren Gemische und deren Salze.
Eine zweite bevorzugte Untergruppe des ersten Erfindungsgegenstandes sind Verbindungen der allgemeinen Formel I, in denen
R¹ eine [2-(Methylcarbonylamino)-phenyl]-carbonylmethyl-Gruppe,
eine [2-(Ethylcarbonylamino)-phenyl]-carbonylmethyl-Gruppe oder
eine [2-(Isopropylcarbonylamino)-phenyl]-carbonylmethyl-Gruppe,
R² eine Methylgruppe und
R³ eine 2-Buten-1-yl- oder eine 2-Butin-1-yl-Gruppe bedeuten,
deren Tautomere, Enantiomere, Diastereomere, deren Gemische und deren Salze.

Eine dritte bevorzugte Untergruppe des ersten Erfindungsgegenstandes sind Verbindungen der allgemeinen Formel I gemäß Anspruch 1, in denen
R¹ eine [2-(Aminocarbonylmethoxy)-phenyl]-carbonylmethyl-Gruppe,
[2-(Methylaminocarbonylmethoxy)-phenyl]-carbonylmethyl-Gruppe,
eine [2-(Ethylaminocarbonylmethoxy)-phenyl]-carbonylmethyl-Gruppe oder
eine [2-(Isopropylaminocarbonylmethoxy)-phenyl]-carbonylmethyl-Gruppe,
R² eine Methylgruppe und
R³ eine 2-Buten-1-yl-Gruppe,
eine 2-Butin-1-yl-Gruppe oder
eine 1-Cyclopenten-1-ylmethyl-Gruppe bedeuten,
deren Tautomere, Enantiomere, Diastereomere, deren Gemische und deren Salze.

Eine zweiter Erfindungsgegenstand betrifft Verbindungen der allgemeinen Formel I, in denen
R¹ eine Methylgruppe, die durch eine Naphthyl-, Fluornaphthyl-, Methylnaphthyl-, Methoxynaphthyl-, (Difluormethoxy)-naphthyl-, Cyanonaphthyl-, Nitronaphthyl- oder (Dimethylamino)-naphthyl-Gruppe substituiert ist,
eine Methylgruppe, die durch eine Phenanthrenylgruppe substituiert ist,
eine Methylgruppe, die durch eine 2-Phenylethenyl-, 2-[(Trifluormethyl)-phenyl]-ethenyl-, 2-(Nitrophenyl)ethenyl-, 2-(Pentafluorphenyl)ethenyl- oder eine Biphenylyl-Gruppe substituiert ist,
eine Methylgruppe, die durch eine Phenyloxadiazolyl-, Phenylpyridinyl-, Indolyl-, Methylindolyl-, Dimethyl-6,7-dihydro-5*H*-[2]pyrindinyl-, Benzimidazolyl-, Methylbenzimidazolyl-, (Cyanoethyl)-benzimidazolyl-, (Methylaminocarbonylmethyl)benzimidazolyl-, Benzylbenzimidazolyl-, Benzofuranyl-, Acetylbenzofuranyl-, Cyanobenzofuranyl-, Benzoxazolyl-, Nitrobenzoxazolyl-, Benzothiophenyl-, Methylbenzothiazolyl-, Chinolinyl-, Methoxychinolinyl-, Isochinolinyl-, Methylisochinolinyl-, (Difluormethyl)-isochinolinyl-, (Trifluormethyl)-isochinolinyl-, Dimethylisochinolinyl-, (1-Cyano-1-methyl-ethyl)isochinolinyl-, Phenylisochinolinyl-, Methoxyisochinolinyl-, Methoxychlor-isochinolinyl-, Methoxy-brom-isochinolinyl-, (Methoxycarbonylmethylamino)-isochinolinyl-, Dimethyl-5,6,7,8-tetrahydroisochinolinyl-, 1,2,3,4-Tetrahydrophenanthridinyl-, Cinnolinyl-, Chinazolinyl-, Methylchinazolinyl-, Isopropylchinazolinyl-, Cyclopropylchinazolinyl-, Phenylchinazolinyl-, Aminochinazolinyl-, (Dimethylamino)-chinazolinyl-, Pyrrolidin-1-ylchinazolinyl-, Piperidin-1-ylchinazolinyl-, Piperazin-1-ylchinazo-linyl-, Morpholin-4-ylchinazolinyl-, Ethoxychinazolinyl-, Isopropyloxychinazolinyl-, Phenyloxychinazolinyl-, Imidazo[1,2-a]pyridinyl-, Methylimidazo[1,2-a]pyridinyl-, Phenylimidazo[1,2-a]pyridinyl-, Benzylimidazo[1,2-a]pyridinyl-, Pyrazolo[1,5-a]pyridinyl-, Chinoxalinyl-, Methylchinoxalinyl-, Dimethylchinoxalinyl-, Trimethylchinoxalinyl-, Phenylchinoxalinyl-, Methylphthalazinyl-, Naphthyridinyl-, 2,3-Dihydro-benzo[1,4]-dioxinyl-, 1,2-Dihydro-2-oxo-chinolinyl-, 1,2-Dihydro-1-methyl-2-oxo-chinolinyl-, 1,2-Dihydro-2-methyl-1-oxo-isochinolinyl-, 3,4-Dihydro-4-oxo-phthalazinyl-, 3,4-Dihydro-3-methyl-4-oxo-phthalazinyl-, 3,4-Dihydro-4-oxo-chinazolinyl-, 3,4-Dihydro-3-methyl-4-oxo-chinazolinyl- oder eine 2-Oxo-2H-chromenyl-Gruppe substituiert ist,
eine Phenylcarbonylmethyl-Gruppe,
eine Phenylcarbonylmethylgruppe, in der der Phenylteil durch eine Amino-, Cyanmethylamino-, (Ethyloxycarbonylmethyl)amino-, (Methylaminocarbonyl)methylamino-, Methylcarbonylamino-, Ethylcarbonylamino-, Isopropylcarbonylamino-, Phenylcarbonylamino-, Methoxycarbonylamino-, (Ethyloxycarbonylamino)-carbonylamino- oder eine 2-Oxo-imidazolidin-1-yl-Gruppe substituiert ist,
eine Phenylcarbonylmethylgruppe, in der der Phenylteil durch eine Phenylgruppe substituiert ist,
eine Phenylcarbonylmethylgruppe, in der der Phenylteil durch eine Carboxy-, Methoxycarbonyl-, Ethyloxycarbonyl-, Aminocarbonyl-, Methylaminocarbonyl-, Dimethylaminocarbonyl- oder Morpholin-4-ylcarbonylgruppe substituiert ist,
eine Phenylcarbonylmethylgruppe, in der der Phenylteil durch eine Methylsulfanyl-, Methylsulfinyl- oder Methylsulfonylgruppe substituiert ist,
eine Phenylcarbonylmethylgruppe, in der der Phenylteil durch eine Methoxy-, Difluormethoxy-, Trifluormethoxy-, Ethyloxy-, Isopropyloxy- oder Phenyloxygruppe substituiert ist,
eine Phenylcarbonylmethylgruppe, in der der Phenylteil durch eine Methylsulfinylmethoxy-, Carboxymethoxy-, Ethyloxycarbonylmethoxy-, Isopropyloxycarbonylmethoxy-, Aminocarbonylmethoxy-, Methylaminocarbonylmethoxy-, Ethylaminocarbonylmethoxy-, Isopropylaminocarbonylmethoxy-, Dimethylaminocarbonylmethoxy-, Pyrrolidin-1-ylcarbonylmethoxy- oder Morpholin-4-ylcarbonylmethoxy-Gruppe substituiert ist,
eine Phenylcarbonylmethylgruppe, in der der Phenylteil durch eine 1-(Ethyloxycarbonyl)-1-methyl-ethyloxy-, 1-(Methoxycarbonyl)-ethyloxy- oder eine 1-(Aminocarbonyl)-ethyloxy-Gruppe substituiert ist,
eine Phenylcarbonylmethylgruppe, in der der Phenylteil durch zwei Methoxygruppen substituiert ist,
eine Phenylcarbonylmethylgruppe, in der der Phenylteil durch eine Methoxygruppe und eine Nitrogruppe substituiert ist,
eine Phenylcarbonylmethylgruppe, in der der Phenylteil durch eine Methoxygruppe und eine Aminogruppe substituiert ist,
eine Phenylcarbonylmethylgruppe, in der im Phenylteil zwei benachbarte Wasserstoffatome durch eine -O-CH₂-O-, -O-CF₂-O-, -O-CH₂-CH₂-O-, -NH-CO-NH-, -N(CH₃)-CO-NH-, -N(CH₃)-CO-N(CH₃)-, -NH-CO-O- oder eine -N(CH₃)-CO-O-Gruppe ersetzt sind,
eine (2-Phenylethyl)carbonylmethylgruppe,
eine Naphthylcarbonylmethyl-, Indolylcarbonylmethyl- oder Chinolinylcarbonylmethylgruppe oder
eine 2-Cyanimino-2-phenyl-ethylgruppe,
R² eine Methyl-, Isopropyl-, Cyclopropyl-, Phenyl- oder Fluorphenylgruppe und
R³ eine 2-Methyl-2-propen-1-yl-, 2-Chlor-2-propen-1-yl- oder 3-Brom-2-propen-1-yl-Gruppe
eine 1-Buten-1-yl-, 3-Methyl-1-buten-1-yl-, 2-Buten-1-yl-, 2-Methyl-2-buten-1-yl- oder 2,3-Dimethyl-2-buten-1-yl-Gruppe,
eine 2-Butin-1-yl-Gruppe,
eine 1-Cyclopenten-1-ylmethyl-Gruppe oder
eine 2-Furanylmethyl-Gruppe bedeuten,
deren Tautomere, Enantiomere, Diastereomere, deren Gemische, deren Prodrugs und deren Salze.

Eine bevorzugte Untergruppe des zweiten Erfindungsgegenstandes sind Verbindungen der allgemeinen Formel I, in denen
R¹ und R² wie oben erwähnt definiert sind und
R³ eine 1-Buten-1-yl-, 2-Buten-1-yl- oder 2-Butin-1-yl-Gruppe bedeutet,
deren Tautomere, Enantiomere, Diastereomere, deren Gemische und deren Salze.

Eine besonders bevorzugte Untergruppe des zweiten Erfindungsgegenstandes sind Verbindungen der allgemeinen Formel I, in denen
R¹ eine Methylgruppe, die durch eine Naphthyl-, Fluornaphthyl-, Methylnaphthyl-, Methoxynaphthyl-, (Difluormethoxy)-naphthyl-, Cyanonaphthyl- oder Nitronaphthyl-Gruppe substituiert ist,
eine Methylgruppe, die durch eine 2-(Pentafluorphenyl)ethenyl-Gruppe substituiert ist,
eine Methylgruppe, die durch eine Benzofuranyl-, Methylbenzothiazolyl-, Chinolinyl-, Methoxychinolinyl-, Isochinolinyl-, Methylisochinolinyl-, (Difluormethyl)-isochinolinyl-, (Trifluormethyl)-isochinolinyl-, Dimethylisochinolinyl-, (1-Cyano-1-methyl-ethyl)isochinolinyl-, Phenylisochinolinyl-, Methoxyisochinolinyl-, 1,2,3,4-Tetrahydrophenanthridinyl-, Chinazolinyl-, Methylchinazolinyl-, Isopropylchinazolinyl-, Cyclopropylchinazolinyl-, Phenylchinazolinyl-, Aminochinazolinyl-, (Dimethylamino)-chinazolinyl-, Pyrrolidin-1-ylchinazolinyl-, Piperidin-1-ylchinazolinyl-, Piperazin-1-ylchinazolinyl-, Morpholin-4-ylchinazolinyl-, Ethoxychinazolinyl-, Isopropyloxychinazolinyl-, Chinoxalinyl-, Methylchinoxalinyl-, Dimethylchinoxalinyl-, Trimethylchinoxalinyl-, Phenylchinoxalinyl-, [1,5]Naphthyridinyl-, [1,6]Naphthyridinyl-, [1,8]Naphthyridinyl- oder eine 1,2-Dihydro-1-methyl-2-oxo-chinolinyl-Gruppe substituiert ist,
eine Phenylcarbonylmethyl-Gruppe,
eine Phenylcarbonylmethylgruppe, in der der Phenylteil durch eine Phenylgruppe substituiert ist,
eine Phenylcarbonylmethylgruppe, in der der Phenylteil durch eine Methoxy-, Difluormethoxy-, Trifluormethoxy-, Ethyloxy-, Isopropyloxy- oder Phenyloxygruppe substituiert ist,
eine Phenylcarbonylmethylgruppe, in der im Phenylteil zwei benachbarte Wasserstoffatome durch eine -O-CH₂-O-, -O-CF₂-O-, -O-CH₂-CH₂-O-, -N(CH₃)-CO-N(CH₃)- oder eine -N(CH₃)-CO-O-Gruppe ersetzt sind,
eine Naphthylcarbonylmethyl-, Indolylcarbonylmethyl- oder Chinolinylcarbonylmethylgruppe oder
eine 2-Cyanimino-2-phenyl-ethylgruppe,
R² eine Methyl-, Isopropyl-, Cyclopropyl-, Phenyl- oder 4-Fluorphenylgruppe und
R³ eine 1-Buten-1-yl-, 2-Buten-1-yl- oder eine 2-Butin-1-yl-Gruppe bedeuten,
deren Tautomere, Enantiomere, Diastereomere, deren Gemische und deren Salze.

Eine zweite bevorzugte Untergruppe des zweiten Erfindungsgegenstandes sind Verbindungen der allgemeinen Formel I, in denen R¹ und R² wie unmittelbar zuvor erwähnt definiert sind und R³ eine 1-Buten-1-yl-Gruppe bedeutet,
deren Tautomere, Enantiomere, Diastereomere, deren Gemisch und deren Salze.

Eine dritte bevorzugte Untergruppe des zweiten Erfindungsgegenstandes sind Verbindungen der allgemeinen Formel I, in denen R¹ und R² wie unmittelbar zuvor erwähnt definiert sind und R³ eine 2-Buten-1-yl-Gruppe bedeutet,
deren Tautomere, Enantiomere, Diastereomere, deren Gemische und deren Salze.

Eine vierte bevorzugte Untergruppe des zweiten Erfindungsgegenstandes sind Verbindungen der allgemeinen Formel I, in denen R¹ und R² wie unmittelbar zuvor erwähnt definiert sind und R³ eine 2-Butin-1-yl-Gruppe bedeutet,
deren Tautomere, Enantiomere, Diastereomere, deren Gemische und deren Salze.

Eine dritter Erfindungsgegenstand betrifft Verbindungen der allgemeinen Formel I, in denen

R¹ eine Methylgruppe, die durch eine Naphthyl-, Fluornaphthyl-, Methylnaphthyl-, Methoxynaphthyl-, (Difluormethoxy)-naphthyl-, Cyanonaphthyl- oder Nitronaphthyl-Gruppe substituiert ist,
eine Methylgruppe, die durch eine 2-(Pentafluorphenyl)ethenyl-Gruppe substituiert ist, oder
eine Methylgruppe, die durch eine Benzofuranyl-, Methylbenzothiazolyl-, Chinolinyl-, Methoxychinolinyl-, Isochinolinyl-, Methylisochinolinyl-, (Difluormethyl)-isochinolinyl-, (Trifluormethyl)-isochinolinyl-, Dimethylisochinolinyl-, (1-Cyano-1-methyl-ethyl)isochinolinyl-, Phenylisochinolinyl-, Methoxyisochinolinyl-, 1,2,3,4-Tetrahydrophenanthridinyl-, Chinazolinyl-, Methylchinazolinyl-, Isopropylchinazolinyl-, Cyclopropylchinazolinyl-, Phenylchinazolinyl-, Aminochinazolinyl-, (Dimethylamino)-chinazolinyl-, Pyrrolidin-1-ylchinazolinyl-, Piperidin-1-ylchinazolinyl-, Piperazin-1-ylchinazolinyl-, Morpholin-4-ylchinazolinyl-, Ethoxychinazolinyl-, Isopropyloxychinazolinyl-, Chinoxalinyl-, Methylchinoxalinyl-, Dimethylchinoxalinyl-, Trimethylchinoxalinyl-, Phenylchinoxalinyl-, [1,5]Naphthyridinyl-, [1,6]Naphthyridinyl-, [1,8]Naphthyridinyl- oder eine 1,2-Dihydro-1-methyl-2-oxo-chinolinyl-Gruppe substituiert ist,
R² eine Methyl-, Isopropyl-, Cyclopropyl- oder Phenylgruppe und
R³ eine 2-Chlorbenzyl-, 2-Brombenzyl-, 2-Ethinylbenzyl- oder 2-Cyanbenzyl-Gruppe bedeuten,
deren Tautomere, Enantiomere, Diastereomere, deren Gemische und deren Salze.

Eine erste bevorzugte Untergruppe des dritten Erfindungsgegenstandes sind Verbindungen der allgemeinen Formel I, in denen
R¹ eine (3-Methyl-isochinolin-1-yl)methylgruppe,
R² eine Methylgruppe und
R³ eine 2-Chlorbenzyl-, 2-Brombenzyl-, 2-Ethinylbenzyl- oder 2-Cyanbenzyl-Gruppe bedeuten,
deren Tautomere, Enantiomere, Diastereomere, deren Gemische und deren Salze.

Eine zweite bevorzugte Untergruppe des dritten Erfindungsgegenstandes sind Verbindungen der allgemeinen Formel I, in denen R¹ und R² wie oben erwähnt definiert sind und R³ eine 2-Chlorbenzyl-Gruppe bedeutet, deren Tautomere, Enantiomere, Diastereomere, deren Gemische und deren Salze.

Eine dritte bevorzugte Untergruppe des dritten Erfindungsgegenstandes sind Verbindungen der allgemeinen Formel I, in denen R¹ und R² wie oben erwähnt definiert sind und R³ eine 2-Brombenzyl-Gruppe bedeutet, deren Tautomere, Enantiomere, Diastereomere, deren Gemische und deren Salze.

Eine vierte bevorzugte Untergruppe des dritten Erfindungsgegenstandes sind Verbindungen der allgemeinen Formel I, in denen R¹ und R² wie oben erwähnt definiert sind und R³ eine 2-Ethinylbenzyl-Gruppe bedeutet, deren Tautomere, Enantiomere, Diastereomere, deren Gemische und deren Salze.

Eine fünfte bevorzugte Untergruppe des dritten Erfindungsgegenstandes sind Verbindungen der allgemeinen Formel I, in denen R¹ und R² wie oben erwähnt definiert sind und R³ eine 2-Cyanbenzyl-Gruppe bedeutet, deren Tautomere, Enantiomere, Diastereomere, deren Gemische und deren Salze.

Ganz besonders bevorzugt sind folgende Verbindungen der allgemeinen Formel I:
(1) 1-[(Chinazolin-2-yl)methyl]-3-methyl-7-(2-butin-1-yl)-8-((*R*)-3-amino-piperidin-1-yl)-xanthin,
(2) 1-(2-{2-[(Ethylaminocarbonyl)methoxy]-phenyl}-2-oxo-ethyl)-3-methyl-7-(2-butin-1-yl)-8-((*R*)-3-amino-piperidin-1-yl)-xanthin,
(3) 1-(2-{2-[(Methylaminocarbonyl)methoxy]-phenyl}-2-oxo-ethyl)-3-methyl-7-(2-butin-1-yl)-8-((R)-3-amino-piperidin-1-yl)-xanthin,
(4) 1-(2-Phenyl-2-oxo-ethyl)-3-methyl-7-((E)-2-buten-1-yl)-8-((R)-3-amino-piperidin-1-yl)-xanthin,
(5) 1-[(3-Methyl-isochinolin-1-yl)methyl]-3-methyl-7-(2-butin-1-yl)-8-((S)-3-amino-piperidin-1-yl)-xanthin,
(6) 1-[(3-Methyl-isochinolin-1-yl)methyl]-3-methyl-7-(2-butin-1-yl)-8-((R)-3-amino-piperidin-1-yl)-xanthin,
(7) 1-[(4-Methyl-isochinolin-1-yl)methyl]-3-methyl-7-(2-butin-1-yl)-8-((S)-3-amino-piperidin-1-yl)-xanthin,
(8) 1-[(4-Methyl-isochinolin-1-yl)methyl]-3-methyl-7-(2-butin-1-yl)-8-((R)-3-amino-piperidin-1-yl)-xanthin,
(9) 1-[2-(2,3-Dihydro-benzo[1,4]dioxin-5-yl)-2-oxo-ethyl]-3-methyl-7-(2-butin-1-y1)-8-(3-(*R*)-amino-piperidin-1-yl)-xanthin,
(10) 1-[(4-Methoxy-naphthalin-1-yl)methyl]-3-methyl-7-(2-butin-1-yl)-8-((S)-3-amino-piperidin-1-yl)-xanthin,
(11) 1-[(4-Methoxy-naphthalin-1-yl)methyl]-3-methyl-7-(2-butin-1-yl)-8-((R)-3-amino-piperidin-1-yl)-xanthin,
(12) 1-[2-(Benzo[1,3]dioxol-4-yl)-2-oxo-ethyl]-3-methyl-7-(2-butin-1-yl)-8-((R)-3-amino-piperidin-1-yl)-xanthin,
(13) 1-[(4-Methyl-chinazolin-2-yl)methyl]-3-methyl-7-(2-butin-1-yl)-8-(3-(R)-amino-piperidin-1-yl)-xanthin,
(14) 1-(2-{2-[(Isopropylcarbonyl)amino]-phenyl}-2-oxo-ethyl)-3-methyl-7-((E)-2-buten-1-yl)-8-((S)-3-amino-piperidin-1-yl)-xanthin,
(15) 1-(2-{2-[(Methylaminocarbonyl)methoxy]-phenyl}-2-oxo-ethyl)-3-methyl-7-((E)-2-buten-1-yl)-8-((R)-3-amino-piperidin-1-yl)-xanthin,
(16) 1-(2-{2-[(Methylaminocarbonyl)methoxy]-phenyl}-2-oxo-ethyl)-3-methyl-7-((E)-2-buten-1-yl)-8-((S)-3-amino-piperidin-1-yl)-xanthin,
(17) 1-(2-{2-[(Isopropylcarbonyl)amino]-phenyl}-2-oxo-ethyl)-3-methyl-7-(2-butin-1-yl)-8-((R)-3-amino-piperidin-1-yl)-xanthin,
(18) 1-(2-{2-[(Isopropylcarbonyl)amino]-phenyl}-2-oxo-ethyl)-3-methyl-7-((E)-2-buten-1-yl)-8-((*R*)-3-amino-piperidin-1-yl)-xanthin,
(19) 1-[(4-Cyano-naphthalin-1-yl)methyl]-3-methyl-7-(2-butin-1-yl)-8-((R)-3-amino-piperidin-1-yl)-xanthin,
(20) 1-[(4-Phenyl-chinazolin-2-yl)methyl]-3-methyl-7-(2-butin-1-yl)-8-((R)-3-aminopiperidin-1-yl)-xanthin,
(21) 1-[(8-Methyl-chinoxalin-6-yl)methyl]-3-methyl-7-(2-butin-1-yl)-8-((R)-3-amino-piperidin-1-yl)-xanthin,
(22) 1-[(4-Fluor-naphthalin-1-yl)methyl]-3-methyl-7-(2-butin-1-yl)-8-((*R*)-3-amino-piperidin-1-yl)-xanthin ,
(23) 1-((E)-3-Pentafluorphenyl-allyl)-3-methyl-7-(2-butin-1-yl)-8-((R)-3-aminopiperidin-1-yl)-xanthin ,
(24) 1-[(3-Trifluormethyl-isochinolin-1-yl)methyl]-3-methyl-7-(2-butin-1-yl)-8-((R)-3-amino-piperidin-1-yl)-xanthin,
(25) 1-[(3-Difluormethyl-isochinolin-1-yl)methyl]-3-methyl-7-(2-butin-1-yl)-8-((*R*)-3-amino-piperidin-1-yl)-xanthin,
(26) 1-[2-(Biphenyl-2-yl)-2-oxo-ethyl]-3-methyl-7-(2-butin-1-yl)-8-((R)-3-amino-piperidin-1-yl)-xanthin,
(27) 1-[(3-Methyl-isochinolin-1-yl)methyl]-3-cyclopropyl-7-(2-butin-1-yl)-8-((R)-3-amino-piperidin-1-yl)-xanthin,
(28) 1-[2-(3-Methoxy-phenyl)-2-oxo-ethyl]-3-methyl-7-(2-butin-1-yl)-8-((R)-3-amino-piperidin-1-yl)-xanthin,
(29) 1-[(3-Methyl-isochinolin-1-yl)methyl]-3-methyl-7-(2-chlor-benzyl)-8-((R)-3-amino-piperidin-1-yl)-xanthin und
(30) 1-[(3-Methyl-isochinolin-1-yl)methyl]-3-methyl-7-(2-brom-benzyl)-8-((R)-3-amino-piperidin-1-yl)-xanthin
sowie deren Tautomere, Enantiomere, Diastereomere, deren Gemische und deren Salze.

Erfindungsgemäß erhält man die Verbindungen der allgemeinen Formel I nach an sich bekannten Verfahren, beispielsweise nach folgenden Verfahren:
a) Umsetzung einer Verbindung der allgemeinen Formel 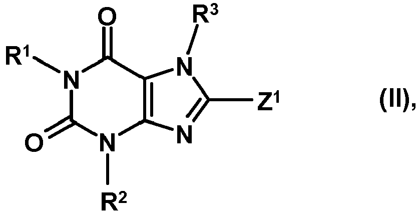 in der
   R¹ bis R³ wie eingangs erwähnt definiert sind und
   Z¹ eine Austrittsgruppe wie ein Halogenatom, eine substituierte Hydroxy-, Mercapto-, Sulfinyl-, Sulfonyl- oder Sulfonyloxygruppe wie ein Chlor- oder Bromatom, eine Methansulfonyl- oder Methansulfonyloxygruppe darstellt, mit 3-Aminopiperidin, dessen Enantiomeren oder dessen Salzen.
   Die Umsetzung wird zweckmäßigerweise in einem Lösungsmittel wie Isopropanol, Butanol, Tetrahydrofuran, Dioxan, Dimethylformamid, Dimethylsulfoxid, Ethylenglycolmonomethylether, Ethylenglycoldiethylether oder Sulfolan gegebenenfalls in Gegenwart einer anorganischen oder tertiären organischen Base, z.B. Natriumcarbonat, Kaliumcarbonat oder Kaliumhydroxid, einer tertiären organischen Base, z.B. Triethylamin, oder in Gegenwart von N-Ethyl-diisopropylamin (Hünig-Base), wobei diese organischen Basen gleichzeitig auch als Lösungsmittel dienen können, und gegebenenfalls in Gegenwart eines Reaktionsbeschleunigers wie einem Alkalihalogenid oder einem Katalysator auf Palladiumbasis bei Temperaturen zwischen -20 und 180°C, vorzugsweise jedoch bei Temperaturen zwischen -10 und 120°C, durchgeführt. Die Umsetzung kann jedoch auch ohne Lösungsmittel oder in einem Überschuß des 3-Aminopiperidins durchgeführt werden.
b) Entschützung einer Verbindung der allgemeinen Formel 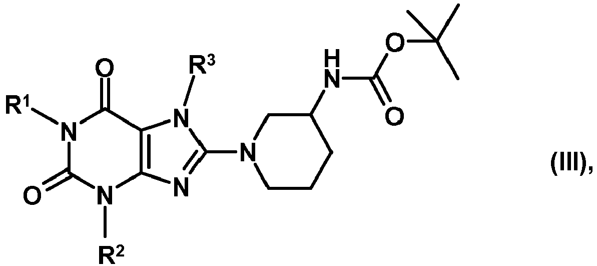 in der R¹, R² und R³ wie eingangs definiert sind.
   Die Abspaltung des tert.-Butyloxycarbonylrestes erfolgt vorzugsweise durch Behandlung mit einer Säure wie Trifluoressigsäure oder Salzsäure oder durch Behandlung mit Bromtrimethylsilan oder lodtrimethylsilan gegebenenfalls unter Verwendung eines Lösungsmittels wie Methylenchlorid, Essigester, Dioxan, Methanol, Isopropanol oder Diethylether bei Temperaturen zwischen 0 und 80°C.
c) Zur Herstellung einer Verbindung der allgemeinen Formel I, in der R¹ gemäß der eingangs erwähnten Definition eine Carboxygruppe enthält:
   Entschützung einer Verbindung der allgemeinen Formel 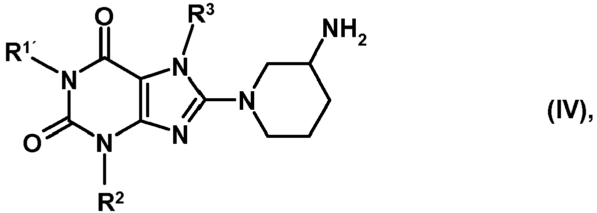 in der R² und R³ wie eingangs definiert sind und R^{1'} eine durch eine C₁₋₄-Alkylgruppe geschützte Carboxygruppe enthält.

Die Abspaltung des Schutzrestes erfolgt beispielsweise hydrolytisch mit Hilfe einer Säure wie Salzsäure oder Schwefelsäure oder eines Alkalihydroxids wie Lithiumhydroxid, Natriumhydroxid oder Kaliumhydroxid in einem Lösungsmittel wie Methanol, Ethanol, Isopropanol, Tetrahydrofuran oder Dioxan in Gegenwart von Wasser.

Bei den vorstehend beschriebenen Umsetzungen können gegebenenfalls vorhandene reaktive Gruppen wie Carboxy-, Amino-, Alkylamino- oder Iminogruppen während der Umsetzung durch übliche Schutzgruppen geschützt werden, welche nach der Umsetzung wieder abgespalten werden.

Beispielsweise kommen als Schutzreste für eine Carboxygruppe die Trimethylsilyl-, Methyl-, Ethyl-, tert.-Butyl-, Benzyl- oder Tetrahydropyranylgruppe,
als Schutzreste für eine Amino-, Alkylamino- oder Iminogruppe die Formyl-, Acetyl-, Trifluoracetyl-, Ethoxycarbonyl-, tert.-Butoxycarbonyl-, Benzyloxycarbonyl-, Benzyl-, Methoxybenzyl- oder 2,4-Dimethoxybenzylgruppe und für die Aminogruppe zusätzlich die Phthalylgruppe in Betracht.

Die gegebenenfalls anschließende Abspaltung eines verwendeten Schutzrestes erfolgt beispielsweise hydrolytisch in einem wässrigen Lösungsmittel, z.B. in Wasser, Isopropanol/Wasser, Essigsäure/Wasser, Tetrahydrofuran/Wasser oder Dioxan/Wasser, in Gegenwart einer Säure wie Trifluoressigsäure, Salzsäure oder Schwefelsäure oder in Gegenwart einer Alkalibase wie Natriumhydroxid oder Kaliumhydroxid oder aprotisch, z.B. in Gegenwart von Jodtrimethylsilan, bei Temperaturen zwischen 0 und 120°C, vorzugsweise bei Temperaturen zwischen 10 und 100°C.

Die Abspaltung eines Benzyl-, Methoxybenzyl- oder Benzyloxycarbonylrestes erfolgt jedoch beispielsweise hydrogenolytisch, z.B. mit Wasserstoff in Gegenwart eines Katalysators wie Palladium/Kohle in einem geeigneten Lösungsmittel wie Methanol, Ethanol, Essigsäureethylester oder Eisessig gegebenenfalls unter Zusatz einer Säure wie Salzsäure bei Temperaturen zwischen 0 und 100°C, vorzugsweise jedoch bei Raumtemperaturen zwischen 20 und 60°C, und bei einem Wasserstoffdruck von 1 bis 7 bar, vorzugsweise jedoch von 3 bis 5 bar. Die Abspaltung eines 2,4-Dimethoxybenzylrestes erfolgt jedoch vorzugsweise in Trifluoressigsäure in Gegenwart von Anisol.

Die Abspaltung eines tert.-Butyl- oder tert.-Butyloxycarbonylrestes erfolgt vorzugsweise durch Behandlung mit einer Säure wie Trifluoressigsäure oder Salzsäure oder durch Behandlung mit Jodtrimethylsilan gegebenenfalls unter Verwendung eines Lösungsmittels wie Methylenchlorid, Dioxan, Methanol oder Diethylether.

Die Abspaltung eines Trifluoracetylrestes erfolgt vorzugsweise durch Behandlung mit einer Säure wie Salzsäure gegebenenfalls in Gegenwart eines Lösungsmittels wie Essigsäure bei Temperaturen zwischen 50 und 120°C oder durch Behandlung mit Natronlauge gegebenenfalls in Gegenwart eines Lösungsmittels wie Tetrahydrofuran bei Temperaturen zwischen 0 und 50°C.

Die Abspaltung eines Phthalylrestes erfolgt vorzugsweise in Gegenwart von Hydrazin oder eines primären Amins wie Methylamin, Ethylamin oder n-Butylamin in einem Lösungsmittel wie Methanol, Ethanol, Isopropanol, Toluol/Wasser oder Dioxan bei Temperaturen zwischen 20 und 50°C.

Ferner können die erhaltenen Verbindungen der allgemeinen Formel I, wie bereits eingangs erwähnt wurde, in ihre Enantiomeren und/oder Diastereomeren aufgetrennt werden. So können beispielsweise cis-/trans-Gemische in ihre cis- und trans-Isomere, und Verbindungen mit mindestens einem optisch aktiven Kohlenstoffatom in ihre Enantiomeren aufgetrennt werden.

So lassen sich beispielsweise die erhaltenen cis-/trans-Gemische durch Chromatographie in ihre cis- und trans-Isomeren, die erhaltenen Verbindungen der allgemeinen Formel I, welche in Racematen auftreten, nach an sich bekannten Methoden (siehe Allinger N. L. und Eliel E. L. in "Topics in Stereochemistry", Vol. 6, Wiley Interscience, 1971) in ihre optischen Antipoden und Verbindungen der allgemeinen Formel I mit mindestens 2 asymmetrischen Kohlenstoffatomen auf Grund ihrer physikalisch-chemischen Unterschiede nach an sich bekannten Methoden, z.B. durch Chromatographie und/oder fraktionierte Kristallisation, in ihre Diastereomeren auftrennen, die, falls sie in racemischer Form anfallen, anschließend wie oben erwähnt in die Enantiomeren getrennt werden können.

Die Enantiomerentrennung erfolgt vorzugsweise durch Säulentrennung an chiralen Phasen oder durch Umkristallisieren aus einem optisch aktiven Lösungsmittel oder durch Umetzen mit einer, mit der racemischen Verbindung Salze oder Derivate wie z.B. Ester oder Amide bildenden optisch aktiven Substanz, insbesondere Säuren und ihre aktivierten Derivate oder Alkohole, und Trennen des auf diese Weise erhaltenen diastereomeren Salzgemisches oder Derivates, z.B. auf Grund von verschiedenen Löslichkeiten, wobei aus den reinen diastereomeren Salzen oder Derivaten die freien Antipoden durch Einwirkung geeigneter Mittel freigesetzt werden können. Besonders gebräuchliche, optisch aktive Säuren sind z.B. die D- und L-Formen von Weinsäure oder Dibenzoylweinsäure, Di-O-p-toluoyl-weinsäure, Äpfelsäure, Mandelsäure, Camphersulfonsäure, Glutaminsäure, Asparaginsäure oder Chinasäure. Als optisch aktiver Alkohol kommt beispielsweise (+)- oder (-)-Menthol und als optisch aktiver Acylrest in Amiden beispielsweise (+)-oder (-)-Menthyloxycarbonyl in Betracht.

Desweiteren können die erhaltenen Verbindungen der Formel I in ihre Salze, insbesondere für die pharmazeutische Anwendung in ihre physiologisch verträglichen Salze mit anorganischen oder organischen Säuren, übergeführt werden. Als Säuren kommen hierfür beispielsweise Salzsäure, Bromwasserstoffsäure, Schwefelsäure, Methansulfonsäure, Phosphorsäure, Fumarsäure, Bernsteinsäure, Milchsäure, Zitronensäure, Weinsäure oder Maleinsäure in Betracht.

Außerdem lassen sich die so erhaltenen neuen Verbindungen der Formel I, falls diese eine Carboxygruppe enthalten, gewünschtenfalls anschließend in ihre Salze mit anorganischen oder organischen Basen, insbesondere für die pharmazeutische Anwendung in ihre physiologisch verträglichen Salze, überführen. Als Basen kommen hierbei beispielsweise Natriumhydroxid, Kaliumhydroxid, Arginin, Cyclohexylamin, Ethanolamin, Diethanolamin und Triethanolamin in Betracht.

Die als Ausgangsstoffe verwendeten Verbindungen der allgemeinen Formeln II bis IV sind entweder literaturbekannt oder man erhält diese nach an sich literaturbekannten Verfahren (siehe Beispiele I bis LXXI).

Wie bereits eingangs erwähnt, weisen die erfindungsgemäßen Verbindungen der allgemeinen Formel I und ihre physiologisch verträglichen Salze wertvolle pharmakologische Eigenschaften auf, insbesondere eine Hemmwirkung auf das Enzym DPP-IV.

Die biologischen Eigenschaften der neuen Verbindungen wurden wie folgt geprüft:
Die Fähigkeit der Substanzen und ihrer entsprechenden Salze, die DPP-IV Aktivität zu hemmen, kann in einem Versuchsaufbau gezeigt werden, in dem ein Extrakt der humanen Koloncarcinomzelllinie Caco-2 als DPP-IV Quelle benutzt wird. Die Differenzierung der Zellen, um die DPP-IV Expression zu induzieren, wurde nach der Beschreibung von Reiher et al. in einem Artikel mit dem Titel "Increased expression of intestinal cell line Caco-2" , erschienen in Proc. Natl. Acad. Sci. Vol. 90, Seiten 5757-5761 (1993), durchgeführt. Der Zellextrakt wurde von in einem Puffer (10 mM Tris HCl, 0.15 M NaCl, 0.04 t.i.u. Aprotinin, 0.5% Nonidet-P40, pH 8.0) solubilisierten Zellen durch Zentrifugation bei 35,000 g für 30 Minuten bei 4°C (zur Entfernung von Zelltrümmern) gewonnen.

Der DPP-IV Assay wurde wie folgt durchgeführt:
50 µl Substratlösung (AFC; AFC ist Amido-4-trifluormethylcoumarin), Endkonzentration 100 µM, wurden in schwarze Mikrotiterplatten vorgelegt. 20 µl Assay Puffer (Endkonzentrationen 50 mM Tris HCl pH 7.8, 50 mM NaCl, 1 % DMSO) wurde zupipettiert. Die Reaktion wurde durch Zugabe von 30 µl solubilisiertem Caco-2 Protein (Endkonzentration 0.14 µg Protein pro Well) gestartet. Die zu überprüfenden Testsubstanzen wurden typischerweise in 20 µl vorverdünnt zugefügt, wobei das Assaypuffervolumen dann entsprechend reduziert wurde. Die Reaktion wurde bei Raumtemperatur durchgeführt, die Inkubationsdauer betrug 60 Minuten. Danach wurde die Fluoreszenz in einem Victor 1420 Multilabel Counter gemessen, wobei die Anregungswellenlänge bei 405 nm und die Emissionswellenlänge bei 535 nm lag. Leerwerte (entsprechend 0 % Aktivität) wurden in Ansätzen ohne Caco-2 Protein (Volumen ersetzt durch Assay Puffer), Kontrollwerte (entsprechend 100 % Aktivität) wurden in Ansätzen ohne Substanzzusatz erhalten. Die Wirkstärke der jeweiligen Testsubstanzen, ausgedrückt als IC₅₀ Werte, wurden aus Dosis-Wirkungs Kurven berechnet, die aus jeweils 11 Meßpunkten bestanden. Hierbei wurden folgende Ergebnisse erhalten:

| Verbindung (Beispiel Nr.) | DPP IV-Hemmung IC₅₀ [nM] |
|---|---|
| 2(3) | 2160 |
| 2(9) | 264 |
| 2(12) | 16 |
| 2(17) | 32 |
| 2(20) | 12 |
| 2(25) | 4 |
| 2(27) | 9 |
| 2(35) | 5 |
| 2(37) | 5 |
| 2(43) | 6 |
| 2(51) | 6 |
| 2(52) | 9 |
| 2(59) | 250 |
| 2(66) | 22 |
| 2(80) | 1 |
| 2(86) | 2 |
| 2(96) | 2 |
| 2(99) | 1 |
| 2(100) | 3 |
| 2(108) | 3 |
| 2(129) | 3 |
| 2(130) | 3 |
| 2(131) | 3 |
| 2(132) | 1 |
| 2(135) | 3 |
| 2(137) | 13 |
| 2(138) | 8 |
| 2(139) | 4 |
| 2(142) | 1 |
| 2(145) | 4 |
| 2(148) | 1 |
| 2(150) | 1 |
| 2(151) | 3 |
| 2(152) | 4 |
| 2(185) | 3 |
| 2(217) | 4 |
| 2(247) | 2 |
| 2(251) | 12 |
| 2(256) | 8 |
| 2(260) | 13 |
| 2(264) | 6 |
| 2(277) | 6 |
| 2(280) | 5 |
| 2(285) | 3 |
| 2(287) | 11 |
| 2(288) | 14 |

Die erfindungsgemäß hergestellten Verbindungen sind gut verträglich, da beispielsweise nach oraler Gabe von 10 mg/kg der Verbindung des Beispiels 2(80) an Ratten keine Änderungen im Verhalten der Tiere beobachtet werden konnten.

Im Hinblick auf die Fähigkeit, die DPP-IV Aktivität zu hemmen, sind die erfindungsgemäßen Verbindungen der allgemeinen Formel I und ihre entsprechenden pharmazeutisch akzeptablen Salze geeignet, alle diejenigen Zustände oder Krankheiten zu beeinflussen, die durch eine Hemmung der DPP-IV Aktivität beeinflusst werden können. Es ist daher zu erwarten, daß die erfindungsgemäßen Verbindungen zur Prävention oder Behandlung von Krankheiten oder Zuständen wie Diabetes mellitus Typ 1 und Typ 2, diabetische Komplikationen (wie z.B. Retinopathie, Nephropathie oder Neuropathien), metabolische Azidose oder Ketose, reaktiver Hypoglykämie, Insulinresistenz, Metabolischem Syndrom, Dyslipidämien unterschiedlichster Genese, Arthritis, Atherosklerose und verwandte Erkrankungen, Adipositas, Allograft Transplantation und durch Calcitonin verursachte Osteoporose geeignet sind. Darüberhinaus sind diese Substanzen geeignet, die B-Zelldegeneration wie z.B. Apoptose oder Nekrose von pankreatischen B-Zellen zu verhindern. Die Substanzen sind weiter geeignet, die Funktionalität von pankreatischen Zellen zu verbessern oder wiederherzustellen, daneben die Anzahl und Größe von pankreatischen B-Zellen zu erhöhen. Zusätzlich und begründet durch die Rolle der Glucagon-Like Peptide, wie z.B. GLP-1 und GLP-2 und deren Verknüpfung mit DPP-IV Inhibition, wird erwartet, daß die erfindungsgemäßen Verbindungen geeignet sind, um unter anderem einen sedierenden oder angstlösenden Effekt zu erzielen, darüberhinaus katabole Zustände nach Operationen oder hormonelle Stressantworten günstig zu beeinflussen oder die Mortalität und Morbidität nach Myokardinfarkt reduzieren zu können. Darüberhinaus sind sie geeignet zur Behandlung von allen Zuständen, die im Zusammenhang mit oben genannten Effekten stehen und durch GLP-1 oder GLP-2 vermittelt sind. Die erfindungsgemäßen Verbindungen sind ebenfalls als Diuretika oder Antihypertensiva einsetzbar und zur Prävention und Behandlung des akuten Nierenversagens geeignet. Weiterhin sind die erfindungsgemäßen Verbindungen zur Behandlung entzündlicher Erkrankungen der Atemwege einsetzbar. Ebenso sind sie zur Prävention und Therapie von chronischen entzündlichen Darmerkrankungen wie z.B. Reizdarmsyndrom (IBS), Morbus Crohn oder Colitis ulcerosa ebenso wie bei Pankreatitis geeignet. Des weiteren wird erwartet, daß sie bei jeglicher Art von Verletzung oder Beeinträchtigung im Gastrointestinaltrakt eingesetzt werden können wie auch z.B. bei Kolitiden und Enteriden. Darüberhinaus wird erwartet, daß DPP-IV Inhibitoren und somit auch die erfindungsgemäßen Verbindungen zur Behandlung der Unfruchtbarkeit oder zur Verbesserung der Fruchtbarkeit beim Menschen oder im Säugetierorganismus verwendet werden können, insbesondere dann, wenn die Unfruchtbarkeit im Zusammenhang mit einer Insulinresistenz oder mit dem polyzystischen Ovarialsyndrom steht. Auf der anderen Seite sind diese Substanzen geeignet, die Motilität der Spermien zu beeinflussen und sind damit als Kontrazeptiva zur Verwendung beim Mann einsetzbar. Des weiteren sind die Substanzen geeignet, Mangelzustände von Wachstumshormon, die mit Minderwuchs einhergehen, zu beeinflussen, sowie bei allen Indikationen sinnvoll eingesetzt werden können, bei denen Wachstumshormon verwendet werden kann. Die erfindungsgemäßen Verbindungen sind auf Grund ihrer Hemmwirkung gegen DPP IV auch geeignet zur Behandlung von verschiedenen Autoimmunerkrankungen wie z.B. rheumatoide Arthritis, Multiple Sklerose, Thyreoditiden und Basedow' scher Krankheit etc.. Darüberhinaus können sie eingesetzt werden bei viralen Erkrankungen wie auch z.B. bei HIV Infektionen, zur Stimulation der Blutbildung, bei benigner Prostatahyperplasie, bei Gingivitiden, sowie zur Behandlung von neuronalen Defekten und neurdegenerativen Erkrankungen wie z.B. Morbus Alzheimer. Beschriebene Verbindungen sind ebenso zu verwenden zur Therapie von Tumoren, insbesondere zur Veränderung der Tumorinvasion wie auch Metastatisierung, Beispiele hier sind die Anwendung bei T-Zell Lymphomen, akuter lymphoblastischer Leukämie, zellbasierende Schilddrüsenkarzinome, Basalzellkarzinome oder Brustkarzinome. Weitere Indikationen sind Schlaganfall, Ischämien verschiedenster Genese, Morbus Parkinson und Migräne. Darüberhinaus sind weitere Indikationsgebiete follikuläre und epidermale Hyperkeratosen, erhöhte Keratinozytenproliferation, Psoriasis, Enzephalomyelitiden, Glomerulonephritiden, Lipodystrophien, sowie psychosomatische, depressive und neuropsychiatrische Erkrankungen verschiedenster Genese.

Die erfindungsgemäßen Verbindungen können auch in Kombination mit anderen Wirkstoffen verwendet werden. Zu den zu einer solchen Kombination geeigneten Therapeutika gehören z.B. Antidiabetika, wie etwa Metformin, Sulfonylharnstoffe (z.B. Glibenclamid, Tolbutamid, Glimepiride), Nateglinide, Repaglinide, Thiazolidindione (z.B. Rosiglitazone, Pioglitazone), PPAR-gamma-Agonisten (z.B. GI 262570) und -Antagonisten, PPAR-gamma/alpha Modulatoren (z.B. KRP 297), alpha-Glucosidasehemmer (z.B. Acarbose, Voglibose),andere DPP-IV Inhibitoren, alpha2-Antagonisten, Insulin und Insulinanaloga, GLP-1 und GLP-1 Analoga (z.B. Exendin-4) oder Amylin. Daneben SGLT2-Inhibitoren wie T-1095, Inhibitoren der Proteintyrosinphosphatase 1, Substanzen, die eine deregulierte Glucoseproduktion in der Leber beeinflussen, wie z.B. Inhibitoren der Glucose-6-phosphatase, oder der Fructose-1,6-bisphosphatase, der Glycogenphosphorylase, Glucagonrezeptor Antagonisten und Inhibitoren der Phosphoenolpyruvatcarboxykinase, der Glykogensynthasekinase oder der Pyruvatdehydrokinase, Lipidsenker, wie etwa HMG-CoA-Reduktasehemmer (z.B. Simvastatin, Atorvastatin), Fibrate (z.B. Bezafibrat, Fenofibrat), Nikotinsäure und deren Derivate, PPAR-alpha agonisten, PPAR-delta agonisten, ACAT Inhibitoren (z.B. Avasimibe) oder Cholesterolresorptionsinhibitoren wie zum Beispiel Ezetimibe, gallensäurebindende Substanzen wie zum Beispiel Colestyramin, Hemmstoffe des ilealen Gallensäuretransportes, HDL-erhöhende Verbindungen wie zum Beispiel Inhibitoren von CETP oder Regulatoren von ABC1 oder Wirkstoffe zur Behandlung von Obesitas, wie etwa Sibutramin oder Tetrahydrolipstatin, Dexfenfluramin, Axokine, Antagonisten des Cannbinoid1 Rezeptors, MCH-1 Rezeptorantagonisten, MC4 Rezeptor Agonisten, NPY5 oder NPY2 Antagonisten oder β₃-Agonisten wie SB-418790 oder AD-9677 ebenso wie Agonisten des 5HT2c Rezeptors.

Daneben ist eine Kombination mit Medikamenten zur Beeinflussung des Bluthochdrucks wie z.B. All Antagonisten oder ACE Inhibitoren, Diuretika, β-Blocker, Ca-Antagonisten und anderen oder Kombinationen daraus geeignet.

Die zur Erzielung einer entsprechenden Wirkung erforderliche Dosierung beträgt zweckmäßigerweise bei intravenöser Gabe 1 bis 100 mg, vorzugsweise 1 bis 30 mg, und bei oraler Gabe 1 bis 1000 mg, vorzugsweise 1 bis 100 mg, jeweils 1 bis 4 x täglich. Hierzu lassen sich die erfindungsgemäß hergestellten Verbindungen der Formel I, gegebenenfalls in Kombination mit anderen Wirksubstanzen, zusammen mit einem oder mehreren inerten üblichen Trägerstoffen und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure, Wasser, Wasser/Ethanol, Wasser/Glycerin, Wasser/Sorbit, Wasser/Polyethylenglykol, Propylenglykol, Cetylstearylalkohol, Carboxymethylcellulose oder fetthaltigen Substanzen wie Hartfett oder deren geeigneten Gemischen, in übliche galenische Zubereitungen wie Tabletten, Dragees, Kapseln, Pulver, Suspensionen oder Zäpfchen einarbeiten.

Die nachfolgenden Beispiele sollen die Erfindung näher erläutern:

### Herstellung der Ausgangsverbindungen:

### Beispiel I

### 1.3-Dimethyl-7-(2,6-dicyano-benzyl)-8-brom-xanthin

Eine Mischung aus 555 mg 8-Bromtheophyllin und 0.39 ml Hünigbase in 9 ml N,N-Dimethylformamid wird mit 600 mg 2-Bromomethyl-isophthalonitril versetzt und über Nacht bei Raumtemperatur gerührt. Zur Aufarbeitung wird das Reaktionsgemisch auf Wasser gegossen. Der ausgefallene Niederschlag wird abgesaugt, mit Wasser nachgewaschen und getrocknet.
Ausbeute: 686 mg (83 % der Theorie)
R_{f}-Wert: 0.56 (Kieselgel, Methylenchlorid/Methanol = 95:5)
Massenspektrum (ESI⁺): m/z = 399, 401 [M+H]⁺

### Analog Beispiel I werden folgende Verbindungen erhalten:

(1) 3-Methyl-7-(3-methyl-2-buten-1-yl)-8-chlor-xanthin
   Massenspektrum (ESI⁺): m/z = 269, 271 [M+H]⁺
(2) 3-Methyl-7-(2-cyano-benzyl)-8-chlor-xanthin
   Massenspektrum (ESI⁺): m/z = 316, 318 [M+H]⁺
(3) 1-(2-Phenyl-2-oxo-ethyl)-3-methyl-7-(2-butin-1-yl)-8-brom-xanthin
   Massenspektrum (ESI⁺): m/z = 415, 417 [M+H]⁺
(4) 3-Methyl-7-[(2-trimethylsilanyl-ethoxy)methyl]-8-brom-xanthin
   (Durchführung in Gegenwart von Kaliumcarbonat)
   Massenspektrum (ESI⁺): m/z = 375, 377 [M+H]⁺
(5) 3-Methyl-7-(3-methyl-2-buten-1-yl)-8-brom-xanthin
   Massenspektrum (ESI⁺): m/z = 313, 315 [M+H]⁺
(6) 3-Methyl-7-(2,3-dimethyl-2-buten-1-yl)-8-brom-xanthin R_{f}-Wert: 0.43 (Kieselgel, Methylenchlorid/Methanol = 9:1)
   Massenspektrum (ESI⁺): m/z = 327, 329 [M+H]⁺
(7) 3-Methyl-7-(2-butin-1-yl)-8-brom-xanthin
   R_{f}-Wert: 0.72 (Kieselgel, Essigester)
   Massenspektrum (ESI⁺): m/z = 297/299 [M+H]⁺
(8) 3-Methyl-7-((E)-2-buten-1-yl)-8-brom-xanthin
   (Das Produkt ist mit ca. 10-20 % Z-Verbindung verunreinigt)
   R_{f}-Wert: 0.55 (Kieselgel, Cyclohexan/Essigester/Methanol = 6:3:1)
   Massenspektrum (ESI⁺): m/z = 299, 301 [M+H]⁺
(9) 3-Methyl-7-[(1-cyclopenten-1-yl)methyl]-8-brom-xanthin
   Massenspektrum (ESI⁺): m/z = 325, 327 [M+H]⁺
(10) 1-(2-Phenyl-2-oxo-ethyl)-3-methyl-7-[(1-cyclopenten-1-yl)methyl]-8-brom-xanthin
   Massenspektrum (ESI⁺): m/z = 443, 445 [M+H]⁺
(11) 1-(2-Phenyl-2-oxo-ethyl)-3-methyl-7-((E)-2-buten-1-yl)-8-brom-xanthin
   (Produkt enthält ca. 25 % Z-Isomer)
   Massenspektrum (ESI⁺): m/z = 417, 419 [M+H]⁺
(12) 1-(2-Phenyl-2-oxo-ethyl)-3-methyl-7-(2-methyl-allyl)-8-brom-xanthin
   R_{f}-Wert: 0.71 (Kieselgel, Methylenchlorid/Methanol = 95:5)
   Massenspektrum (ESI⁺): m/z = 417, 419 [M+H]⁺
(13) 1-(2-Phenyl-2-oxo-ethyl)-3-methyl-7-(3-brom-allyl)-8-brom-xanthin
   R_{f}-Wert: 0.68 (Kieselgel, Methylenchlorid/Methanol = 95:5)
   Massenspektrum (ESI⁺): m/z = 481, 483, 485 [M+H]⁺
(14) 1-(2-Phenyl-2-oxo-ethyl)-3-methyl-7-[(furan-2-yl)methyl]-8-brom-xanthin
   R_{f}-Wert: 0.60 (Kieselgel, Methylenchlorid/Methanol = 95:5)
   Massenspektrum (ESI⁺): m/z = 443, 445 [M+H]⁺
(15) 1-(2-Phenyl-2-oxo-ethyl)-3-methyl-7-(2-chlor-allyl)-8-brom-xanthin
   R_{f}-Wert: 0.77 (Kieselgel, Methylenchlorid/Methanol = 95:5)
   Massenspektrum (ESI⁺): m/z = 437, 439, 441 [M+H]⁺
(16) 1-(2-Phenyl-2-oxo-ethyl)-3-methyl-7-((Z)-2-methyl-2-buten-1-yl)-8-brom-xanthin
   R_{f}-Wert: 0.77 (Kieselgel, Methylenchlorid/Methanol = 95:5)
   Massenspektrum (ESI⁺): m/z = 431, 433 [M+H]⁺
(17) 1-(2-Phenyl-2-oxo-ethyl)-3-methyl-7-((E)-2-methyl-2-buten-1-yl)-8-brom-xanthin
   R_{f}-Wert: 0.77 (Kieselgel, Methylenchlorid/Methanol = 95:5)
   Massenspektrum (ESI⁺): m/z = 431, 433 [M+H]⁺
(18) 1-(2-Phenyl-2-oxo-ethyl)-3-methyl-7-(1-phenylsulfanyl-butyl)-8-brom-xanthin
   R_{f}-Wert: 0.83 (Kieselgel, Methylenchlorid/Methanol = 95:5)
   Massenspektrum (ESI⁺): m/z = 527, 529 [M+H]⁺
(19) 3-Methyl-7-(3-methyl-1-phenylsulfanyl-butyl)-8-brom-xanthin
   (Das als Ausgangsmaterial für die Umsetzung verwendete [(1-Chlor-3-methylbutyl)sulfanyl]-benzol wird durch Chlorierung von [(3-Methyl-butyl)sulfanyl]-benzol mit N-Chlor-succinimid inTetrachlorkohlenstoff erhalten)
   R_{f}-Wert: 0.38 (Kieselgel, Methylenchlorid/Methanol = 95:5)
   Massenspektrum (ESI⁺): m/z = 423, 425 [M+H]⁺
(20) 1,3-Dimethyl-7-(2-brom-benzyl)-8-chlor-xanthin
   R_{f}-Wert: 0.50 (Kieselgel, Cyclohexan/Essigester = 1:1)
(21) 1,3-Dimethyl-7-(2-chlor-benzyl)-8-chlor-xanthin
   R_{f}-Wert: 0.50 (Kieselgel, Cyclohexan/Essigester = 1:1)
(22) 3-Cyclopropyl-7-(2-butin-1-yl)-8-brom-xanthin
   R_{f}-Wert: 0.45 (Reversed Phase DC-Fertigplatte (E. Merck), Acetonitril/Wasser/ Trifluoressigsäure = 50:50:1)
   Massenspektrum (ESI⁺): m/z = 223/225 [M+H]⁺

### Beispiel II

### 1-(2-{2-[(Ethoxycarbonyl)methoxy]-phenyl}-2-oxo-ethyl)-3-methyl-7-(3-methyl-2-buten-1-yl)-8-[3-(tert.-butyloxycarbonylamino)-piperidin-1-yl]-xanthin

Zu einem Gemisch aus 200 mg 1-[2-(2-Hydroxy-phenyl)-2-oxo-ethyl]-3-methyl-7-(3-methyl-2-buten-1-yl)-8-[3-(tert.-butyloxycarbonylamino)-piperidin-1-yl]-xanthin und 63 mg Kaliumcarbonat in 3 ml N,N-Dimethylformamid werden 63 mg Bromessigsäureethylester gegeben. Das Reaktionsgemisch wird fünf Stunden bei Raumtemperatur gerührt. Zur Aufarbeitung wird es mit Wasser versetzt und der ausgefallene Niederschlag wird abgesaugt, mit Wasser nachgewaschen und drei Stunden bei 80°C im Trockenschrank getrocknet.
Ausbeute: 216 mg (94 % der Theorie)
Massenspektrum (ESI⁺): m/z = 653 [M+H]⁺

### Analog Beispiel II werden folgende Verbindungen erhalten:

(1) 1-(2-{2-[(Aminocarbonyl)methoxy]-phenyl}-2-oxo-ethyl)-3-methyl-7-(3-methyl-2-buten-1-yl)-8-[3-(tert.-butyloxycarbonylamino)-piperidin-1-yl]-xanthin
   Massenspektrum (ESI⁺): m/z = 624 [M+H]⁺
(2) 1-(2-{3-[(Methylsulfanyl)methoxy]-phenyl}-2-oxo-ethyl)-3-methyl-7-(3-methyl-2-buten-1-yl)-8-[3-(tert.-butyloxycarbonylamino)-piperidin-1-yl]-xanthin
   R_{f}-Wert: 0.20 (Kieselgel, Cyclohexan/Essigester = 6:4)
   Massenspektrum (ESI⁺): m/z = 627 [M+H]⁺
(3) 1-(2-{3-[(Ethoxycarbonyl)methoxy]-phenyl}-2-oxo-ethyl)-3-methyl-7-(3-methyl-2-buten-1-yl)-8-[3-(tert.-butyloxycarbonylamino)-piperidin-1-yl]-xanthin
   R_{f}-Wert: 0.50 (Kieselgel, Cyclohexan/Essigester = 3:7)
(4) 1-(2-{2-[(Methylaminocarbonyl)methoxy]-phenyl}-2-oxo-ethyl)-3-methyl-7-(3-methyl-2-buten-1-yl)-8-[3-(tert.-butyloxycarbonylamino)-piperidin-1-yl]-xanthin
   Massenspektrum (ESI⁺): m/z = 638 [M+H]⁺
(5) 1-(2-{2-[(Dimethylaminocarbonyl)methoxy]-phenyl}-2-oxo-ethyl)-3-methyl-7-(3-methyl-2-buten-1-yl)-8-[3-(tert.-butyloxycarbonylamino)-piperidin-1-yl]-xanthin
   Massenspektrum (ESI⁺): m/z = 652 [M+H]⁺
(6) 1-(2-{3-[(Methoxycarbonyl)methoxy]-phenyl}-2-oxo-ethyl)-3-methyl-7-(3-methyl-2-buten-1-yl)-8-[3-(tert.-butyloxycarbonylamino)-piperidin-1-yl]-xanthin
   Massenspektrum (ESI⁺): m/z = 639 [M+H]⁺
(7) 1-(2-{2-[(Ethylaminocarbonyl)methoxy]-phenyl}-2-oxo-ethyl)-3-methyl-7-(2-butin-1-yl)-8-[(*R*)-3-(tert.-butyloxycarbonylamino)-piperidin-1-yl]-xanthin
   Massenspektrum (ESI⁺): m/z = 636 [M+H]⁺
(8) 1-(2-{2-[(Methylaminocarbonyl)methoxy]-phenyl}-2-oxo-ethyl)-3-methyl-7-[(1-cyclopenten-1-yl)methyl]-8-[3-(tert.-butyloxycarbonylamino)-piperidin-1-yl]-xanthin
   Massenspektrum (ESI⁺): m/z = 650 [M+H]⁺
(9) 1-(2-{2-[(Methylaminocarbonyl)methoxy]-phenyl}-2-oxo-ethyl)-3-methyl-7-(2-butin-1-yl)-8-[3-(tert.-butyloxycarbonylamino)-piperidin-1-yl]-xanthin
   Massenspektrum (ESI⁺): m/z = 622 [M+H]⁺
(10) 1-(2-{2-[(Aminocarbonyl)methoxy]-phenyl}-2-oxo-ethyl)-3-methyl-7-(2-butin-1-yl)-8-[3-(tert.-butyloxycarbonylamino)-piperidin-1-yl]-xanthin
   Massenspektrum (ESI⁺): m/z = 608 [M+H]⁺
(11) 1-(2-{2-[(Methoxycarbonyl)methoxy]-phenyl}-2-oxo-ethyl)-3-methyl-7-(2-butin-1-yl)-8-[3-(tert.-butyloxycarbonylamino)-piperidin-1-yl]-xanthin
   Massenspektrum (ESI⁺): m/z = 623 [M+H]⁺
(12) 1-(2-{2-[(Isopropyloxycarbonyl)methoxy]-phenyl}-2-oxo-ethyl)-3-methyl-7-(3-methyl-2-buten-1-yl)-8-[3-(tert.-butyloxycarbonylamino)-piperidin-1-yl]-xanthin
   Massenspektrum (ESI⁺): m/z = 667 [M+H]⁺
(13) 1-(2-{2-[(Methylaminocarbonyl)methoxy]-phenyl}-2-oxo-ethyl)-3-methyl-7-(2-butin-1-yl)-8-[(*R*)-3-(tert.-butyloxycarbonylamino)-piperidin-1-yl]-xanthin
   Massenspektrum (ESI⁺): m/z = 622 [M+H]⁺
(14) 1-(2-{2-[(Methylaminocarbonyl)methoxy]-phenyl}-2-oxo-ethyl)-3-methyl-7-((E)-2-buten-1-yl)-8-[3-(tert.-butyloxycarbonylamino)-piperidin-1-yl]-xanthin
   (Produkt enthält etwas Z-Isomer)
   R_{f}-Wert: 0.35 (Kieselgel, Cyclohexan/Essigester/Methanol = 5:4:1) Massenspektrum (ESI⁺): m/z = 624 [M+H]⁺
(15) 1-(2-{2-[(Ethylaminocarbonyl)methoxy]-phenyl}-2-oxo-ethyl)-3-methyl-7-(2-butin-1-yl)-8-[(S)-3-(tert.-butyloxycarbonylamino)-piperidin-1-yl]-xanthin
   Massenspektrum (ESI⁺): m/z = 636 [M+H]⁺
(16) 1-(2-{2-[(Methylaminocarbonyl)methoxy]-phenyl}-2-oxo-ethyl)-3-methyl-7-(2-butin-1-yl)-8-[(S)-3-(tert.-butyloxycarbonylamino)-piperidin-1-yl]-xanthin
   Massenspektrum (ESI⁺): m/z = 622 [M+H]⁺
(17) 1-(2-{2-[(Methoxycarbonyl)methoxy]-phenyl}-2-oxo-ethyl)-3-methyl-7-(3-methyl-2-buten-1-yl)-8-[3-(tert.-butyloxycarbonylamino)-piperidin-1-yl]-xanthin
   Massenspektrum (ESI⁺): m/z = 639 [M+H]⁺
(18) 1-(2-{2-[(Methylaminocarbonyl)methoxy]-phenyl}-2-oxo-ethyl)-3-methyl-7-(3-methyl-2-buten-1-yl)-8-[(S)-3-(tert.-butyloxycarbonylamino)-piperidin-1-yl]-xanthin
   Massenspektrum (ESI⁺): m/z = 638 [M+H]⁺
(19) 2-(2-Acetyl-phenoxy)-*N*-ethyl-acetamid
   Massenspektrum (ESI⁺): m/z = 222 [M+H]⁺
(20) 1-{2-[2-(1-Methoxycarbonyl-ethoxy)-phenyl]-2-oxo-ethyl}-3-methyl-7-(2-butin-1-yl)-8-[3-(tert.-butyloxycarbonylamino)-piperidin-1-yl]-xanthin
   Massenspektrum (ESI⁺): m/z = 637 [M+H]⁺
(21) 1-{2-[2-(1-Aminocarbonyl-ethoxy)-phenyl]-2-oxo-ethyl}-3-methyl-7-(2-butin-1-yl)-8-[3-(tert.-butyloxycarbonylamino)-piperidin-1-yl]-xanthin
   Massenspektrum (ESI⁺): m/z = 622 [M+H]⁺
(22) 2-(2-Acetyl-phenoxy)-*N*-methyl-acetamid
   Massenspektrum (ESI⁺): m/z = 208 [M+H]⁺
(23) 1-{2-[2-(2-Oxo-propoxy)-phenyl]-2-oxo-ethyl}-3-methyl-7-(2-butin-1-yl)-8-[3-(tert.-butyloxycarbonylamino)-piperidin-1-yl]-xanthin
   Massenspektrum (ESI⁺): m/z = 607 [M+H]⁺
(24) 1-{2-[2-(1-Ethoxycarbonyl-1-methyl-ethoxy)-phenyl]-2-oxo-ethyl}-3-methyl-7-(2-butin-1-yl)-8-[3-(tert.-butyloxycarbonylamino)-piperidin-1-yl]-xanthin
   Massenspektrum (ESI⁺): m/z = 665 [M+H]⁺
(25) 1-{2-[2-Cyanomethoxy-phenyl]-2-oxo-ethyl}-3-methyl-7-(2-butin-1-yl)-8-[3-(tert.-butyloxycarbonylamino)-piperidin-1-yl]-xanthin
   Massenspektrum (ESI⁺): m/z = 590 [M+H]⁺
(26) 1-(2-{2-[(Methylsulfanyl)methoxy]-phenyl}-2-oxo-ethyl)-3-methyl-7-(2-butin-1-yl)-8-[3-(tert.-butyloxycarbonylamino)-piperidin-1-yl]-xanthin
   Massenspektrum (ESI⁺): m/z = 611 [M+H]⁺
(27) 1-{[2-(tert.-Butylcarbonyl)-benzofuran-3-yl]methyl}-3-methyl-7-(2-butin-1-yl)-8-[3-(tert.-butyloxycarbonylamino)-piperidin-1-yl]-xanthin
   (Entsteht als Hauptprodukt bei der Umsetzung von 1-[2-(2-Hydroxy-phenyl)-2-oxo-ethyl]-3-methyl-7-(2-butin-1-yl)-8-[3-(tert.-butyloxycarbonylamino)-piperidin-1-yl]-xanthin mit 1-Chlor-3,3-dimethyl-butan-2-on)
   Massenspektrum (ESI⁺): m/z = 631 [M+H]⁺

### Beispiel III

### 1-[2-(2-Hydroxy-phenyl)-2-oxo-ethyl]-3-methyl-7-(3-methyl-2-buten-1-yl)-8-[3-(tert.-butyloxycarbonylamino)-piperidin-1-yl]-xanthin

Zu einem Gemisch aus 2.51 g 1-[2-(2-Hydroxy-phenyl)-2-oxo-ethyl]-3-methyl-7-(3-methyl-2-buten-1-yl)-8-chlor-xanthin und 880 mg Natriumcarbonat in 8 ml Dimethylsulfoxid werden 1.30 g 3-tert.-Butyloxycarbonylamino-piperidin gegeben. Das Reaktionsgemisch wird 18 Stunden bei 60°C gerührt. Zur Aufarbeitung wird es mit Wasser versetzt und der ausgefallene Niederschlag wird abgesaugt. Das feste Rohprodukt wird in Essigester gelöst, die Lösung über Magnesiumsulfat getrocknet und eingeengt. Der Kolbenrückstand wird über eine Kieselgel-Säule mit Cyclohexan/Essigester (10:1 auf 1:1) als Laufmittel chromatographiert.
Ausbeute: 2.56 g (91 % der Theorie)
Massenspektrum (ESI⁺): m/z = 567 [M+H]⁺

### Analog Beispiel III werden folgende Verbindungen erhalten:

(1) 3-Methyl-7-(3-methyl-2-buten-1-yl)-8-[3-(tert.-butyloxycarbonylamino)-piperidin-1-yl]-xanthin
   Massenspektrum (ESI⁺): m/z = 433 [M+H]⁺
(2) 1-(1-Methyl-2-oxo-2-phenyl-ethyl)-3-methyl-7-(3-methyl-2-buten-1-yl)-8-[3-(tert.-butyloxycarbonylamino)-piperidin-1-yl]-xanthin
   Massenspektrum (ESI⁺): m/z = 565 [M+H]⁺
(3) 3-Methyl-7-(2-cyano-benzyl)-8-[3-(tert.-butyloxycarbonylamino)-piperidin-1-yl]-xanthin
   R_{f}-Wert: 0.90 (Kieselgel, Methylenchlorid/Methanol = 9:1)
   Massenspektrum (ESI⁻): m/z = 478 [M-H]⁻
(4) 1-Methyl-3-[(methoxycarbonyl)methyl]-7-(2-cyano-benzyl)-8-[3-(tert.-butyloxy-carbonylamino)-piperidin-1-yl]-xanthin
   Massenspektrum (ESI⁺): m/z = 552 [M+H]⁺
(5) 1-Methyl-3-cyanomethyl-7-(2-cyano-benzyl)-8-[3-(tert.-butyloxycarbonylamino)-piperidin-1-yl]-xanthin
   Massenspektrum (ESI⁺): m/z = 519 [M+H]⁺
(6) 1-Methyl-3-(2-propin-1-yl)-7-(2-cyano-benzyl)-8-[3-(tert.-butyloxycarbonylamino)-piperidin-1-yl]-xanthin
   Massenspektrum (ESI⁺): m/z = 518 [M+H]⁺
(7) 1-[2-(3-Nitro-phenyl)-2-oxo-ethyl]-3-methyl-7-(3-methyl-2-buten-1-yl)-8-[3-(tert.-butyloxycarbonylamino)-piperidin-1-yl]-xanthin
   R_{f}-Wert: 0.25 (Kieselgel, Cyclohexan/Essigester/Methanol = 7:2:1)
   Massenspektrum (ESI⁺): m/z = 596 [M+H]⁺
(8) 1-Methyl-3-(2-propen-1-yl)-7-(2-cyano-benzyl)-8-[3-(tert.-butyloxycarbonylamino)-piperidin-1-yl]-xanthin
   Massenspektrum (ESI⁺): m/z = 520 [M+H]⁺
(9) 1-(2-Phenyl-2-oxo-ethyl)-3-methyl-7-(2-butin-1-yl)-8-[3-(tert.-butyloxycarbonyl-amino)-piperidin-1-yl]-xanthin
   Massenspektrum (ESI⁺): m/z = 535 [M+H]⁺
(10) 1-[2-(2-Nitro-phenyl)-2-oxo-ethyl]-3-methyl-7-(3-methyl-2-buten-1-yl)-8-[3-(tert.-butyloxycarbonylamino)-piperidin-1-yl]-xanthin
   R_{f}-Wert: 0.52 (Kieselgel, Cyclohexan/Essigester = 3:7)
   Massenspektrum (ESI⁺): m/z = 596 [M+H]⁺
(11) 1-Methyl-3-phenyl-7-(2-cyano-benzyl)-8-[3-(tert.-butyloxycarbonylamino)-piperidin-1-yl]-xanthin
   Massenspektrum (ESI⁺): m/z = 556 [M+H]⁺
(12) 1-[2-(2-Nitro-phenyl)-2-oxo-ethyl]-3-methyl-7-(3-methyl-2-buten-1-yl)-8-[(S)-3-(tert.-butyloxycarbonylamino)-piperidin-1-yl]-xanthin
   Massenspektrum (ESI⁺): m/z = 596 [M+H]⁺
(13) 1-[(Cinnolin-4-yl)methyl]-3-methyl-7-(3-methyl-2-buten-1-yl)-8-[3-(tert.-butyloxy-carbonylamino)-piperidin-1-yl]-xanthin im Gemisch mit 1-[(1,4-Dihydro-cinnolin-4-yl)methyl]-3-methyl-7-(3-methyl-2-buten-1-yl)-8-[3-(tert.-butyloxycarbonylamino)-piperidin-1-yl]-xanthin
   R_{f}-Wert: 0.62 (Kieselgel, Essigester)
(14) 1-({4-Oxo-3-[(2-trimethylsilanyl-ethoxy)methyl]-3,4-dihydro-phthalazin-1-yl}-methyl)-3-methyl-7-(3-methyl-2-buten-1-yl)-8-[3-(tert.-butyloxycarbonylamino)-piperidin-1-yl]-xanthin
   (Durchführung mit Kaliumcarbonat in Gegenwart von Hünigbase)
   R_{f}-Wert: 0.27 (Kieselgel, Cyclohexan/Essigester = 1:1)
   Massenspektrum (ESI⁺): m/z = 720 [M+H]⁺
(15) 1-[(Isochinolin-3-yl)methyl]-3-methyl-7-(3-methyl-2-buten-1-yl)-8-[3-(tert.-butyloxycarbonylamino)-piperidin-1-yl]-xanthin
   R_{f}-Wert: 0.31 (Kieselgel, Essigester/Petrolether = 7:3)
   Massenspektrum (ESI⁺): m/z = 574 [M+H]⁺
(16) 1-[(3-Methyl-4-oxo-3,4-dihydro-phthalazin-1-yl)methyl]-3-methyl-7-(3-methyl-2-buten-1-yl)-8-[3-(tert.-butyloxycarbonylamino)-piperidin-1-yl]-xanthin
   R_{f}-Wert: 0.45 (Kieselgel, Methylenchlorid/Methanol/konz. wässriges Ammoniak = 95:5)
   Massenspektrum (ESI⁺): m/z = 605 [M+H]⁺
(17) 3-Methyl-7-(2,3-dimethyl-2-buten-1-yl)-8-[3-(tert.-butyloxycarbonylamino)-piperidin-1-yl]-xanthin
   (Durchführung mit Kaliumcarbonat)
   R_{f}-Wert: 0.42 (Kieselgel, Methylenchlorid/Methanol = 20:1)
   Massenspektrum (ESI⁺): m/z = 447 [M+H]⁺
(18) 3-Methyl-7-(2-butin-1-yl)-8-[3-(tert.-butyloxycarbonylamino)-piperidin-1-yl]-xanthin
   (Durchführung mit Kaliumcarbonat)
   Schmelzpunkt: 235-237°C
   Massenspektrum (ESI⁺): m/z = 417 [M+H]⁺
(19) 1-[(Chinolin-4-yl)methyl]-3-methyl-7-(2-butin-1-yl)-8-[3-(tert.-butyloxycarbonyl-amino)-piperidin-1-yl]-xanthin
   (Durchführung mit Kaliumcarbonat)
   R_{f}-Wert: 0.36 (Kieselgel, Essigester)
   Massenspektrum (ESI⁺): m/z = 558 [M+H]⁺
(20) 1-[(Isochinolin-1-yl)methyl]-3-methyl-7-(2-butin-1-yl)-8-[3-(tert.-butyloxycarbonyl-amino)-piperidin-1-yl]-xanthin
   (Durchführung mit Kaliumcarbonat)
   R_{f}-Wert: 0.71 (Kieselgel, Essigester)
   Massenspektrum (ESI⁺): m/z = 558 [M+H]⁺
(21) 1-[(lsochinolin-1-yl)methyl]-3-methyl-7-((E)-2-buten-1-yl)-8-[3-(tert.-butyloxy-carbonylamino)-piperidin-1-yl]-xanthin
   (Durchführung mit Kaliumcarbonat; das Produkt enthält ca. 20 % Z-Isomer)
   R_{f}-Wert: 0.24 (Kieselgel, Essigester/Petrolether = 1:1)
   Massenspektrum (ESI⁺): m/z = 560 [M+H]⁺
(22) 3-Methyl-7-(2-butin-1-yl)-8-[(R)-3-(tert.-butyloxycarbonylamino)-piperidin-1-yl]-xanthin
   (Durchführung mit Kaliumcarbonat)
   R_{f}-Wert: 0.64 (Kieselgel, Essigester)
   Massenspektrum (ESI⁺): m/z = 417 [M+H]⁺
(23) 3-Methyl-7-(2-butin-1-yl)-8-[(S)-3-(tert.-butyloxycarbonylamino)-piperidin-1-yl]-xanthin
   (Durchführung mit Kaliumcarbonat)
   R_{f}-Wert: 0.64 (Kieselgel, Essigester)
   Massenspektrum (ESI⁺): m/z = 417 [M+H]⁺
(24) 3-Methyl-7-((E)-2-buten-1-yl)-8-[(S)-3-(tert.-butyloxycarbonylamino)-piperidin-1-yl]-xanthin
   (Produkt enthält ca. 15 % Z-Isomer)
   R_{f}-Wert: 0.35 (Kieselgel, Cyclohexan/ Essigester = 3:7)
   Massenspektrum (ESI⁺): m/z = 419 [M+H]⁺
(25) 3-Methyl-7-((E)-2-buten-1-yl)-8-[(R)-3-(tert.-butyloxycarbonylamino)-piperidin-1-yl]-xanthin
   (Produkt enthält ca. 15 % Z-Isomer)
   R_{f}-Wert: 0.35 (Kieselgel, Cyclohexan/ Essigester = 3:7)
   Massenspektrum (ESI⁺): m/z = 419 [M+H]⁺
(26) 1-[2-(2-Hydroxy-phenyl)-2-oxo-ethyl]-3-methyl-7-(2-butin-1-yl)-8-[(*R*)-3-(tert.-butyloxycarbonylamino)-piperidin-1-yl]-xanthin
   Massenspektrum (ESI⁺): m/z = 551 [M+H]⁺
(27) 1-[2-(2-Amino-phenyl)-2-oxo-ethyl]-3-methyl-7-[(1-cyclopenten-1-yl)methyl]-8-[3-(tert.-butyloxycarbonylamino)-piperidin-1-yl]-xanthin
   Massenspektrum (ESI⁺): m/z = 578 [M+H]⁺
(28) 1-(2-Phenyl-2-oxo-ethyl)-3-methyl-7-[(1-cyclopenten-1-yl)methyl]-8-[3-(tert.-butyloxycarbonylamino)-piperidin-1-yl]-xanthin
   Massenspektrum (ESI⁺): m/z = 563 [M+H]⁺
(29) 1-[2-(2-Hydroxy-phenyl)-2-oxo-ethyl]-3-methyl-7-[(1-cyclopenten-1-yl)methyl]-8-[3-(tert.-butyloxycarbonylamino)-piperidin-1-yl]-xanthin
   Massenspektrum (ESI⁺): m/z = 579 [M+H]⁺
(30) 1-Methyl-3-isopropyl-7-(2-cyano-benzyl)-8-[3-(tert.-butyloxycarbonylamino)-piperidin-1-yl]-xanthin
   Massenspektrum (ESI⁺): m/z = 522 [M+H]⁺
(31) 1-[2-(2-Hydroxy-phenyl)-2-oxo-ethyl]-3-methyl-7-(2-butin-1-yl)-8-[3-(tert.-butyloxycarbonylamino)-piperidin-1-yl]-xanthin
   Massenspektrum (ESI⁺): m/z = 551 [M+H]⁺
(32) 1-[2-(2-Amino-phenyl)-2-oxo-ethyl]-3-methyl-7-((E)-2-buten-1-yl)-8-[3-(tert.-butyloxycarbonylamino)-piperidin-1-yl]-xanthin
   (Produkt enthält ca. 10 % Z-Isomer)
   R_{f}-Wert: 0.20 (Kieselgel, Cyclohexan/Essigester =1:1)
   Massenspektrum (ESI⁺): m/z = 552 [M+H]⁺
(33) 1-(2-Phenyl-2-oxo-ethyl)-3-methyl-7-((E)-2-buten-1-yl)-8-[3-(tert.-butyloxy-carbonylamino)-piperidin-1-yl]-xanthin
   (Produkt enthält ca. 25 % Z-Isomer)
   Massenspektrum (ESI⁺): m/z = 537 [M+H]⁺
(34) 1-[2-(2-Hydroxy-phenyl)-2-oxo-ethyl]-3-methyl-7-(2-butin-1-yl)-8-[(*R*)-3-(tert.-butyloxycarbonylamino)-piperidin-1-yl]-xanthin
(35) 1-[2-(2-Hydroxy-phenyl)-2-oxo-ethyl]-3-methyl-7-((E)-2-buten-1-yl)-8-[3-(tert.-butyloxycarbonylamino)-piperidin-1-yl]-xanthin
   (Produkt enthält etwas Z-Isomer)
   R_{f}-Wert: 0.30 (Kieselgel, Cyclohexan/Essigester = 4:6)
   Massenspektrum (ESI⁺): m/z = 553 [M+H]⁺
(36) 1-[2-(2-Hydroxy-phenyl)-2-oxo-ethyl]-3-methyl-7-(2-butin-1 -yl)-8-[(S)-3-(tert.-butyloxycarbonylamino)-piperidin-1-yl]-xanthin
   Massenspektrum (ESI⁺): m/z = 551 [M+H]⁺
(37) 1-[2-(2-Amino-phenyl)-2-oxo-ethyl]-3-methyl-7-(2-butin-1 -yl)-8-[3-(tert.-butyloxy-carbonylamino)-piperidin-1-yl]-xanthin
   R_{f}-Wert: 0.50 (Kieselgel, Methylenchlorid/Methanol = 95:5)
   Massenspektrum (ESI⁺): m/z = 550 [M+H]⁺
(38) 1-[2-(2-Hydroxy-phenyl)-2-oxo-ethyl]-3-methyl-7-(3-methyl-2-buten-1-yl)-8-[(*S*)-3-(tert.-butyloxycarbonylamino)-piperidin-1-yl]-xanthin
   Massenspektrum (ESI⁺): m/z = 567 [M+H]⁺
(39) 1-(2-Phenyl-2-oxo-ethyl)-3-methyl-7-(2-butin-1-yl)-8-[(*R*)-3-(tert.-butyloxy-carbonylamino)-piperidin-1-yl]-xanthin
   Massenspektrum (ESI⁺): m/z = 535 [M+H]⁺
(40) 1-Methyl-3-[(2-trimethylsilanyl-ethoxy)methyl]-7-(2-cyano-benzyl)-8-[3-(tert.-butyloxycarbonylamino)-piperidin-1-yl]-xanthin
   Massenspektrum (ESI⁺): m/z = 610 [M+H]⁺
(41) 3-Methyl-7-(2-butin-1-yl)-8-[(*S*)-3-(tert.-butyloxycarbonylamino)-piperidin-1-yl]-xanthin
   (Durchführung mit Kaliumcarbonat)
   R_{f}-Wert: 0.52 (Kieselgel, Essigester)
   Massenspektrum (ESI⁺): m/z = 417 [M+H]⁺
(42) 1-(2-Phenyl-2-oxo-ethyl)-3-methyl-7-(2-methyl-allyl)-8-[3-(tert.-butyloxycarbonyl-amino)-piperidin-1-yl]-xanthin
   R_{f}-Wert: 0.46 (Kieselgel, Methylenchlorid/Methanol = 95:5)
(43) 1-(2-Phenyl-2-oxo-ethyl)-3-methyl-7-(3-brom-allyl)-8-[3-(tert.-butyloxycarbonyl-amino)-piperidin-1-yl]-xanthin
   R_{f}-Wert: 0.22 (Kieselgel, Methylenchlorid/Methanol = 95:5)
   Massenspektrum (ESI⁺): m/z = 601, 603 [M+H]⁺
(44) 1-(2-Phenyl-2-oxo-ethyl)-3-methyl-7-[(furan-2-yl)methyl]-8-[3-(tert.-butyloxy-carbonylamino)-piperidin-1-yl]-xanthin
   R_{f}-Wert: 0.41 (Kieselgel, Methylenchlorid/Methanol = 95:5)
(45) 1-(2-Phenyl-2-oxo-ethyl)-3-methyl-7-(2-chlor-allyl)-8-[3-(tert.-butyloxycarbonyl-amino)-piperidin-1-yl]-xanthin
   R_{f}-Wert: 0.49 (Kieselgel, Methylenchlorid/Methanol = 95:5)
   Massenspektrum (ESI⁺): m/z = 557, 559 [M+H]⁺
(46) 1-(2-Phenyl-2-oxo-ethyl)-3-methyl-7-(2-butin-1-yl)-8-[(S)-3-(tert.-butyloxy-carbonylamino)-piperidin-1-yl]-xanthin
   Massenspektrum (ESI⁺): m/z = 535 [M+H]⁺
(47) 1-[2-(2-Amino-phenyl)-2-oxo-ethyl]-3-methyl-7-((E)-2-buten-1-yl)-8-[(*S*)-3-(tert.-butyloxycarbonylamino)-piperidin-1-yl]-xanthin
   R_{f}-Wert: 0.40 (Kieselgel, Cyclohexan/Essigester = 4:6)
   Massenspektrum (ESI⁺): m/z = 552 [M+H]⁺
(48) 1-[2-(2-Nitro-phenyl)-2-oxo-ethyl]-3-methyl-7-((E)-2-buten-1-yl)-8-[(*R*)-3-(tert.-butyloxycarbonylamino)-piperidin-1-yl]-xanthin
   R_{f}-Wert: 0.50 (Kieselgel, Cyclohexan/Essigester = 1:2)
(49) 1-[2-(2-Nitro-phenyl)-2-oxo-ethyl]-3-methyl-7-((E)-2-buten-1-yl)-8-[(*S*)-3-(tert.-butyloxycarbonylamino)-piperidin-1-yl]-xanthin
   Massenspektrum (ESI⁺): m/z = 582 [M+H]⁺
(50) 1-[2-(2-Nitro-3-methoxy-phenyl)-2-oxo-ethyl]-3-methyl-7-(3-methyl-2-buten-1-yl)-8-[3-(tert.-butyloxycarbonylamino)-piperidin-1-yl]-xanthin
   Massenspektrum (ESI⁺): m/z = 626 [M+H]⁺
(51) 1-(2-{2-Oxo-3-[(2-trimethylsilanyl-ethoxy)methyl]-2,3-dihydro-benzooxazol-7-yl}-2-oxo-ethyl)-3-methyl-7-(3-methyl-2-buten-1-yl)-8-[3-(tert.-butyloxycarbonylamino)-piperidin-1-yl]-xanthin
   Massenspektrum (ESI⁺): m/z = 738 [M+H]⁺
(52) 1-(2-Phenyl-2-oxo-ethyl)-3-methyl-7-((Z)-2-methyl-2-buten-1-yl)-8-[3-(tert.-butyloxycarbonylamino)-piperidin-1-yl]-xanthin
   R_{f}-Wert: 0.48 (Kieselgel, Methylenchlorid/Methanol = 95:5)
   Massenspektrum (ESI⁺): m/z = 551 [M+H]⁺
(53) 1-(2-Phenyl-2-oxo-ethyl)-3-methyl-7-((E)-2-methyl-2-buten-1-yl)-8-[3-(tert.-butyloxycarbonylamino)-piperidin-1-yl]-xanthin
   Massenspektrum (ESI⁺): m/z = 551 [M+H]⁺
(54) 1-(2-{2-Oxo-3-[(2-trimethylsilanyl-ethoxy)methyl]-2,3-dihydro-benzooxazol-4-yl}-2-oxo-ethyl)-3-methyl-7-(2-butin-1-yl)-8-[3-(tert.-butyloxycarbonylamino)-piperidin-1-yl]-xanthin
   R_{f}-Wert: 0.40 (Kieselgel, Petrolether/Essigester = 1:1)
   Massenspektrum (ESI⁺): m/z = 722 [M+H]⁺
(55) 1-[2-(2,2-Difluor-benzo[1,3]dioxol-4-yl)-2-oxo-ethyl]-3-methyl-7-(2-butin-1-yl)-8-[(*R*)-3-(tert.-butyloxycarbonylamino)-piperidin-1-yl]-xanthin
   Massenspektrum (ESI⁺): m/z = 615 [M+H]⁺
(56) 1-[2-(2,2-Difluor-benzo[1,3]dioxol-4-yl)-2-oxo-ethyl]-3-methyl-7-(2-butin-1-yl)-8-[(*S*)-3-(tert.-butyloxycarbonylamino)-piperidin-1-yl]-xanthin
   Massenspektrum (ESI⁺): m/z = 615 [M+H]⁺
(57) 1-[(1-Methyl-1*H*-indol-2-yl)methyl]-3-methyl-7-(2-butin-1-yl)-8-[3-(tert.-butyloxy-carbonylamino)-piperidin-1-yl]-xanthin
   R_{f}-Wert: 0.80 (Kieselgel, Methylenchlorid/Methanol = 95:5)
   Massenspektrum (ESI⁺): m/z = 560 [M+H]⁺
(58) 1-[(Chinolin-3-yl)methyl]-3-methyl-7-(2-butin-1-yl)-8-[3-(tert.-butyloxycarbonyl-amino)-piperidin-1-yl]-xanthin
   R_{f}-Wert: 0.50 (Kieselgel, Essigester)
   Massenspektrum (ESI⁺): m/z = 558 [M+H]⁺
(59) 1-{[1-(tert.-Butyloxycarbonylamino)-1*H*-indol-2-yl]methyl}-3-methyl-7-(2-butin-1-yl)-8-[3-(tert.-butyloxycarbonylamino)-piperidin-1-yl]-xanthin
   R_{f}-Wert: 0.40 (Kieselgel, Petrolether/Essigester = 1:1)
   Massenspektrum (ESI⁺): m/z = 646 [M+H]⁺
(60) 1-[(2-Methyl-1-[(2-trimethylsilanyl-ethoxy)methyl]-1*H*-benzoimidazol-5-yl)methyl]-3-methyl-7-(2-butin-1-yl)-8-[3-(tert.-butyloxycarbonylamino)-piperidin-1-yl]-xanthin (im Gemisch mit 1-[(2-Methyl-3-[(2-trimethylsilanyl-ethoxy)methyl]-3*H*-benzoimidazol-5-yl)methyl]-3-methyl-7-(2-butin-1-yl)-8-[3-(tert.-butyloxycarbonylamino)-piperidin-1-yl]-xanthin)
   R_{f}-Wert: 0.15 (Kieselgel, Essigester)
   Massenspektrum (ESI⁺): m/z = 691 [M+H]⁺
(61) 1-[2-(Chinolin-8-yl-]-2-oxo-ethyl]-3-methyl-7-(2-butin-1-yl)-8-[3-(tert.-butyloxy-carbonylamino)-piperidin-1-yl]-xanthin
   R_{f}-Wert: 0.35 (Kieselgel, Methylenchlorid/Methanol = 95:5)
   Massenspektrum (ESI⁺): m/z = 586 [M+H]⁺
(62) 1-[(1-[(2-Trimethylsilanyl-ethoxy)methyl]-1*H*-benzoimidazol-5-yl)methyl]-3-methyl--yl)-8-[3-(tert.-butyloxycarbonylamino)-piperidin-1-yl]-xanthin (im Gemisch mit 1-[(3-[(2-Trimethylsilanyl-ethoxy)methyl]-3*H*-benzoimidazol-5-yl)methyl]-3-methyl-7-(2-butin-1-yl)-8-[3-(tert.-butyloxycarbonylamino)-piperidin-1-yl]-xanthin)
   R_{f}-Wert: 0.23 (Kieselgel, Essigester)
   Massenspektrum (ESI⁺): m/z = 677 [M+H]⁺
(63) 1-[(Pyrazolo[1,5-a]pyridin-2-yl)methyl]-3-methyl-7-(2-butin-1-yl)-8-[3-(tert.-butyl-oxycarbonylamino)-piperidin-1-yl]-xanthin
   R_{f}-Wert: 0.46 (Kieselgel, Methylenchlorid/Methanol = 95:5)
   Massenspektrum (ESI⁺): m/z = 547 [M+H]⁺
(64) 1-(2-Phenyl-2-oxo-ethyl)-3-methyl-7-((E)-1-buten-1-yl)-8-[3-(tert.-butyloxycarbonylamino)-piperidin-1-yl]-xanthin
   R_{f}-Wert: 0.48 (Kieselgel, Methylenchlorid/Methanol = 95:5)
   Massenspektrum (ESI⁺): m/z = 537 [M+H]⁺
(65) 1-{2-[1-(tert.-Butyloxycarbonyl)-1*H*-indol-7-yl]-2-oxo-ethyl}-3-methyl-7-(2-butin-1-yl)-8-[3-(tert.-butyloxycarbonylamino)-piperidin-1-yl]-xanthin
   R_{f}-Wert: 0.38 (Kieselgel, Petrolether/Essigester = 1:1)
(66) 1-[(3-Methyl-isochinolin-1-yl)methyl]-3-methyl-7-(3-methyl-1-buten-1-yl)-8-[(*R*)-3-(tert.-butyloxycarbonylamino)-piperidin-1-yl]-xanthin
   R_{f}-Wert: 0.40 (Kieselgel, Methylenchlorid/Methanol = 95:5)
   Massenspektrum (ESI⁺): m/z = 588 [M+H]⁺
(67) 1,3-Dimethyl-7-(2-brom-benzyl)-8-[3-(tert.-butyloxycarbonylamino)-piperidin-1-yl]-xanthin
   R_{f}-Wert: 0.40 (Kieselgel, Cyclohexan/Essigester = 1:1)
(68) 1,3-Dimethyl-7-(2-chlor-benzyl)-8-[3-(tert.-butyloxycarbonylamino)-piperidin-1-yl]-xanthin
   R_{f}-Wert: 0.42 (Kieselgel, Cyclohexan/Essigester = 1:1)
(69) 1-[(3-Methyl-isochinolin-1-yl)methyl]-3-methyl-7-(2-cyano-benzyl)-8-[3-(tert.-butyloxycarbonylamino)-piperidin-1-yl]-xanthin
   Massenspektrum (ESI⁺): m/z = 635 [M+H]⁺
   R_{f}-Wert: 0.40 (Kieselgel, Cyclohexan/Essigester = 3:7)
(70) 3-Cyclopropyl-7-(2-butin-1-yl)-8-[3-(tert.-butyloxycarbonylamino)-piperidin-1-yl]-xanthin
   Massenspektrum (ESI⁺): m/z = 443 [M+H]⁺
   R_{f}-Wert: 0.70 (Kieselgel, Essigester)
(71) 3-Cyclopropyl-7-(2-butin-1-yl)-8-[(R)-3-(tert.-butyloxycarbonylamino)-piperidin-1-yl]-xanthin
   R_{f}-Wert: 0.35 (Reversed Phase DC-Fertigplatte (E. Merck), Acetonitril/Wasser/ Trifluoressigsäure = 50:50:1)
   Massenspektrum (ESI⁺): m/z = 443 [M+H]⁺
(72) 1-[(3-Methyl-isochinolin-1-yl)methyl]-3-methyl-7-(2-chlor-benzyl)-8-[(R)-3-(tert.-butyloxycarbonylamino)-piperidin-1-yl]-xanthin
   Massenspektrum (ESI⁺): m/z = 644, 646 [M+H]⁺
   R_{f}-Wert: 0.39 (Kieselgel, Cyclohexan/Essigester = 1:1)
(73) 1-[(3-Methyl-isochinolin-1-yl)methyl]-3-methyl-7-(2-brom-benzyl)-8-[(R)-3-(tert.-butyloxycarbonylamino)-piperidin-1-yl]-xanthin
   Massenspektrum (ESI⁺): m/z = 644, 646 [M+H]⁺
(74) 1-[(4-Methyl-chinazolin-2-yl)methyl]-3-methyl-7-(2-chlor-benzyl)-8-[(R)-3-(tert.-butyloxycarbonylamino)-piperidin-1-yl]-xanthin
   Hergestellt durch Umsetzung von (4-Methyl-chinazolin-2-yl)-methylchlorid und 3-Methyl-7-(2-chlor-benzyl)-8-brom-xanthin und nachfolgender Umsetzung mit (R)-3-(tert.-Butyloxycarbonylamino)-piperidin
   Massenspektrum (ESI⁺): m/z = 645, 647 [M+H]⁺
(75) 1-[(4-Phenyl-chinazolin-2-yl)methyl]-3-methyl-7-(2-chlor-benzyl)-8-[(R)-3-(tert.-butyloxycarbonylamino)-piperidin-1-yl]-xanthin
   Hergestellt durch Umsetzung von (4-Phenyl-chinazolin-2-yl)-methylchlorid und 3-Methyl-7-(2-chlor-benzyl)-8-brom-xanthin und nachfolgender Umsetzung mit (R)-3-(tert.-Butyloxycarbonylamino)-piperidin
   Massenspektrum (ESI⁺): m/z = 707, 709 [M+H]⁺

### Beispiel IV

### 1-[2-(2-Hydroxy-phenyl)-2-oxo-ethyl]-3-methyl-7-(3-methyl-2-buten-1-yl)-8-chlorxanthin

hergestellt durch Behandeln von 1-[2-(2-Methoxy-phenyl)-2-oxo-ethyl]-3-methyl-7-(3-methyl-2-buten-1-yl)-8-chlor-xanthin mit Bortribromid in Methylenchlorid. Das gewünschte Produkt ist mit ca. 20 % 1-[2-(2-Hydroxy-phenyl)-2-oxo-ethyl]-3-methyl-7-(3-brom-3-methyl-butyl)-8-chlor-xanthin verunreinigt.
Massenspektrum (ESI⁺): m/z = 403, 405 [M+H]⁺

### Analog Beispiel IV werden folgende Verbindungen erhalten:

(1) 1-[2-(2-Hydroxy-phenyl)-2-oxo-ethyl]-3-methyl-7-(2-butin-1-yl)-8-brom-xanthin (Produkt ist mit ca. 20 % 1-[2-(2-Hydroxy-phenyl)-2-oxo-ethyl]-3-methyl-7-(3-brom-2-buten-1-yl)-8-brom-xanthin verunreinigt)
   Massenspektrum (ESI⁺): m/z = 431, 433 [M+H]⁺
(2) 1-[2-(2-Hydroxy-phenyl)-2-oxo-ethyl]-3-methyl-7-[(1-cyclopenten-1-yl)methyl]-8-brom-xanthin
   Massenspektrum (ESI⁺): m/z = 459, 461 [M+H]⁺
(3) 1-[2-(2-Hydroxy-phenyl)-2-oxo-ethyl]-3-methyl-7-((E)-2-buten-1-yl)-8-brom-xanthin (Produkt enthält etwas Z-Isomer)
   R_{f}-Wert: 0.60 (Kieselgel, Cyclohexan/Essigester = 4:6)
   Massenspektrum (ESI⁺): m/z = 433, 435 [M+H]⁺
(4) 1-[2-(2-Hydroxy-phenyl)-2-oxo-ethyl]-3-methyl-7-(3-methyl-2-buten-1-yl)-8-bromxanthin
   Massenspektrum (ESI⁺): m/z = 447, 449 [M+H]⁺

### Beispiel V

### 1-[2-(2-Methoxy-phenyl)-2-oxo-ethyl]-3-methyl-7-(3-methyl-2-buten-1-yl)-8-chlorxanthin

Zu einem Gemisch aus 2.00 g 3-Methyl-7-(3-methyl-2-buten-1-yl)-8-chlor-xanthin und 1.38 mg Kaliumcarbonat in 15 ml N,N-Dimethylformamid werden 1.71 g 2-Brom-1-(2-methoxy-phenyl)-ethanon gegeben. Das Reaktionsgemisch wird acht Stunden bei Raumtemperatur gerührt. Nach wässriger Aufarbeitung wird das Rohprodukt chromatographisch über eine Kieselgel-Säule mit Cyclohexan/Essigester (8:1 auf 8:1) als Laufmittel gereinigt.
Ausbeute: 2.61 g (84 % der Theorie)
Massenspektrum (ESI⁺): m/z = 417, 419 [M+H]⁺

### Analog Beispiel V werden folgende Verbindungen erhalten:

(1) 1-[2-(3-Hydroxy-phenyl)-2-oxo-ethyl]-3-methyl-7-(3-methyl-2-buten-1-yl)-8-[3-(tert.-butyloxycarbonylamino)-piperidin-1-yl]-xanthin
   (Die Umsetzung erfolgt mit 2-Brom-1-[3-(tert.-butyldimethylsilanyloxy)-phenyl]-ethanon)
   R_{f}-Wert: 0.40 (Kieselgel, Cyclohexan/Essigester = 3:7)
   Massenspektrum (ESI⁺): m/z = 567 [M+H]⁺
(2) 1-(1-Methyl-2-oxo-2-phenyl-ethyl)-3-methyl-7-(3-methyl-2-buten-1-yl)-8-chlor-xanthin
   Massenspektrum (ESI⁺): m/z = 401, 403 [M+H]⁺
(3) 1-(2-Cyano-ethyl)-3-methyl-7-(2-cyano-benzyl)-8-chlor-xanthin
   Massenspektrum (ESI⁺): m/z = 391, 393 [M+Na]⁺
(4) 1-(2-Phenoxy-ethyl)-3-methyl-7-(2-cyano-benzyl)-8-[3-(tert.-butyloxycarbonyl-amino)-piperidin-1-yl]-xanthin
   R_{f}-Wert: 0.90 (Kieselgel, Methylenchlorid/Methanol = 9:1)
   Massenspektrum (ESI⁺): m/z = 600 [M+H]⁺
(5) 1-(2-Phenyl-2-oxo-ethyl)-3-[(2-trimethylsilanyl-ethoxy)methyl]-7-(3-methyl-2-buten-1-yl)-8-[3-(tert.-butyloxycarbonylamino)-piperidin-1-yl]-xanthin
   Massenspektrum (ESI⁺): m/z = 667 [M+H]⁺
(6) 1-(2-Methoxy-ethyl)-3-methyl-7-(2-cyano-benzyl)-8-[3-(tert.-butyloxycarbonyl-amino)-piperidin-1-yl]-xanthin
   R_{f}-Wert: 0.90 (Kieselgel, Methylenchlorid/Methanol = 9:1)
   Massenspektrum (ESI⁺): m/z = 538 [M+H]⁺
(7) 1-Methyl-3-[(2-trimethylsilanyl-ethoxy)methyl]-7-(2-cyano-benzyl)-xanthin R_{f}-Wert: 0.60 (Kieselgel, Cyclohexan/Essigester = 1:1)
   Massenspektrum (ESI⁺): m/z = 412 [M+H]⁺
(8) 1-[2-(3-Nitro-phenyl)-2-oxo-ethyl]-3-methyl-7-(3-methyl-2-buten-1-yl)-8-chlor-xanthin
   R_{f}-Wert: 0.40 (Kieselgel, Cyclohexan/Essigester/Methanol = 7:2:1)
   Massenspektrum (ESI⁺): m/z = 432, 434 [M+H]⁺
(9) 1-(2-Phenyl-2-oxo-ethyl)-3-methyl-7-[(2-trimethylsilanyl-ethoxy)methyl]-8-brom-xanthin
   Massenspektrum (ESI⁺): m/z = 493, 495 [M+H]⁺
(10) 1-[2-(2-Nitro-phenyl)-2-oxo-ethyl]-3-methyl-7-(3-methyl-2-buten-1-yl)-8-chlor-xanthin
   R_{f}-Wert: 0.64 (Kieselgel, Cyclohexan/Essigester = 3:7)
   Massenspektrum (ESI⁺): m/z = 432, 434 [M+H]⁺
(11) 1-[2-(2-Nitro-phenyl)-2-oxo-ethyl]-3-methyl-7-(3-methyl-2-buten-1-yl)-8-brom-xanthin
   Massenspektrum (ESI⁺): m/z = 476, 478 [M+H]⁺
(12) 1-[(Chinolin-2-yl)methyl]-3-methyl-7-(3-methyl-2-buten-1-yl)-8-[3-(tert.-butyloxy-carbonylamino)-piperidin-1-yl]-xanthin
   R_{f}-Wert: 0.45 (Kieselgel, Essigester/Petrolether = 7:3)
   Massenspektrum (ESI⁺): m/z = 574 [M+H]⁺
(13) 1-[(2-Oxo-2*H*-chromen-4-yl)methyl]-3-methyl-7-(3-methyl-2-buten-1-yl)-8-[3-(tert.-butyloxycarbonylamino)-piperidin-1-yl]-xanthin
   (Das Ausgangsmaterial 4-Brommethyl-chromen-2-on wird analog Kimura et al., Chem. Pharm. Bull. 1982, 30, 552-558 hergestellt.)
   R_{f}-Wert: 0.52 (Kieselgel, Methylenchlorid/Methanol = 95:5)
   Massenspektrum (ESI⁺): m/z = 591 [M+H]⁺
(14) 1-[(1-Methyl-2-oxo-1,2-dihydro-chinolin-4-yl)methyl]-3-methyl-7-(3-methyl-2-buten-1-yl)-8-[3-(tert.-butyloxycarbonylamino)-piperidin-1-yl]-xanthin
   R_{f}-Wert: 0.54 (Kieselgel, Methylenchlorid/Methanol = 95:5)
   Massenspektrum (ESI⁺): m/z = 604 [M+H]⁺
(15) 1-[(Chinazolin-4-yl)methyl]-3-methyl-7-(3-methyl-2-buten-1-yl)-8-[3-(tert.-butyloxycarbonylamino)-piperidin-1-yl]-xanthin
   Schmelzpunkt: 195-197°C
   Massenspektrum (ESI⁺): m/z = 575 [M+H]⁺
(16) 1-[(5-Methyl-3-phenyl-isoxazol-4-yl)methyl]-3-methyl-7-(3-methyl-2-buten-1-yl)-8-[3-(tert.-butyloxycarbonylamino)-piperidin-1-yl]-xanthin
   R_{f}-Wert: 0.40 (Kieselgel, Cyclohexan/Essigester = 1:1)
   Massenspektrum (ESI⁺): m/z = 604 [M+H]⁺
(17) 1-[(3-Phenyl-[1,2,4]oxadiazol-5-yl)methyl]-3-methyl-7-(3-methyl-2-buten-1-yl)-8-[3-(tert.-butyloxycarbonylamino)-piperidin-1-yl]-xanthin
   R_{f}-Wert: 0.18 (Kieselgel, Petrolether/Essigester = 2:1)
   Massenspektrum (ESI⁺): m/z = 591 [M+H]⁺
(18) 1-[(4-Phenyl-pyridin-2-yl)methyl]-3-methyl-7-(3-methyl-2-buten-1-yl)-8-[3-(tert.-butyloxycarbonylamino)-piperidin-1-yl]-xanthin
   R_{f}-Wert: 0.53 (Kieselgel, Methylenchlorid/Methanol = 95:5)
   Massenspektrum (ESI⁺): m/z = 600 [M+H]⁺
(19) 1-[(5-Phenyl-pyridin-2-yl)methyl]-3-methyl-7-(3-methyl-2-buten-1-yl)-8-[3-(tert.-butyloxycarbonylamino)-piperidin-1-yl]-xanthin
   R_{f}-Wert: 0.73 (Kieselgel, Essigester)
   Massenspektrum (ESI⁺): m/z = 600 [M+H]⁺
(20) 1-[2-(3-Methylsulfanyl-phenyl)-2-oxo-ethyl]-3-methyl-7-(3-methyl-2-buten-1-yl)-8-[3-(tert.-butyloxycarbonylamino)-piperidin-1-yl]-xanthin
   R_{f}-Wert: 0.43 (Kieselgel, Cyclohexan/Essigester = 1:1)
   Massenspektrum (ESI⁺): m/z = 597 [M+H]⁺
(21) 1-[2-(3-Methoxycarbonyl-phenyl)-2-oxo-ethyl]-3-methyl-7-(3-methyl-2-buten-1-yl)-8-[3-(tert.-butyloxycarbonylamino)-piperidin-1-yl]-xanthin
   (Durchführung in N-Methylpyrrolidin-2-on bei 60°C)
   R_{f}-Wert: 0.27 (Kieselgel, Methylenchlorid/Methanol = 20:1)
   Massenspektrum (ESI⁺): m/z = 609 [M+H]⁺
(22) 1-[2-(2-Ethoxycarbonyl-phenyl)-2-oxo-ethyl]-3-methyl-7-(3-methyl-2-buten-1-yl)-8-[3-(tert.-butyloxycarbonylamino)-piperidin-1-yl]-xanthin
   (Durchführung in N-Methylpyrrolidin-2-on bei 60°C)
   R_{f}-Wert: 0.35 (Kieselgel, Methylenchlorid/Methanol = 20:1)
   Massenspektrum (ESI⁺): m/z = 623 [M+H]⁺
(23) 1-[2-(2,6-Dimethoxy-phenyl)-2-oxo-ethyl]-3-methyl-7-(3-methyl-2-buten-1-yl)-8-[3-(tert.-butyloxycarbonylamino)-piperidin-1-yl]-xanthin
   (Durchführung in N-Methylpyrrolidin-2-on bei 60°C)
   R_{f}-Wert: 0.53 (Kieselgel, Methylenchlorid/Methanol = 20:1)
   Massenspektrum (ESI⁺): m/z = 611 [M+H]⁺
(24) 1-(2-Phenyl-2-oxo-ethyl)-3-methyl-7-(2,3-dimethyl-2-buten-1-yl)-8-[3-(tert.-butyloxycarbonylamino)-piperidin-1-yl]-xanthin
   (Durchführung in N-Methylpyrrolidin-2-on bei 60°C)
   R_{f}-Wert: 0.38 (Kieselgel, Methylenchlorid/Methanol = 20:1)
   Massenspektrum (ESI⁺): m/z = 565 [M+H]⁺
(25) 1-((E)-3-Phenyl-allyl)-3-methyl-7-(3-methyl-2-buten-1-yl)-8-[3-(tert.-butyloxycarbonylamino)-piperidin-1-yl]-xanthin
   R_{f}-Wert: 0.54 (Kieselgel, Methylenchlorid/Methanol/konz. wässriges Ammoniak = 95:5:0.1)
   Massenspektrum (ESI⁺): m/z = 549 [M+H]⁺
(26) 1-[(1-Benzo[b]thiophen-3-yl)methyl]-3-methyl-7-(3-methyl-2-buten-1-yl)-8-[3-(tert.-butyloxycarbonylamino)-piperidin-1-yl]-xanthin
   R_{f}-Wert: 0.75 (Kieselgel, Methylenchlorid/Methanol = 95:5)
   Massenspektrum (ESI⁺): m/z = 579 [M+H]⁺
(27) 1-{[1-(tert.-Butyloxycarbonyl)-indol-3-yl]methyl}-3-methyl-7-(3-methyl-2-buten-1-yl)-8-[3-(tert.-butyloxycarbonylamino)-piperidin-1-yl]-xanthin
   R_{f}-Wert: 0.61 (Kieselgel, Methylenchlorid/Methanol = 9:1)
   Massenspektrum (ESI⁺): m/z = 662 [M+H]⁺
(28) 1-[(Biphenyl-4-yl)methyl]-3-methyl-7-(3-methyl-2-buten-1-yl)-8-[3-(tert.-butyloxycarbonylamino)-piperidin-1-yl]-xanthin
   R_{f}-Wert: 0.68 (Kieselgel, Cyclohexan/Essigester = 1:1)
   Massenspektrum (ESI⁺): m/z = 599 [M+H]⁺
(29) 1-[(1-Naphthyl)methyl]-3-methyl-7-(2-butin-1-yl)-8-[3-(tert.-butyloxycarbonyl-amino)-piperidin-1-yl]-xanthin
   R_{f}-Wert: 0.83 (Kieselgel, Essigester/Petrolether = 4:1)
   Massenspektrum (ESI⁺): m/z = 557 [M+H]⁺
(30) 1-[(1-Methyl-2-oxo-1,2-dihydro-chinolin-4-yl)methyl]-3-methyl-7-(2-butin-1-yl)-8-[3-(tert.-butyloxycarbonylamino)-piperidin-1-yl]-xanthin
   R_{f}-Wert: 0.25 (Kieselgel, Essigester/Petrolether = 4:1)
   Massenspektrum (ESI⁺): m/z = 588 [M+H]⁺
(31) 1-(2-Cyclohexyl-2-oxo-ethyl)-3-methyl-7-(3-methyl-2-buten-1-yl)-8-[3-(tert.-butyloxycarbonylamino)-piperidin-1-yl]-xanthin
   Schmelzpunkt: 163-165°C
   Massenspektrum (ESI⁺): m/z = 557 [M+H]⁺
(32) 1-(3,3-Dimethyl-2-oxo-butyl)-3-methyl-7-(3-methyl-2-buten-1-yl)-8-[3-(tert.-butyloxycarbonylamino)-piperidin-1-yl]-xanthin
   R_{f}-Wert: 0.95 (Kieselgel, Essigester/Petrolether = 4:1)
   Massenspektrum (ESI⁺): m/z = 531 [M+H]⁺
(33) 1-[(Chinazolin-2-yl)methyl]-3-methyl-7-(2-butin-1-yl)-8-[3-(tert.-butyloxycarbonyl-amino)-piperidin-1-yl]-xanthin
   R_{f}-Wert: 0.40 (Kieselgel, Essigester)
   Massenspektrum (ESI⁺): m/z = 559 [M+H]⁺
(34) 1-[(2-Methyl-naphthalin-1-yl)methyl]-3-methyl-7-(2-butin-1-yl)-8-[3-(tert.-butyloxycarbonylamino)-piperidin-1-yl]-xanthin
   R_{f}-Wert: 0.80 (Kieselgel, Essigester)
   Massenspektrum (ESI⁺): m/z = 571 [M+H]⁺
(35) 1-[(5-Nitro-isochinolin-1-yl)methyl]-3-methyl-7-(3-methyl-2-buten-1-yl)-8-[3-(tert.-butyloxycarbonylamino)-piperidin-1-yl]-xanthin
   R_{f}-Wert: 0.54 (Kieselgel, Methylenchlorid/Methanol = 95:5)
(36) 1-(2-Dimethylamino-2-oxo-ethyl)-3-methyl-7-(3-methyl-2-buten-1-yl)-8-[3-(tert.-butyloxycarbonylamino)-piperidin-1-yl]-xanthin
   R_{f}-Wert: 0.23 (Kieselgel, Essigester)
   Massenspektrum (ESI⁺): m/z = 518 [M+H]⁺
(37) 1-[2-(Piperidin-1-yl)-2-oxo-ethyl]-3-methyl-7-(3-methyl-2-buten-1-yl)-8-[3-(tert.-butyloxycarbonylamino)-piperidin-1-yl]-xanthin
   R_{f}-Wert: 0.44 (Kieselgel, Essigester)
   Massenspektrum (ESI⁺): m/z = 558 [M+H]⁺
(38) 1-[(2-Methyl-1-oxo-1,2-dihydro-isochinolin-4-yl)methyl]-7-(2-butin-1-yl)-8-[3-(tert.-butyloxycarbonylamino)-piperidin-1-yl]-xanthin
   R_{f}-Wert: 0.25 (Kieselgel, Essigester)
   Massenspektrum (ESI⁺): m/z = 588 [M+H]⁺
(39) 1-[(2-Methyl-1-oxo-1,2-dihydro-isochinolin-4-yl)methyl]-7-(3-methyl-2-buten-1-yl)-8-[3-(tert.-butyloxycarbonylamino)-piperidin-1-yl]-xanthin
   R_{f}-Wert: 0.30 (Kieselgel, Essigester)
   Massenspektrum (ESI⁺): m/z = 604 [M+H]⁺
(40) 1-[(2-Methoxy-naphthalin-1-yl)methyl]-3-methyl-7-(2-butin-1-yl)-8-[3-(tert.-butyloxycarbonylamino)-piperidin-1-yl]-xanthin
   R_{f}-Wert: 0.75 (Kieselgel, Essigester)
   Massenspektrum (ESI⁺): m/z = 587 [M+H]⁺
(41) 1-[(4-Methoxy-naphthalin-1-yl)methyl]-3-methyl-7-(2-butin-1-yl)-8-[3-(tert.-butyloxycarbonylamino)-piperidin-1-yl]-xanthin
   R_{f}-Wert: 0.80 (Kieselgel, Essigester)
   Massenspektrum (ESI⁺): m/z = 587 [M+H]⁺
(42) 1-[(3-Methyl-isochinolin-1-yl)methyl]-3-methyl-7-(2-butin-1-yl)-8-[3-(tert.-butyloxycarbonylamino)-piperidin-1-yl]-xanthin
   R_{f}-Wert: 0.56 (Kieselgel, Methylenchlorid/Methanol = 95:5)
   Massenspektrum (ESI⁺): m/z = 572 [M+H]⁺
(43) 1-[2-(2,3-Dimethoxy-phenyl)-2-oxo-ethyl]-3-methyl-7-(3-methyl-2-buten-1-yl)-8-[3-(tert.-butyloxycarbonylamino)-piperidin-1-yl]-xanthin
   R_{f}-Wert: 0.83 (Kieselgel, Essigester)
   Massenspektrum (ESI⁺): m/z = 611 [M+H]⁺
(44) 1-[(5-Nitro-naphthalin-1-yl)methyl]-3-methyl-7-(2-butin-1-yl)-8-[3-(tert.-butyloxy-carbonylamino)-piperidin-1-yl]-xanthin
   R_{f}-Wert: 0.78 (Kieselgel, Essigester)
   Massenspektrum (ESI⁺): m/z = 602 [M+H]⁺
(45) 1-[2-(Pyrrolidin-1-yl)-2-oxo-ethyl]-3-methyl-7-(3-methyl-2-buten-1-yl)-8-[3-(tert.-butyloxycarbonylamino)-piperidin-1-yl]-xanthin
   R_{f}-Wert: 0.39 (Kieselgel, Essigester)
   Massenspektrum (ESI⁺): m/z = 544 [M+H]⁺
(46) 1-[(4-Methyl-isochinolin-1-yl)methyl]-3-methyl-7-(2-butin-1-yl)-8-[3-(tert.-butyloxycarbonylamino)-piperidin-1-yl]-xanthin
   R_{f}-Wert: 0.56 (Kieselgel, Methylenchlorid/Methanol = 95:5)
   Massenspektrum (ESI⁺): m/z = 572 [M+H]⁺
(47) 1-[(2-Naphthyl)methyl]-3-methyl-7-(2-butin-1-yl)-8-[3-(tert.-butyloxycarbonylamino)-piperidin-1-yl]-xanthin
   R_{f}-Wert: 0.78 (Kieselgel, Essigester)
   Massenspektrum (ESI⁺): m/z = 557 [M+H]⁺
(48) 1-Cyanomethyl-3-methyl-7-(2-butin-1-yl)-8-[3-(tert.-butyloxycarbonylamino)-piperidin-1-yl]-xanthin
   R_{f}-Wert: 0.80 (Kieselgel, Essigester)
   Massenspektrum (ESI⁺): m/z = 456 [M+H]⁺
(49) 1-[(Chinolin-6-yl)methyl]-3-methyl-7-(2-butin-1-yl)-8-[3-(tert.-butyloxycarbonyl-amino)-piperidin-1-yl]-xanthin
   R_{f}-Wert: 0.40 (Kieselgel, Essigester)
   Massenspektrum (ESI⁺): m/z = 558 [M+H]⁺
(50) 1-[(3-Methoxy-naphthalin-2-yl)methyl]-3-methyl-7-(2-butin-1-yl)-8-[3-(tert.-butyloxycarbonylamino)-piperidin-1-yl]-xanthin
   R_{f}-Wert: 0.83 (Kieselgel, Essigester)
   Massenspektrum (ESI⁺): m/z = 587 [M+H]⁺
(51) 1-[2-(2,3-Dihydro-benzo[1,4]dioxin-5-yl)-2-oxo-ethyl]-3-methyl-7-(3-methyl-2-buten-1-yl)-8-[3-(tert.-butyloxycarbonylamino)-piperidin-1-yl]-xanthin
   R_{f}-Wert: 0.38 (Kieselgel, Essigester/Petrolether = 1:1)
   Massenspektrum (ESI⁺): m/z = 609 [M+H]⁺
(52) 1-[2-(3-Methyl-2-oxo-2,3-dihydro-benzooxazol-7-yl)-2-oxo-ethyl]-3-methyl-7-(3-methyl-2-buten-1-yl)-8-[3-(tert.-butyloxycarbonylamino)-piperidin-1-yl]-xanthin
   (Durchführung mit Kalium-tert.-butylat in Dimethylsulfoxid)
   R_{f}-Wert: 0.48 (Kieselgel, Essigester/Petrolether = 2:1)
   Massenspektrum (ESI⁺): m/z = 622 [M+H]⁺
(53) 1-[2-(Benzo[1,3]dioxol-4-yl)-2-oxo-ethyl]-3-methyl-7-(3-methyl-2-buten-1-yl)-8-[3-(tert.-butyloxycarbonylamino)-piperidin-1-yl]-xanthin
   Massenspektrum (ESI⁺): m/z = 595 [M+H]⁺
(54) 1-[(Chinazolin-2-yl)methyl]-3-methyl-7-(2-butin-1-yl)-8-[(*R*)-3-(tert.-butyloxy-carbonylamino)-piperidin-1-yl]-xanthin
   Massenspektrum (ESI⁺): m/z = 559 [M+H]⁺
(55) 1-[(Chinazolin-2-yl)methyl]-3-methyl-7-(2-butin-1-yl)-8-[(*S*)-3-(tert.-butyloxy-carbonylamino)-piperidin-1-yl]-xanthin
   Massenspektrum (ESI⁺): m/z = 559 [M+H]⁺
(56) 1-[(Chinazolin-2-yl)methyl]-3-methyl-7-((E)-2-buten-1-yl)-8-[(*S*)-3-(tert.-butyloxy-carbonylamino)-piperidin-1-yl]-xanthin
   (Produkt enthält ca. 15 % Z-Isomer)
   R_{f}-Wert: 0.30 (Kieselgel, Essigester/Cyclohexan = 8:2)
   Massenspektrum (ESI⁺): m/z = 561 [M+H]⁺
(57) 1-[(Chinazolin-2-yl)methyl]-3-methyl-7-((E)-2-buten-1-yl)-8-[(*R*)-3-(tert.-butyloxy-carbonylamino)-piperidin-1-yl]-xanthin
   (Produkt enthält ca. 15 % Z-Isomer)
   R_{f}-Wert: 0.30 (Kieselgel, Essigester/Cyclohexan = 8:2)
   Massenspektrum (ESI⁺): m/z = 561 [M+H]⁺
(58) 1-[(3-Methyl-isochinolin-1-yl)methyl]-3-methyl-7-((E)-2-buten-1-yl)-8-[(*R*)-3-(tert.-butyloxycarbonylamino)-piperidin-1-yl]-xanthin
   (Produkt enthält ca. 17 % Z-Isomer)
   R_{f}-Wert: 0.58 (Kieselgel, Methylenchlorid/Methanol = 95:5)
   Massenspektrum (ESI⁺): m/z = 574 [M+H]⁺
(59) 1-[(3-Methyl-isochinolin-1-yl)methyl]-3-methyl-7-((E)-2-buten-1-yl)-8-[(*S*)-3-(tert.-butyloxycarbonylamino)-piperidin-1-yl]-xanthin
   (Produkt enthält ca. 17 % Z-Isomer)
   R_{f}-Wert: 0.58 (Kieselgel, Methylenchlorid/Methanol = 95:5)
   Massenspektrum (ESI⁺): m/z = 574 [M+H]⁺
(60) 1-[2-(2-Methoxy-phenyl)-2-oxo-ethyl]-3-methyl-7-(2-butin-1-yl)-8-brom-xanthin Massenspektrum (ESI⁺): m/z = 445, 447 [M+H]⁺
(61) 1-[2-(2-Nitro-phenyl)-2-oxo-ethyl]-3-methyl-7-[(1-cyclopenten-1-yl)methyl]-8-brom-xanthin
   Massenspektrum (ESI⁺): m/z = 488, 490 [M+H]⁺
(62) 1-[2-(2-Methoxy-phenyl)-2-oxo-ethyl]-3-methyl-7-[(1-cyclopenten-1-yl)methyl]-8-brom-xanthin
   Massenspektrum (ESI⁺): m/z = 473, 475 [M+H]⁺
(63) 1-[(Isochinolin-1-yl)methyl]-3-[(methoxycarbonyl)methyl]-7-(3-methyl-2-buten-1-yl)-8-[3-(tert.-butyloxycarbonylamino)-piperidin-1-yl]-xanthin
   R_{f}-Wert: 0.35 (Kieselgel, Methylenchlorid/Methanol = 95:5)
(64) 1-[2-(2-Nitro-phenyl)-2-oxo-ethyl]-3-methyl-7-((E)-2-buten-1-yl)-8-brom-xanthin (Produkt enthält ca. 10 % Z-Isomer)
   R_{f}-Wert: 0.60 (Kieselgel, Cyclohexan/Essigester = 4:6)
   Massenspektrum (ESI⁺): m/z = 462, 464 [M+H]⁺
(65) 1-[2-(2-Methoxy-phenyl)-2-oxo-ethyl]-3-methyl-7-((E)-2-buten-1-yl)-8-brom-xanthin
   (Produkt enthält etwas Z-Isomer)
   R_{f}-Wert: 0.30 (Kieselgel, Cyclohexan/Essigester = 1:1)
   Massenspektrum (ESI⁺): m/z = 447, 449 [M+H]⁺
(66) 1-[2-(2-Nitro-phenyl)-2-oxo-ethyl]-3-methyl-7-(2-butin-1-yl)-8-brom-xanthin
   R_{f}-Wert: 0.77 (Kieselgel, Methylenchlorid/Methanol = 95:5)
   Massenspektrum (ESI⁺): m/z = 460, 462 [M+H]⁺
(67) 1-(2-Phenyl-2-oxo-ethyl)-3-methyl-7-((E)-2-buten-1-yl)-8-[(*R*)-3-(tert.-butyloxy-carbonylamino)-piperidin-1-yl]-xanthin
   (Produkt enthält ca. 20 % Z-Isomer)
   Massenspektrum (ESI⁺): m/z = 537 [M+H]⁺
(68) 1-[2-(2-Methoxy-phenyl)-2-oxo-ethyl]-3-methyl-7-(3-methyl-2-buten-1-yl)-8-brom-xanthin
   Massenspektrum (ESI⁺): m/z = 461, 463 [M+H]⁺
(69) 1-(2-Phenyl-2-oxo-ethyl)-3-methyl-7-(2-butin-1-yl)-8-brom-xanthin
   R_{f}-Wert: 0.61 (Kieselgel, Cyclohexan/Essigester = 4:6)
(70) 1-(2-{2-[(Ethylaminocarbonyl)methoxy]-phenyl}-2-oxo-ethyl)-3-methyl-7-((E)-2-buten-1-yl)-8-[(*R*)-3-(tert.-butyloxycarbonylamino)-piperidin-1-yl]-xanthin
   (Produkt enthält ca. 17 % Z-Isomer)
   Massenspektrum (ESI⁺): m/z = 638 [M+H]⁺
(71) 1-(2-Phenyl-2-oxo-ethyl)-3-methyl-7-((E)-2-buten-1-yl)-8-[(*S*)-3-(tert.-butyloxy-carbonylamino)-piperidin-1-yl]-xanthin
   (Produkt enthält ca. 18 % Z-Isomer)
   R_{f}-Wert: 0.35 (Kieselgel, Cyclohexan/Essigester = 6:4)
   Massenspektrum (ESI⁺): m/z = 537 [M+H]⁺
(72) 1-[2-(2-Nitro-phenyl)-2-oxo-ethyl]-3-methyl-7-(2-butin-1-yl)-8-[(*S*)-3-(tert.-butyloxycarbonylamino)-piperidin-1-yl]-xanthin
   R_{f}-Wert: 0.60 (Kieselgel, Methylenchlorid/Methanol = 95:5)
   Massenspektrum (ESI⁺): m/z = 580 [M+H]⁺
(73) 1-[(3-Methyl-isochinolin-1-yl)methyl]-3-methyl-7-(2-butin-1-yl)-8-[(S)-3-(tert.-butyloxycarbonylamino)-piperidin-1-yl]-xanthin
   R_{f}-Wert: 0.52 (Kieselgel, Methylenchlorid/Methanol = 95:5)
   Massenspektrum (ESI⁺): m/z = 572 [M+H]⁺
(74) 1-[(3-Methyl-isochinolin-1-yl)methyl]-3-methyl-7-(2-butin-1-yl)-8-[(R)-3-(tert.-butyloxycarbonylamino)-piperidin-1-yl]-xanthin
   Massenspektrum (ESI⁺): m/z = 572 [M+H]⁺
(75) 1-[(4-Methyl-isochinolin-1-yl)methyl]-3-methyl-7-(2-butin-1-yl)-8-[(S)-3-(tert.-butyloxycarbonylamino)-piperidin-1-yl]-xanthin
   Massenspektrum (ESI⁺): m/z = 572 [M+H]⁺
(76) 1-[(4-Methyl-isochinolin-1-yl)methyl]-3-methyl-7-(2-butin-1-yl)-8-[(R)-3-(tert.-butyloxycarbonylamino)-piperidin-1-yl]-xanthin
   Massenspektrum (ESI⁺): m/z = 572 [M+H]⁺
(77) 1-[(4-Methyl-isochinolin-1-yl)methyl]-3-methyl-7-((E)-2-buten-1-yl)-8-[(S)-3-(tert.-butyloxycarbonylamino)-piperidin-1-yl]-xanthin
   R_{f}-Wert: 0.52 (Kieselgel, Methylenchlorid/Methanol = 95:5)
   Massenspektrum (ESI⁺): m/z = 574 [M+H]⁺
(78) 1-[(4-Methyl-isochinolin-1-yl)methyl]-3-methyl-7-((E)-2-buten-1-yl)-8-[(*R*)-3-(tert.-butyloxycarbonylamino)-piperidin-1-yl]-xanthin
   R_{f}-Wert: 0.52 (Kieselgel, Methylenchlorid/Methanol = 95:5)
   Massenspektrum (ESI⁺): m/z = 574 [M+H]⁺
(79) 1-[2-(2,3-Dihydro-benzo[1,4]dioxin-5-yl)-2-oxo-ethyl]-3-methyl-7-(2-butin-1-y1)-8-[(*R*)-3-(tert.-butyloxycarbonylamino)-piperidin-1-yl]-xanthin
   R_{f}-Wert: 0.18 (Kieselgel, Essigester/Petrolether = 1:1)
   Massenspektrum (ESI⁺): m/z = 593 [M+H]⁺
(80) 1-[2-(2,3-Dihydro-benzo[1,4]dioxin-5-yl)-2-oxo-ethyl]-3-methyl-7-(2-butin-1-yl)-8-[(*S*)-3-(tert.-butyloxycarbonylamino)-piperidin-1-yl]-xanthin
   Massenspektrum (ESI⁺): m/z = 593 [M+H]⁺
(81) 1-[(4-Methoxy-naphthalin-1-yl)methyl]-3-methyl-7-(2-butin-1-yl)-8-[(S)-3-(tert.-butyloxycarbonylamino)-piperidin-1-yl]-xanthin
   R_{f}-Wert: 0.56 (Kieselgel, Petrolether/Essigester = 1:2)
   Massenspektrum (ESI⁺): m/z = 587 [M+H]⁺
(82) 1-[(4-Methoxy-naphthalin-1-yl)methyl]-3-methyl-7-(2-butin-1-yl)-8-[(R)-3-(tert.-butyloxycarbonylamino)-piperidin-1-yl]-xanthin
   Massenspektrum (ESI⁺): m/z = 587 [M+H]⁺
(83) 1-[2-(Benzo[1,3]dioxol-4-yl)-2-oxo-ethyl]-3-methyl-7-(2-butin-1-yl)-8-[(*R*)-3-(tert.-butyloxycarbonylamino)-piperidin-1-yl]-xanthin
   R_{f}-Wert: 0.86 (Kieselgel, Essigester/Petrolether = 4:1)
   Massenspektrum (ESI⁺): m/z = 579 [M+H]⁺
(84) 1-[2-(Benzo[1,3]dioxol-4-yl)-2-oxo-ethyl]-3-methyl-7-(2-butin-1-yl)-8-[(*S*)-3-(tert.-butyloxycarbonylamino)-piperidin-1-yl]-xanthin
   R_{f}-Wert: 0.86 (Kieselgel, Essigester/Petrolether = 4:1)
   Massenspektrum (ESI⁺): m/z = 579 [M+H]⁺
(85) 1-[(4-Methyl-chinazolin-2-yl)methyl]-3-methyl-7-(2-butin-1-yl)-8-[(*S*)-3-(tert.-butyloxycarbonylamino)-piperidin-1-yl]-xanthin
   R_{f}-Wert: 0.48 (Kieselgel, Methylenchlorid/Methanol = 95:5)
   Massenspektrum (ESI⁺): m/z = 573 [M+H]⁺
(86) 1-[(4-Methyl-chinazolin-2-yl)methyl]-3-methyl-7-(2-butin-1-yl)-8-[(*R*)-3-(tert.-butyloxycarbonylamino)-piperidin-1-yl]-xanthin
   R_{f}-Wert: 0.48 (Kieselgel, Methylenchlorid/Methanol = 95:5)
   Massenspektrum (ESI⁺): m/z = 573 [M+H]⁺
(87) 1-[2-(3-Methyl-2-oxo-2,3-dihydro-benzooxazol-4-yl)-2-oxo-ethyl]-3-methyl-7-(3-methyl-2-buten-1-yl)-8-[3-(tert.-butyloxycarbonylamino)-piperidin-1-yl]-xanthin
   R_{f}-Wert: 0.50 (Kieselgel, Petrolether/Essigester = 1:2)
   Massenspektrum (ESI⁺): m/z = 622 [M+H]⁺
(88) 1-(2-{2-[(Ethylaminocarbonyl)methoxy]-phenyl}-2-oxo-ethyl)-3-methyl-7-((E)-2-buten-1-yl)-8-[(*S*)-3-(tert.-butyloxycarbonylamino)-piperidin-1-yl]-xanthin
   Massenspektrum (ESI⁺): m/z = 638 [M+H]⁺
(89) 1-(2-{2-[(Methylaminocarbonyl)methoxy]-phenyl}-2-oxo-ethyl)-3-methyl-7-((E)-2-buten-1-yl)-8-[(*R*)-3-(tert.-butyloxycarbonylamino)-piperidin-1-yl]-xanthin
   Massenspektrum (ESI⁺): m/z = 624 [M+H]⁺
(90) 1-(2-{2-[(Methylaminocarbonyl)methoxy]-phenyl}-2-oxo-ethyl)-3-methyl-7-((E)-2-buten-1-yl)-8-[(*S*)-3-(tert.-butyloxycarbonylamino)-piperidin-1-yl]-xanthin
   Massenspektrum (ESI⁺): m/z = 624 [M+H]⁺
(91) 1-[2-(2-Nitro-phenyl)-2-oxo-ethyl]-3-methyl-7-(2-butin-1-yl)-8-[(*R*)-3-(tert.-butyloxycarbonylamino)-piperidin-1-yl]-xanthin
   R_{f}-Wert: 0.60 (Kieselgel, Methylenchlorid/Methanol = 95:5)
(92) 1-[2-(2-Nitro-3-methoxy-phenyl)-2-oxo-ethyl]-3-methyl-7-(3-methyl-2-buten-1-yl)-8-brom-xanthin
   Massenspektrum (ESI⁺): m/z = 506, 508 [M+H]⁺
(93) 1-[(4-Dimethylamino-chinazolin-2-yl)methyl]-3-methyl-7-(2-butin-1-yl)-8-[3-(tert.-butyloxycarbonylamino)-piperidin-1-yl]-xanthin
   (Durchführung in Gegenwart von Cäsiumcarbonat)
   R_{f}-Wert: 0.40 (Kieselgel, Methylenchlorid/Methanol = 95:5)
   Massenspektrum (ESI⁺): m/z = 602 [M+H]⁺
(94) 1-(2-{2-Oxo-3-[(2-trimethylsilanyl-ethoxy)methyl]-2,3-dihydro-benzooxazol-7-yl}-2-oxo-ethyl)-3-methyl-7-(3-methyl-2-buten-1-yl)-8-brom-xanthin
   R_{f}-Wert: 0.75 (Kieselgel, Essigester/Petrolether = 1:1)
   Massenspektrum (ESI⁺): m/z = 618, 620 [M+H]⁺
(95) 1-[(Imidazo[1,2-a]pyridin-2-yl)methyl]-3-methyl-7-(2-butin-1-yl)-8-[3-(tert.-butyloxycarbonylamino)-piperidin-1-yl]-xanthin
   R_{f}-Wert: 0.44 (Kieselgel, Methylenchlorid/Methanol = 95:5)
   Massenspektrum (ESI⁺): m/z = 547 [M+H]⁺
(96) 1-[(Chinoxalin-2-yl)methyl]-3-methyl-7-(2-butin-1-yl)-8-[3-(tert.-butyloxycarbonyl-amino)-piperidin-1-yl]-xanthin
   R_{f}-Wert: 0.50 (Kieselgel, Methylenchlorid/Methanol = 95:5)
   Massenspektrum (ESI⁺): m/z = 559 [M+H]⁺
(97) 1-[2-(1,3-Dimethyl-2-oxo-2,3-dihydro-1H-benzoimidazol-4-yl)-2-oxo-ethyl]-3-methyl-7-(2-butin-1-yl)-8-[3-(tert.-butyloxycarbonylamino)-piperidin-1-yl]-xanthin
   R_{f}-Wert: 0.50 (Kieselgel, Methylenchlorid/Methanol = 95:5)
   Massenspektrum (ESI⁺): m/z = 619 [M+H]⁺
(98) 1-[(Chinoxalin-6-yl)methyl]-3-methyl-7-(2-butin-1-yl)-8-[3-(tert.-butyloxycarbonylamino)-piperidin-1-yl]-xanthin
   R_{f}-Wert: 0.35 (Kieselgel, Essigester)
   Massenspektrum (ESI⁺): m/z = 559 [M+H]⁺
(99) 1-(2-{2-Oxo-3-[(2-trimethylsilanyl-ethoxy)methyl]-2,3-dihydro-benzooxazol-4-yl}-2-oxo-ethyl)-3-methyl-7-(2-butin-1-yl)-8-brom-xanthin
   R_{f}-Wert: 0.30 (Kieselgel, Petrolether/Essigester = 2:1)
   Massenspektrum (ESI⁻): m/z = 600, 602 [M-H]⁻
(100) 1-[(3-Methyl-chinoxalin-2-yl)methyl]-3-methyl-7-(2-butin-1-yl)-8-[3-(tert.-butyloxycarbonylamino)-piperidin-1-yl]-xanthin
   R_{f}-Wert: 0.44 (Kieselgel, Petrolether/Essigester = 1:2)
   Massenspektrum (ESI⁺): m/z = 573 [M+H]⁺
(101) 1-[(3-Phenyl-isochinolin-1-yl)methyl]-3-methyl-7-(2-butin-1-yl)-8-[3-(tert.-butyloxycarbonylamino)-piperidin-1-yl]-xanthin
   R_{f}-Wert: 0.85 (Kieselgel, Essigester)
   Massenspektrum (ESI⁺): m/z = 634 [M+H]⁺
(102) 1-[(3,4-Dimethyl-isochinolin-1-yl)methyl]-3-methyl-7-(2-butin-1-yl)-8-[3-(tert.-butyloxycarbonylamino)-piperidin-1-yl]-xanthin
   R_{f}-Wert: 0.60 (Kieselgel, Essigester/Methanol = 3:1)
   Massenspektrum (ESI⁺): m/z = 586 [M+H]⁺
(103) 1-[(Benzofuran-2-yl)methyl]-3-methyl-7-(2-butin-1-yl)-[(*R*)-3-(tert.-butyl-oxycarbonylamino)-piperidin-1-yl]-xanthin
   Massenspektrum (ESI⁺): m/z = 547 [M+H]⁺
(104) 1-{[4-(Morpholin-4-yl)-chinazolin-2-yl]methyl}-3-methyl-7-(2-butin-1-yl)-8-[3-(tert.-butyloxycarbonylamino)-piperidin-1-yl]-xanthin
   (Durchführung in Gegenwart von Cäsiumcarbonat)
   R_{f}-Wert: 0.28 (Kieselgel, Essigester)
   Massenspektrum (ESI⁺): m/z = 644 [M+H]⁺
(105) 1-{[4-(Piperidin-1-yl)-chinazolin-2-yl]methyl}-3-methyl-7-(2-butin-1-yl)-8-[3-(tert.-butyloxycarbonylamino)-piperidin-1-yl]-xanthin
   (Durchführung in Gegenwart von Cäsiumcarbonat)
   R_{f}-Wert: 0.35 (Kieselgel, Essigester)
   Massenspektrum (ESI⁺): m/z = 642 [M+H]⁺
(106) 1-({4-[4-(tert.-Butyloxycarbonyl)-piperazin-1-yl]-chinazolin-2-yl}methyl)-3-methyl-7-(2-butin-1-yl)-8-[3-(tert.-butyloxycarbonylamino)-piperidin-1-yl]-xanthin
   (Durchführung in Gegenwart von Cäsiumcarbonat)
   R_{f}-Wert: 0.50 (Kieselgel, Essigester)
   Massenspektrum (ESI⁺): m/z = 743 [M+H]⁺
(107) 1-{[4-(Pyrrolidin-1-yl)-chinazolin-2-yl]methyl}-3-methyl-7-(2-butin-1-yl)-8-[3-(tert.-butyloxycarbonylamino)-piperidin-1-yl]-xanthin
   (Durchführung in Gegenwart von Cäsiumcarbonat)
   R_{f}-Wert: 0.59 (Kieselgel, Essigester/Methanol/konz. wässriges Ammoniak = 95:5:0.1) Massenspektrum (ESI⁺): m/z = 628 [M+H]⁺
(108) 1-[2-(1-Ethoxycarbonyl-3-methyl-2-oxo-2,3-dihydro-1H-benzoimidazol-4-yl)-2-oxo-ethyl]-3-methyl-7-(2-butin-1-yl)-8-[3-(tert.-butyloxycarbonylamino)-piperidin-1-yl]-xanthin
   R_{f}-Wert: 0.25 (Kieselgel, Petrolether/Essigester = 1:2)
   Massenspektrum (ESI⁺): m/z = 677 [M+H]⁺
(109) 1-[(4-Cyano-naphthalin-1-yl)methyl]-3-methyl-7-(2-butin-1-yl)-8-[(*R*)-3-(tert.-butyloxycarbonylamino)-piperidin-1-yl]-xanthin
   R_{f}-Wert: 0.77 (Kieselgel, Methylenchlorid/Methanol/konz. wässriges Ammoniak = 90:10:1)
   Massenspektrum (ESI⁺): m/z = 582 [M+H]⁺
(110) 1-[(Imidazo[1,2-a]pyridin-3-yl)methyl]-3-methyl-7-(2-butin-1-yl)-8-[3-(tert.-butyloxycarbonylamino)-piperidin-1-yl]-xanthin
   Massenspektrum (ESI⁺): m/z = 547 [M+H]⁺
(111) 1-[(8-Methyl-imidazo[1,2-a]pyridin-2-yl)methyl]-3-methyl-7-(2-butin-1-yl)-8-[3-(tert.-butyloxycarbonylamino)-piperidin-1-yl]-xanthin
   R_{f}-Wert: 0.25 (Kieselgel, Essigester)
   Massenspektrum (ESI⁺): m/z = 561 [M+H]⁺
(112) 1-[(8-Methoxy-chinolin-5-yl)methyl]-3-methyl-7-(2-butin-1-yl)-8-[3-(tert.-butyloxycarbonylamino)-piperidin-1-yl]-xanthin
   R_{f}-Wert: 0.60 (Kieselgel, Essigester/Methanol = 9:1)
   Massenspektrum (ESI⁺): m/z = 588 [M+H]⁺
(113) 1-[(5-Methoxy-chinolin-8-yl)methyl]-3-methyl-7-(2-butin-1-yl)-8-[3-(tert.-butyloxycarbonylamino)-piperidin-1-yl]-xanthin
   R_{f}-Wert: 0.40 (Kieselgel, Methylenchlorid/Methanol = 20:1)
   Massenspektrum (ESI⁺): m/z = 588 [M+H]⁺
(114) 1-[(4-Phenyl-chinazolin-2-yl)methyl]-3-methyl-7-(2-butin-1-yl)-8-[3-(tert.-butyloxycarbonylamino)-piperidin-1-yl]-xanthin
   Massenspektrum (ESI⁺): m/z = 635 [M+H]⁺
(115) 1-[(7-Methyl-imidazo[1,2-a]pyridin-2-yl)methyl]-3-methyl-7-(2-butin-1-yl)-8-[3-(tert.-butyloxycarbonylamino)-piperidin-1-yl]-xanthin
   R_{f}-Wert: 0.50 (Kieselgel, Methylenchlorid/Methanol = 95:5)
   Massenspektrum (ESI⁺): m/z = 561 [M+H]⁺
(116) 1-(2-Oxo-4-phenyl-butyl)-3-methyl-7-(2-butin-1-yl)-8-[(R)-3-(tert.-butyloxycarbonylamino)-piperidin-1-yl]-xanthin
   Massenspektrum (ESI⁺): m/z = 563 [M+H]⁺
(117) 1-(2-{2-Oxo-1,3-bis-[(2-trimethylsilanyl-ethoxy)methyl]-2,3-dihydro-1*H-*benzoimidazol-4-yl}-2-oxo-ethyl)-3-methyl-7-(2-butin-1-yl)-8-[3-(tert.-butyloxy-carbonylamino)-piperidin-1-yl]-xanthin
   R_{f}-Wert: 0.50 (Kieselgel, Essigester/Petrolether = 1:1)
   Massenspektrum (ESI⁺): m/z = 851 [M+H]⁺
(118) 1-[(3-Difluormethyl-isochinolin-1-yl)methyl]-3-methyl-7-(2-butin-1-yl)-8-[3-(tert.-butyloxycarbonylamino)-piperidin-1-yl]-xanthin
   (Nebenprodukt bei der Umsetzung von 3-Methyl-7-(2-butin-1-yl)-8-[3-(tert.-butyloxy-carbonylamino)-piperidin-1-yl]-xanthin mit 1-Chlormethyl-3-trifluormethyl-3,4-dihydro-isochinolin)
   R_{f}-Wert: 0.75 (Aluminiumoxid, Petrolether/Essigester = 1:2)
   Massenspektrum (ESI⁺): m/z = 608 [M+H]⁺
(119) 1-[2-(2,2-Difluor-benzo[1,3]dioxol-4-yl)-2-oxo-ethyl]-3-methyl-7-(2-butin-1-yl)-8-brom-xanthin
   Massenspektrum (ESI⁺): m/z = 495, 497 [M+H]⁺
(120) 1-[(3-Methyl-imidazo[1,2-a]pyridin-2-yl)methyl]-3-methyl-7-(2-butin-1-yl)-8-[3-(tert.-butyloxycarbonylamino)-piperidin-1-yl]-xanthin
   R_{f}-Wert: 0.50 (Kieselgel, Methylenchlorid/Methanol = 95:5)
   Massenspektrum (ESI⁺): m/z = 561 [M+H]⁺
(121) 1-[(5-Methyl-imidazo[1,2-a]pyridin-2-yl)methyl]-3-methyl-7-(2-butin-1-yl)-8-[3-(tert.-butyloxycarbonylamino)-piperidin-1-yl]-xanthin
   R_{f}-Wert: 0.50 (Kieselgel, Methylenchlorid/Methanol/konz. wässriges Ammoniak = 90:10:1)
   Massenspektrum (ESI⁺): m/z = 561 [M+H]⁺
(122) 1-[(6-Methyl-imidazo[1,2-a]pyridin-2-yl)methyl]-3-methyl-7-(2-butin-1-yl)-8-[3-(tert.-butyloxycarbonylamino)-piperidin-1-yl]-xanthin
   R_{f}-Wert: 0.10 (Kieselgel, Essigester/Methanol = 98:2)
   Massenspektrum (ESI⁺): m/z = 561 [M+H]⁺
(123) 1-[(3-Benzyl-imidazo[1,2-a]pyridin-2-yl)methyl]-3-methyl-7-(2-butin-1-yl)-8-[3-(tert.-butyloxycarbonylamino)-piperidin-1-yl]-xanthin
   R_{f}-Wert: 0.60 (Kieselgel, Methylenchlorid/Methanol = 95:5)
   Massenspektrum (ESI⁺): m/z = 637 [M+H]⁺
(124) 1-[(4-Isopropyl-chinazolin-2-yl)methyl]-3-methyl-7-(2-butin-1-yl)-8-[3-(tert.-butyloxycarbonylamino)-piperidin-1-yl]-xanthin
   R_{f}-Wert: 0.50 (Kieselgel, Essigester/Petrolether = 8:2)
   Massenspektrum (ESI⁺): m/z = 601 [M+H]⁺
(125) 1-[(2,3-Dihydro-benzo[1,4]dioxin-6-yl)methyl]-3-methyl-7-(2-butin-1-yl)-8-[3-(tert.-butyloxycarbonylamino)-piperidin-1-yl]-xanthin
   R_{f}-Wert: 0.53 (Kieselgel, Essigester/Petrolether = 3:2)
(126) 1-[(3-Phenyl-imidazo[1,2-a]pyridin-2-yl)methyl]-3-methyl-7-(2-butin-1-yl)-8-[3-(tert.-butyloxycarbonylamino)-piperidin-1-yl]-xanthin
   R_{f}-Wert: 0.50 (Kieselgel, Methylenchlorid/Methanol = 95:5)
   Massenspektrum (ESI⁺): m/z = 623 [M+H]⁺
(127) 1-[2-(Naphthalin-1-yl]-2-oxo-ethyl]-3-methyl-7-(2-butin-1-yl)-8-[3-(tert.-butyloxycarbonylamino)-piperidin-1-yl]-xanthin
   R_{f}-Wert: 0.54 (Kieselgel, Methylenchlorid/Methanol = 95:5)
   Massenspektrum (ESI⁺): m/z = 585 [M+H]⁺
(128) 1-[(5-Methoxy-isochinolin-8-yl)methyl]-3-methyl-7-(2-butin-1-yl)-8-[3-(tert.-butyloxycarbonylamino)-piperidin-1-yl]-xanthin
   R_{f}-Wert: 0.50 (Kieselgel, Essigester/Methanol = 24:1)
   Massenspektrum (ESI⁺): m/z = 588 [M+H]⁺
(129) 1-[(3-Difluormethyl-isochinolin-1-yl)methyl]-3-methyl-7-(2-butin-1-yl)-8-[3-(tert.-butyloxycarbonylamino)-piperidin-1-yl]-xanthin
   (Nebenprodukt bei der Umsetzung von 3-Methyl-7-(2-butin-1-yl)-8-[3-(tert.-butyloxy-carbonylamino)-piperidin-1-yl]-xanthin mit 1-Chlormethyl-3-trifluormethyl-3,4-dihydro-isochinolin)
   R_{f}-Wert: 0.75 (Aluminiumoxid, Petrolether/Essigester = 1:2)
   Massenspektrum (ESI⁺): m/z = 608 [M+H]⁺
(130) 1-{[1-(1-Cyano-1-methyl-ethyl)-isochinolin-3-yl]methyl}-3-methyl-7-(2-butin-1-yl)-8-[3-(tert.-butyloxycarbonylamino)-piperidin-1-yl]-xanthin
   R_{f}-Wert: 0.75 (Kieselgel, Essigester)
   Massenspektrum (ESI⁺): m/z = 625 [M+H]⁺
(132) 1-Methoxycarbonylmethyl-3-methyl-7-(2-butin-1-yl)-8-[3-(tert.-butyloxycarbonyl-amino)-piperidin-1-yl]-xanthin
   Massenspektrum (ESI⁺): m/z = 489 [M+H]⁺
(133) 1-[(4-Phenyl-chinazolin-2-yl)methyl]-3-methyl-7-(2-butin-1-yl)-8-[(R)-3-(tert.-butyloxycarbonylamino)-piperidin-1-yl]-xanthin
   Massenspektrum (ESI⁺): m/z = 635 [M+H]⁺
(134) 1-[(2,3-Dimethyl-chinoxalin-6-yl)methyl]-3-methyl-7-(2-butin-1-yl)-8-[3-(tert.-butyloxycarbonylamino)-piperidin-1-yl]-xanthin
   (Durchführung in Gegenwart von Cäsiumcarbonat)
   R_{f}-Wert: 0.40 (Kieselgel, Essigester)
   Massenspektrum (ESI⁺): m/z = 587 [M+H]⁺
(135) 1-[(4-Phenyl-chinazolin-2-yl)methyl]-3-methyl-7-(2-butin-1-yl)-8-[(*S*)-3-(tert.-butyloxycarbonylamino)-piperidin-1-yl]-xanthin
   R_{f}-Wert: 0.55 (Kieselgel, Essigester/Petrolether = 8:2)
   Massenspektrum (ESI⁺): m/z = 635 [M+H]⁺
(136) 1-[2-(Chinolin-8-yl-]-2-oxo-ethyl]-3-methyl-7-(2-butin-1-yl)-8-brom-xanthin
   R_{f}-Wert: 0.55 (Kieselgel, Methylenchlorid/Methanol = 95:5)
   Massenspektrum (ESI⁺): m/z = 466, 468 [M+H]⁺
(137) 1-[(3,4-Dimethyl-6,7-dihydro-5H-[2]pyrindin-1-yl)methyl]-3-methyl-7-(2-butin-1-yl)-8-[3-(tert.-butyloxycarbonylamino)-piperidin-1-yl]-xanthin
   R_{f}-Wert: 0.65 (Aluminiumoxid, Essigester/Petrolether = 3:1)
   Massenspektrum (ESI⁺): m/z = 576 [M+H]⁺
(138) 1-[(3,4-Dimethyl-5,6,7,8-tetrahydro-isochinolin-1-yl)methyl]-3-methyl-7-(2-butin-1-yl)-8-[3-(tert.-butyloxycarbonylamino)-piperidin-1-yl]-xanthin
   R_{f}-Wert: 0.40 (Aluminiumoxid, Methylenchlorid/Methanol = 20:1)
   Massenspektrum (ESI⁺): m/z = 590 [M+H]⁺
(139) 1-{2-[1-(tert.-Butyloxycarbonyl)-1H-indol-4-yl]-2-oxo-ethyll-3-methyl-7-(2-butin-1-yl)-8-[3-(tert.-butyloxycarbonylamino)-piperidin-1-yl]-xanthin
   R_{f}-Wert: 0.55 (Kieselgel, Petrolether/Essigester = 1:2)
   Massenspektrum (ESI⁺): m/z = 674 [M+H]⁺
(140) 1-[(1-Methyl-2-oxo-1,2-dihydro-chinolin-6-yl)methyl]-3-methyl-7-(2-butin-1-yl)-8-[3-(tert.-butyloxycarbonylamino)-piperidin-1-yl]-xanthin
   Massenspektrum (EI): m/z = 587 [M]⁺
(141) 1-({1-[(2-Trimethylsilanyl-ethoxy)methyl]-2-oxo-1,2-dihydro-chinolin-6-yl}methyl)-3-methyl-7-(2-butin-1-yl)-8-[3-(tert.-butyloxycarbonylamino)-piperidin-1-yl]-xanthin
   Massenspektrum (ESI⁺): m/z = 704 [M+H]⁺
(142) 1-[(2,3,8-Trimethyl-chinoxalin-6-yl)methyl]-3-methyl-7-(2-butin-1-yl)-8-[3-(tert.-butyloxycarbonylamino)-piperidin-1-yl]-xanthin
   Massenspektrum (ESI⁺): m/z = 601 [M+H]⁺
(143) 1-[(8-Methyl-chinoxalin-6-yl)methyl]-3-methyl-7-(2-butin-1-yl)-8-[3-(tert.-butyloxycarbonylamino)-piperidin-1-yl]-xanthin
   Massenspektrum (ESI⁺): m/z = 573 [M+H]⁺
(144) 1-[(4-Methyl-phthalazin-1-yl)methyl]-3-methyl-7-(2-butin-1-yl)-8-[3-(tert.-butyloxycarbonylamino)-piperidin-1-yl]-xanthin
   R_{f}-Wert: 0.65 (Kieselgel, Methylenchlorid/Methanol = 9:1)
   Massenspektrum (ESI⁺): m/z = 573 [M+H]⁺
(145) 1-[(4-Brom-3-methoxy-isochinolin-1-yl)methyl]-3-methyl-7-(2-butin-1-yl)-8-[3-(tert.-butyloxycarbonylamino)-piperidin-1-yl]-xanth in
   R_{f}-Wert: 0.65 (Kieselgel, Methylenchlorid/Essigester = 1:1)
   Massenspektrum (ESI⁺): m/z = 666, 668 [M+H]⁺
(146) 1-[(4-Difluormethoxy-naphthalin-1-yl)methyl]-3-methyl-7-(2-butin-1-yl)-8-[(R)-3-(tert.-butyloxycarbonylamino)-piperidin-1-yl]-xanth in
   R_{f}-Wert: 0.80 (Kieselgel, Methylenchlorid/Methanol/konz. wässriges Ammoniak = 90:10:1)
   Massenspektrum (ESI⁺): m/z = 623 [M+H]⁺
(147) 1-{2-[1-(tert.-Butyloxycarbonyl)-1*H*-indol-7-yl]-2-oxo-ethyl}-3-methyl-7-(2-butin-1-yl)-8-brom-xanthin
   R_{f}-Wert: 0.83 (Kieselgel, Methylenchlorid/Methanol = 95:5)
(148) 1-[(E)-3-(2-Nitro-phenyl)-2-propen-1-yl]-3-methyl-7-(2-butin-1-yl)-8-[3-(tert.-butyloxycarbonylamino)-piperidin-1-yl]-xanth in
   Massenspektrum (ESI⁺): m/z = 578 [M+H]⁺
(149) 1-((E)-3-Pentafluorphenyl-2-propen-1-yl)-3-methyl-7-(2-butin-1-yl)-8-[3-(tert.-butyloxycarbonylamino)-piperidin-1-yl]-xanthin
   Massenspektrum (ESI⁺): m/z = 623 [M+H]⁺
(150) 1-[(4-Nitro-naphthalin-1-yl)methyl]-3-methyl-7-(2-butin-1-yl)-8-[(R)-3-(tert.-butyloxycarbonylamino)-piperidin-1-yl]-xanthin
   R_{f}-Wert: 0.41 (Kieselgel, Methylenchlorid/Methanol = 95:5)
   Massenspektrum (ESI⁺): m/z = 602 [M+H]⁺
(151) 1-[(Benzooxazol-2-y)methyl]-3-methyl-7-(2-butin-1-yl)-8-[3-(tert.-butyloxycarbo-nylamino)-piperidin-1-yl]-xanthin
   R_{f}-Wert: 0.40 (Kieselgel, Cyclohexan/Essigester = 3:7)
   Massenspektrum (ESI⁺): m/z = 548 [M+H]⁺
(152) 1-[(5-Nitro-benzooxazol-2-y)methyl]-3-methyl-7-(2-butin-1-yl)-8-[3-(tert.-butyloxycarbonylamino)-piperidin-1-yl]-xanthin
   R_{f}-Wert: 0.50 (Kieselgel, Cyclohexan/Essigester/Methanol = 5:4:1)
   Massenspektrum (ESI⁺): m/z = 593 [M+H]⁺
(153) 1-[(3-Methyl-isochinolin-1-yl)methyl]-3-methyl-7-(3-methyl-1-buten-1-yl)-8-brom-xanthin
   R_{f}-Wert: 0.65 (Kieselgel, Methylenchlorid/Methanol = 95:5)
   Massenspektrum (ESI⁺): m/z = 468, 470 [M+H]⁺
(154) 1-[(Chinolin-7-yl)methyl]-3-methyl-7-(2-butin-1-yl)-8-[3-(tert.-butyloxycarbonyl-amino)-piperidin-1-yl]-xanth in
   Massenspektrum (ESI⁺): m/z = 558 [M+H]⁺
(155) 1-[([1,5]Naphthyridin-2-yl)methyl]-3-methyl-7-(2-butin-1-yl)-8-[3-(tert.-butyloxycarbonylamino)-piperidin-1-yl]-xanthin
   Massenspektrum (ESI⁺): m/z = 559 [M+H]⁺
(156) 1-[(8-Methyl-chinoxalin-6-yl)methyl]-3-methyl-7-(2-butin-1-yl)-8-[(R)-3-(tert.-butyloxycarbonylamino)-piperidin-1-yl]-xanthin
   R_{f}-Wert: 0.45 (Kieselgel, Methylenchlorid/Methanol = 19:1)
   Massenspektrum (ESI⁺): m/z = 573 [M+H]⁺
(157) 1-[(2,3,8-Trimethyl-chinoxalin-6-yl)methyl]-3-methyl-7-(2-butin-1-yl)-8-[(R)-3-(tert.-butyloxycarbonylamino)-piperidin-1-yl]-xanthin
   R_{f}-Wert: 0.32 (Kieselgel, Methylenchlorid/Methanol = 96:4)
   Massenspektrum (ESI⁺): m/z = 601 [M+H]⁺
(158) 1-[([1,6]Naphthyridin-5-yl)methyl]-3-methyl-7-(2-butin-1-yl)-8-[3-(tert.-butyloxycarbonylamino)-piperidin-1-yl]-xanthin
   R_{f}-Wert: 0.20 (Kieselgel, Essigester/Methanol = 98:2)
   Massenspektrum (ESI⁺): m/z = 559 [M+H]⁺
(159) 1-[([1,8]Naphthyridin-2-yl)methyl]-3-methyl-7-(2-butin-1-yl)-8-[3-(tert.-butyloxycarbonylamino)-piperidin-1-yl]-xanthin
   R_{f}-Wert: 0.12 (Kieselgel, Essigester/Methanol = 98:2)
   Massenspektrum (ESI⁺): m/z = 559 [M+H]⁺
(160) 1-[(4-Fluor-naphthalin-1-yl)methyl]-3-methyl-7-(2-butin-1-yl)-8-[(R)-3-(tert.-butyloxycarbonylamino)-piperidin-1-yl]-xanthin
   R_{f}-Wert: 0.47 (Kieselgel, Petrolether/Essigester = 1:1)
   Massenspektrum (ESI⁺): m/z = 575 [M+H]⁺
(161) 1-[([1,5]Naphthyridin-2-yl)methyl]-3-methyl-7-(2-butin-1-yl)-8-[(*R*)-3-(tert.-butyloxycarbonylamino)-piperidin-1-yl]-xanthin
   R_{f}-Wert: 0.39 (Kieselgel, Essigester)
   Massenspektrum (ESI⁺): m/z = 559 [M+H]⁺
(162) 1-[2-(3-Methyl-2-oxo-2,3-dihydro-benzooxazol-4-yl)-2-oxo-ethyl]-3-methyl-7-(2-butin-1-yl)-8-[(R)-3-(tert.-butyloxycarbonylamino)-piperidin-1-yl]-xanthin
   R_{f}-Wert: 0.60 (Kieselgel, Methylenchlorid/Methanol = 95:5)
   Massenspektrum (ESI⁺): m/z = 606 [M+H]⁺
(163) 1-[(8-Phenyl-chinoxalin-6-yl)methyl]-3-methyl-7-(2-butin-1-yl)-8-[(R)-3-(tert.-butyloxycarbonylamino)-piperidin-1-yl]-xanthin
   R_{f}-Wert: 0.48 (Kieselgel, Methylenchlorid/Methanol = 95:5)
   Massenspektrum (ESI⁺): m/z = 356 [M+H]⁺
(164) 1-[([1,5]Naphthyridin-4-yl)methyl]-3-methyl-7-(2-butin-1-yl)-8-[(R)-3-(tert.-butyloxycarbonylamino)-piperidin-1-yl]-xanthin
   R_{f}-Wert: 0.25 (Kieselgel, Essigester/Petrolether = 4:1)
   Massenspektrum (ESI⁺): m/z = 559 [M+H]⁺
(165) 1-((E)-3-Pentafluorphenyl-allyl)-3-methyl-7-(2-butin-1-yl)-8-[(R)-3-(tert.-butyloxycarbonylamino)-piperidin-1-yl]-xanthin
   Massenspektrum (ESI⁺): m/z = 623 [M+H]⁺
(166) 1-[(E)-3-(2-Trifluormethyl-phenyl)-allyl]-3-methyl-7-(2-butin-1-yl)-8-[(R)-3-(tert.-butyloxycarbonylamino)-piperidin-1-yl]-xanthin
   Massenspektrum (ESI⁺): m/z = 601 [M+H]⁺
(167) 1-[(E)-3-(3-Trifluormethyl-phenyl)-allyl]-3-methyl-7-(2-butin-1-yl)-8-[(R)-3-(tert.-butyloxycarbonylamino)-piperidin-1-yl]-xanthin
   Massenspektrum (ESI⁺): m/z = 601 [M+H]⁺
(168) 1-[(E)-3-(4-Trifluormethyl-phenyl)-allyl]-3-methyl-7-(2-butin-1-yl)-8-[(R)-3-(tert.-butyloxycarbonylamino)-piperidin-1-yl]-xanthin
   Massenspektrum (ESI⁺): m/z = 601 [M+H]⁺
(169) 1-[(3-Trifluormethyl-isochinolin-1-yl)methyl]-3-methyl-7-(2-butin-1-yl)-8-[(R)-3-(tert.-butyloxycarbonylamino)-piperidin-1-yl]-xanthin
   R_{f}-Wert: 0.68 (Kieselgel, Cyclohexan/Essigester = 3:7)
   Massenspektrum (ESI⁺): m/z = 626 [M+H]⁺
(170) 1-[(3-Methyl-isochinolin-1-yl)methyl]-3-methyl-7-(2-cyano-benzyl)-8-chlor-xanthin
(171) 1-[(3-Difluormethyl-isochinolin-1-yl)methyl]-3-methyl-7-(2-butin-1-yl)-8-[(R)-3-(tert.-butyloxycarbonylamino)-piperidin-1-yl]-xanthin
   R_{f}-Wert: 0.38 (Kieselgel, Cyclohexan/Essigester = 1:1)
   Massenspektrum (ESI⁺): m/z = 608 [M+H]⁺
(172) 1-[(4-Chlor-3-methoxy-isochinolin-1-yl)methyl]-3-methyl-7-(2-butin-1-yl)-8-[3-(tert.-butyloxycarbonylamino)-piperidin-1-yl]-xanthin
   R_{f}-Wert: 0.65 (Kieselgel, Methylenchlorid/Essigester = 1:1)
   Massenspektrum (ESI⁺): m/z = 622, 624 [M+H]⁺
(173) 1-[(4-Ethoxy-chinazolin-2-yl)methyl]-3-methyl-7-(2-butin-1-yl)-8-[3-(tert.-butyloxycarbonylamino)-piperidin-1-yl]-xanthin
   R_{f}-Wert: 0.25 (Kieselgel, Methylenchlorid/Essigester = 1:1)
   Massenspektrum (ESI⁺): m/z = 603 [M+H]⁺
(174) 1-[(4-Isopropyloxy-chinazolin-2-yl)methyl]-3-methyl-7-(2-butin-1-yl)-8-[3-(tert.-butyloxycarbonylamino)-piperidin-1-yl]-xanthin
   R_{f}-Wert: 0.40 (Kieselgel, Methylenchlorid/Essigester = 1:1)
   Massenspektrum (ESI⁺): m/z = 617 [M+H]⁺
(175) 1-[(2-Methyl-benzothiazol-6-yl)methyl]-3-methyl-7-(2-butin-1-yl)-8-[3-(tert.-butyloxycarbonylamino)-piperidin-1-yl]-xanthin
   R_{f}-Wert: 0.56 (Kieselgel, Methylenchlorid/Essigester = 1:1)
   Massenspektrum (ESI⁺): m/z = 578 [M+H]⁺
(176) 1-[(3-Phenyl-isochinolin-1-yl)methyl]-3-methyl-7-(2-butin-1-yl)-8-[(R)-3-(tert.-butyloxycarbonylamino)-piperidin-1-yl]-xanthin
   R_{f}-Wert: 0.75 (Kieselgel, Methylenchlorid/Essigester = 1:1)
   Massenspektrum (ESI⁺): m/z = 634 [M+H]⁺
(177) 1-[(4-Phenyloxy-chinazolin-2-yl)methyl]-3-methyl-7-(2-butin-1-yl)-8-[3-(tert.-butyloxycarbonylamino)-piperidin-1-yl]-xanthin
   R_{f}-Wert: 0.35 (Kieselgel, Methylenchlorid/Essigester = 1:1)
   Massenspektrum (ESI⁺): m/z = 651 [M+H]⁺
(178) 1-[(4-Phenyl-chinazolin-2-yl)methyl]-3-cyclopropyl-7-(2-butin-1-yl)-8-[3-(tert.-butyloxycarbonylamino)-piperidin-1-yl]-xanthin
   R_{f}-Wert: 0.45 (Kieselgel, Methylenchlorid/Essigester = 1:1)
   Massenspektrum (ESI⁺): m/z = 661 [M+H]⁺
(179) 1-[(3-Methyl-isochinolin-1-yl)methyl]-3-cyclopropyl-7-(2-butin-1-yl)-8-[3-(tert.-butyloxycarbonylamino)-piperidin-1-yl]-xanthin
   R_{f}-Wert: 0.50 (Kieselgel, Methylenchlorid/Essigester = 1:1)
   Massenspektrum (ESI⁺): m/z = 598 [M+H]⁺
(180) 1-[2-(3-Difluormethoxy-phenyl)-2-oxo-ethyl]-3-methyl-7-(2-butin-1-yl)-8-[(R)-3-(tert.-butyloxycarbonylamino)-piperidin-1-yl]-xanth in
   R_{f}-Wert: 0.77 (Kieselgel, Methylenchlorid/Essigester = 1:1)
   Massenspektrum (ESI⁺): m/z = 601 [M+H]⁺
(181) 1-[(2-Phenyl-chinazolin-4-yl)methyl]-3-methyl-7-(2-butin-1-yl)-8-[3-(tert.-butyloxycarbonylamino)-piperidin-1-yl]-xanthin
   R_{f}-Wert: 0.65 (Kieselgel, Methylenchlorid/Essigester = 1:1)
   Massenspektrum (ESI⁺): m/z = 635 [M+H]⁺
(182) 1-[2-(2-Methoxy-phenyl)-2-oxo-ethyl]-3-methyl-7-(2-butin-1-yl)-8-[3-(tert.-butyloxycarbonylamino)-piperidin-1-yl]-xanthin
   R_{f}-Wert: 0.57 (Kieselgel, Methylenchlorid/Essigester = 1:1)
   Massenspektrum (ESI⁺): m/z = 565 [M+H]⁺
(183) 1-[2-(3-Methoxy-phenyl)-2-oxo-ethyl]-3-methyl-7-(2-butin-1-yl)-8-[3-(tert.-butyloxycarbonylamino)-piperidin-1-yl]-xanthin
   R_{f}-Wert: 0.63 (Kieselgel, Methylenchlorid/Essigester = 1:1)
   Massenspektrum (ESI⁺): m/z = 565 [M+H]⁺
(184) 1-[2-(3-Trifluormethoxy-phenyl)-2-oxo-ethyl]-3-methyl-7-(2-butin-1-yl)-8-[(R)-3-(tert.-butyloxycarbonylamino)-piperidin-1-yl]-xanthin
   R_{f}-Wert: 0.64 (Kieselgel, Methylenchlorid/Essigester = 1:1)
   Massenspektrum (ESI⁺): m/z = 619 [M+H]⁺
(185) 1-[2-(Biphenyl-2-yl)-2-oxo-ethyl]-3-methyl-7-(2-butin-1-yl)-8-[(R)-3-(tert.-butyloxycarbonylamino)-piperidin-1-yl]-xanthin
   R_{f}-Wert: 0.70 (Kieselgel, Methylenchlorid/Essigester = 1:1)
   Massenspektrum (ESI⁺): m/z = 611 [M+H]⁺
(186) 1-[2-(Biphenyl-3-yl)-2-oxo-ethyl]-3-methyl-7-(2-butin-1-yl)-8-[(R)-3-(tert.-butyloxycarbonylamino)-piperidin-1-yl]-xanthin
   R_{f}-Wert: 0.75 (Kieselgel, Methylenchlorid/Essigester = 1:1)
   Massenspektrum (ESI⁺): m/z = 611 [M+H]⁺
(187) 1-[2-(3-Isopropyloxy-phenyl)-2-oxo-ethyl]-3-methyl-7-(2-butin-1-yl)-8-[(R)-3-(tert.-butyloxycarbonylamino)-piperidin-1-yl]-xanthin
   R_{f}-Wert: 0.66 (Kieselgel, Methylenchlorid/Essigester = 1:1)
   Massenspektrum (ESI⁺): m/z = 593 [M+H]⁺
(188) 1-[(3-Methyl-isochinolin-1-yl)methyl]-3-cyclopropyl-7-(2-butin-1-yl)-8-[(R)-3-(tert.-butyloxycarbonylamino)-piperidin-1-yl]-xanth in
   R_{f}-Wert: 0.50 (Kieselgel, Methylenchlorid/Essigester = 1:1)
   Massenspektrum (ESI⁺): m/z = 598 [M+H]⁺
(189) 1-[(4-Phenyl-chinazolin-2-yl)methyl]-3-cyclopropyl-7-(2-butin-1-yl)-8-[(*R*)-3-(tert.-butyloxycarbonylamino)-piperidin-1-yl]-xanthin
   R_{f}-Wert: 0.50 (Kieselgel, Methylenchlorid/Essigester = 1:1)
   Massenspektrum (ESI⁺): m/z = 661 [M+H]⁺
(190) 1-[(4-Cyano-naphthalin-1-yl)methyl]-3-cyclopropyl-7-(2-butin-1-yl)-8-[(R)-3-(tert.-butyloxycarbonylamino)-piperidin-1-yl]-xanth in
   R_{f}-Wert: 0.75 (Kieselgel, Methylenchlorid/Essigester = 1:1)
   Massenspektrum (ESI⁺): m/z = 608 [M+H]⁺
(191) 1-[2-(2-Phenyloxy-phenyl)-2-oxo-ethyl]-3-methyl-7-(2-butin-1-yl)-8-[(*R*)-3-(tert.-butyloxycarbonylamino)-piperidin-1-yl]-xanthin
   R_{f}-Wert: 0.85 (Kieselgel, Methylenchlorid/Essigester = 1:1)
   Massenspektrum (ESI⁺): m/z = 627 [M+H]⁺
(192) 1-[2-(3-Ethoxy-phenyl)-2-oxo-ethyl]-3-methyl-7-(2-butin-1-yl)-8-[(R)-3-(tert.-butyloxycarbonylamino)-piperidin-1-yl]-xanthin
   R_{f}-Wert: 0.72 (Kieselgel, Methylenchlorid/Essigester = 1:1)
   Massenspektrum (ESI⁺): m/z = 579 [M+H]⁺
(193) 1-[2-(3-Methoxy-phenyl)-2-oxo-ethyl]-3-methyl-7-(2-butin-1-yl)-8-[(R)-3-(tert.-butyloxycarbonylamino)-piperidin-1-yl]-xanthin
   R_{f}-Wert: 0.67 (Kieselgel, Methylenchlorid/Essigester = 1:1)
   Massenspektrum (ESI⁺): m/z = 565 [M+H]⁺
(194) 1-[2-(2-Methoxy-phenyl)-2-oxo-ethyl]-3-methyl-7-(2-butin-1-yl)-8-[(R)-3-(tert.-butyloxycarbonylamino)-piperidin-1-yl)-xanthin
   R_{f}-Wert: 0.57 (Kieselgel, Methylenchlorid/Essigester = 1:1)
   Massenspektrum (ESI⁺): m/z = 565 [M+H]⁺
(195) 1-[(3-Methyl-isochinolin-1-yl)methyl]-3-methyl-7-(2-chlor-benzyl)-8-brom-xanthin
(196) 1-[(3-Methyl-isochinolin-1-yl)methyl]-3-methyl-7-(2-brom-benzyl)-8-[(R)-3-(tert.-butyloxycarbonylamino)-piperidin-1-yl]-xanthin
(197) 1-[(1,2,3,4-Tetrahydro-phenanthridin-6-yl)methyl]-3-methyl-7-(2-butin-1-yl)-8-[3-(tert.-butyloxycarbonylamino)-piperidin-1-yl]-xanthin
   R_{f}-Wert: 0.55 (Kieselgel, Essigester/ Petrolether = 2:1)
   Massenspektrum (ESI⁺): m/z = 612 [M+H]⁺

### Beispiel VI

### 1-(2-{3-[(Methansulfinyl)methoxy]-phenyl}-2-oxo-ethyl)-3-methyl-7-(3-methyl-2-buten-1-yl)-8-[3-(tert.-butyloxycarbonylamino)-piperidin-1-yl]-xanthin

Zu einer Lösung aus 402 mg 1-(2-{3-[(Methylsulfanyl)methoxy]-phenyl}-2-oxo-ethyl)-3-methyl-7-(3-methyl-2-buten-1-yl)-8-[3-(tert.-butyloxycarbonylamino)-piperidin-1-yl]-xanthin in 10 ml Hexafluorisopropanol werden 0.15 ml einer 35 %igen Wasserstoffperoxid-Lösung gegeben. Das Reaktionsgemisch wird eine halbe Stunde bei Raumtemperatur gerührt. Dann werden 5 ml einer 10 %igen Natriumthiosulfat-Lösung zugegeben. Die wässrige Phase wird zweimal mit je 5 ml Methylenchlorid extrahiert. Die vereinigten Extrakte werden über Natriumsulfat getrocknet und eingeengt. Der gelbe Rückstand wird chromatographisch über eine Kieselgel-Säule mit Cyclohexan/ Essigester/Methanol (5:4:1) als Laufmittel gereinigt.
Ausbeute: 299 mg (73 % der Theorie)
R_{f}-Wert: 0.28 (Kieselgel, Cyclohexan/Essigester/Methanol = 5:4:1)
Massenspektrum (ESI⁺): m/z = 643 [M+H]⁺

### Analog Beispiel VI werden folgende Verbindungen erhalten:

(1) 1-[2-(3-Methansulfinyl-phenyl)-2-oxo-ethyl]-3-methyl-7-(3-methyl-2-buten-1-yl)-8-[3-(tert.-butyloxycarbonylamino)-piperidin-1-yl]-xanthin
   R_{f}-Wert: 0.05 (Kieselgel, Essigester/Cyclohexan = 3:1)
   Massenspektrum (ESI⁺): m/z = 613 [M+H]⁺
(2) 1-(2-{2-[(Methansulfinyl)methoxy]-phenyl}-2-oxo-ethyl)-3-methyl-7-(2-butin-1-yl)-8-[3-(tert.-butyloxycarbonylamino)-piperidin-1-yl]-xanthin
   Massenspektrum (ESI⁺): m/z = 627 [M+H]⁺

### Beispiel VII

### 3-[(2-Trimethylsilanyl-ethoxy)methyl]-7-(3-methyl-2-buten-1-yl)-8-[3-(tert.-butyloxycarbonylamino)-piperidin-1-yl]-xanthin

Zu 630 mg 7-(3-Methyl-2-buten-1-yl)-8-[3-(tert.-butyloxycarbonylamino)-piperidin-1-yl]-xanthin in 11 ml Acetonitril werden 236 µl 1,8-Diazabicyclo[5.4.0]undec-7-en getropft. Die Lösung wird zwei Stunden bei Raumtemperatur gerührt, dann wird das Acetonitril im Vakuum abdestilliert. Der Kolbenrückstand wird in 11 ml N,N-Dimethylformamid aufgenommen und mit 258 mg (2-Trimethylsilanyl-ethoxy)methylchlorid versetzt. Das Reaktionsgemisch wird drei Stunden bei 120°C gerührt. Zur Aufarbeitung wird Wasser zugegeben, der ausgefallene Niederschlag abfiltriert und in Essigester aufgenommen. Die Lösung wird über Magnesiumsulfat getrocknet, eingeengt und über eine Kieselgel-Säule mit Cyclohexan/Essigester/Methanol (6:1:0 auf 0:5:1) als Laufmittel chromatographiert.
Ausbeute: 435 mg (53 % der Theorie)
Massenspektrum (ESI⁺): m/z = 549 [M+H]⁺

### Analog Beispiel VII werden folgende Verbindungen erhalten:

(1) 3-[(2-Trimethylsilanyl-ethoxy)methyl]-7-(2-cyano-benzyl)-xanthin Massenspektrum (ESI⁻): m/z = 396 [M-H]⁻
(2) 3-[(Methoxycarbonyl)methyl]-7-(3-methyl-2-buten-1-yl)-8-[3-(tert.-butyloxy-carbonylamino)-piperidin-1-yl]-xanthin
   R_{f}-Wert: 0.31 (Kieselgel, Methylenchlorid/Methanol/konz. wässriges Ammoniak = 90:10:1)
   Massenspektrum (ESI⁺): m/z = 491 [M+H]⁺

### Beispiel VIII

### 7-(3-Methyl-2-buten-1-yl)-8-[3-(tert.-butyloxycarbonylamino)-piperidin-1-yl]-xanthin

Zu 2.32 g 2-[3-(tert.-Butyloxycarbonylamino)-piperidin-1-yl]-3-(3-methyl-2-buten-1-yl)-4-ethoxycarbonyl-5-{[(ethoxycarbonylamino)carbonyl]amino}-3*H*-imidazol in 35 ml Ethanol werden 510 mg Kalium-tert.-butylat gegeben. Die gelbe Lösung wird fünf Stunden unter Rückfluß erhitzt. Nach Abkühlung auf Raumtemperatur wird mit Methylenchlorid verdünnt. Die organische Phase wird mit gesättigter Ammoniumchlorid-Lösung und gesättigter Natriumchlorid-Lösung gewaschen, über Magnesiumsulfat getrocknet und eingeengt. Das Rohprodukt wird chromatographisch über eine Kieselgel-Säule mit Methylenchlorid/Methanol/konz. methanolischem Ammoniak (95:5:1 auf 90:10:1) als Laufmittel gereinigt.
Ausbeute: 630 mg (35 % der Theorie)
R_{f}-Wert: 0.24 (Kieselgel, Methylenchlorid/Methanol/konz. wässriges Ammoniak = 90:10:1)
Massenspektrum (ESI⁺): m/z = 419 [M+H]⁺

### Beispiel IX

### 2-[3-(tert.-Butyloxycarbonylamino)-piperidin-1-yl]-3-(3-methyl-2-buten-1-yl)-4-ethoxycarbonyl-5-{[(ethoxycarbonylamino)carbonyl]amino}-3H-imidazol

Zu 4.00 g 2-[3-(tert.-Butyloxycarbonylamino)-piperidin-1-yl]-3-(3-methyl-2-buten-1-yl)-4-ethoxycarbonyl-5-amino-3*H*-imidazol in 90 ml 1,2-Dimethoxyethan werden 2.97 ml Isocanatameisensäureethylester gegeben und die hellbraune Lösung wird über Nacht bei 120°C im Ölbad erhitzt. Dann werden nochmals 0.6 ml Isocanatameisensäureethylester zugegeben und es wird weitere vier Stunden erhitzt. Zur Aufarbeitung wird das Reaktionsgemisch mit gesättigter Kaliumcarbonatlösung versetzt und mit Essigester extrahiert. Die organische Phase wird über Magnesiumsulfat getrocknet, eingeengt und über eine Kieselgel-Säule mit Methylenchlorid/Methanol/konz. methanolischem Ammoniak (98:2:1 auf 90:10:1) als Laufmittel gereinigt.
Ausbeute: 2.27 g (45 % der Theorie)
R_{f}-Wert: 0.29 (Kieselgel, Methylenchlorid/Methanol/konz. wässriges Ammoniak = 90:10:1)
Massenspektrum (ESI⁺): m/z = 537 [M+H]⁺

### Beispiel X

### 2-[3-(tert.-Butyloxycarbonylamino)-piperidin-1-yl]-3-(3-methyl-2-buten-1-yl)-4-ethoxycarbonyl-5-amino-3H-imidazol

hergestellt durch Umsetzung von Cyanimino-[N-(3-methyl-2-buten-1-yl)-N-(ethoxy-carbonylmethyl)-amino]-[3-(tert.-butyloxycarbonylamino)-piperidin-1-yl]-methan mit Natrium in Ethanol unter Rückfluß.
R_{f}-Wert: 0.26 (Aluminiumoxid, Essigester/Petrolether = 8:2)
Massenspektrum (ESI⁺): m/z = 422 [M+H]⁺

### Beispiel XI

### Cyanimino-[N-(3-methyl-2-buten-1-yl)-N-(ethoxycarbonylmethyl)-amino]-[3-(tert.-butyloxycarbonylamino)-piperidin-1-yl]-methan

hergestellt durch Umsetzung von Cyanimino-[N-(3-methyl-2-buten-1-yl)-N-(ethoxy-carbonylmethyl)-amino]-phenyloxy-methan mit 3-(tert.-Butyloxycarbonylamino)-piperidin in Gegenwart von Kaliumcarbonat in N,N-Dimethylformamid bei Raumtemperatur.
R_{f}-Wert: 0.10 (Kieselgel, Petrolether/Essigester = 6:4)
Massenspektrum (ESI⁺): m/z = 422 [M+H]⁺

### Beispiel XII

### Cyanimino-[N-(3-methyl-2-buten-1-yl)-N-(ethoxycarbonylmethyl)-amino]-phenyloxy-Methan

hergestellt durch Umsetzung von Cyanimino-[(ethoxycarbonylmethyl)amino]-phenyloxy-methan mit 1-Brom-3-methyl-2-buten in Gegenwart von Kaliumcarbonat in Aceton bei Raumtemperatur.
R_{f}-Wert: 0.70 (Kieselgel, Cyclohexan/Essigester = 1:1)
Massenspektrum (ESI⁺): m/z = 316 [M+H]⁺

### Beispiel XIII

### Cyanimino-[(ethoxvcarbonylmethyl)aminol-phenyloxy-methan

hergestellt durch Umsetzung von Diphenylcyanocarbonimidat mit Aminoessigsäureethylester-hydrochlorid in Gegenwart von Triethylamin in Isopropanol bei Raumtemperatur (analog R. Besse et al., Tetrahedron 1990, 46, 7803-7812).
R_{f}-Wert: 0.73 (Kieselgel, Petrolether/Essigester = 8:2)
Massenspektrum (ESI⁺): m/z = 248 [M+H]⁺

### Beispiel XIV

### 1-Methyl-3-[(methoxycarbonyl)methyl]-7-(2-cyano-benzyl)-8-chlor-xanthin

hergestellt durch Umsetzung von 1-Methyl-7-(2-cyano-benzyl)-8-chlor-xanthin mit Bromessigsäuremethylester in Gegenwart von Kaliumcarbonat in N,N-Dimethylformamid bei Raumtemperatur.
R_{f}-Wert: 0.80 (Kieselgel, Methylenchlorid/Methanol = 9:1)
Massenspektrum (ESI⁺): m/z = 388, 390 [M+H]⁺

### Analog Beispiel XIV werden folgende Verbindungen erhalten:

(1) 1-Methyl-3-cyanomethyl-7-(2-cyano-benzyl)-8-chlor-xanthin
   Massenspektrum (ESI⁺): m/z = 355, 357 [M+H]⁺
(2) 1-Methyl-3-(2-propin-1-yl)-7-(2-cyano-benzyl)-8-chlor-xanthin R_{f}-Wert: 0.80 (Kieselgel, Methylenchlorid/Methanol = 95:5)
   Massenspektrum (ESI⁺): m/z = 354, 356 [M+H]⁺
(3) 1-Methyl-3-(2-propen-1-yl)-7-(2-cyano-benzyl)-8-chlor-xanthin R_{f}-Wert: 0.90 (Kieselgel, Methylenchlorid/Methanol = 95:5)
   Massenspektrum (ESI⁺): m/z = 356, 358 [M+H]⁺
(4) 1-(2-Phenyl-2-oxo-ethyl)-3-cyanomethyl-7-(3-methyl-2-buten-1-yl)-8-[3-(tert.-butyloxycarbonylamino)-piperidin-1-yl]-xanthin
   R_{f}-Wert: 0.78 (Kieselgel, Methylenchlorid/Methanol/konz. wässriges Ammoniak = 90:10:1)
   Massenspektrum (ESI⁺): m/z = 576 [M+H]⁺
(5) 1-Methyl-3-isopropyl-7-(2-cyano-benzyl)-8-chlor-xanthin
   Massenspektrum (ESI⁺): m/z = 358, 360 [M+H]⁺

### Beispiel XV

### 1-Methyl-7-(2-cyano-benzyl)-8-chlor-xanthin

hergestellt durch Behandeln von 1-Methyl-3-[(2-trimethylsilanyl-ethoxy)methyl]-7-(2-cyano-benzyl)-8-chlor-xanthin mit Trifluoressigsäure in Methylenchlorid bei Raumtemperatur.
R_{f}-Wert: 0.50 (Kieselgel, Methylenchlorid/Methanol = 9:1)
Massenspektrum (ESI⁺): m/z = 316, 318 [M+H]⁺

### Analog Beispiel XV werden folgende Verbindungen erhalten:

(1) 1-(2-Phenyl-2-oxo-ethyl)-3-methyl-8-brom-xanthin
   R_{f}-Wert: 0.26 (Kieselgel, Methylenchlorid/Methanol = 95:5)
   Massenspektrum (ESI⁻): m/z = 361, 363 [M-H]⁻
(2) 1-[(4-Oxo-3,4-dihydro-phthalazin-1-yl)methyl]-3-methyl-7-(3-methyl-2-buten-1-yl)-8-(3-amino-piperidin-1-yl)-xanthin
   (Da die Verbindung noch Verunreinigungen enthält, die chromatographisch nicht entfernt werden können, wird das Material nochmals in das BOC-geschützte Derivat überführt und dann chromatographisch gereinigt, vgl. Bsp. XXV(1).)
   Massenspektrum (ESI⁺): m/z = 491 [M+H]⁺

### Beispiel XVI

### 1-Methyl-3-[(2-trimethylsilanyl-ethoxy)methyl]-7-(2-cyano-benzyl)-8-chlor-xanthin

hergestellt durch Chlorierung von 1-Methyl-3-[(2-trimethylsilanyl-ethoxy)methyl]-7-(2-cyano-benzyl)-xanthin mit N-Chlorsuccinimid in Dichlorethan unter Rückfluß.
Massenspektrum (EI): m/z = 445, 447 [M]⁺

### Beispiel XVII

### 7-(2-Cyano-benzyl)-xanthin

hergestellt durch Behandeln von 16.68 g 2-Amino-7-(2-cyano-benzyl)-1,7-dihydro-purin-6-on mit 17.00 g Natriumnitrit in einem Gemisch aus 375 ml konz. Essigsäure, 84 ml Wasser und 5.2 ml konz. Salzsäure bei 50°C.
Ausbeute: 8.46 g (50 % der Theorie)
Massenspektrum (ESI⁺): m/z = 268 [M+H]⁺

### Beispiel XVIII

### 2-Amino-7-(2-cyano-benzyl)-1,7-dihydro-purin-6-on

hergestellt durch Umsetzung von 20.00 g Guanosin-hydrat mit 22.54 g 2-Cyanobenzylbromid in Dimethylsulfoxid bei 60°C und anschließende Behandlung mit 57 ml konz. Salzsäure.
Ausbeute: 18.00 g (97% der Theorie)
Massenspektrum (ESI⁺): m/z = 267 [M+H]⁺

### Beispiel XIX

### 1-{2-[3-(2-Oxo-imidazolidin-1-yl)-phenyl]-2-oxo-ethyl}-3-methyl-7-(3-methyl-2-buten-1-yl)-8-[3-(tert.-butyloxycarbonylamino)-piperidin-1-yl]-xanthin

hergestellt durch Behandeln von 1-[2-(3-{[(2-Chlor-ethylamino)carbonyl]amino}-phenyl)-2-oxo-ethyl]-3-methyl-7-(3-methyl-2-buten-1-yl)-8-[3-(tert.-butyloxy-carbonylamino)-piperidin-1-yl]-xanthin mit Kalium-tert.-butylat in N,N-Dimethyl-formamid bei Raumtemperatur.
R_{f}-Wert: 0.50 (Kieselgel, Methylenchlorid/Methanol = 9:1)

### Beispiel XX

### 1-[2-(3-{[(2-Chlor-ethylamino)carbonyl]amino}-phenyl)-2-oxo-ethyl]-3-methyl-7-(3-methyl-2-buten-l-yl)-8-[3-(tert.-butyloxycarbonylamino)-piperidin-1-yl]-xanthin

hergestellt durch Umsetzung von 221 mg 1-[2-(3-Amino-phenyl)-2-oxo-ethyl]-3-methyl-7-(3-methyl-2-buten-1-yl)-8-[3-(tert.-butyloxycarbonylamino)-piperidin-1-yl]-xanthin mit 60 µl 2-Chlorethylisocyanat in 3 ml Methylenchlorid bei Raumtemperatur.
Ausbeute: 163 mg (64 % der Theorie)
R_{f}-Wert: 0.20 (Kieselgel, Cyclohexan/Essigester/Methanol = 6:3:1) Massenspektrum (ESI⁺): m/z = 671, 673 [M+H]⁺

### Analog Beispiel XX werden folgende Verbindungen erhalten:

(1) 1-[2-(2-{[(Ethoxycarbonylamino)carbonyl]amino}-phenyl)-2-oxo-ethyl]-3-methyl-7-(3-methyl-2-buten-1-yl)-8-[3-(tert.-butyloxycarbonylamino)-piperidin-1-yl]-xanthin
   (Durchführung in N,N-Dimethylformamid bei 30°C)
   R_{f}-Wert: 10.26 (Kieselgel, Cyclohexan/Essigester = 4:6)
   Massenspektrum (ESI⁺): m/z = 681 [M+H]⁺

### Beispiel XXI

### 1-[2-(3-Amino-phenyl)-2-oxo-ethyl]-3-methyl-7-(3-methyl-2-buten-1-yl)-8-[3-(tert.-butyloxycarbonylamino)-piperidin-1-yl]-xanthin

hergestellt durch Behandeln von 1-[2-(3-Nitro-phenyl)-2-oxo-ethyl]-3-methyl-7-(3-methyl-2-buten-1-yl)-8-[3-(tert.-butyloxycarbonylamino)-piperidin-1-yl]-xanthin mit Eisenpulver in einem Gemisch aus Ethanol, Wasser und Eisessig (80:25:10) bei 100°C.
R_{f}-Wert: 0.55 (Kieselgel, Cyclohexan/Essigester/Methanol/konz. wässriges Ammoniak = 50:30:20:1)
Massenspektrum (ESI⁺): m/z = 566 [M+H]⁺

### Analog Beispiel XXI werden folgende Verbindungen erhalten:

(1) 1-[2-(2-Amino-phenyl)-2-oxo-ethyl]-3-methyl-7-(3-methyl-2-buten-1-yl)-8-[3-(tert.-butyloxycarbonylamino)-piperidin-1-yl]-xanthin
   Massenspektrum (ESI⁺): m/z = 566 [M+H]⁺
(2) 1-[2-(2-Amino-phenyl)-2-oxo-ethyl]-3-methyl-7-(3-methyl-2-buten-1-yl)-8-[(S)-3-(tert.-butyloxycarbonylamino)-piperidin-1-yl]-xanthin
   Massenspektrum (ESI⁺): m/z = 566 [M+H]⁺
(3) 1-[(5-Amino-isochinolin-1-yl)methyl]-3-methyl-7-(3-methyl-2-buten-1-yl)-8-[3-(tert.-butyloxycarbonylamino)-piperidin-1-yl]-xanthin
   R_{f}-Wert: 0.22 (Kieselgel, Essigester)
   Massenspektrum (ESI⁺): m/z = 589 [M+H]⁺
(4) 1-[2-(2-Amino-phenyl)-2-oxo-ethyl]-3-methyl-7-[(1-cyclopenten-1-yl)methyl]-8-brom-xanthin
   Massenspektrum (ESI⁺): m/z = 458, 460 [M+H]⁺
(5) 1-[2-(2-Amino-phenyl)-2-oxo-ethyl]-3-methyl-7-((E)-2-buten-1-yl)-8-brom-xanthin (Produkt enthält ca. 10 % Z-Isomer)
   R_{f}-Wert: 0.55 (Kieselgel, Cyclohexan/Essigester = 4:6)
   Massenspektrum (ESI⁺): m/z = 432, 434 [M+H]⁺
(6) 1-[2-(2-Amino-phenyl)-2-oxo-ethyl]-3-methyl-7-(2-butin-1-yl)-8-brom-xanthin
   R_{f}-Wert: 0.50 (Kieselgel, Methylenchlorid/Methanol = 95:5)
   Massenspektrum (ESI⁺): m/z = 430, 432 [M+H]⁺
(7) 1-[2-(2-Amino-phenyl)-2-oxo-ethyl]-3-methyl-7-((E)-2-buten-1-yl)-8-[(R)-3-(tert.-butyloxycarbonylamino)-piperidin-1-yl]-xanthin
   Massenspektrum (ESI⁺): m/z = 552 [M+H]⁺
(8) 1-[2-(2-Amino-phenyl)-2-oxo-ethyl]-3-methyl-7-((E)-2-buten-1-yl)-8-[(S)-3-(tert.-butyloxycarbonylamino)-piperidin-1-yl]-xanthin
   Massenspektrum (ESI⁺): m/z = 552 [M+H]⁺
(9) 1-[2-(2-Amino-3-methoxy-phenyl)-2-oxo-ethyl]-3-methyl-7-(3-methyl-2-buten-1-yl)-8-[3-(tert.-butyloxycarbonylamino)-piperidin-1-yl]-xanthin
   R_{f}-Wert: 0.82 (Kieselgel, Essigester/Petrolether = 4:1)
   Massenspektrum (ESI⁺): m/z = 596 [M+H]⁺

### Beispiel XXII

### 1-(2-{2-[(Ethylcarbonyl)amino]-phenyl}-2-oxo-ethyl)-3-methyl-7-(3-methyl-2-buten-1-yl)-8-[3-(tert.-butyloxycarbonylamino)-piperidin-1-yl]-xanthin

hergestellt durch Umsetzung von 248 mg 1-[2-(2-Amino-phenyl)-2-oxo-ethyl]-3-methyl-7-(3-methyl-2-buten-1-yl)-8-[3-(tert.-butyloxycarbonylamino)-piperidin-1-yl]-xanthin mit 40 µl Propionsäurechlorid in Gegenwart von 60 µl Pyridin in N,N-Dimethylformamid bei 80°C.
Ausbeute: 168 mg (62 % der Theorie)
R_{f}-Wert: 0.55 (Kieselgel, Cyclohexan/Essigester = 3:7)
Massenspektrum (ESI⁺): m/z = 622 [M+H]⁺

### Analog Beispiel XXII werden folgende Verbindungen erhalten:

(1) 1-({5-[(Methoxycarbonyl)methylamino]-isochinolin-1-yl}methyl)-3-methyl-7-(3-methyl-2-buten-1-yl)-8-[3-(tert.-butyloxycarbonylamino)-piperidin-1-yl]-xanthin
   (Durchführung mit Bromessigsäuremethylester und Kaliumcarbonat)
   R_{f}-Wert: 0.42 (Kieselgel, Methylenchlorid/Methanol = 95:5)
   Massenspektrum (ESI⁺): m/z = 661 [M+H]⁺
(2) 1-[2-(2-Acetylamino-phenyl)-2-oxo-ethyl]-3-methyl-7-((E)-2-buten-1-yl)-8-[3-(tert.-butyloxycarbonylamino)-piperidin-1-yl]-xanthin
   (Produkt enthält ca. 10 % Z-Isomer)
   Massenspektrum (ESI⁺): m/z = 594 [M+H]⁺
(3) 1-(2-{2-[(Isopropylcarbonyl)amino]-phenyl}-2-oxo-ethyl)-3-methyl-7-((E)-2-buten-1-yl)-8-[3-(tert.-butyloxycarbonylamino)-piperidin-1-yl]-xanthin
   (Produkt enthält ca. 10 % Z-Isomer)
   Massenspektrum (ESI⁺): m/z = 622 [M+H]⁺
(4) 1-[2-(2-Acetylamino-phenyl)-2-oxo-ethyl]-3-methyl-7-(3-methyl-2-buten-1-yl)-8-[(S)- 3-(tert.-butyloxycarbonylamino)-piperidin-1-yl]-xanthin
   R_{f}-Wert: 0.50 (Kieselgel, Cyclohexan/Essigester = 3:7)
   Massenspektrum (ESI⁺): m/z = 608 [M+H]⁺
(5) 1-[2-(2-Acetylamino-phenyl)-2-oxo-ethyl]-3-methyl-7-(2-butin-1-yl)-8-[3-(tert.-butyloxycarbonylamino)-piperidin-1-yl]-xanthin
   R_{f}-Wert: 0.34 (Kieselgel, Cyclohexan/Essigester = 3:7)
   Massenspektrum (ESI⁺): m/z = 592 [M+H]⁺
(6) 1-(2-{2-[(Isopropylcarbonyl)amino]-phenyl}-2-oxo-ethyl)-3-methyl-7-(3-methyl-2-buten-1-yl)-8-[3-(tert.-butyloxycarbonylamino)-piperidin-1-yl]-xanthin
   R_{f}-Wert: 0.25 (Kieselgel, Cyclohexan/Essigester = 1:1)
   Massenspektrum (ESI⁺): m/z = 636 [M+H]⁺
(7) 1-(2-{2-[(Isopropylcarbonyl)amino]-phenyl}-2-oxo-ethyl)-3-methyl-7-(2-butin-1-yl)-8-[3-(tert.-butyloxycarbonylamino)-piperidin-1-yl]-xanthin
   R_{f}-Wert: 0.44 (Kieselgel, Methylenchlorid/Methanol = 95:5)
   Massenspektrum (ESI⁺): m/z = 620 [M+H]⁺
(8) 1-[2-(2-Acetylamino-phenyl)-2-oxo-ethyl]-3-methyl-7-(2-butin-1-yl)-8-[(S)-3-(tert.-butyloxycarbonylamino)-piperidin-1-yl]-xanthin
   R_{f}-Wert: 0.34 (Kieselgel, Cyclohexan/Essigester = 3:7)
   Massenspektrum (ESI⁺): m/z = 592 [M+H]⁺
(9) 1-(2-{2-[(Isopropylcarbonyl)amino]-phenyl}-2-oxo-ethyl)-3-methyl-7-(2-butin-1-yl)-8-[(S)-3-(tert.-butyloxycarbonylamino)-piperidin-1-yl]-xanthin
   R_{f}-Wert: 0.44 (Kieselgel, Methylenchlorid/Methanol = 95:5)
   Massenspektrum (ESI⁺): m/z = 620 [M+H]⁺
(10) 1-(2-{2-[(Methoxycarbonyl)amino]-phenyl}-2-oxo-ethyl)-3-methyl-7-(3-methyl-2-buten-1-yl)-8-[3-(tert.-butyloxycarbonylamino)-piperidin-1-yl]-xanthin
   (Durchführung in Acetonitril bei 55°C)
   R_{f}-Wert: 0.25 (Kieselgel, Cyclohexan/Essigester = 1:1)
   Massenspektrum (ESI⁺): m/z = 624 [M+H]⁺
(11) 1-(2-{2-[(Isopropylcarbonyl)amino]-phenyl}-2-oxo-ethyl)-3-methyl-7-((E)-2-buten-1-yl)-8-[(S)-3-(tert.-butyloxycarbonylamino)-piperidin-1-yl]-xanthin
   (Durchführung in Acetonitril bei 65°C)
   R_{f}-Wert: 0.50 (Kieselgel, Cyclohexan/Essigester/Isopropanol = 14:3:3)
   Massenspektrum (ESI⁺): m/z = 622 [M+H]⁺
(12) 1-(2-{2-[(Ethylcarbonyl)amino]-phenyl}-2-oxo-ethyl)-3-methyl-7-((E)-2-buten-1-yl)-8-[(R)-3-(tert.-butyloxycarbonylamino)-piperidin-1-yl]-xanthin
   Massenspektrum (ESI⁺): m/z = 608 [M+H]⁺
(13) 1-[2-(2-Acetylamino-phenyl)-2-oxo-ethyl]-3-methyl-7-((E)-2-buten-1-yl)-8-[(*R*)-3-(tert.-butyloxycarbonylamino)-piperidin-1-yl]-xanthin
   Massenspektrum (ESI⁺): m/z = 594 [M+H]⁺
(14) 1-[2-(2-Acetylamino-phenyl)-2-oxo-ethyl]-3-methyl-7-((E)-2-buten-1-yl)-8-[(S)-3-(tert.-butyloxycarbonylamino)-piperidin-1-yl]-xanthin
   R_{f}-Wert: 0.28 (Kieselgel, Cyclohexan/Essigester/Isopropanol = 8:1:1)
   Massenspektrum (ESI⁺): m/z = 594 [M+H]⁺
(15) 1-(2-{2-[(Isopropylcarbonyl)amino]-phenyl}-2-oxo-ethyl)-3-methyl-7-(2-butin-1-yl)-8-[(*R*)-3-(tert.-butyloxycarbonylamino)-piperidin-1-yl]-xanthin
   R_{f}-Wert: 0.90 (Kieselgel, Methylenchlorid/Methanol = 9:1)
(16) 1-(2-{2-[(Isopropylcarbonyl)amino]-phenyl}-2-oxo-ethyl)-3-methyl-7-((E)-2-buten-1-yl)-8-[(*R*)-3-(tert.-butyloxycarbonylamino)-piperidin-1-yl])-xanthin
   (Durchführung in 1,2-Dichlorethan bei 45°C)
   R_{f}-Wert: 0.30 (Kieselgel, Cyclohexan/Essigester/Isopropanol = 8:1:1)
   Massenspektrum (ESI⁺): m/z = 622 [M+H]⁺
(17) 1-(2-{2-[(Ethylcarbonyl)amino]-phenyl}-2-oxo-ethyl)-3-methyl-7-((E)-2-buten-1-yl)-8-[(S)-3-(tert.-butyloxycarbonylamino)-piperidin-1-yl]-xanthin
   R_{f}-Wert: 0.48 (Kieselgel, Cyclohexan/Essigester/Isopropanol = 14:3:3)
   Massenspektrum (ESI⁺): m/z = 608 [M+H]⁺
(18) 1-(2-{2-[(Ethylcarbonyl)amino]-phenyl}-2-oxo-ethyl)-3-methyl-7-(2-butin-1-yl)-8-[(*R*)-3-(tert.-butyloxycarbonylamino)-piperidin-1-yl]-xanthin
   Massenspektrum (ESI⁺): m/z = 606 [M+H]⁺
(19) 1-(2-{2-[(Ethylcarbonyl)amino]-phenyl}-2-oxo-ethyl)-3-methyl-7-(2-butin-1-yl)-8-[(*S*)-3-(tert.-butyloxycarbonylamino)-piperidin-1-yl]-xanthin
   R_{f}-Wert: 0.22 (Kieselgel, Methylenchlorid/Methanol = 95:5)
(20) 1-(2-{2-[(Phenylcarbonyl)amino]-phenyl}-2-oxo-ethyl)-3-methyl-7-((E)-2-buten-1-yl)-8-[3-(tert.-butyloxycarbonylamino)-piperidin-1-yl]-xanthin
   R_{f}-Wert: 0.55 (Kieselgel, Cyclohexan/Essigester/Isopropanol = 14:3:3)
   Massenspektrum (ESI⁺): m/z = 656 [M+H]⁺
(21) 1-(2-{2-[(Cyclopropylcarbonyl)amino]-phenyl}-2-oxo-ethyl)-3-methyl-7-[(1-cyclopenten-1-yl)methyl]-8-[3-(tert.-butyloxycarbonylamino)-piperidin-1-yl]-xanthin (Durchführung mit Hünigbase und 4-Dimethylamino-pyridin in Methylenchlorid)
   R_{f}-Wert: 0.60 (Kieselgel, Methylenchlorid/Methanol = 18:1)

### Beispiel XXIII

### 1-(2-{3-[(Methoxycarbonyl)methoxy]-phenyl}-2-oxo-ethyl)-3-methyl-7-(3-methyl-2-buten-1-yl)-8-(3-amino-piperidin-1-yl)-xanthin

hergestellt durch Behandeln von 1-(2-{3-[(Methoxycarbonyl)methoxy]-phenyl}-2-oxo-ethyl)-3-methyl-7-(3-methyl-2-buten-1-yl)-8-[3-(tert.-butyloxycarbonylamino)-piperidin-1-yl]-xanthin mit Trifluoressigsäure in Methylenchlord bei Raumtemperatur.
Massenspektrum (ESI⁺): m/z = 539 [M+H]⁺

### Analog Beispiel XXIII werden folgende Verbindungen erhalten:

(1) 1-(2-{2-[(Methoxycarbonyl)methoxy]-phenyl}-2-oxo-ethyl)-3-methyl-7-(3-methyl-2-buten-1-yl)-8-(3-amino-piperidin-1-yl)-xanthin
   Massenspektrum (ESI⁺): m/z = 539 [M+H]⁺

### Beispiel XXIV

### 1-Methyl-3-phenyl-7-(2-cyano-benzyl)-8-chlor-xanthin

Ein Gemisch aus 829 mg 1-Methyl-7-(2-cyano-benzyl)-8-chlor-xanthin, 640 mg Phenylboronsäure, 509 mg wasserfreiem Kupferacetat und 0.43 ml Pyridin in 20 ml Methylenchlorid wird in Gegenwart von 100 mg Molsieb 4Å vier Tage bei Raumtemperatur gerührt. Dann werden nochmals 320 mg Phenylboronsäure zugesetzt und das Reaktionsgemisch wird einen weiteren Tag bei Raumtemperatur gerührt. Zur Aufarbeitung wird das Gemisch über Talkum filtriert und mit Essigester nachgewaschen. Das Filtrat wird eingeengt und über eine Kieselgel-Säule mit Cyclohexan/Essigester (7:3 auf 1:1) als Laufmittel chromatogaphiert.
Ausbeute: 142 mg (14 % der Theorie)
Massenspektrum (ESI⁺): m/z = 392, 394 [M+H]⁺

### Beispiel XXV

### 1-(2-Phenyl-2-oxo-ethyl)-7-(3-methyl-2-buten-1-yl)-8-[3-(tert.-butyloxycarbonyl-amino)-piperidin-1-yl]-xanthin

hergestellt durch Umsetzung von 1-(2-Phenyl-2-oxo-ethyl)-7-(3-methyl-2-buten-1-yl)-8-(3-amino-piperidin-1-yl)-xanthin mit Pyrokohlensäure-di-tert.-butylester in Gegenwart von Hünigbase in Methylenchlorid bei Raumtemperatur.
R_{f}-Wert: 0.27 (Kieselgel, Methylenchlorid/Methanol/konz. wässriges Ammoniak = 90:10:1)

### Analog Beispiel XXV werden folgende Verbindungen erhalten:

(1) 1-[(4-Oxo-3,4-dihydro-phthalazin-1-yl)methyl]-3-methyl-7-(3-methyl-2-buten-1-yl)-8-[3-(tert.-butyloxycarbonyl-amino)-piperidin-1-yl]-xanthin
   R_{f}-Wert: 0.27 (Kieselgel, Methylenchlorid/Methanol = 95:5)
   Massenspektrum (ESI⁺): m/z = 591 [M+H]⁺
(2) 7-Acetyl-1-(tert.-butyloxycarbonyl)-1*H*-indol
   R_{f}-Wert: 0.82 (Kieselgel, Methylenchlorid/Petrolether/Essigester= 5:4:1)
   Massenspektrum (ESI⁺): m/z = 260 [M+H]⁺

### Beispiel XXVI

### 1-[(Cinnolin-4-yl)methyl]-3-methyl-7-(3-methyl-2-buten-1-yl)-8-chlor-xanthin und 1-[(1,4-Dihydro-cinnolin-4-yl)methyl]-3-methyl-7-(3-methyl-2-buten-l-yl)-8-chlor-xanthin

Zu 830 mg 3-Methyl-7-(3-methyl-2-buten-1-yl)-8-chlor-xanthin und 1.25 g Triphenylphosphin in 25 ml Tetrahydrofuran werden 510 mg eines Gemisches aus (Cinnolin-4-yl)-methanol und (1,4-Dihydro-cinnolin-4-yl)-methanol (siehe Bsp. XXVII) gegeben. Das Reaktionsgemisch wird mit 0.92 ml Azodicarbonsäurediethylester versetzt und über Nacht bei Raumtemperatur gerührt. Anschließend wird es eingeengt und über eine Kieselgel-Säule mit Essigester/Petrolether (7:3 auf 0:1) als Laufmittel chromatographiert. Es wird ein Gemisch aus Cinnolin- und 1,4-Dihydro-Cinnolin-Verbindung erhalten.
Aubeute: 660 mg (52 % der Theorie)
R_{f}-Wert: 0.60 (Kieselgel, Essigester/Petrolether = 7:3)

### Analog Beispiel XXVI werden folgende Verbindungen erhalten:

(1) 1-({4-Oxo-3-[(2-trimethylsilanyl-ethoxy)methyl]-3,4-dihydro-phthalazin-1-yl}methyl)-3-methyl-7-(3-methyl-2-buten-1-yl)-8-chlor-xanthin
   R_{f}-Wert: 0.85 (Kieselgel, Methylenchlorid/Methanol = 95:5)
   Massenspektrum (ESI⁺): m/z = 557, 559 [M+H]⁺
(2) 1-[(lsochinolin-3-yl)methyl]-3-methyl-7-(3-methyl-2-buten-1-yl)-8-chlor-xanthin Schmelzpunkt: 194-195°C
   Massenspektrum (ESI⁺): m/z = 410, 412 [M+H]⁺
(3) 1-[(3-Methyl-4-oxo-3,4-dihydro-phthalazin-1-yl)methyl]-3-methyl-7-(3-methyl-2-buten-1-yl)-8-chlor-xanthin
   R_{f}-Wert: 0.66 (Kieselgel, Essigester)
   Massenspektrum (ESI⁺): m/z = 441, 443 [M+H]⁺
(4) 1-[(Chinolin-4-yl)methyl]-3-methyl-7-(2-butin-1-yl)-8-brom-xanthin
   (Durchführung mit Kaliumcarbonat)
   R_{f}-Wert: 0.45 (Kieselgel, Essigester)
   Massenspektrum (ESI⁺): m/z = 438, 440 [M+H]⁺
(5) 1-[(Isochinolin-1-yl)methyl]-3-methyl-7-(2-butin-1-yl)-8-brom-xanthin
   R_{f}-Wert: 0.78 (Kieselgel, Essigester)
   Massenspektrum (ESI⁺): m/z = 438, 440 [M+H]⁺
(6) 1-[(4-Dimethylamino-naphthalin-1-yl)methyl]-3-methyl-7-(2-butin-1-yl)-8-[3-(tert.-butyloxycarbonylamino)-piperidin-1-yl]-xanthin
   R_{f}-Wert: 0.80 (Kieselgel, Essigester)
   Massenspektrum (ESI⁺): m/z = 600 [M+H]⁺
(7) 1-[(Isochinolin-1-yl)methyl]-3-methyl-7-((E)-2-buten-1-yl)-8-brom-xanthin
   (Das Produkt enthält ca. 20 % Z-Isomer)
   R_{f}-Wert: 0.71 (Kieselgel, Essigester)
   Massenspektrum (ESI⁺): m/z = 440, 442 [M+H]⁺
(8) 1-[(1-Methyl-1*H*-indol-2-yl)methyl]-3-methyl-7-(2-butin-1-yl)-8-brom-xanthin
   R_{f}-Wert: 0.95 (Kieselgel, Methylenchlorid/Methanol = 95:5)
   Massenspektrum (ESI⁺): m/z = 440, 442 [M+H]⁺
(9) 1-[(Chinolin-3-yl)methyl]-3-methyl-7-(2-butin-1-yl)-8-brom-xanthin
   R_{f}-Wert: 0.55 (Kieselgel, Essigester/Petrolether = 8:2)
   Massenspektrum (ESI⁺): m/z = 438, 440 [M+H]⁺
(10) 1-{[1-(tert.-Butyloxycarbonylamino)-1H-indol-2-yl]methyl}-3-methyl-7-(2-butin-1-yl)-8-brom-xanthin
   R_{f}-Wert: 0.74 (Kieselgel, Petrolether/Essigester = 1:1)
   Massenspektrum (ESI⁺): m/z = 526, 528 [M+H]⁺
(11) 1-({2-Methyl-1-[(2-trimethylsilanyl-ethoxy)methyl]-1*H*-benzoimidazol-5-yl}methyl)-3-methyl-7-(2-butin-1-yl)-8-brom-xanthin (im Gemisch mit 1-({2-Methyl-3-[(2-trimethylsilanyl-ethoxy)methyl]-3H-benzoimidazol-5-yl}methyl)-3-methyl-7-(2-butin-1-yl)-8-brom-xanthin)
   Massenspektrum (ESI⁺): m/z = 571, 573 [M+H]⁺
(12) 1-[(1-[(2-Trimethylsilanyl-ethoxy)methyl]-1*H*-benzoimidazol-5-yl)methyl]-3-methyl-7-(2-butin-1-yl)-8-brom-xanthin (im Gemisch mit 1-[(3-[(2-Trimethylsilanyl-ethoxy)methyl]-3H-benzoimidazol-5-yl)methyl]-3-methyl-7-(2-butin-1-yl)-8-brom-xanthin)
   R_{f}-Wert: 0.50 (Kieselgel, Methylenchlorid/Methanol = 95:5)
   Massenspektrum (ESI⁺): m/z = 557, 559 [M+H]⁺
(13) 1-[(Pyrazolo[1,5-a]pyridin-2-yl)methyl]-3-methyl-7-(2-butin-1-yl)-8-brom-xanthin
   R_{f}-Wert: 0.35 (Kieselgel, Petrolether/Essigester = 1:2)
   Massenspektrum (ESI⁺): m/z = 427, 429 [M+H]⁺

### Beispiel XXVII

### (Cinnolin-4-vl)-methanol und (1,4-Dihydro-cinnolin-4-yl)-methanol

Eine Lösung aus 1.00 g Cinnolin-4-carbonsäure-methylester in 15 ml Diethylether wird bei 0°C zu einer Suspension aus 222 mg Lithiumaluminiumhydrid in 5 ml Diethylether getropft. Nach 1.5 Stunden wird das Reaktionsgemisch vorsichtig tropfenweise mit Wasser versetzt, mit Methylenchlorid verrührt und über einen Glasfaserfilter abgesaugt. Die wässrige Phase wird mit Methylenchlorid extrahiert und die vereinigten organischen Phasen werden über Magnesiumsulfat getrocknet und eingeengt. Laut ¹H-NMR wird ein Gemisch aus Cinnolin- und 1,4-Dihydro-Cinnolin-Verbindung als gelbes Öl erhalten, welches ohne weitere Reinigung weiter umgesetzt wird.
Aubeute: 530 mg (62 % der Theorie)
R_{f}-Wert: 0.63 (Kieselgel, Essigester)
Massenspektrum (ESI⁺): m/z = 161 [M1+H]⁺ und 163 [M2+H]⁺

### Analog Beispiel XXVII werden folgende Verbindungen erhalten:

(1) {2-Methyl-1-[(2-trimethylsilanyl-ethoxy)methyl]-1*H*-benzoimidazol-5-yl}-methanol (im Gemisch mit {2-Methyl-3-[(2-trimethylsilanyl-ethoxy)methyl]-3*H*-benzoimidazol-5-yl}-methanol)
   Massenspektrum (ESI⁺): m/z = 293 [M+H]⁺
(2) (2,3,8-Trimethyl-chinoxalin-6-yl)-methanol
   R_{f}-Wert: 0.45 (Kieselgel, Petrolether/Essigester = 1:2)
   Massenspektrum (ESI⁺): m/z = 203 [M+H]⁺
(3) (8-Methyl-chinoxalin-6-yl)-methanol
   R_{f}-Wert: 0.18 (Kieselgel, Cyclohexan/Essigester = 1:1)
   Massenspektrum (ESI⁺): m/z = 175 [M+H]⁺
(4) (E)-3-Pentafluorphenyl-2-propen-1-ol
   (Durchführung mit Diisobutylaluminiumhydrid in Toluol)
   Massenspektrum (EI): m/z = 224 [M]⁺
(5) (E)-3-(2-Trifluormethyl-phenyl)-2-propen-1-ol
   (Durchführung mit Diisobutylaluminiumhydrid in Toluol)
(6) (E)-3-(3-Trifluormethyl-phenyl)-2-propen-1-ol
   (Durchführung mit Diisobutylaluminiumhydrid in Toluol)
   Massenspektrum (EI): m/z = 202 [M]⁺
(7) (E)-3-(4-Trifluormethyl-phenyl)-2-propen-1-ol
   (Durchführung mit Diisobutylaluminiumhydrid in Toluol)

### Beispiel XXVIII

### 4-Hydroxymethyl-2-[(2-trimethylsilanyl-ethoxy)methyl]-2H-phthalazin-1-on

hergestellt durch Behandeln von 4-Oxo-3-[(2-trimethylsilanyl-ethoxy)methyl]-3,4-dihydro-phthalazin-1-carbonsäure-methylester mit Natriumborhydrid in Tetrahydrofuran bei 40°C.
R_{f}-Wert: 0.55 (Kieselgel, Cyclohexan/Essigester 1:1)
Massenspektrum (ESI⁺): m/z = 307 [M+H]⁺

### Analog Beispiel XXVIII werden folgende Verbindungen erhalten:

(1) (3,4-Dimethyl-isochinolin-1-yl)-methanol
   (Durchführung mit Lithiumborhydrid in Tetrahydrofuran)
   R_{f}-Wert: 0.35 (Kieselgel, Petrolether/Essigester = 2:1)
   Massenspektrum (ESI⁺): m/z = 188 [M+H]⁺
(2) (3-Methyl-imidazo[1,2-a]pyridin-2-yl)-methanol
   (Durchführung mit Lithiumborhydrid in Tetrahydrofuran)
   R_{f}-Wert: 0.48 (Kieselgel, Methylenchlorid/Methanol/konz. wässriges Ammoniak = 90:10:1)
   Massenspektrum (ESI⁺): m/z = 163 [M+H]⁺
(3) (3,4-Dimethyl-6,7-dihydro-5H-[2]pyrindin-1-yl)-methanol
   (Durchführung mit Lithiumborhydrid in Tetrahydrofuran)
   R_{f}-Wert: 0.40 (Aluminiumoxid, Petrolether/Essigester = 1:1)
   Massenspektrum (ESI⁺): m/z = 178 [M+H]⁺
(4) (3,4-Dimethyl-5,6,7,8-tetrahydro-isochinolin-1-yl)-methanol
   (Durchführung mit Lithiumborhydrid in Tetrahydrofuran)
   R_{f}-Wert: 0.45 (Aluminiumoxid, Petrolether/Essigester = 3:1)
   Massenspektrum (ESI⁺): m/z = 192 [M+H]⁺
(5) 6-Hydroxymethyl-1,2,3,4-tetrahydro-phenanthridin
   (Durchführung mit Lithiumborhydrid in Tetrahydrofuran bei Raumtemperatur)
   R_{f}-Wert: 0.40 (Kieselgel, Petrolether/Essigester = 2:1)
   Massenspektrum (ESI⁺): m/z = 214 [M+H]⁺

### Beispiel XXIX

### 4-Oxo-3-[(2-trimethylsilanyl-ethoxy)methyl]-3,4-dihydro-phthalazin-1-carbonsäure-methylester

hergestellt durch Umsetzung von 4-Oxo-3,4-dihydro-phthalazin-1-carbonsäure-methylester mit (2-Trimethylsilanyl-ethoxy)methylchlorid in Gegenwart von Hünigbase in Methylenchlorid bei Raumtemperatur.
R_{f}-Wert: 0.75 (Kieselgel, Cyclohexan/Essigester 6:4)
Massenspektrum (ESI⁺): m/z = 335 [M+H]⁺

### Analog Beispiel XXIX werden folgende Verbindungen erhalten:

(1) 7-Acetyl-3-[(2-trimethylsilanyl-ethoxy)methyl]-3H-benzooxazol-2-on
   Massenspektrum (ESI⁺): m/z = 308 [M+H]⁺
(2) 4-Acetyl-3-[(2-trimethylsilanyl-ethoxy)methyl]-3H-benzooxazol-2-on
   R_{f}-Wert: 0.87 (Kieselgel, Methylenchlorid/Methanol = 99:1)
(3) 4-Acetyl-1,3-bis-[(2-trimethylsilanyl-ethoxy)methyl]-1,3-dihydro-benzoimidazol-2-on
   (Durchführung mit Kalium-tert.-butylat in N,N-Dimethylformamid)
   R_{f}-Wert: 0.90 (Kieselgel, Petrolether/Essigester = 1:1)
   Massenspektrum (ESI⁺): m/z = 437 [M+H]⁺
(4) 6-Methyl-1-[(2-trimethylsilanyl-ethoxy)methyl]-1*H*-chinolin-2-on
   R_{f}-Wert: 0.78 (Kieselgel, Petrolether/Essigester = 1:1)
   Massenspektrum (ESI⁺): m/z = 290 [M+H]⁺
(5) {2-Methyl-1-[(2-trimethylsilanyl-ethoxy)methyl]-1*H*-benzoimidazol-5-yl}-carbonsäuremethylester (im Gemisch mit {2-Methyl-3-[(2-trimethylsilanyl-ethoxy)methyl]-3*H*-benzoimidazol-5-yl}-carbonsäuremethylester)
   Massenspektrum (ESI⁺): m/z = 321 [M+H]⁺

### Beispiel XXX

### 1-[2-(3-Methansulfonyl-phenyl)-2-oxo-ethyl]-3-methyl-7-(3-methyl-2-buten-1-yl)-8-[3-(tert.-butyloxycarbonylamino-yl]-xanthin

Zu 500 mg 1-[2-(3-Methylsulfanyl-phenyl)-2-oxo-ethyl]-3-methyl-7-(3-methyl-2-buten-1-yl)-8-[3-(tert.-butyloxycarbonylamino)-piperidin-1-yl]-xanthin in 5 ml Methylenchlorid werden 0.22 ml einer 35 %igen Wasserstoffperoxid-Lösung und 20 mg Natriumwolframat gegeben. Das Reaktionsgemisch wird über Nacht bei Raumtemperatur gerührt, dann wird 1 ml Methanol zugesetzt. Nach weiteren 48 Stunden werden nochmals 1.5 ml 35 %ige Wasserstoffperoxid-Lösung, eine Spatelspitze Natriumwolframat und zwei Tropfen Wasser zugegeben. Am nächsten Morgen ist die Oxidation laut Dünnschichtchromatographie vollständig und das Reaktionsgemisch wird mit 50 ml Methylenchlorid verdünnt und zweimal mit je 30 ml 10 %-iger Natriumthiosulfatlösung gewaschen. Die organische Phase wird über Magnesiumsulfat getrocknet und eingeengt, wobei ein zähes Harz zurückbleibt, welches ohne weitere Reinigung weiter umgesetzt wird.
Ausbeute: 530 mg (100 % der Theorie)
R_{f}-Wert: 0.72 (Kieselgel, Essigester)
Massenspektrum (ESI⁺): m/z = 629 [M+H]⁺

### Beispiel XXXI

### 1-[2-(3-Carboxy-phenyl)-2-oxo-ethyl]-3-methyl-7-(3-methyl-2-buten-1-yl)-8-[3-(tert.-butyloxycarbonylamino)-piperidin-1-yl]-xanthin

hergestellt durch Behandeln von 1-[2-(3-Methoxycarbonyl-phenyl)-2-oxo-ethyl]-3-methyl-7-(3-methyl-2-buten-1-yl)-8-[3-(tert.-butyloxycarbonylamino)-piperidin-1-yl]-xanthin mit 3 M Natronlauge in Methanol bei Raumtemperatur.
R_{f}-Wert: 0.34 (Kieselgel, Methylenchlorid/Methanol = 9:1)
Massenspektrum (ESI⁺): m/z = 595 [M+H]⁺

### Analog Beispiel XXXI werden folgende Verbindungen erhalten:

(1) 1-[2-(2-Carboxy-phenyl)-2-oxo-ethyl]-3-methyl-7-(3-methyl-2-buten-1-yl)-8-[3-(tert.-butyloxycarbonylamino)-piperidin-1-yl]-xanthin
   R_{f}-Wert: 0.49 (Kieselgel, Methylenchlorid/Methanol = 9:1)
(2) 1-[2-(2-Carboxymethoxy-phenyl)-2-oxo-ethyl]-3-methyl-7-(2-butin-1-yl)-8-[3-(tert.-butyloxycarbonylamino)-piperidin-1-yl]-xanthin
   (Durchführung mit 4 M Kalilauge in Tetrahydrofuran)
   Massenspektrum (ESI⁺): m/z = 609 [M+H]⁺
(3) 1-[2-(2-Carboxymethylamino-phenyl)-2-oxo-ethyl]-3-methyl-7-((E)-2-buten-1-yl)-8-[(*R*)-3-(tert.-butyloxycarbonylamino)-piperidin-1-yl]-xanthin
   (Durchführung mit 4 M Kalilauge in Tetrahydrofuran)
   R_{f}-Wert: 0.65 (Kieselgel, Cyclohexan/Essigester = 3:7)
   Massenspektrum (ESI⁺): m/z = 610 [M+H]⁺
(4) 1-Carboxymethyl-3-methyl-7-(2-butin-1-yl)-8-[3-(tert.-butyloxycarbonylamino)-piperidin-1-yl]-xanthin
   Massenspektrum (ESI⁺): m/z = 475 [M+H]⁺

### Beispiel XXXII

### 1-{2-[3-(Methylaminocarbonyl)-phenyl]-2-oxo-ethyl}-3-methyl-7-(3-methyl-2-buten-1-yl)-8-[3-(tert.-butyloxycarbonylamino)-piperidin-1-yl]-xanthin

Ein Gemisch aus 190 mg 1-[2-(3-Carboxy-phenyl)-2-oxo-ethyl]-3-methyl-7-(3-methyl-2-buten-1-yl)-8-[3-(tert.-butyloxycarbonylamino)-piperidin-1-yl]-xanthin, 43 µl einer 40 %igen wässrigen Methylamin-Lösung, 103 mg O-(Benzotriazol-1-yl)-N,N,N',N'-tetramethyluroniumtetrafluoroborat, 43 mg N-Hydroxybenzotriazol und 45 µl Triethylamin in 3 ml Tetrahydrofuran wird acht Stunden bei Raumtemperatur gerührt. Zur Aufarbeitung wird das Reaktionsgemisch mit Essigester verdünnt und mit Wasser, 10 %-iger Zitronensäure-Lösung, 10 %-iger Kaliumcarbonat-Lösung und gesättigter Natriumchlorid-Lösung gewaschen. Die organische Phase wird eingeengt und über eine Kieselgel-Säule mit Methylenchlorid/Methanol (98:2 auf 80:20) als Laufmittel chromatographiert.
Ausbeute: 173 mg (89 % der Theorie)
R_{f}-Wert: 0.30 (Kieselgel, Methylenchlorid/Methanol = 20:1)
Massenspektrum (ESI⁺): m/z = 608 [M+H]⁺

### Analog Beispiel XXXII werden folgende Verbindungen erhalten:

(1) 1-{2-[3-(Dimethylaminocarbonyl)-phenyl]-2-oxo-ethyl}-3-methyl-7-(3-methyl-2-buten-1-yl)-8-[3-(tert.-butyloxycarbonylamino)-piperidin-1-yl]-xanthin
   R_{f}-Wert: 0.28 (Kieselgel, Methylenchlorid/Methanol = 20:1)
   Massenspektrum (ESI⁺): m/z = 622 [M+H]⁺
(2) 1-{2-[3-(Morpholin-4-yl-carbonyl)-phenyl]-2-oxo-ethyl}-3-methyl-7-(3-methyl-2-buten-1-yl)-8-[3-(tert.-butyloxycarbonylamino)-piperidin-1-yl]-xanthin
   R_{f}-Wert: 0.30 (Kieselgel, Methylenchlorid/Methanol = 20:1)
   Massenspektrum (ESI⁺): m/z = 664 [M+H]⁺
(3) 1-{2-[2-(Dimethylaminocarbonyl)-phenyl]-2-oxo-ethyl}-3-methyl-7-(3-methyl-2-buten-1-yl)-8-[3-(tert.-butyloxycarbonylamino)-piperidin-1-yl]-xanthin
   R_{f}-Wert: 0.30 (Kieselgel, Methylenchlorid/Methanol = 20:1)
   Massenspektrum (ESI⁺): m/z = 622 [M+H]⁺
(4) 1-{2-[2-(Morpholin-4-yl-carbonyl)-phenyl]-2-oxo-ethyl}-3-methyl-7-(3-methyl-2-buten-1-yl)-8-[3-(tert.-butyloxycarbonylamino)-piperidin-1-yl]-xanthin
   R_{f}-Wert: 0.30 (Kieselgel, Methylenchlorid/Methanol = 20:1)
   Massenspektrum (ESI⁺): m/z = 664 [M+H]⁺
(5) 1-(2-{2-[(Isopropylaminocarbonyl)methoxy]-phenyl}-2-oxo-ethyl)-3-methyl-7-(2-butin-1-yl)-8-[3-(tert.-butyloxycarbonylamino)-piperidin-1-yl]-xanthin
   (Durchführung mit Hünigbase in N,N-Dimethylformamid)
   Massenspektrum (ESI⁺): m/z = 650 [M+H]⁺
(6) 1-(2-{2-[(Ethylaminocarbonyl)methoxy]-phenyl}-2-oxo-ethyl)-3-methyl-7-(2-butin-1-yl)-8-[3-(tert.-butyloxycarbonylamino)-piperidin-1-yl]-xanthin
   (Durchführung mit Hünigbase in N,N-Dimethylformamid)
   Massenspektrum (ESI⁺): m/z = 636 [M+H]⁺
(7) 1-(2-{2-[2-Oxo-2-(pyrrolidin-1-yl)-ethoxy]-phenyl}-2-oxo-ethyl)-3-methyl-7-(2-butin-1-yl)-8-[3-(tert.-butyloxycarbonylamino)-piperidin-1-yl]-xanthin
   (Durchführung mit Hünigbase in N,N-Dimethylformamid)
   Massenspektrum (ESI⁺): m/z = 662 [M+H]⁺
(8) 1-(2-{2-[2-(Morpholin-4-yl)-2-oxo-ethoxy]-phenyl}-2-oxo-ethyl)-3-methyl-7-(2-butin-1-yl)-8-[3-(tert.-butyloxycarbonylamino)-piperidin-1-yl]-xanthin
   (Durchführung mit Hünigbase in N,N-Dimethylformamid)
   Massenspektrum (ESI⁺): m/z = 678 [M+H]⁺
(9) 1-(2-{2-[(Methylaminocarbonyl)methylamino]-phenyl}-2-oxo-ethyl)-3-methyl-7-((E)-2-buten-1-yl)-8-[(R)-3-(tert.-butyloxycarbonylamino)-piperidin-1-yl]-xanthin
   R_{f}-Wert: 0.40 (Kieselgel, Cyclohexan/Essigester/Methanol = 5:4:1)
   Massenspektrum (ESI⁺): m/z = 623 [M+H]⁺
(10) 1-[(2-Amino-phenylaminocarbonyl)methyl]-3-methyl-7-(2-butin-1-yl)-8-[3-(tert.-butyloxycarbonylamino)-piperidin-1-yl]-xanthin
   R_{f}-Wert: 0.40 (Kieselgel, Cyclohexan/Essigester/Methanol = 5:4:1)
   Massenspektrum (ESI⁺): m/z = 565 [M+H]⁺

### Beispiel XXXIII

### 1-Chlormethyl-4-methyl-isochinolin-hydrochlorid

hergestellt durch Behandeln von (4-Methyl-isochinolin-1-yl)-methanol mit Thionylchlorid in Methylenchlorid.
R_{f}-Wert: 0.76 (Kieselgel, Methylenchlorid/Methanol/konz. wässriges Ammoniak = 95:5:0.1)
Massenspektrum (ESI⁺): m/z = 192, 194 [M+H]⁺

### Analog Beispiel XXXIII werden folgende Verbindungen erhalten:

(1) 1-Chlormethyl-3,4-dimethyl-isochinolin-hydrochlorid
   R_{f}-Wert: 0.65 (Kieselgel, Petrolether/Essigester = 2:1)
   Massenspektrum (ESI⁺): m/z = 206, 208 [M+H]⁺
(2) 5-Chlormethyl-8-methoxy-chinolin-hydrochlorid
   Massenspektrum (ESI⁺): m/z = 208, 210 [M+H]⁺
(3) 8-Chlormethyl-5-methoxy-chinolin-hydrochlorid
   Massenspektrum (EI): m/z = 207, 209 [M]⁺
(4) 2-Chlormethyl-3-methyl-imidazo[1,2-a]pyridin-hydrochlorid
   R_{f}-Wert: 0.55 (Kieselgel, Methylenchlorid/Methanol/konz. wässriges Ammoniak = 90:10:1)
   Massenspektrum (ESI⁺): m/z = 181, 183 [M+H]⁺
(5) 8-Chlormethyl-5-methoxy-isochinolin-hydrochlorid
   Massenspektrum (ESI⁺): m/z = 208, 210 [M+H]⁺
(6) 1-Chlormethyl-3,4-dimethyl-6,7-dihydro-5H-[2]pyrindin-hydrochlorid
   R_{f}-Wert: 0.50 (Aluminiumoxid, Petrolether/Essigester = 10:1)
   Massenspektrum (ESI⁺): m/z = 196, 198 [M+H]⁺
(7) 1-Chlormethyl-3,4-dimethyl-5,6,7,8-tetrahydro-isochinolin-hydrochlorid
   R_{f}-Wert: 0.50 (Aluminiumoxid, Petrolether/Essigester = 10:1)
   Massenspektrum (ESI⁺): m/z = 210, 212 [M+H]⁺
(8) 6-Chlormethyl-2,3,8-trimethyl-chinoxalin-hydrochlorid
   Massenspektrum (ESI⁺): m/z = 221, 223 [M+H]⁺
(9) 6-Chlormethyl-8-methyl-chinoxalin-hydrochlorid
   Massenspektrum (ESI⁺): m/z = 193,195 [M+H]⁺
(10) 6-Chlormethyl-1,2,3,4-tetrahydro-phenanthridin-hydrochlorid
   R_{f}-Wert: 0.50 (Kieselgel, Petrolether/Essigester = 5:1)
   Massenspektrum (ESI⁺): m/z = 232, 234 [M+H]⁺

### Beispiel XXXIV

### 1-[(4-Oxo-3,4-dihydro-chinazolin-2-yl)methyl]-3-methyl-7-(2-butin-1-yl)-8-[3-(tert.-butyloxycarbonylamino)-piperidin-1-yl]-xanthin

Zu einer Lösung aus 428 mg 1-Cyanomethyl-3-methyl-7-(2-butin-1-yl)-8-[3-(tert.-butyloxycarbonylamino)-piperidin-1-yl]-xanthin in 3 ml Methanol werden bei Raumtemperatur 0.5 ml einer 1 M Natriummethanolat-Lösung in Methanol getropft. Nach etwa 20 Minuten wird die entstandene dicke Suspension im Wasserbad leicht erwärmt und mit 2 ml Methanol verdünnt. Sobald die Umsetzung zum Iminoester laut Dünnschichtchromatographie vollständig ist, wird das Reaktionsgemisch mit 0.5 ml 1 M Eisessig-Lösung in Methanol neutralisiert und mit einer Lösung aus 130 mg Anthranilsäure in 2 ml Methanol versetzt. Durch leichtes Erwärmen entsteht eine klare Lösung, welche 2.5 Stunden bei Raumtemperatur gerührt wird. Anschließend wird das Reaktionsgemisch etwa 3.5 Stunden unter leichtem Rückfluß erhitzt. Nach Stehen über Nacht bei Raumtemperatur wird das Methanol abdestilliert und der Rückstand mit kaltem Wasser verrührt, abgesaugt und getrocknet. Das Rohprodukt wird in 5 ml Methanol suspendiert, leicht erwärmt und nach Abkühlung abgesaugt, mit Methanol nachgewaschen und im Exsikkator getrocknet.
Ausbeute: 302 mg (56 % der Theorie)
R_{f}-Wert: 0.55 (Kieselgel, Essigester)
Massenspektrum (ESI⁺): m/z = 575 [M+H]⁺

### Analog Beispiel XXXV werden folgende Verbindungen erhalten:

(1) (4-Difluormethoxy-naphthalin-1-yl)-methanol
   R_{f}-Wert: 0.33 (Kieselgel, Cyclohexan/Essigester = 6:4)
   Massenspektrum (ESI⁻): m/z = 223 [M-H]⁻

### Beispiel XXXV

### (4-Dimethylamino-naphthalin-1-yl)-methanol

hergestellt durch Reduktion von 4-Dimethylamino-naphthalin-1-carbaldehyd mit Natriumborhydrid in wässrigem Tetrahydrofuran.
R_{f}-Wert: 0.67 (Kieselgel, Cyclohexan/Essigester = 1:1)

### Beispiel XXXVI

### 2-Brom-1-(2,3-dihydro-benzo[1,4]dioxin-5-yl)-ethanon

hergestellt durch Bromierung von 1-(2,3-Dihydro-benzo[1,4]dioxin-5-yl)-ethanon in Methylenchlorid unter leichter Eisbad-Kühlung. Die als Nebenprodukt entstehende Dibrom-Verbindung wird säulenchromatographisch abgetrennt.
Massenspektrum (ESI⁺): m/z = 257, 259 [M+H]⁺
R_{f}-Wert: 0.92 (Kieselgel, Methylenchlorid)

### Analog Beispiel XXXVI werden folgende Verbindungen erhalten:

(1) 7-(2-Brom-acetyl)-3-methyl-3H-benzooxazol-2-on
   (Bromierung wird in Dioxan bei 40°C durchgeführt; das Produkt ist mit ca. 20 % Dibrom-Verbindung verunreinigt)
   R_{f}-Wert: 0.44 (Kieselgel, Petrolether/Essigester = 1:1)
   Massenspektrum (ESI⁺): m/z = 270, 272 [M+H]⁺
(2) 1-Benzo[1,3]dioxol-4-yl-2-brom-ethanon
   Massenspektrum (ESI⁺): m/z = 243, 245 [M+H]⁺
   R_{f}-Wert: 0.94 (Kieselgel, Methylenchlorid)
(3) 2-[2-(2-Brom-acetyl)-phenoxy]-*N*-ethyl-acetamid
   (Bromierung wird mit Kupfer(II)bromid in Dioxan durchgeführt)
   Massenspektrum (ESI⁺): m/z = 300, 302 [M+H]⁺
(4) 4-(2-Brom-acetyl)-3-methyl-3*H*-benzooxazol-2-on
   R_{f}-Wert: 0.67 (Kieselgel, Methylenchlorid/Methanol = 99:1)
   Massenspektrum (ESI⁺): m/z = 270, 272 [M+H]⁺
(5) 2-[2-(2-Brom-acetyl)-phenoxy]-*N*-methyl-acetamid
   Massenspektrum (ESI⁺): m/z = 386, 388 [M+H]⁺
(6) 7-(2-Brom-acetyl)-3-[(2-trimethylsilanyl-ethoxy)methyl]-3*H*-benzooxazol-2-on
   R_{f}-Wert: 0.84 (Kieselgel, Methylenchlorid/Methanol = 99:1)
   Massenspektrum (ESI⁺): m/z = 384, 386 [M+H]⁺
(7) 4-(2-Brom-acetyl)-1,3-dimethyl-1,3-dihydro-benzoimidazol-2-on
   R_{f}-Wert: 0.38 (Kieselgel, Essigester/Petrolether = 1:1)
   Massenspektrum (ESI⁺): m/z = 283, 285 [M+H]⁺
(8) 4-(2-Brom-acetyl)-3-[(2-trimethylsilanyl-ethoxy)methyl]-3*H*-benzooxazol-2-on
   R_{f}-Wert: 0.82 (Kieselgel, Methylenchlorid/Methanol = 99:1)
(9) 4-(2-Brom-acetyl)-1-ethoxycarbonyl-3-methyl-1,3-dihydro-benzoimidazo1-2-on
   R_{f}-Wert: 0.39 (Kieselgel, Petrolether/Essigester = 2:1)
   Massenspektrum (ESI⁺): m/z = 341, 343 [M+H]⁺
(10) 2-Brom-1-(2,2-difluor-benzo[1,3]dioxol-4-yl)-ethanon
   Massenspektrum (ESI⁻): m/z = 277, 279 [M-H]⁻

### Beispiel XXXVII

### (2.3-Dihydro-benzo[1,4]dioxin-5-yl)-ethanon

hergestellt durch Umsetzung von 1-(2,3-Dihydroxy-phenyl)-ethanon mit 1,2-Dibromethan in Gegenwart von Kaliumcarbonat in N,N-Dimethylformamid bei 100°C.
R_{f}-Wert: 0.43 (Kieselgel, Essigester/Petrolether = 1:4)
Massenspektrum (ESI⁺): m/z = 179 [M+H]⁺

### Beispiel XXXVIII

### 1-[(3-Methyl-4-oxo-3,4-dihydro-chinazolin-2-yl)methyl]-3-methyl-7-(2-butin-1-yl)-8-[3-(tert.-butyloxycarbonylamino)-piperidin-1-yl]-xanthin

hergestellt durch Umsetzung von 1-[(4-Oxo-3,4-dihydro-chinazolin-2-yl)methyl]-3-methyl-7-(2-butin-1-yl)-8-[3-(tert.-butyloxycarbonylamino)-piperidin-1-yl]-xanthin mit Methyliodid in Gegenwart von Kaliumcarbonat in N,N-Dimethylformamid bei Raumtemperatur.
R_{f}-Wert: 0.50 (Kieselgel, Essigester)
Massenspektrum (ESI⁺): m/z = 589 [M+H]⁺

### Analog Beispiel XXXVIII werden folgende Verbindungen erhalten:

(1) 7-Acetyl-3-methyl-3*H*-benzooxazol-2-on
   (Die Methylierung erfolgt in Gegenwart von Natriumcarbonat in Methanol)
   R_{f}-Wert: 0.46 (Kieselgel, Petrolether/Essigester = 1:1)
   Massenspektrum (ESI⁺): m/z = 192 [M+H]⁺
(2) 4-Acetyl-3-methyl-3*H*-benzooxazol-2-on
   (Die Methylierung erfolgt in Gegenwart von Natriumcarbonat in Methanol unter Rückfluß)
   R_{f}-Wert: 0.67 (Kieselgel, Methylenchlorid/Methanol = 99:1)
   Massenspektrum (ESI⁺): m/z = 192 [M+H]⁺
(3) 4-Acetyl-1,3-dimethyl-1,3-dihydro-benzoimidazol-2-on
   (Durchführung in Gegenwart von Kalium-tert.-butylat)
   R_{f}-Wert: 0.40 (Kieselgel, Essigester/Petrolether = 2:1)
   Massenspektrum (ESI⁺): m/z = 205 [M+H]⁺
(4) 4-Acetyl-1-ethoxycarbonyl-3-methyl-1,3-dihydro-benzoimidazol-2-on
   R_{f}-Wert: 0.23 (Kieselgel, Petrolether/Essigester = 2:1)
   Massenspektrum (ESI⁺): m/z = 263 [M+H]⁺
(5) 1-[(1-Methyl-1*H*-benzoimidazol-2-yl)methyl]-3-methyl-7-(2-butin-1-yl)-8-[3-(tert.-butyloxycarbonylamino)-piperidin-1-yl]-xanthin
   Massenspektrum (ESI⁺): m/z = 561 [M+H]⁺
(6) 1-{[1-(2-Cyano-ethyl)-1H-benzoimidazol-2-yl]methyl}-3-methyl-7-(2-butin-1-yl)-8-[3-(tert.-butyloxycarbonylamino)-piperidin-1-yl]-xanthin
   R_{f}-Wert: 0.50 (Kieselgel, Cyclohexan/Essigester/Methanol = 5:4:1)
   Massenspektrum (ESI⁺): m/z = 600 [M+H]⁺
(7) 1-({1-[(Methylaminocarbonyl)methyl]-1H-benzoimidazol-2-yl}methyl)-3-methyl-7-(2-butin-1-yl)-8-[3-(tert.-butyloxycarbonylamino)-piperidin-1-yl]-xanthin
   R_{f}-Wert: 0.45 (Kieselgel, Cyclohexan/Essigester/Methanol = 5:4:1)
   Massenspektrum (ESI⁺): m/z = 618 [M+H]⁺
(8) 1-[(1-Benzyl-1H-benzoimidazol-2-yl)methyl]-3-methyl-7-(2-butin-1-yl)-8-[3-(tert.-butyloxycarbonylamino)-piperidin-1-yl]-xanthin
   R_{f}-Wert: 0.50 (Kieselgel, Cyclohexan/Essigester/Methanol = 5:4:1)
   Massenspektrum (ESI⁺): m/z = 637 [M+H]⁺

### Beispiel XXXIX

### 1-[2-(2-Cyanomethylamino-phenyl)-2-oxo-ethyl]-3-methyl-7-(3-methyl-2-buten-1-yl)-8-[3-(tert.-butyloxycarbonylamino)-piperidin-1-yl]-xanthin

hergestellt durch Umsetzung von 1-[2-(2-Amino-phenyl)-2-oxo-ethyl]-3-methyl-7-(3-methyl-2-buten-1-yl)-8-[3-(tert.-butyloxycarbonylamino)-piperidin-1-yl]-xanthin mit Paraformaldehyd und Kaliumcyanid in Gegenwart von Zinkchlorid in Eisessig bei 40°C.
R_{f}-Wert: 0.45 (Kieselgel, Cyclohexan/Essigester = 3:7)
Massenspektrum (ESI⁺): m/z = 605 [M+H]⁺

### Beispiel XL

### 1-[2-(2-Amino-phenyl)-2-oxo-ethyl]-3-methyl-7-(2-butin-1-yl)-8-[(S)-3-(tert.-butyloxycarbonylamino)-piperidin-1-yl]-xanthin

hergestellt durch Reduktion von 1-[2-(2-Nitro-phenyl)-2-oxo-ethyl]-3-methyl-7-(2-butin-1-yl)-8-[(*S*)-3-(tert.-butyloxycarbonylamino)-piperidin-1-yl]-xanthin mit Natriumdithionit in einem Gemisch aus Methylglykol und Wasser (2:1) bei 100°C.
R_{f}-Wert: 0.34 (Kieselgel, Methylenchlorid/Methanol = 95:5)

### Analog Beispiel XL werden folgende Verbindungen erhalten:

(1) 1-[2-(2-Amino-phenyl)-2-oxo-ethyl]-3-methyl-7-(2-butin-1-yl)-8-[(*R*)-3-(tert.-butyloxycarbonylamino)-piperidin-1-yl]-xanthin
   R_{f}-Wert: 0.50 (Kieselgel, Cyclohexan/Essigester = 4:6)

### Beispiel XLI

### 2-Chlormethyl-4-methyl-chinazolin

hergestellt durch Behandlung von 2.95 g 2-Chlormethyl-4-methyl-chinazolin-3-oxid mit 6 ml Phosphortrichlorid in 150 ml Chloroform unter Rückfluß.
Ausbeute: 1.75 g (57 % der Theorie)
R_{f}-Wert: 0.81 (Kieselgel, Methylenchlorid/Methanol = 95:5)
Massenspektrum (ESI⁺): m/z = 193, 195 [M+H]⁺

### Beispiel XLII

### 2-Chlormethyl-4-dimethylamino-chinazolin

Zu 500 mg 4-Chlor-2-chlormethyl-chinazolin in 5 ml Tetrahydrofuran wird unter Eisbad-Kühlung eine frisch hergestellte Lösung aus 202 mg Dimethylamin in 3.2 ml Tetrahydrofuran getropft. Anschließend wird das Reaktionsgemisch noch 3.5 Stunden unter Eisbad-Kühlung und weitere 30 Minuten bei Raumtemperatur gerührt. Dann wird das Lösungsmittel am Rotationsverdampfer schonend abdestilliert und der Rückstand in Methylenchlorid aufgenommen. Die Lösung wird mit gesättigter Natriumhydrogencarbonat-Lösung und mit Wasser gewaschen, über Magnesiumsulfat getrocknet und eingeengt. Der feste Rückstand wird mit wenig tert.-Butylmethylether verrührt, abgesaugt, mit Petrolether nachgewaschen und im Vakuum getrocknet.
Ausbeute: 323 mg (62 % der Theorie)
R_{f}-Wert: 0.60 (Kieselgel, Cyclohexan/Essigester = 1:1)
Massenspektrum (ESI⁺): m/z = 222, 224 [M+H]⁺

### Analog Beispiel XLII werden folgende Verbindungen erhalten:

(1) 2-Chlormethyl-4-(morpholin-4-yl)-chinazolin
   R_{f}-Wert: 0.50 (Kieselgel, Cyclohexan/Essigester = 1:1)
   Massenspektrum (ESI⁺): m/z = 264, 266 [M+H]⁺
(2) 2-Chlormethyl-4-(piperidin-1-yl)-chinazolin
   R_{f}-Wert: 0.70 (Kieselgel, Cyclohexan/Essigester = 1:1)
   Massenspektrum (ESI⁺): m/z = 262, 264 [M+H]⁺
(3) 4-[4-(tert.-Butyloxycarbonyl)-piperazin-1-yl]-2-chlormethyl-chinazolin
   R_{f}-Wert: 0.57 (Kieselgel, Cyclohexan/Essigester = 1:1)
   Massenspektrum (ESI⁺): m/z = 363, 365 [M+H]⁺
(4) 2-Chlormethyl-4-(pyrrolidin-1-yl)-chinazolin
   R_{f}-Wert: 0.50 (Kieselgel, Cyclohexan/Essigester = 1:1)
   Massenspektrum (ESI⁺): m/z = 248, 250 [M+H]⁺
(5) 2-Chlormethyl-4-ethoxy-chinazolin
   (Die Umsetzung erfolgt mit Natriumethanolat in Ethanol bei Raumtemperatur.)
   R_{f}-Wert: 0.50 (Kieselgel, Cyclohexan/Essigester = 3:1)
   Massenspektrum (ESI⁺): m/z = 223, 225 [M+H]⁺
(6) 2-Chlormethyl-4-isopropyloxy-chinazolin
   (Die Umsetzung erfolgt mit Natriumisopropanolat in Isopropanol bei Raumtemperatur.)
   R_{f}-Wert: 0.70 (Kieselgel, Cyclohexan/Essigester = 3:1)
   Massenspektrum (ESI⁺): m/z = 237, 239 [M+H]⁺
(7) 2-Chlormethyl-4-phenyloxy-chinazolin
   (Die Umsetzung erfolgt mit Natriumhydrid und Phenol in Tetrahydrofuran bei Raumtemperatur.)
   R_{f}-Wert: 0.65 (Kieselgel, Cyclohexan/Essigester = 3:1)
   Massenspektrum (ESI⁺): m/z = 271, 273 [M+H]⁺

### Beispiel XLIII

### 1-(2-{2-[(Ethoxycarbonyl)methylamino]-phenyl}-2-oxo-ethyl)-3-methyl-7-((E)-2-buten-1-yl)-8-[3-(tert.-butyloxycarbonylamino)-piperidin-1-yl]-xanthin

Zu 531 mg 1-[2-(2-Amino-phenyl)-2-oxo-ethyl]-3-methyl-7-((E)-2-buten-1-yl)-8-[3-(tert.-butyloxycarbonylamino)-piperidin-1-yl]-xanthin und 10 mg Methyltrioxorhenium in 4.5 ml Toluol wird bei Raumtemperatur unter Argon-Atmosphäre eine Lösung aus 110 µL Diazoessigsäureethylester in 0.5 ml Toluol getropft. Das Reaktionsgemisch wird 15 Stunden bei Raumtemperatur gerührt. Dann werden nochmals ca. 5 mg Methyltrioxorhenium und 20 µL Diazoessigsäureethylester zugegeben und das Reaktionsgemisch wird zwei Stunden auf 50°C erwärmt. Nach Abkühlung auf Raumtemperatur werden erneut 5 mg Methyltrioxorhenium und 20 µL Diazoessigsäureethylester zugegeben. Nach weiteren 16 Stunden bei Raumteperatur wird das Reaktionsgemisch mit 5 ml konz. wässrigem Ammoniak versetzt, durchgeschüttelt und auf eine Extrelut-Packung gegeben. Nach 15 min wird mit 200 ml Methylenchlorid nachgespült. Die Methylenchlorid-Lösung wird eingeengt und über eine Kieselgelsäule mit Cyclohexan/Essigester/Isopropanol (8:2:0 auf 8:1:1) als Laufmittel chromatographiert.
Ausbeute: 220 mg (36 % der Theorie)
R_{f}-Wert: 0.40 (Kieselgel, Cyclohexan/Essigester = 1:1)
Massenspektrum (ESI⁺): m/z = 638 [M+H]⁺

### Beispiel XLIV

### 1-[(2-Cyano-benzofuran-3-yl)methyl]-3-methyl-7-(2-butin-1-yl)-8-[3-(tert.-butyloxycarbonylamino)-piperidin-1-yl]-xanthin

Ein Gemisch aus 215 mg 1-{2-[2-Cyanomethoxy-phenyl]-2-oxo-ethyl}-3-methyl-7-(2-butin-1-yl)-8-[3-(tert.-butyloxycarbonylamino)-piperidin-1-yl]-xanthin und 244 mg Cäsiumcarbonat in 4 ml N,N-Dimethylformamid wird zwei Stunden bei 50°C, dann weitere drei Stunden bei 70°C gerührt. Zur Aufarbeitung wird das Reaktionsgemisch mit Wasser versetzt und der entstandene Niederschlag abgesaugt und getrocknet.
Ausbeute: 130 mg (62 % der Theorie)
Massenspektrum (ESI⁺): m/z = 572 [M+H]⁺

### Beispiel XLV

### 1-[2-(3-Methyl-2-oxo-2,3-dihydro-1H-benzoimidazol-4-yl)-2-oxo-ethyl]-3-methyl-7-(2-butin-1-yl)-8-[3-(tert.-butyloxycarbonylamino)-piperidin-1-yl]-xanthin

hergestellt durch Behandeln von 1-[2-(1-Ethoxycarbonyl-3-methyl-2-oxo-2,3-dihydro-1*H*-benzoimidazol-4-yl)-2-oxo-ethyl]-3-methyl-7-(2-butin-1-yl)-8-[3-(tert.-butyloxy-carbonylamino)-piperidin-1-yl]-xanthin mit 1 N Natronlauge in Methanol bei Raumtemperatur.
R_{f}-Wert: 0.36 (Kieselgel, Essigester)
Massenspektrum (ESI⁺): m/z = 605 [M+H]⁺

### Beispiel XLVI

### 4-Acetyl-1-ethoxycarbonyl-1,3-dihydro-benzoimidazol-2-on

Zu 1.50 g 1-(2,3-Diamino-phenyl)-ethanon in 75 ml Methylenchlorid werden 5.29 g Diethyldicarbonat und 611 mg Dimethylaminopyridin gegeben. Das Reaktionsgemisch wird drei Stunden bei Raumtemperatur gerührt, dann werden nochmals 100 mg Dimethylaminopyridin und 1ml Diethyldicarbonat zugegeben und weitere 20 Stunden bei Raumtemperatur gerührt. Zur Aufarbeitung wird das Reaktionsgemisch mit Methylenchlorid verdünnt, mit 2 N Zitronensäure-Lösung sowie gesättigter Natriumhydrogencarbonat-Lösung und gesättigter Natriumchlorid-Lösung gewaschen, über Magnesiumsulfat getrocknet und eingeengt. Der Rückstand wird über eine Kieselgelsäule mit Petrolether/Essigester (3:1 auf 1:2) als Laufmittel chromatographiert. Das gewünschte Produkt wird mit wenig tert.-Butylmethylether verrührt, abgesaugt, mit wenig Essigester und tert.-Butylmethylether nachgewaschen und getrocknet.
Ausbeute: 900 mg (36 % der Theorie)
R_{f}-Wert: 0.15 (Kieselgel, Petrolether/Essigester = 2:1)
Massenspektrum (ESI⁺): m/z = 249 [M+H]⁺

### Beispiel XLVII

### 1-[(4-Amino-chinazolin-2-yl)methyl]-3-methyl-7-(2-butin-1-yl)-8-[3-(tert.-butyloxy-carbonylamino)-piperidin-1-yl]-xanthin

Zu einem Gemisch aus 17 mg Kalium-tert.-butylat in 10 ml Methanol werden 501 mg 1-Cyanomethyl-3-methyl-7-(2-butin-1-yl)-8-[3-(tert.-butyloxycarbonylamino)-piperidin-1-yl]-xanthin gegeben. Nach kurzem Erwärmen unter Rühren ensteht eine klare Lösung und nach etwa 20 Minuten ist das Nitril laut Dünnschichtchromatogramm weitgehend zum Iminoester umgesetzt. Nun werden 206 mg 2-Amino-benzamidinhydrochlorid zugegeben und das Reaktionsgemisch wird vier Stunden unter Rückfluß erhitzt. Nach Abkühlung auf Raumtemperatur wird der entstandene Niederschlag abgesaugt, mit Methanol gewaschen und getrocknet.
Ausbeute: 143 mg (23 % der Theorie)
R_{f}-Wert: 0.15 (Kieselgel, Essigester)
Massenspektrum (ESI⁺): m/z = 574 [M+H]⁺

### Beispiel XLVIII

### 1-(2-Phenyl-2-oxo-ethyl)-3-methyl-7-((Z)-2-buten-1-yl)-8-[3-(tert.-butyloxycarbonylamino)-piperidin-1-yl-xanthin

150 mg 1-(2-Phenyl-2-oxo-ethyl)-3-methyl-7-(2-butin-1-yl)-8-[3-(tert.-butyloxy-carbonylamino)-piperidin-1-yl]-xanthin werden in einem Gemisch aus 5 ml Tetrahydrofuran und 5 ml Methanol in Gegenwart von 30 mg 5 %igem Palladium auf Aktivkohle (vergiftet mit Chinolin) bei Raumtemperatur hydriert, bis die berechnete Menge Wasserstoff aufgenommen ist. Dann wird eine Spatelspitze Aktivkohle zugegeben und abgesaugt. Das Filtrat wird eingeengt und das Rohprodukt chromatographisch über eine Kieselgelsäule mit Cyclohexan/Essigester (7:3 auf 4:6) gereinigt.
Ausbeute: 120 mg (85 % der Theorie)
R_{f}-Wert: 0.40 (Kieselgel, Cyclohexan/Essigester = 4:6)
Massenspektrum (ESI⁺): m/z = 537 [M+H]⁺

### Beispiel XLIX

### 8-Hvdroxvmethyl-5-methoxy-chinolin

Zu einer Lösung aus 640 mg 8-Hydroxymethyl-chinolin-5-ol in N,N-Dimethylformamid werden unter Eisbad-Kühlung portionsweise 148 mg Natriumhydrid (ca. 60 %ig in Mineralöl) gegeben und das Reaktionsgemisch wird langsam auf Raumtemperatur erwärmt. Nachdem die Gasentwicklung beendet ist werden unter Eisbad-Kühlung 230 µl Methyliodid zugetropft, anschließend wird das Reaktionsgemisch noch ca. zwei Stunden bei Raumtemperatur gerührt. Zur Aufarbeitung wird es auf Eiswasser gegossen, mit Natriumchlorid gesättigt und mit einem Gemisch aus Diethylether und Essigester extrahiert. Die vereinigten Extrakte werden mit gesättigter Natriumchlorid-Lösung gewaschen, über Magnesiumsulfat getrocknet und eingeengt. Der Kolbenrückstand wird mit Petrolether verrieben und der Überstand abdekantiert. Das Rohprodukt wird über eine Kieselgelsäule mit Essigester als Laufmittel gereinigt.
Ausbeute: 470 mg (68 % der Theorie)
R_{f}-Wert: 0.70 (Kieselgel, Essigester)
Massenspektrum (ESI⁺): m/z = 190 [M+H]⁺

### Analog Beispiel XLIX werden folgende Verbindungen erhalten:

(1) 8-Hydroxymethyl-5-methoxy-isochinolin
   R_{f}-Wert: 0.40 (Kieselgel, Methylenchlorid/Methanol = 10:1)
   Massenspektrum (ESI⁺): m/z = 190 [M+H]⁺

### Beispiel L

### 8-Hydroxymethyl-chinolin-5-ol

3.40 g Chinolin-5-ol wird unter Eisbad-Kühlung mit 8 ml konz. Salzsäure und 8 ml 37 %iger Formalin-Lösung versetzt. Dann wird etwa zwei Stunden Chlorwasserstoffgas durch das Reaktionsgemisch geleitet, wobei die Temperatur langsam ansteigt. Das Reaktionsgemisch wird über Nacht zunächst noch unter Eisbad-Kühlung, dann bei Raumtemperatur gerührt und anschließend im Vakuum eingeengt. Der Kolbenrückstand wird in Wasser aufgenommen, mit Diethylether überschichtet und unter Eisbad-Kühlung und starkem Rühren mit verdünnter Ammoniak-Lösung auf pH 10 eingestellt. Nach weiteren zwei Stunden kräftigem Rühren bei Raumtemperatur wird die organische Phase abgetrennt und die wässrige Phase mit Diethylether extrahiert. Die vereinigten organischen Phasen werden mit Wasser und gesättigter Natriumchlorid-Lösung gewaschen, über Magnesiumsulfat getrocknet und eingeengt. Der Kolbenrückstand wird über eine Kieselgelsäule mit Methylenchlorid/Methanol (20:1) als Laufmittel chromatographiert.
Ausbeute: 660 mg (16 % der Theorie)
Massenspektrum (ESI⁺): m/z = 176 [M+H]⁺

### Analog Beispiel L werden folgende Verbindungen erhalten:

(1) 8-Hydroxymethyl-isochinolin-5-ol
   R_{f}-Wert: 0.50 (Kieselgel, Methylenchlorid/Methanol = 5:1)
   Massenspektrum (ESI⁺): m/z = 176 [M+H]⁺

### Beispiel LI

### 1-[(2-Cyclopropyl-chinazolin-4-yl)methyl]-3-methyl-7-[(1-cyclopenten-1-yl)methyl]-8-[3-(tert.-butyloxycarbonylamino)-piperidin-1-yl]-xanthin

Ein Gemisch aus 250 mg 1-(2-{2-[(Cyclopropylcarbonyl)amino]-phenyl}-2-oxo-ethyl)-3-methyl-7-[(1-cyclopenten-1-yl)methyl]-8-[3-(tert.-butyloxycarbonylamino)-piperidin-1-yl]-xanthin und 7.5 ml ethanolischer Ammoniaklösung (6 M) wird in einer Bombe sieben Stunden auf 150 °C erhitzt. Zur Aufarbeitung wird das Reaktionsgemisch eingeengt und über eine Kieselgelsäule mit Methylenchlorid/Methanol (100:0 auf 70:30) als Laufmittel chromatographiert. Die verunreinigte Produktfraktion wird eingeengt und nochmals über eine Reversed Phase-HPLC-Säule mit Wasser/ Acetonitril/Trifluoressigsäure (65:15:0.08 auf 0:100:0.1) als Laufmittel gereinigt. Die Produktfraktionen werden eingeengt, mit verdünnter Natronlauge alkalisch gestellt und mit Methylenchlorid extrahiert. Die vereinigten Extrakte werden über Magnesiumsulfat getrocknet und eingeengt.
Ausbeute: 40 mg (14 % der Theorie)
R_{f}-Wert: 0.40 (Kieselgel, Methylenchlorid /Essigester = 1:1)
Massenspektrum (ESI⁺): m/z = 627 [M+H]⁺

### Beispiel LII

### 4-(2-Brom-acetyl)-1,3-bis-[(2-trimethylsilanyl-ethoxy)methyl]-1,3-dihydro-benzo-imidazol-2-on

Zu 420 mg 4-Acetyl-1,3-bis-[(2-trimethylsilanyl-ethoxy)methyl]-1,3-dihydro-benzoimidazol-2-on in 5 ml Tetrahydrofuran werden unter Argonatmosphäre 520 mg 2-Pyrrolidinon-hydrotribromid und 89 mg 2-Pyrrolidinon gegeben. Das Reaktionsgemisch wird zwei Stunden unter Rückfluß erhitzt und anschließend noch warm abgesaugt. Der Filterkuchen wird mit Tetrahydrofuran nachgewaschen und das Filtrat eingeengt, wobei 660 mg eines gelbbraunen Feststoffes zurückbleiben. Dieser wird mit wenig Methanol verrührt, abgesaugt, mit etwas Methanol nachgewaschen und getrocknet. Das Rohprodukt wird ohne weitere Reinigung weiter umgesetzt.
Ausbeute: 430 mg (87 % der Theorie)
R_{f}-Wert: 0.23 (Kieselgel, Petrolether/Essigester = 9:1)
Massenspektrum (EI): m/z = 514, 516 [M]⁺

### Analog Beispiel LII werden folgende Verbindungen erhalten:

(1) 7-(2-Brom-acetyl)-1-(tert.-butyloxycarbonyl)-1*H*-indol
   R_{f}-Wert: 0.33 (Kieselgel, Petrolether/Essigester = 9:1)
   Massenspektrum (ESI⁺): m/z = 338, 340 [M+H]⁺
(2) 2-Brom-1-(3-isopropyloxy-phenyl)-ethanon
   (Durchführung mit Phenyltrimethylammoniumtribromid in Methylenchlorid)
   R_{f}-Wert: 0.39 (Kieselgel, Cyclohexan/Essigester = 9:1)
(3) 2-Brom-1-(3-difluormethoxy-phenyl)-ethanon
   (Durchführung mit Phenyltrimethylammoniumtribromid in Methylenchlorid)
   R_{f}-Wert: 0.24 (Reversed Phase DC-Fertigplatte (E. Merck), Acetonitril/Wasser/ Trifluoressigsäure = 50:50:1)

### Beispiel LIII

### 3-Methyl-imidazo[1,2-a]pyridin-2-carbonsäure-methylester

Ein Gemisch aus 1.91 g 2-Aminopyridin und 4.40 g 3-Brom-2-oxo-buttersäuremethylester in 40 ml Ethanol wird sechs Stunden unter Rückfluß erhitzt und anschließend 2 Tage bei Raumtemperatur stehengelassen. Das Lösungsmittel wird am Rotationsverdampfer abdestilliert und das Rohprodukt chromatographisch über eine Kieselgelsäule mit Methylenchlorid/Methanol/metanolischer Ammoniaklösung (95:4:1 auf 90:9:1) als Laufmittel gereinigt. Als Nebenprodukt werden 560 mg des Ethylesters isoliert.
Ausbeute: 2.09 g (54 % der Theorie)
R_{f}-Wert: 0.20 (Kieselgel, Methylenchlorid/Essigester = 1:1)
Massenspektrum (ESI⁺): m/z = 191 [M+H]⁺

### Beispiel LIV

### 2-Chlormethyl-4-isopropyl-chinazolin

Durch eine Lösung aus 2.86 g 1-(2-Amino-phenyl)-2-methyl-propan-1-on und 1.33 ml Chloracetonitril in 14 ml Dioxan wird unter Rühren bei Raumtemperatur ca. fünf Stunden trockenes Chlorwasserstoffgas geleitet. Anschließend wird das Dioxan im Wasserstrahlvakuum größtenteils abdestilliert. Der honigartige Rückstand wird mit Eiswasser versetzt und die entstandene Suspension unter Eisbad-Kühlung mit gesättigter Kaliumcarbonatlösung alkalisch gestellt. Der Niederschlag wird abgesaugt, mit Wasser gewaschen und getrocknet. Das Rohprodukt wird chromatographisch über eine Kieselgelsäule mit Petrolether/Methylenchlorid (8:2 auf 0:1) als Laufmittel gereinigt.
Ausbeute: 1.80 g (58 % der Theorie)
R_{f}-Wert: 0.30 (Kieselgel, Methylenchlorid/Petrolether = 1:1)
Massenspektrum (ESI⁺): m/z = 221, 223 [M+H]⁺

### Beispiel LV

### 1-Chlormethyl-3-trifluormethyl-3,4-dihvdro-isochinolin

Zu 4.00 g Polyphosphorsäure werden 530 mg *N*-(1-Benzyl-2,2,2-trifluor-ethyl)-2-chlor-acetamid (hergestellt durch Umsetzung von 1-Benzyl-2,2,2-trifluoro-ethylamin mit Chloracetylchlorid in Gegenwart von Triethylamin) und 0.74 ml Posphoroxychlorid gegeben. Das zähe Gemisch wird 1.5 Stunden bei 130°C gerührt. Zur Aufarbeitung wird das abgekühlte Reaktionsgemisch und mit Eiswasser versetzt, zehn Minuten kräftig gerührt und abgesaugt. Der Filterkuchen wird in Essigester gelöst und die Lösung über Magnesiumsulfat getrocknet und eingeengt, wobei ein weißer Feststoff zurückbleibt.
Ausbeute: 415 mg (84 % der Theorie)
R_{f}-Wert: 0.55 (Aluminiumoxid, Petrolether/Essigester = 10:1)
Massenspektrum (ESI⁺): m/z = 248, 250 [M+H]⁺

### Analog Beispiel LV wird folgende Verbindung erhalten:

(1) 1-Methyl-3-trifluormethyl-3,4-dihydro-isochinolin
   (Das Ausgangsmaterial *N*-(1-Benzyl-2,2,2-trifluoro-ethyl)-acetamid wird durch Umsetzung von 1-Benzyl-2,2,2-trifluoro-ethylamin mit Acetanhydrid erhalten.)

### Beispiel LVI

### 3-Brommethyl-1-(1-cyano-1-methyl-ethyl)-isochinolin

Ein Gemisch aus 375 mg 1-(1-Cyano-1-methyl-ethyl)-3-methyl-isochinolin und 321 mg N-Bromsuccinimid in 5 ml Tetrachlorkohlenstoff wird mit einer Spatelspitze 2,2-Azoisobuttersäuredinitril versetzt und etwa sechs Stunden unter Rückfluß erhitzt. Das abgekühlte Reaktionsgemisch wird filtriert und eingeengt. Der Kolbenrückstand wird ohne weitere Reinigung weiter umgesetzt.
R_{f}-Wert: 0.70 (Kieselgel, Cyclohexan/Essigester = 3:1)

### Analog Beispiel LVI werden folgende Verbindungen erhalten:

(1) 6-Brommethyl-1-[(2-trimethylsilanyl-ethoxy)methyl]-1*H*-chinolin-2-on
(2) 1-Brommethyl-4-brom-3-methoxy-isochinolin
(3) 2-Brommethyl-[1,5]naphthyridin
   Massenspektrum (ESI+): m/z = 223, 225 [M+H]+
(4) 5-Brommethyl-[1,6]naphthyridin
   R_{f}-Wert: 0.48 (Kieselgel, Essigester/Methanol = 98:2)
(5) 7-Brommethyl-5-phenyl-chinoxalin
   R_{f}-Wert: 0.85 (Kieselgel, Methylenchlorid/Methanol = 95:5)
   Massenspektrum (ESI⁺): m/z = 299, 301 [M+H]⁺
(6) 4-Brommethyl-[1,5]naphthyridin
   R_{f}-Wert: 0.56 (Kieselgel, Methylenchlorid/Essigester = 7:3)
   Massenspektrum (ESI⁺): m/z = 223, 225 [M+H]⁺
(7) 1-Brommethyl-3-trifluormethyl-isochinolin
   Massenspektrum (ESI⁺): m/z = 290, 292 [M+H]⁺
(8) 1-Brommethyl-3-difluormethyl-isochinolin
   Massenspektrum (ESI⁺): m/z = 272, 274 [M+H]⁺
(9) 1-Brommethyl-4-chlor-3-methoxy-isochinolin

### Beispiel LVII

### 1-(1-Cyano-1-methyl-ethyl)-3-methyl-isochinolin

Zu 1.60 g 3-Methyl-isochinolin-N-oxid in 30 ml Toluol werden 3.30 g 2,2-Azoisobuttersäuredinitril gegeben. Das Reaktionsgemisch wird sechs Stunden bei 85°C gerührt und anschließend zwei Tage bei Raumtemperatur stehengelassen. Zur Aufarbeitung wird das Reaktionsgemisch mit 20%iger Salzsäure extrahiert. Die vereinigten wässrigen Phasen werden mit Methylenchlorid verdünnt, mit gesättigter Kaliumcarbonatlösung unter Eisbad-Kühlung alkalisch gestellt und mit Methylenchlorid extrahiert. Die vereinigten Methylenchlorid-Extrakte werden über Magnesiumsulfat getrocknet und eingeengt. Der Rückstand wird über eine Kieselgelsäule mit Cyclohexan als Laufmittel chromatographiert.
Ausbeute: 375 mg (18 % der Theorie)
Massenspektrum (ESI⁺): m/z = 211 [M+H]⁺
R_{f}-Wert: 0.75 (Kieselgel, Cyclohexan/Essigester = 3:1)

### Beispiel LVIII

### 1-(2-Cyanoimino-2-phenyl-ethyl)-3-methyl-7-(2-butin-1-yl)-8-[3-(tert.-butyloxycar-bonylamino)-piperidin-1-yl]-xanthin (E/Z-Gemisch)

Zu 244 mg 1-(2-Phenyl-2-oxo-ethyl)-3-methyl-7-(2-butin-1-yl)-8-[3-(tert.-butyloxy-carbonylamino)-piperidin-1-yl]-xanthin in 7 ml Methylenchlorid werden 0.48 ml einer 1M Lösung von Titantetrachlorid in Methylenchlorid getropft. Anschließend werden 88 µl 1,3-Bis(trimethylsilyl)carbodiimid zugegeben und das Gemisch wird vier Stunden bei Raumtemperatur gerührt. Zur Aufarbeitung wird das Reaktionsgemisch mit Methylenchlorid verdünnt und auf Eiswasser gegossen. Die organische Phase wird mit 0.5 N Citronensäure gewaschen, über Magnesiumsulfat getrocknet und eingeengt. Das Rohprodukt wird chromatographisch über eine Kieselgelsäule mit Methylenchlorid/Methanol (98:2 auf 95:5) als Laufmittel gereinigt.
Ausbeute: 206 mg (97 % der Theorie)
Massenspektrum (ESI⁻): m/z = 557 [M-H]⁻
R_{f}-Wert: 0.16 (Kieselgel, Cyclohexan/Essigester = 1:1)

### Beispiel LIX

### 1-[(1H-Benzoimidazol-2-yl)methyl]-3-methyl-7-(2-butin-1-yl)-8-[3-(tert.-butyloxy-carbonylamino)-piperidin-1-yl]-xanthin

350 mg 1-[(2-Amino-phenylaminocarbonyl)methyl]-3-methyl-7-(2-butin-1-yl)-8-[3-(tert.-butyloxycarbonylamino)-piperidin-1-yl]-xanthin werden in 3 ml Eisessig zwei Stunden unter Rückfluß erhitzt. Anschließend wird das Reaktionsgemisch eingeengt, der Kolbenrückstand mit 5 ml 1 M Natronlauge versetzt und mit Methylenchlorid gewaschen. Dann wird die wässrige Phase wird mit 1 M Salzsäure angesäuert und mit Methylenchlorid extrahiert. Die vereinigten Extrakte werden eingeengt und über eine Kieselgelsäule mit Cyclohexan/Essigester/Methanol (6:4:0 auf 5:4:1) als Laufmittel chromatographiert.
Ausbeute: 250 mg (74 % der Theorie)
Massenspektrum (ESI⁺): m/z = 547 [M+H]⁺

### Beispiel LX

### 3,4-Dimethyl-6,7-dihydro-5H-[2]pyrindin-1-carbonsäure-ethylester

Hergestellt durch Behandlung von 1.16 g 3,4-Dimethyl-4a-(pyrrolidin-1-yl)-5,6,7,7a-tetrahydro-4a*H*-[2]pyrindin-1-carbonsäure-ethylester mit 1.08 g 70 %iger 3-Chlorperbenzoesäure in 50 ml Methylenchlorid bei Raumtemperatur.
Ausbeute: 850 mg (97 % der Theorie)
R_{f}-Wert: 0.30 (Aluminiumoxid, Petrolether/Essigester = 5:1)
Massenspektrum (ESI⁺): m/z = 220 [M+H]⁺

### Analog Beispiel LX werden folgende Verbindungen erhalten:

(1) 3,4-Dimethyl-5,6,7,8-tetrahydro-isochinolin-1-carbonsäure-ethylester
   R_{f}-Wert: 0.35 (Aluminiumoxid, Petrolether/Essigester = 5:1)
   Massenspektrum (ESI⁺): m/z = 234 [M+H]⁺

### Beispiel LXI

### 3,4-Dimethyl-4a-(pyrrolidin-1-yl)-5,6,7,7a-tetrahydro-4aH-[2]pyrindin-1-carbonsäure-ethylester

Hergestellt durch Umsetzung von 2.50 g 5,6-Dimethyl-[1,2,4]triazin-3-carbonsäure-ethylester mit 2.74 g 1-(Cyclopenten-1-yl)-pyrrolidin in 25 ml Chloroform bei Raumtemperatur.
Ausbeute: 3.00 g (75 % der Theorie)
R_{f}-Wert: 0.60 (Aluminiumoxid, Petrolether/Essigester = 5:1)
Massenspektrum (ESI⁺): m/z = 291 [M+H]⁺

### Analog Beispiel LXI werden folgende Verbindungen erhalten:

(1) 3,4-Dimethyl-4a-(pyrrolidin-1-yl)-4a,5,6,7,8,8a-hexahydro-isochinolin-1-carbonsäure-ethylester
   R_{f}-Wert: 0.60 (Aluminiumoxid, Petrolether/Essigester = 5:1)
   Massenspektrum (ESI⁺): m/z = 305 [M+H]⁺

### Beispiel LXII

### 2,3,8-Trimethyl-chinoxalin-6-carbonsäure-methylester

Hergestellt durch Umsetzung von 1.60 g 3,4-Diamino-5-methyl-benzoesäure-methylester mit 0.86 ml Diacetyl in einem Gemisch aus Wasser und Ethanol unter Rückfluß.
Ausbeute: 1.53 g (80 % der Theorie)
R_{f}-Wert: 0.63 (Kieselgel, Cyclohexan/Essigester = 1:1)
Massenspektrum (ESI⁺): m/z = 231 [M+H]⁺

### Analog Beispiel LXII werden folgende Verbindungen erhalten:

(1) 8-Methyl-chinoxalin-6-carbonsäure-methylester
   (Umsetzung erfolgt mit Glyoxal in Wasser.)
   R_{f}-Wert: 0.55 (Kieselgel, Cyclohexan/Essigester = 1:1)
   Massenspektrum (ESI⁺): m/z = 203 [M+H]⁺
(2) 5-Brom-7-methyl-chinoxalin
   (Umsetzung erfolgt mit Glyoxal in einem Wasser/Ethanol-Gemisch.)
   R_{f}-Wert: 0.75 (Kieselgel, Methylenchlorid/Methanol = 95:5)
   Massenspektrum (ESI⁺): m/z = 223, 225 [M+H]⁺

### Beispiel LXIII

### 3,4-Diamino-5-methyl-benzoesäure-methylester

Hergestellt durch Reduktion von 3-Nitro-4-amino-5-methyl-benzoesäure-methylester bei einem Wasserstoffpartialdruck von 50 psi in Gegenwart von Raney-Nickel in Methanol bei Raumtemperatur.
R_{f}-Wert: 0.40 (Kieselgel, tert.-Butylmethylether)

### Beispiel LXIV

### 3-Nitro-4-amino-5-methyl-benzoesäure-methylester

Hergestellt durch Behandlung von 3-Nitro-4-acetylamino-5-methyl-benzoesäure mit Chlorwasserstoffgas in Methanol bei Raumtemperatur und anschließendes Erhitzen unter Rückfluß.
Massenspektrum (ESI⁺): m/z = 211 [M+H]⁺
R_{f}-Wert: 0.75 (Kieselgel, tert.-Butylmethylether/Essigsäure = 99:1)

### Beispiel LXV

### 1-(2-Phenyl-2-oxo-ethyl)-3-methyl-7-((E)-1-buten-1-yl)-8-brom-xanthin

Zu 290 mg 1-(2-Phenyl-2-oxo-ethyl)-3-methyl-7-(1-phenylsulfanyl-butyl)-8-brom-xanthin in 6 ml Hexafluorisopropanol werden 0.13 ml 35 %ige Wasserstoffperoxid-Lösung gegeben. Das Reaktionsgemisch wird eine Stunde bei Raumtemperatur gerührt, mit Methylenchlorid verdünnt und mit Natriumthiosulfat-Lösung gewaschen. Die organische Phase wird über Magnesiumsulfat getrocknet und eingeengt. Der Kolbenrückstand wird in 6 ml Toluol aufgenommen und acht Stunden unter Rückfluß erhitzt. Anschließend wird das Toluol im Vakuum abdestilliert und das Rohprodukt über eine Kieselgelsäule mit Methylenchlorid/Methanol (100:0 auf 95:5) als Laufmittel gereinigt.
Ausbeute: 104 mg (45 % der Theorie)
R_{f}-Wert: 0.61 (Kieselgel, Methylenchlorid/Methanol = 95:5)
Massenspektrum (ESI⁺): m/z = 417, 419 [M+H]⁺

### Analog Beispiel LXV werden folgende Verbindungen erhalten:

(1) 3-Methyl-7-(3-methyl-1-buten-1-yl)-8-brom-xanthin
   R_{f}-Wert: 0.24 (Kieselgel, Methylenchlorid/Methanol = 95:5)
   Massenspektrum (ESI⁺): m/z = 313, 315 [M+H]⁺

### Beispiel LXVI

### 1-Methansulfonyloxymethyl-4-difluormethoxy-naphthalin

Hergestellt durch Umsetzung von (4-Difluormethoxy-naphthalin-1-yl)-methanol mit Methansulfonsäurechlorid in Methylenchlorid in Gegenwart von Triethylamin.

### Analog Beispiel LXVI werden folgende Verbindungen erhalten:

(1) (E)-1-Methansulfonyloxy-3-(2-nitro-phenyl)-2-propen
(2) (E)-1-Methansulfonyloxy-3-pentafluorphenyl-2-propen
(3) (E)-1-Methansulfonyloxy-3-(2-trifluormethyl-phenyl)-2-propen
(4) (E)-1-Methansulfonyloxy-3-(3-trifluormethyl-phenyl)-2-propen
(5) (E)-1-Methansulfonyloxy-3-(4-trifluormethyl-phenyl)-2-propen

### Beispiel LXVII

### 7-Methyl-5-phenyl-chinoxalin

Ein Gemisch aus 400 mg 5-Brom-7-methyl-chinoxalin, 244 mg Phenylboronsäure und 100 mg Tetrakis(triphenylphosphin)palladium in 12 ml Dioxan, 4 ml Methanol und 3.6 ml 1 M wässriger Natriumcarbonatlösung wird unter einer Argonatmosphäre drei Stunden unter Rückfluss erhitzt. Anschließend wird das Reaktionsgemisch eingeengt und der Rückstand zwischen Essigester und Wasser verteilt. Die Essigesterphase wird abgetrennt, über Magnesiumsulfat getrocknet und eingeengt. Das Rohprodukt wird chromatographisch über eine Kieselgelsäule mit Cyclohexan/Essigester (85:15 auf 70:30) als Laufmittel gereinigt.
Ausbeute: 390 mg (66% der Theorie)
R_{f}-Wert: 0.36 (Kieselgel, Petrolether/Essigester = 5:1)
Massenspektrum (ESI⁺): m/z = 221 [M+H]⁺

### Beispiel LXVIII

### 1-Methyl-3-trifluormethyl-isochinolin

Hergestellt durch Behandlung von 905 mg 1-Chlormethyl-3-trifluoromethyl-3,4-dihydro-isochinolin mit 420 mg Kalium-tert.-butylat in 10 ml Tetrahydrofuran bei Raumtemperatur.
Ausbeute: 755 mg (98% der Theorie)
Massenspektrum (ESI⁺): m/z = 212 [M+H]⁺

### Analog Beispiel LXVIII werden folgende Verbindungen erhalten:

(1) 1-Methyl-3-difluormethyl-isochinolin
   (Hergestellt aus 1-Methyl-3-trifluormethyl-3,4-dihydro-isochinolin)
   Massenspektrum (ESI⁺): m/z = 194 [M+H]⁺

### Beispiel LXIX

### 4-Chlor-3-methoxy-1-methyl-isochinolin

Hergestellt durch Behandlung von 3-Methoxy-1-methyl-isochinolin mit Sulfurylchlorid in Methylenchlorid.
R_{f}-Wert: 0.30 (Kieselgel, Cyclohexan)
Massenspektrum (ESI⁺): m/z = 208, 210 [M+H]⁺

### Beispiel LXX

### 3-Cyclopropyl-8-brom-xanthin

Hergestellt durch Umsetzung von 3-Cyclopropyl-xanthin mit Brom in Gegenwart Kaliumcarbonat in Acetonitril bei 60°C.
R_{f}-Wert: 0.65 (Reversed Phase DC-Fertigplatte (E. Merck), Acetonitril/Wasser/ Trifluoressigsäure = 50:50:1)
Massenspektrum (ESI⁺): m/z = 271, 273 [M+H]⁺

### Beispiel LXXI

### 1,2,3,4-Tetrahydro-phenanthridin-6-yl-carbonsäure-ethylester

Analog dem von Gonsalves et al. beschriebenen Verfahren (Tetrahedron 1992, 48, 6821) wird eine Lösung aus 3.90 g 5,6,7,8-Tetrahydro-benzo[1,2,4]triazin-3-carbonsäure-ethylester (Sagi et al., Heterocycles 1989, 29, 2253) in 20 ml Dioxan zum Rückfluß erhitzt. Dann werden mit Hilfe von zwei Tropftrichtern simultan 8.22 g Anthranilsäure und 7.02 g Isoamylnitrit, jeweils in 20 ml Dioxan gelöst, innerhalb von 25 Minuten zugetropft. Das Reaktionsgemisch wird noch weitere 30 Minuten unter Rückfluß erhitzt. Zur Aufarbeitung wird die abgekühlte tiefbraune Reaktionslösung mit 150 ml Diethylether verdünnt, mit 100 ml 2 N Natronlauge und mit Wasser gewaschen, über Magnesiumsulfat getrocknet und eingeengt. Der braune, ölige Kolbenrückstand wird über eine Kieselgelsäule mit Essigester/Petrolether (20:80 auf 50:50) als Laufmittel chromatographiert. Das erhaltene Produkt ist noch etwas verunreinigt, wird aber ohne weitere Reinigung weiter umgesetzt.
Ausbeute: 380 mg (8 % der Theorie)
R_{f}-Wert: 0.55 (Kieselgel, Petrolether/Essigester = 2:1)
Massenspektrum (ESI⁺): m/z = 256 [M+H]⁺

### Herstellung der Endverbindungen:

### Beispiel 1

### 1,3-Dimethyl-7-(2,6-dicyano-benzyl)-8-(3-amino-piperidin-1-yl)-xanthin

Zu einem Gemisch aus 298 mg 1,3-Dimethyl-7-(2,6-dicyano-benzyl)-8-brom-xanthin und 420 mg Kaliumcarbonat in 9 ml N,N-Dimethylformamid werden 129 mg 3-Aminopiperidin-dihydrochlorid gegeben. Das Reaktionsgemisch wird drei Stunden bei 80°C gerührt. Zur Aufarbeitung wird das Gemisch mit Methylenchlorid verdünnt und mit gesättigter Natriumchlorid-Lösung gewaschen. Die organische Phase wird über Magnesiumsulfat getrocknet und eingeengt. Das Rohprodukt wird chromatographisch über eine Kieselgel-Säule mit Methylenchlorid/Methanol/konz. methanolischem Ammoniak (95:5:1 auf 80:20:1) als Laufmittel gereinigt.
Ausbeute: 43 mg (14 % der Theorie)
R_{f}-Wert: 0.67 (Kieselgel, Methylenchlorid/Methanol/konz. wässriges Ammoniak = 80:20:1)
Massenspektrum (ESI⁺): m/z = 419 [M+H]⁺

### Analog Beispiel 1 werden folgende Verbindungen erhalten:

(1) 1-(2-Cyano-ethyl)-3-methyl-7-(2-cyano-benzyl)-8-(3-amino-piperidin-1-yl)-xanthin
   R_{f}-Wert: 0.35 (Kieselgel, Methylenchlorid/Methanol = 9:1)
   Massenspektrum (ESI⁺): m/z = 433 [M+H]⁺

### Beispiel 2

### 1-(2-{2-[(Ethoxycarbonyl)methoxy]-phenyl}-2-oxo-ethyl)-3-methyl-7-(3-methyl-2-buten-1-yl)-8-(3-amino-piperidin-1-yl)-xanthin

Eine Lösung aus 209 mg 1-(2-{2-[(Ethoxycarbonyl)methoxy]-phenyl}-2-oxo-ethyl)-3-methyl-7-(3-methyl-2-buten-1-yl)-8-[3-(tert.-butyloxycarbonylamino)-piperidin-1-yl]-xanthin in 4 ml Methylenchlorid wird mit 1 ml Trifluoressigsäure versetzt und eine halbe Stunde bei Raumtemperatur gerührt. Zur Aufarbeitung wird das Reaktionsgemisch mit Methylenchlorid verdünnt und mit gesättigter Kaliumcarbonat-Lösung gewaschen. Die organische Phase wird getrocknet, eingeengt und über eine Kieselgel-Säule mit Methylenchlorid/Methanol (1:0 auf 4:1) als Laufmittel chromatographiert.
Ausbeute: 153 mg (87 % der Theorie)
Massenspektrum (ESI⁺): m/z = 553 [M+H]⁺

### Analog Beispiel 2 werden folgende Verbindungen erhalten:

(1) 1-(2-{2-[(Aminocarbonyl)methoxy]-phenyl}-2-oxo-ethyl)-3-methyl-7-(3-methyl-2-buten-1-yl)-8-(3-amino-piperid-in-1-yl)-xanthin
   Massenspektrum (ESI⁺): m/z = 524 [M+H]⁺
(2) 1-(2-{3-[(Methansulfinyl)methoxy]-phenyl}-2-oxo-ethyl)-3-methyl-7-(3-methyl-2-buten-1-yl)-8-(3-amino-piperidin-1-yl)-xanthin
   R_{f}-Wert: 0.58 (Reversed Phase DC-Fertigplatte (E. Merck), Acetonitril/Wasser/ Trifluoressigsäure = 100:100:0.1)
   Massenspektrum (ESI⁺): m/z = 543 [M+H]⁺
(3) 1-(1-Methyl-2-oxo-2-phenyl-ethyl)-3-methyl-7-(3-methyl-2-buten-1-yl)-8-(3-amino-piperidin-1-yl)-xanthin
   Massenspektrum (ESI⁺): m/z = 465 [M+H]⁺
(4) 1-(2-Phenoxy-ethyl)-3-methyl-7-(2-cyano-benzyl)-8-(3-amino-piperidin-1-yl)-xanthin
   R_{f}-Wert: 0.50 (Kieselgel, Methylenchlorid/Methanol = 9:1)
   Massenspektrum (ESI⁺): m/z = 500 [M+H]⁺
(5) 1-(2-Phenyl-2-oxo-ethyl)-7-(3-methyl-2-buten-1-yl)-8-(3-amino-piperidin-1-yl)-xanthin
   R_{f}-Wert: 0.58 (Kieselgel, Methylenchlorid/Methanol/konz. wässriges Ammoniak = 80:20:1)
   Massenspektrum (ESI⁻): m/z = 435 [M-H]⁻
(6) 1-(2-{3-[(Ethoxycarbonyl)methoxy]-phenyl}-2-oxo-ethyl)-3-methyl-7-(3-methyl-2-buten-1-yl)-8-(3-amino-piperidin-1-yl)-xanthin
   Massenspektrum (ESI⁺): m/z = 553 [M+H]⁺
(7) 1-(2-{2-[(Methylaminocarbonyl)methoxy]-phenyl}-2-oxo-ethyl)-3-methyl-7-(3-methyl-2-buten-1-yl)-8-(3-amino-piperidin-1-yl)-xanthin
   Massenspektrum (ESI⁺): m/z = 538 [M+H]⁺
(8) 1-(2-{2-[(Dimethylaminocarbonyl)methoxy]-phenyl}-2-oxo-ethyl)-3-methyl-7-(3-methyl-2-buten-1-yl)-8-(3-amino-piperidin-1-yl)-xanthin
   Massenspektrum (ESI⁺): m/z = 552 [M+H]⁺
(9) 1-(2-Methoxy-ethyl)-3-methyl-7-(2-cyano-benzyl)-8-(3-amino-piperidin-1-yl)-xanthin
   R_{f}-Wert: 0.40 (Kieselgel, Methylenchlorid/Methanol = 9:1)
   Massenspektrum (ESI⁺): m/z = 438 [M+H]⁺
(10) 1-Methyl-3-[(methoxycarbonyl)methyl]-7-(2-cyano-benzyl)-8-(3-amino-piperidin-1-yl)-xanthin
   R_{f}-Wert: 0.40 (Kieselgel, Methylenchlorid/Methanol = 9:1)
   Massenspektrum (ESI⁺): m/z = 452 [M+H]⁺
(11) 1-Methyl-3-cyanomethyl-7-(2-cyano-benzyl)-8-(3-amino-piperidin-1-yl)-xanthin
   (Durchführung mit isopropanolischer Salzsäure 5-6 M in Methylenchlorid)
   R_{f}-Wert: 0.20 (Kieselgel, Methylenchlorid/Methanol = 9:1)
   Massenspektrum (ESI⁺): m/z = 419 [M+H]⁺
(12) 1-Methyl-3-(2-propin-1-yl)-7-(2-cyano-benzyl)-8-(3-amino-piperidin-1-yl)-xanthin
   R_{f}-Wert: 0.40 (Kieselgel, Methylenchlorid/Methanol = 9:1)
   Massenspektrum (ESI⁺): m/z = 418 [M+H]⁺
(13) 1-{2-[3-(2-Oxo-imidazolidin-1-yl)-phenyl]-2-oxo-ethyl}-3-methyl-7-(3-methyl-2-buten-1-yl)-8-(3-amino-piperidin-1-yl)-xanthin
   R_{f}-Wert: 0.54 (Reversed Phase DC-Fertigplatte (E. Merck), Acetonitril/Wasser/ Trifluoressigsäure = 100:100:0.1)
   Massenspektrum (ESI⁺): m/z = 535 [M+H]⁺
(14) 1-Methyl-3-(2-propen-1-yl)-7-(2-cyano-benzyl)-8-(3-amino-piperidin-1-yl)-xanthin
   R_{f}-Wert: 0.40 (Kieselgel, Methylenchlorid/Methanol = 9:1)
   Massenspektrum (ESI⁺): m/z = 420 [M+H]⁺
(15) 1-(2-Phenyl-2-oxo-ethyl)-3-methyl-7-(2-butin-1-yl)-8-(3-amino-piperidin-1-yl)-xanthin
   Massenspektrum (ESI⁺): m/z = 435 [M+H]⁺
(16) 1-(2-{2-[(Ethylcarbonyl)amino]-phenyl}-2-oxo-ethyl)-3-methyl-7-(3-methyl-2-buten-1-yl)-8-(3-amino-piperidin-1-yl)-xanthin
   R_{f}-Wert: 0.50 (Reversed Phase DC-Fertigplatte (E. Merck), Acetonitril/Wasser/ Trifluoressigsäure = 100:100:0.1)
   Massenspektrum (ESI⁺): m/z = 522 [M+H]⁺
(17) 1-Methyl-3-phenyl-7-(2-cyano-benzyl)-8-(3-amino-piperidin-1-yl)-xanthin
   R_{f}-Wert: 0.40 (Kieselgel, Methylenchlorid/Methanol = 9:1)
   Massenspektrum (ESI⁺): m/z = 456 [M+H]⁺
(18) 1-[2-(2-Amino-phenyl)-2-oxo-ethyl]-3-methyl-7-(3-methyl-2-buten-1-yl)-8-((S)-3-amino-piperidin-1-yl)-xanthin
   R_{f}-Wert: 0.50 (Reversed Phase DC-Fertigplatte (E. Merck), Acetonitril/Wasser/ Trifluoressigsäure = 50:50:0.1)
   Massenspektrum (ESI⁺): m/z = 466 [M+H]⁺
(19) 1-(2-Phenyl-2-oxo-ethyl)-3-cyanomethyl-7-(3-methyl-2-buten-1-yl)-8-(3-amino-piperidin-1-yl)-xanthin
   R_{f}-Wert: 0.07 (Kieselgel, Methylenchlorid/Methanol/konz. wässriges Ammoniak = 90:10:1)
   Massenspektrum (ESI⁺): m/z = 476 [M+H]⁺
(20) 1-[(Chinolin-2-yl)methyl]-3-methyl-7-(3-methyl-2-buten-1-yl)-8-(3-amino-piperidin-1-yl)-xanthin
   (Durchführung mit isopropanolischer Salzsäure 5-6 M in Methylenchlorid)
   R_{f}-Wert: 0.50 (Kieselgel, Methylenchlorid/Methanol/konz. wässriges Ammoniak = 90:10:1)
   Massenspektrum (ESI⁺): m/z = 474 [M+H]⁺
(21) 1-[(2-Oxo-2H-chromen-4-yl)methyl]-3-methyl-7-(3-methyl-2-buten-1-y1)-8-(3-amino-piperidin-1-yl)-xanthin
   (Durchführung mit isopropanolischer Salzsäure 5-6 M in Methylenchlorid)
   Massenspektrum (ESI⁺): m/z = 491 [M+H]⁺
   R_{f}-Wert: 0.16 (Kieselgel, Methylenchlorid/Methanol/konz. wässriges Ammoniak = 95:5:0.1)
(22) 1-[(Cinnolin-4-yl)methyl]-3-methyl-7-(3-methyl-2-buten-1-yl)-8-(3-amino-piperidin-1-yl)-xanthin (1:1 Gemisch mit 1-[(1,4-Dihydro-cinnolin-4-yl)methyl]-3-methyl-7-(3-methyl-2-buten-1-yl)-8-(3-amino-piperidin-1-yl)-xanthin)
   (Durchführung mit isopropanolischer Salzsäure 5-6 M in Methylenchlorid)
   R_{f}-Wert: 0.49 (Kieselgel, Methylenchlorid/Methanol/konz. wässriges Ammoniak = 90:10:1)
   Massenspektrum (ESI⁺): m/z = 475 [M+H]⁺
(23) 1-[(1-Methyl-2-oxo-1,2-dihydro-chinolin-4-yl)methyl]-3-methyl-7-(3-methyl-2-buten-1-yl)-8-(3-amino-piperidin-1-yl)-xanthin
   (Durchführung mit isopropanolischer Salzsäure 5-6 M in Methylenchlorid)
   Schmelzpunkt: 178-181°C
   Massenspektrum (ESI⁺): m/z = 504 [M+H]⁺
(24) 1-[(4-Oxo-3,4-dihydro-phthalazin-1-yl)methyl]-3-methyl-7-(3-methyl-2-buten-1-yl)-8-(3-amino-piperidin-1-yl)-xanthin
   (Durchführung mit isopropanolischer Salzsäure 5-6 M in Methylenchlorid)
   R_{f}-Wert: 0.06 (Kieselgel, Methylenchlorid/Methanol/konz. wässriges Ammoniak = 95:5:0.1)
   Massenspektrum (ESI⁺): m/z = 491 [M+H]⁺
(25) 1-[(Chinazolin-4-yl)methyl]-3-methyl-7-(3-methyl-2-buten-1-yl)-8-(3-amino-piperidin-1-yl)-xanthin
   (Durchführung mit isopropanolischer Salzsäure 5-6 M in Methylenchlorid)
   R_{f}-Wert: 0.48 (Kieselgel, Methylenchlorid/Methanol/konz. wässriges Ammoniak = 90:10:1)
   Massenspektrum (ESI⁺): m/z = 475 [M+H]⁺
(26) 1-[(5-Methyl-3-phenyl-isoxazol-4-yl)methyl]-3-methyl-7-(3-methyl-2-buten-1-yl)-8-(3-amino-piperidin-1-yl)-xanthin
   (Durchführung mit isopropanolischer Salzsäure 5-6 M in Methylenchlorid)
   R_{f}-Wert: 0.40 (Kieselgel, Methylenchlorid/Methanol/konz. wässriges Ammoniak = 90:10:0.1)
   Massenspektrum (ESI⁺): m/z = 504 [M+H]⁺
(27) 1-[(lsochinolin-3-yl)methyl]-3-methyl-7-(3-methyl-2-buten-1-yl)-8-(3-amino-piperidin-1-yl)-xanthin
   (Durchführung mit isopropanolischer Salzsäure 5-6 M in Methylenchlorid)
   R_{f}-Wert: 0.51 (Kieselgel, Methylenchlorid/Methanol/konz. wässriges Ammoniak = 90:10:1)
   Massenspektrum (ESI⁺): m/z = 474 [M+H]⁺
(28) 1-[(3-Phenyl-[1,2,4]oxadiazol-5-yl)methyl]-3-methyl-7-(3-methyl-2-buten-1-yl)-8-(3-amino-piperidin-1-yl)-xanthin
   (Durchführung mit isopropanolischer Salzsäure 5-6 M in Methylenchlorid)
   R_{f}-Wert: 0.23 (Kieselgel, Methylenchlorid/Methanol/konz. wässriges Ammoniak = 95:5:1)
   Massenspektrum (ESI⁺): m/z = 491 [M+H]⁺
(29) 1-[(4-Phenyl-pyridin-2-yl)methyl]-3-methyl-7-(3-methyl-2-buten-1-yl)-8-(3-amino-piperidin-1-yl)-xanthin
   (Durchführung mit isopropanolischer Salzsäure 5-6 M in Methylenchlorid)
   R_{f}-Wert: 0.51 (Kieselgel, Methylenchlorid/Methanol/konz. wässriges Ammoniak = 90:10:1)
   Massenspektrum (ESI⁺): m/z = 500 [M+H]⁺
(30) 1-[(5-Phenyl-pyridin-2-yl)methyl]-3-methyl-7-(3-methyl-2-buten-1-yl)-8-(3-amino-piperidin-1-yl)-xanthin
   (Durchführung mit isopropanolischer Salzsäure 5-6 M in Methylenchlorid)
   R_{f}-Wert: 0.58 (Kieselgel, Methylenchlorid/Methanol/konz. wässriges Ammoniak = 90:10:1)
   Massenspektrum (ESI⁺): m/z = 500 [M+H]⁺
(31) 1-[(3-Methyl-4-oxo-3,4-dihydro-phthalazin-1-yl)methyl]-3-methyl-7-(3-methyl-2-buten-1-yl)-8-(3-amino-piperidin-1-yl)-xanthin-hydrochlorid
   (Durchführung mit isopropanolischer Salzsäure 5-6 M in Methylenchlorid; Produkt fällt als Hydrochlorid aus)
   R_{f}-Wert: 0.55 (Kieselgel, Methylenchlorid/Methanol/konz. wässriges Ammoniak = 90:10:0.1)
   Massenspektrum (ESI⁺): m/z = 505 [M+H]⁺
(32) 1-[2-(3-Methylsulfanyl-phenyl)-2-oxo-ethyl]-3-methyl-7-(3-methyl-2-buten-1-yl)-8-(3-amino-piperidin-1-yl)-xanthin
   R_{f}-Wert: 0.34 (Kieselgel, Methylenchlorid/Methanol/konz. wässriges Ammoniak = 90:10:1)
   Massenspektrum (ESI⁺): m/z = 497 [M+H]⁺
(33) 1-[2-(3-Methansulfinyl-phenyl)-2-oxo-ethyl]-3-methyl-7-(3-methyl-2-buten-1-yl)-8-(3-amino-piperidin-1-yl)-xanthin
   R_{f}-Wert: 0.21 (Kieselgel, Methylenchlorid/Methanol/konz. wässriges Ammoniak = 90:10:1)
   Massenspektrum (ESI⁺): m/z = 513 [M+H]⁺
(34) 1-[2-(3-Methansulfonyl-phenyl)-2-oxo-ethyl]-3-methyl-7-(3-methyl-2-buten-1-yl)-8-(3-amino-piperidin-1-yl)-xanthin
   R_{f}-Wert: 0.66 (Reversed Phase DC-Fertigplatte (E. Merck), Acetonitril/Wasser/ Trifluoressigsäure = 50:50:1)
   Massenspektrum (ESI⁺): m/z = 529 [M+H]⁺
(35) 1-[2-(3-Carboxy-phenyl)-2-oxo-ethyl]-3-methyl-7-(3-methyl-2-buten-1-yl)-8-(3-amino-piperidin-1-yl)-xanthin-hydrochlorid
   (Durchführung mit isopropanolischer Salzsäure 5-6 M in Methylenchlorid; Produkt fällt als Hydrochlorid aus)
   R_{f}-Wert: 0.54 (Reversed Phase DC-Fertigplatte (E. Merck), Acetonitril/Wasser/ Trifluoressigsäure = 50:50:1)
   Massenspektrum (ESI⁺): m/z = 495 [M+H]⁺
(36) 1-[2-(3-Methoxycarbonyl-phenyl)-2-oxo-ethyl]-3-methyl-7-(3-methyl-2-buten-1-yl)-8-(3-amino-piperidin-1-yl)-xanthin-hydrochlorid
   (Durchführung mit isopropanolischer Salzsäure 5-6 M in Methylenchlorid; Produkt fällt als Hydrochlorid aus)
   R_{f}-Wert: 0.47 (Reversed Phase DC-Fertigplatte (E. Merck), Acetonitril/Wasser/ Trifluoressigsäure = 50:50:1)
   Massenspektrum (ESI⁺): m/z = 509 [M+H]⁺
(37) 1-{2-[3-(Methylaminocarbonyl)-phenyl]-2-oxo-ethyl}-3-methyl-7-(3-methyl-2-buten-1-yl)-8-(3-amino-piperidin-1-yl)-xanthin-hydrochlorid
   (Durchführung mit isopropanolischer Salzsäure 5-6 M in Methylenchlorid; Produkt fällt als Hydrochlorid an)
   R_{f}-Wert: 0.53 (Reversed Phase DC-Fertigplatte (E. Merck), Acetonitril/Wasser/ Trifluoressigsäure = 50:50:1)
   Massenspektrum (ESI⁺): m/z = 508 [M+H]⁺
(38) 1-{2-[3-(Dimethylaminocarbonyl)-phenyl]-2-oxo-ethyl}-3-methyl-7-(3-methyl-2-buten-1-yl)-8-(3-amino-piperidin-1-yl)-xanthin-hydrochlorid
   (Durchführung mit isopropanolischer Salzsäure 5-6 M in Methylenchlorid; Produkt fällt als Hydrochlorid an)
   R_{f}-Wert: 0.53 (Reversed Phase DC-Fertigplatte (E. Merck), Acetonitril/Wasser/ Trifluoressigsäure = 50:50:1)
   Massenspektrum (ESI⁺): m/z = 522 [M+H]⁺
(39) 1-{2-[3-(Morpholin-4-yl-carbonyl)-phenyl]-2-oxo-ethyl}-3-methyl-7-(3-methyl-2-buten-1-yl)-8-(3-amino-piperidin-1-yl)-xanthin-hydrochlorid
   (Durchführung mit isopropanolischer Salzsäure 5-6 M in Methylenchlorid; Produkt fällt als Hydrochlorid an)
   R_{f}-Wert: 0.53 (Reversed Phase DC-Fertigplatte (E. Merck), Acetonitril/Wasser/ Trifluoressigsäure = 50:50:1)
   Massenspektrum (ESI⁺): m/z = 564 [M+H]⁺
(40) 1-[2-(2-Carboxy-phenyl)-2-oxo-ethyl]-3-methyl-7-(3-methyl-2-buten-1-yl)-8-(3-amino-piperidin-1-yl)-xanthin-hydrochlorid
   (Durchführung mit isopropanolischer Salzsäure 5-6 M in Methylenchlorid; Produkt fällt als Hydrochlorid an)
   R_{f}-Wert: 0.53 (Reversed Phase DC-Fertigplatte (E. Merck), Acetonitril/Wasser/ Trifluoressigsäure = 50:50:1)
   Massenspektrum (ESI⁺): m/z = 495 [M+H]⁺
(41) 1-[2-(2-Ethoxycarbonyl-phenyl)-2-oxo-ethyl]-3-methyl-7-(3-methyl-2-buten-1-yl)-8-(3-amino-piperidin-1-yl)-xanthin-hydrochlorid
   (Durchführung mit isopropanolischer Salzsäure 5-6 M in Methylenchlorid; Produkt fällt als Hydrochlorid an)
   R_{f}-Wert: 0.41 (Reversed Phase DC-Fertigplatte (E. Merck), Acetonitril/Wasser/ Trifluoressigsäure = 50:50:1)
   Massenspektrum (ESI⁺): m/z = 523 [M+H]⁺
(42) 1-{2-[2-(Dimethylaminocarbonyl)-phenyl]-2-oxo-ethyl}-3-methyl-7-(3-methyl-2-buten-1-yl)-8-(3-amino-piperidin-1-yl)-xanthin-hydrochlorid
   (Durchführung mit isopropanolischer Salzsäure 5-6 M in Methylenchlorid; Produkt fällt als Hydrochlorid an)
   R_{f}-Wert: 0.53 (Reversed Phase DC-Fertigplatte (E. Merck), Acetonitril/Wasser/ Trifluoressigsäure = 50:50:1)
   Massenspektrum (ESI⁺): m/z = 522 [M+H]⁺
(43) 1-{2-[2-(Morpholin-4-yl-carbonyl)-phenyl]-2-oxo-ethyl}-3-methyl-7-(3-methyl-2-buten-1-yl)-8-(3-amino-piperidin-1-yl)-xanthin-hydrochlorid
   (Durchführung mit isopropanolischer Salzsäure 5-6 M in Methylenchlorid; Produkt fällt als Hydrochlorid an)
   R_{f}-Wert: 0.53 (Reversed Phase DC-Fertigplatte (E. Merck), Acetonitril/Wasser/ Trifluoressigsäure = 50:50:1)
   Massenspektrum (ESI⁺): m/z = 564 [M+H]⁺
(44) 1-[2-(2,6-Dimethoxy-phenyl)-2-oxo-ethyl]-3-methyl-7-(3-methyl-2-buten-1 -yl)-8-(3-amino-piperidin-1-yl)-xanthin-hydrochlorid
   (Durchführung mit isopropanolischer Salzsäure 5-6 M in Methylenchlorid; Produkt fällt als Hydrochlorid an)
   R_{f}-Wert: 0.44 (Reversed Phase DC-Fertigplatte (E. Merck), Acetonitril/Wasser/ Trifluoressigsäure = 50:50:1)
   Massenspektrum (ESI⁺): m/z = 511 [M+H]⁺
(45) 1-(2-Phenyl-2-oxo-ethyl)-3-methyl-7-(2,3-dimethyl-2-buten-1-yl)-8-(3-amino-piperidin-1-yl)-xanthin-hydrochlorid
   (Durchführung mit isopropanolischer Salzsäure 5-6 M in Methylenchlorid; Produkt fällt als Hydrochlorid an)
   R_{f}-Wert: 0.68 (Reversed Phase DC-Fertigplatte (E. Merck), Acetonitril/Wasser/ Trifluoressigsäure = 50:50:1)
   Massenspektrum (ESI⁺): m/z = 465 [M+H]⁺
(46) 1-((E)-3-Phenyl-allyl)-3-methyl-7-(3-methyl-2-buten-1-yl)-8-(3-amino-piperidin-1-yl)-xanthin
   (Durchführung mit isopropanolischer Salzsäure 5-6 M in Methylenchlorid)
   R_{f}-Wert: 0.38 (Kieselgel, Methylenchlorid/Methanol/konz. wässriges Ammoniak = 95:5:0.1)
   Massenspektrum (ESI⁺): m/z = 449 [M+H]⁺
(47) 1-[(Benzo[b]thiophen-3-yl)methyl]-3-methyl-7-(3-methyl-2-buten-1-yl)-8-(3-amino-piperidin-1-yl)-xanthin
   (Durchführung mit isopropanolischer Salzsäure 5-6 M in Methylenchlorid)
   R_{f}-Wert: 0.51 (Kieselgel, Methylenchlorid/Methanol/konz. wässriges Ammoniak = 90:10:1)
   Massenspektrum (ESI⁺): m/z = 479 [M+H]⁺
(48) 1-[(1*H*-Indol-3-yl)methyl]-3-methyl-7-(3-methyl-2-buten-1-yl)-8-(3-amino-piperidin-1-yl)-xanthin
   R_{f}-Wert: 0.48 (Kieselgel, Methylenchlorid/Methanol/konz. wässriges Ammoniak = 90:10:1)
   Massenspektrum (ESI⁺): m/z = 462 [M+H]⁺
(49) 1-[(Biphenyl-4-yl)methyl]-3-methyl-7-(3-methyl-2-buten-1-yl)-8-(3-amino-piperidin-1-yl)-xanthin
   (Durchführung mit isopropanolischer Salzsäure 5-6 M in Methylenchlorid)
   R_{f}-Wert: 0.30 (Kieselgel, Methylenchlorid/Methanol/konz. wässriges Ammoniak = 95:5:0.1)
   Massenspektrum (ESI⁺): m/z = 499 [M+H]⁺
(50) 1-[(1-Naphthyl)methyl]-3-methyl-7-(2-butin-1-yl)-8-(3-amino-piperidin-1-yl)-xanthin
   (Durchführung mit isopropanolischer Salzsäure 5-6 M in Methylenchlorid)
   R_{f}-Wert: 0.56 (Kieselgel, Methylenchlorid/Methanol/konz. wässriges Ammoniak = 90:10:1)
   Massenspektrum (ESI⁺): m/z = 457 [M+H]⁺
(51) 1-[(1-Methyl-2-oxo-1,2-dihydro-chinolin-4-yl)methyl]-3-methyl-7-(2-butin-1-yl)-8-(3-amino-piperidin-1-yl)-xanthin
   (Durchführung mit isopropanolischer Salzsäure 5-6 M in Methylenchlorid)
   R_{f}-Wert: 0.40 (Kieselgel, Methylenchlorid/Methanol/konz. wässriges Ammoniak = 90:10:1)
   Massenspektrum (ESI⁺): m/z = 488 [M+H]⁺
(52) 1-[(Chinolin-4-yl)methyl]-3-methyl-7-(2-butin-1-yl)-8-(3-amino-piperidin-1-yl)-xanthin
   (Durchführung mit isopropanolischer Salzsäure 5-6 M in Methylenchlorid)
   R_{f}-Wert: 0.52 (Kieselgel, Methylenchlorid/Methanol/konz. wässriges Ammoniak = 90:10:1)
   Massenspektrum (ESI⁺): m/z = 458 [M+H]⁺
(53) 1-(2-Cyclohexyl-2-oxo-ethyl)-3-methyl-7-(3-methyl-2-buten-1-yl)-8-(3-amino-piperidin-1-yl)-xanthin
   (Durchführung mit isopropanolischer Salzsäure 5-6 M in Methylenchlorid)
   R_{f}-Wert: 0.48 (Kieselgel, Methylenchlorid/Methanol/konz. wässriges Ammoniak = 90:10:1)
   Massenspektrum (ESI⁺): m/z = 457 [M+H]⁺
(54) 1-(3,3-Dimethyl-2-oxo-butyl)-3-methyl-7-(3-methyl-2-buten-1-yl)-8-(3-amino-piperidin-1-yl)-xanthin
   (Durchführung mit isopropanolischer Salzsäure 5-6 M in Methylenchlorid)
   R_{f}-Wert: 0.49 (Kieselgel, Methylenchlorid/Methanol/konz. wässriges Ammoniak = 90:10:1)
   Massenspektrum (ESI⁺): m/z = 431 [M+H]⁺
(55) 1-[(Chinazolin-2-yl)methyl]-3-methyl-7-(2-butin-1-yl)-8-(3-amino-piperidin-1-yl)-xanthin
   (Durchführung mit isopropanolischer Salzsäure 5-6 M in Methylenchlorid)
   R_{f}-Wert: 0.20 (Kieselgel, Methylenchlorid/Methanol/konz. wässriges Ammoniak = 95:5:0.1)
   Massenspektrum (ESI⁺): m/z = 459 [M+H]⁺
(56) 1-[(2-Methyl-naphthalin-1-yl)methyl]-3-methyl-7-(2-butin-1-yl)-8-(3-amino-piperidin-1-yl)-xanthin
   (Durchführung mit isopropanolischer Salzsäure 5-6 M in Methylenchlorid)
   R_{f}-Wert: 0.25 (Kieselgel, Methylenchlorid/Methanol/konz. wässriges Ammoniak = 95:5:0.1)
   Massenspektrum (ESI⁺): m/z = 471 [M+H]⁺
(57) 1-({5-[(Methoxycarbonyl)methylamino]-isochinolin-1-yl}methyl)-3-methyl-7-(3-methyl-2-buten-1-yl)-8-(3-amino-piperidin-1-yl)-xanthin
   (Durchführung mit isopropanolischer Salzsäure 5-6 M in Methylenchlorid)
   R_{f}-Wert: 0.43 (Kieselgel, Methylenchlorid/Methanol/konz. wässriges Ammoniak = 90:10:1)
   Massenspektrum (ESI⁺): m/z = 561 [M+H]⁺
(58) 1-(2-Dimethylamino-2-oxo-ethyl)-3-methyl-7-(3-methyl-2-buten-1-yl)-8-(3-amino-piperidin-1-yl)-xanthin
   (Durchführung mit isopropanolischer Salzsäure 5-6 M in Methylenchlorid)
   R_{f}-Wert: 0.38 (Kieselgel, Methylenchlorid/Methanol/konz. wässriges Ammoniak = 90:10:1)
   Massenspektrum (ESI⁺): m/z = 418 [M+H]⁺
(59) 1-[2-(Piperidin-1-yl)-2-oxo-ethyl]-3-methyl-7-(3-methyl-2-buten-1-yl)-8-(3-amino-piperidin-1-yl)-xanthin
   (Durchführung mit isopropanolischer Salzsäure 5-6 M in Methylenchlorid)
   R_{f}-Wert: 0.35 (Kieselgel, Methylenchlorid/Methanol/konz. wässriges Ammoniak = 90:10:1)
   Massenspektrum (ESI⁺): m/z = 458 [M+H]⁺
(60) 1-[(2-Methyl-1-oxo-1,2-dihydro-isochinolin-4-yl)methyl]-7-(2-butin-1-yl)-8-(3-amino-piperidin-1-yl)-xanthin
   (Durchführung mit isopropanolischer Salzsäure 5-6 M in Methylenchlorid)
   R_{f}-Wert: 0.17 (Kieselgel, Methylenchlorid/Methanol/konz. wässriges Ammoniak = 95:5:0.1)
   Massenspektrum (ESI⁺): m/z = 488 [M+H]⁺
(61) 1-[(2-Methyl-1-oxo-1,2-dihydro-isochinolin-4-yl)methyl]-7-(3-methyl-2-buten-1-yl)-8-(3-amino-piperidin-1-yl)-xanthin
   (Durchführung mit isopropanolischer Salzsäure 5-6 M in Methylenchlorid)
   R_{f}-Wert: 0.13 (Kieselgel, Methylenchlorid/Methanol/konz. wässriges Ammoniak = 95:5:0.1)
   Massenspektrum (ESI⁺): m/z = 504 [M+H]⁺
(62) 1-[(2-Methoxy-naphthalin-1-yl)methyl]-3-methyl-7-(2-butin-1-yl)-8-(3-amino-piperidin-1-yl)-xanthin
   (Durchführung mit isopropanolischer Salzsäure 5-6 M in Methylenchlorid)
   R_{f}-Wert: 0.17 (Kieselgel, Methylenchlorid/Methanol/konz. wässriges Ammoniak = 95:5:0.1)
   Massenspektrum (ESI⁺): m/z = 487 [M+H]⁺
(63) 1-[(Isochinolin-1-yl)methyl]-3-methyl-7-(2-butin-1-yl)-8-(3-amino-piperidin-1-yl)-xanthin
   (Durchführung mit isopropanolischer Salzsäure 5-6 M in Methylenchlorid)
   R_{f}-Wert: 0.42 (Kieselgel, Methylenchlorid/Methanol/konz. wässriges Ammoniak = 90:10:1)
   Massenspektrum (ESI⁺): m/z = 458 [M+H]⁺
(64) 1-[(4-Methoxy-naphthalin-1-yl)methyl]-3-methyl-7-(2-butin-1-yl)-8-(3-amino-piperidin-1-yl)-xanthin
   (Durchführung mit isopropanolischer Salzsäure 5-6 M in Methylenchlorid)
   R_{f}-Wert: 0.14 (Kieselgel, Methylenchlorid/Methanol/konz. wässriges Ammoniak = 95:5:0.1)
   Massenspektrum (ESI⁺): m/z = 487 [M+H]⁺
(65) 1-[(3-Methyl-isochinolin-1-yl)methyl]-3-methyl-7-(2-butin-1-yl)-8-(3-amino-piperidin-1-yl)-xanthin
   (Durchführung mit isopropanolischer Salzsäure 5-6 M in Methylenchlorid)
   Schmelzpunkt: 155-158°C
   Massenspektrum (ESI⁺): m/z = 472 [M+H]⁺
(66) 1-[2-(2,3-Dimethoxy-phenyl)-2-oxo-ethyl]-3-methyl-7-(3-methyl-2-buten-1-yl)-8-(3-amino-piperidin-1-yl)-xanthin
   (Durchführung mit isopropanolischer Salzsäure 5-6 M in Methylenchlorid)
   R_{f}-Wert: 0.40 (Kieselgel, Methylenchlorid/Methanol/konz. wässriges Ammoniak = 90:10:1)
   Massenspektrum (ESI⁺): m/z = 511 [M+H]⁺
(67) 1-[(5-Nitro-naphthalin-1-yl)methyl]-3-methyl-7-(2-butin-1-yl)-8-(3-amino-piperidin-1-yl)-xanthin
   (Durchführung mit isopropanolischer Salzsäure 5-6 M in Methylenchlorid)
   R_{f}-Wert: 0.15 (Kieselgel, Methylenchlorid/Methanol/konz. wässriges Ammoniak = 95:5:0.1)
   Massenspektrum (ESI⁺): m/z = 502 [M+H]⁺
(68) 1-[2-(Pyrrolidin-1-yl)-2-oxo-ethyl]-3-methyl-7-(3-methyl-2-buten-1-yl)-8-(3-amino-piperidin-1-yl)-xanthin
   (Durchführung mit isopropanolischer Salzsäure 5-6 M in Methylenchlorid)
   R_{f}-Wert: 0.56 (Kieselgel, Methylenchlorid/Methanol/konz. wässriges Ammoniak = 90:10:1)
   Massenspektrum (ESI⁺): m/z = 444 [M+H]⁺
(69) 1-[(4-Methyl-isochinolin-1-yl)methyl]-3-methyl-7-(2-butin-1-yl)-8-(3-amino-piperidin-1-yl)-xanthin
   (Durchführung mit isopropanolischer Salzsäure 5-6 M in Methylenchlorid)
   R_{f}-Wert: 0.46 (Kieselgel, Methylenchlorid/Methanol/konz. wässriges Ammoniak = 90:10:1)
   Massenspektrum (ESI⁺): m/z = 472 [M+H]⁺
(70) 1-[(2-Naphthyl)methyl]-3-methyl-7-(2-butin-1-yl)-8-(3-amino-piperidin-1-yl)-xanthin
   (Durchführung mit isopropanolischer Salzsäure 5-6 M in Methylenchlorid)
   R_{f}-Wert: 0.20 (Kieselgel, Methylenchlorid/Methanol/konz. wässriges Ammoniak = 95:5:0.1)
   Massenspektrum (ESI⁺): m/z = 457 [M+H]⁺
(71) 1-[(4-Oxo-3,4-dihydro-chinazolin-2-yl)methyl]-3-methyl-7-(2-butin-1-yl)-8-(3-amino-piperidin-1-yl)-xanthin
   (Durchführung mit isopropanolischer Salzsäure 5-6 M in Methylenchlorid)
   R_{f}-Wert: 0.38 (Kieselgel, Methylenchlorid/Methanol/konz. wässriges Ammoniak = 90:10:0.1)
   Massenspektrum (ESI⁺): m/z = 475 [M+H]⁺
(72) 1-[(Chinolin-6-yl)methyl]-3-methyl-7-(2-butin-1-yl)-8-(3-amino-piperidin-1-yl)-xanthin
   (Durchführung mit isopropanolischer Salzsäure 5-6 M in Methylenchlorid)
   R_{f}-Wert: 0.15 (Kieselgel, Methylenchlorid/Methanol/konz. wässriges Ammoniak = 95:5:0.1)
   Massenspektrum (ESI⁺): m/z = 458 [M+H]⁺
(73) 1-[(4-Dimethylamino-naphthalin-1-yl)methyl]-3-methyl-7-(2-butin-1-yl)-8-(3-amino-piperidin-1-yl)-xanthin
   (Durchführung mit isopropanolischer Salzsäure 5-6 M in Methylenchlorid)
   R_{f}-Wert: 0.18 (Kieselgel, Methylenchlorid/Methanol/konz. wässriges Ammoniak = 95:5:0.1)
   Massenspektrum (ESI⁺): m/z = 500 [M+H]⁺
(74) 1-[(Isochinolin-1-yl)methyl]-3-methyl-7-((E)-2-buten-1-yl)-8-(3-amino-piperidin-1-yl)-xanthin
   (Durchführung mit isopropanolischer Salzsäure 5-6 M in Methylenchlorid; das Produkt enthält noch ca. 20 % Z-Isomer)
   R_{f}-Wert: 0.66 (Kieselgel, Methylenchlorid/Methanol/konz. wässriges Ammoniak = 90:10:0.1)
   Massenspektrum (ESI⁺): m/z = 460 [M+H]⁺
(75) 1-[(3-Methoxy-naphthalin-2-yl)methyl]-3-methyl-7-(2-butin-1-yl)-8-(3-amino-piperidin-1-yl)-xanthin
   (Durchführung mit isopropanolischer Salzsäure 5-6 M in Methylenchlorid)
   R_{f}-Wert: 0.25 (Kieselgel, Methylenchlorid/Methanol/konz. wässriges Ammoniak = 95:5:0.1)
   Massenspektrum (ESI⁺): m/z = 487 [M+H]⁺
(76) 1-[2-(2,3-Dihydro-benzo[1,4]dioxin-5-yl)-2-oxo-ethyl]-3-methyl-7-(3-methyl-2-buten-1-yl)-8-(3-amino-piperidin-1-yl)-xanthin
   (Durchführung mit isopropanolischer Salzsäure 5-6 M in Methylenchlorid)
   Massenspektrum (ESI⁺): m/z = 509 [M+H]⁺
(77) 1-[(3-Methyl-4-oxo-3,4-dihydro-chinazolin-2-yl)methyl]-3-methyl-7-(2-butin-1-yl)-8-(3-amino-piperidin-1-yl)-xanthin
   (Durchführung mit isopropanolischer Salzsäure 5-6 M in Methylenchlorid)
   R_{f}-Wert: 0.20 (Kieselgel, Methylenchlorid/Methanol/konz. wässriges Ammoniak = 95:5:0.1)
   Massenspektrum (ESI⁺): m/z = 489 [M+H]⁺
(78) 1-[2-(3-Methyl-2-oxo-2,3-dihydro-benzooxazol-7-yl)-2-oxo-ethyl]-3-methyl-7-(3-methyl-2-buten-1-yl)-8-(3-amino-piperidin-1-yl)-xanthin
   (Durchführung mit isopropanolischer Salzsäure 5-6 M in Methylenchlorid)
   R_{f}-Wert: 0.47 (Kieselgel, Methylenchlorid/Methanol/konz. wässriges Ammoniak = 90:10:1)
   Massenspektrum (ESI⁺): m/z = 522 [M+H]⁺
(79) 1-[2-(Benzo[1,3]dioxol-4-yl)-2-oxo-ethyl]-3-methyl-7-(3-methyl-2-buten-1-yl)-8-(3-amino-piperidin-1-yl)-xanthin
   (Durchführung mit isopropanolischer Salzsäure 5-6 M in Methylenchlorid)
   Massenspektrum (ESI⁺): m/z = 495 [M+H]⁺
(80) 1-[(Chinazolin-2-yl)methyl]-3-methyl-7-(2-butin-1-yl)-8-((*R*)-3-amino-piperidin-1-yl)-xanthin
   (Durchführung mit isopropanolischer Salzsäure 5-6 M in Methylenchlorid)
   Massenspektrum (ESI⁺): m/z = 459 [M+H]⁺
(81) 1-[(Chinazolin-2-yl)methyl]-3-methyl-7-(2-butin-1-yl)-8-((*S*)-3-amino-piperidin-1-yl)-xanthin
   (Durchführung mit isopropanolischer Salzsäure 5-6 M in Methylenchlorid)
   Massenspektrum (ESI⁺): m/z = 459 [M+H]⁺
(82) 1-[(Chinazolin-2-yl)methyl]-3-methyl-7-((E)-2-buten-1-yl)-8-((*S*)-3-amino-piperidin-1-yl)-xanthin
   (Durchführung mit isopropanolischer Salzsäure 5-6 M in Methylenchlorid; das Produkt enthält noch ca. 20 % Z-Isomer)
   R_{f}-Wert: 0.12 (Kieselgel, Methylenchlorid/Methanol/konz. wässriges Ammoniak = 95:5:0.1)
   Massenspektrum (ESI⁺): m/z = 461 [M+H]⁺
(83) 1-[(Chinazolin-2-yl)methyl]-3-methyl-7-((E)-2-buten-1-yl)-8-((*R*)-3-amino-piperidin-1-yl)-xanthin
   (Durchführung mit isopropanolischer Salzsäure 5-6 M in Methylenchlorid; das Produkt enthält noch ca. 15 % Z-Isomer)
   R_{f}-Wert: 0.12 (Kieselgel, Methylenchlorid/Methanol/konz. wässriges Ammoniak = 95:5:0.1)
   Massenspektrum (ESI⁺): m/z = 461 [M+H]⁺
(84) 1-[(3-Methyl-isochinolin-1-yl)methyl]-3-methyl-7-((E)-2-buten-1-yl)-8-((R)-3-amino-piperidin-1-yl)-xanthin
   (Durchführung mit isopropanolischer Salzsäure 5-6 M in Methylenchlorid; das Produkt enthält noch ca. 17 % Z-Isomer)
   R_{f}-Wert: 0.54 (Kieselgel, Methylenchlorid/Methanol/konz. wässriges Ammoniak = 90:10:1)
   Massenspektrum (ESI⁺): m/z = 474 [M+H]⁺
(85) 1-[(3-Methyl-isochinolin-1-yl)methyl]-3-methyl-7-((E)-2-buten-1-yl)-8-((S)-3-amino-piperidin-1-yl)-xanthin
   (Durchführung mit isopropanolischer Salzsäure 5-6 M in Methylenchlorid; das Produkt enthält noch ca. 17 % Z-Isomer)
   R_{f}-Wert: 0.54 (Kieselgel, Methylenchlorid/Methanol/konz. wässriges Ammoniak = 90:10:1)
   Massenspektrum (ESI⁺): m/z = 474 [M+H]⁺
(86) 1-(2-{2-[(Ethylaminocarbonyl)methoxy]-phenyl}-2-oxo-ethyl)-3-methyl-7-(2-butin-1-yl)-8-((*R*)-3-amino-piperidin-1-yl)-xanthin
   Massenspektrum (ESI⁺): m/z = 536 [M+H]⁺
(87) 1-[2-(2-Amino-phenyl)-2-oxo-ethyl]-3-methyl-7-[(1-cyclopenten-1-yl)methyl]-8-(3-amino-piperidin-1-yl)-xanthin
   Massenspektrum (ESI⁺): m/z = 478 [M+H]⁺
(88) 1-(2-Phenyl-2-oxo-ethyl)-3-methyl-7-[(1-cyclopenten-1-yl)methyl]-8-(3-amino-piperidin-1-yl)-xanthin
   Massenspektrum (ESI⁺): m/z = 463 [M+H]⁺
(89) 1-(2-{2-[(Methylaminocarbonyl)methoxy]-phenyl}-2-oxo-ethyl)-3-methyl-7-[(1-cyclopenten-1-yl)methyl]-8-(3-amino-piperidin-1-yl)-xanthin
   Massenspektrum (ESI⁺): m/z = 550 [M+H]⁺
(90) 1-Methyl-3-isopropyl-7-(2-cyano-benzyl)-8-(3-amino-piperidin-1-yl)-xanthin R_{f}-Wert: 0.40 (Kieselgel, Methylenchlorid/Methanol = 9:1)
   Massenspektrum (ESI⁺): m/z = 422 [M+H]⁺
(91) 1-(2-{2-[(Methylaminocarbonyl)methoxy]-phenyl}-2-oxo-ethyl)-3-methyl-7-(2-butin-1-yl)-8-(3-amino-piperidin-1-yl)-xanthin
   Massenspektrum (ESI⁺): m/z = 522 [M+H]⁺
(92) 1-(2-{2-[(Aminocarbonyl)methoxy]-phenyl}-2-oxo-ethyl)-3-methyl-7-(2-butin-1-yl)-8-(3-amino-piperidin-1-yl)-xanthin
   Massenspektrum (ESI⁺): m/z = 508 [M+H]⁺
(93) 1-[2-(2-Cyanomethylamino-phenyl)-2-oxo-ethyl]-3-methyl-7-(3-methyl-2-buten-1-yl)-8-(3-amino-piperidin-1-yl)-xanthin
   (Durchführung mit isopropanolischer Salzsäure 5-6 M in Methylenchlorid)
   R_{f}-Wert: 0.50 (Reversed Phase DC-Fertigplatte (E. Merck), Acetonitril/Wasser/ Trifluoressigsäure = 50:50:1)
   Massenspektrum (ESI⁺): m/z = 505 [M+H]⁺
(94) 1-(2-{2-[(Isopropylaminocarbonyl)methoxy]-phenyl}-2-oxo-ethyl)-3-methyl-7-(2-butin-1-yl)-8-(3-amino-piperidin-1-yl)-xanthin
   R_{f}-Wert: 0.30 (Kieselgel, Methylenchlorid/Methanol = 9:1)
   Massenspektrum (ESI⁺): m/z = 550 [M+H]⁺
(95) 1-[(Isochinolin-1-yl)methyl]-3-[(methoxycarbonyl)methyl]-7-(3-methyl-2-buten-1-yl)-8-(3-amino-piperidin-1-yl)-xanthin
   R_{f}-Wert: 0.21 (Kieselgel, Methylenchlorid/Methanol/konz. wässriges Ammoniak = 90:10:1)
   Massenspektrum (ESI⁺): m/z = 532 [M+H]⁺
(96) 1-[2-(2-Acetylamino-phenyl)-2-oxo-ethyl]-3-methyl-7-((E)-2-buten-1-yl)-8-(3-amino-piperidin-1-yl)-xanthin
   (Produkt enthält ca. 10 % Z-Isomer)
   Massenspektrum (ESI⁺): m/z = 494 [M+H]⁺
(97) 1-(2-Phenyl-2-oxo-ethyl)-3-methyl-7-((E)-2-buten-1-yl)-8-(3-amino-piperidin-1-yl)-xanthin
   (Produkt enthält ca. 25 % Z-Isomer)
   R_{f}-Wert: 0.30 (Kieselgel, Methylenchlorid/Methanol = 9:1)
   Massenspektrum (ESI⁺): m/z = 437 [M+H]⁺
(98) 1-(2-{2-[(Isopropyloxycarbonyl)methoxy]-phenyl}-2-oxo-ethyl)-3-methyl-7-(3-methyl-2-buten-1-yl)-8-(3-amino-piperidin-1-yl)-xanthin
   Massenspektrum (ESI⁺): m/z = 567 [M+H]⁺
(99) 1-(2-{2-[(Methylaminocarbonyl)methoxy]-phenyl}-2-oxo-ethyl)-3-methyl-7-(2-butin-1-yl)-8-((*R*)-3-amino-piperidin-1-yl)-xanthin
   Massenspektrum (ESI⁺): m/z = 522 [M+H]⁺
(100) 1-(2-{2-[(Isopropylcarbonyl)amino]-phenyl}-2-oxo-ethyl)-3-methyl-7-((E)-2-buten-1-yl)-8-(3-amino-piperidin-1-yl)-xanthin
   (Produkt enthält ca. 10 % Z-Isomer)
   Massenspektrum (ESI⁺): m/z = 522 [M+H]⁺
(101) 1-(2-{2-[(Methylaminocarbonyl)methoxy]-phenyl}-2-oxo-ethyl)-3-methyl-7-((E)-2-buten-1-yl)-8-(3-amino-piperidin-1-yl)-xanthin
   (Produkt enthält ca. 8 % Z-Isomer)
   R_{f}-Wert: 0.51 (Reversed Phase DC-Fertigplatte (E. Merck), Acetonitril/Wasser/ Trifluoressigsäure = 50:50:0.1)
   Massenspektrum (ESI⁺): m/z = 524 [M+H]⁺
(102) 1-(2-{2-[(Ethylaminocarbonyl)methoxy]-phenyl}-2-oxo-ethyl)-3-methyl-7-(2-butin-1-yl)-8-((*S*)-3-amino-piperidin-1-yl)-xanthin
   Massenspektrum (ESI⁺): m/z = 536 [M+H]⁺
(103) 1-[2-(2-{[(Ethoxycarbonylamino)carbonyl]amino}-phenyl)-2-oxo-ethyl]-3-methyl-7-(3-methyl-2-buten-1-yl)-8-(3-amino-piperidin-1-yl)-xanthin
   Massenspektrum (ESI⁺): m/z = 581 [M+H]⁺
(104) 1-[2-(2-Amino-phenyl)-2-oxo-ethyl]-3-methyl-7-((E)-2-buten-1-yl)-8-(3-amino-piperidin-1-yl)-xanthin
   R_{f}-Wert: 0.54 (Reversed Phase DC-Fertigplatte (E. Merck), Acetonitril/Wasser/ Trifluoressigsäure = 50:50:0.1)
   Massenspektrum (ESI⁺): m/z = 452 [M+H]⁺
(105) 1-[2-(2-Acetylamino-phenyl)-2-oxo-ethyl]-3-methyl-7-(3-methyl-2-buten-1-yl)-8-((S)-3-amino-piperidin-1-yl)-xanthin
   R_{f}-Wert: 0.48 (Reversed Phase DC-Fertigplatte (E. Merck), Acetonitril/Wasser/ Trifluoressigsäure = 50:50:0.1)
   Massenspektrum (ESI⁺): m/z = 508 [M+H]⁺
(106) 1-[2-(2-Amino-phenyl)-2-oxo-ethyl]-3-methyl-7-(2-butin-1-yl)-8-(3-amino-piperidin-1-yl)-xanthin
   R_{f}-Wert: 0.31 (Kieselgel, Methylenchlorid/Methanol/konz. wässriges Ammoniak = 90:10:1)
   Massenspektrum (ESI⁺): m/z = 450 [M+H]⁺
(107) 1-(2-{2-[(Methylaminocarbonyl)methoxy]-phenyl}-2-oxo-ethyl)-3-methyl-7-(2-butin-1-yl)-8-((*S*)-3-amino-piperidin-1-yl)-xanthin
   Massenspektrum (ESI⁺): m/z = 522 [M+H]⁺
(108) 1-(2-Phenyl-2-oxo-ethyl)-3-methyl-7-((E)-2-buten-1-yl)-8-((*R*)-3-amino-piperidin-1-yl)-xanthin
   (Produkt enthält ca. 22 % Z-Isomer)
   Massenspektrum (ESI⁺): m/z = 437 [M+H]⁺
(109) 1-(2-{2-[(Ethylaminocarbonyl)methoxy]-phenyl}-2-oxo-ethyl)-3-methyl-7-(2-butin-1-yl)-8-(3-amino-piperidin-1-yl)-xanthin
   R_{f}-Wert: 0.40 (Kieselgel, Methylenchlorid/Methanol = 9:1)
   Massenspektrum (ESI⁺): m/z = 536 [M+H]⁺
(110) 1-[2-(2-Acetylamino-phenyl)-2-oxo-ethyl]-3-methyl-7-(2-butin-1-yl)-8-(3-amino-piperidin-1-yl)-xanthin
   R_{f}-Wert: 0.23 (Kieselgel, Methylenchlorid/Methanol/konz. wässriges Ammoniak = 90:10:1)
   Massenspektrum (ESI⁺): m/z = 492 [M+H]⁺
(111) 1-(2-{2-[2-Oxo-2-(pyrrolidin-1-yl)-ethoxy]-phenyl}-2-oxo-ethyl)-3-methyl-7-(2-butin-1-yl)-8-(3-amino-piperidin-1-yl)-xanthin
   R_{f}-Wert: 0.20 (Kieselgel, Methylenchlorid/Methanol = 9:1)
   Massenspektrum (ESI⁺): m/z = 562 [M+H]⁺
(112) 1-(2-{2-[(Methylaminocarbonyl)methoxy]-phenyl}-2-oxo-ethyl)-3-methyl-7-(3-methyl-2-buten-1-yl)-8-((*S*)-3-amino-piperidin-1-yl)-xanthin
   Massenspektrum (ESI⁺): m/z = 538 [M+H]⁺
(113) 1-(2-Phenyl-2-oxo-ethyl)-3-methyl-7-(2-butin-1-yl)-8-((*R*)-3-amino-piperidin-1-yl)-xanthin
   Massenspektrum (ESI⁺): m/z = 435 [M+H]⁺
(114) 1-(2-{2-[(Ethylaminocarbonyl)methoxy]-phenyl}-2-oxo-ethyl)-3-methyl-7-((E)-2-buten-1-yl)-8-((R)-3-amino-piperidin-1-yl)-xanthin
   (Produkt enthält ca. 30 % Z-Isomer)
   Massenspektrum (ESI⁺): m/z = 538 [M+H]⁺
(115) 1-Methyl-7-(2-cyano-benzyl)-8-(3-amino-piperidin-1-yl)-xanthin
   Massenspektrum (ESI⁺): m/z = 380 [M+H]⁺
(116) 1-(2-{2-[(Isopropylcarbonyl)amino]-phenyl}-2-oxo-ethyl)-3-methyl-7-(3-methyl-2-buten-1-yl)-8-(3-amino-piperidin-1-yl)-xanthin
   R_{f}-Wert: 0.40 (Reversed Phase DC-Fertigplatte (E. Merck), Acetonitril/Wasser/ Trifluoressigsäure = 50:50:0.1)
   Massenspektrum (ESI⁺): m/z = 536 [M+H]⁺
(117) 1-(2-Phenyl-2-oxo-ethyl)-3-methyl-7-((E)-2-buten-1-yl)-8-((*S*)-3-amino-piperidin-1-yl)-xanthin
   (Produkt enthält ca. 23 % Z-Isomer)
   R_{f}-Wert: 0.42 (Reversed Phase DC-Fertigplatte (E. Merck), Acetonitril/Wasser/ Trifluoressigsäure = 50:50:0.1)
   Massenspektrum (ESI⁺): m/z = 437 [M+H]⁺
(118) 1-(2-{2-[(Isopropylcarbonyl)amino]-phenyl}-2-oxo-ethyl)-3-methyl-7-(2-butin-1-yl)-8-(3-amino-piperidin-1-yl)-xanthin
   R_{f}-Wert: 0.20 (Kieselgel, Methylenchlorid/Methanol/konz. wässriges Ammoniak = 90:10:1)
   Massenspektrum (ESI⁺): m/z = 520 [M+H]⁺
(119) 1-[2-(2-Acetylamino-phenyl)-2-oxo-ethyl]-3-methyl-7-(2-butin-1-yl)-8-((S)-3-amino-piperidin-1-yl)-xanthin
   R_{f}-Wert: 0.15 (Kieselgel, Methylenchlorid/Methanol/konz. wässriges Ammoniak = 90:10:1)
   Massenspektrum (ESI⁺): m/z = 492 [M+H]⁺
(120) 1-(2-{2-[(Isopropylcarbonyl)amino]-phenyl}-2-oxo-ethyl)-3-methyl-7-(2-butin-1-yl)-8-((S)-3-amino-piperidin-1-yl)-xanthin
   Massenspektrum (ESI⁺): m/z = 520 [M+H]⁺
(121) 1-(2-Phenyl-2-oxo-ethyl)-3-methyl-7-(2-methyl-allyl)-8-(3-amino-piperidin-1-yl)-xanthin
   R_{f}-Wert: 0.21 (Kieselgel, Methylenchlorid/Methanol/konz. wässriges Ammoniak = 90:10:1)
   Massenspektrum (ESI⁺): m/z = 437 [M+H]⁺
(122) 1-(2-Phenyl-2-oxo-ethyl)-3-methyl-7-(3-brom-allyl)-8-(3-amino-piperidin-1-yl)-xanthin
   R_{f}-Wert: 0.14 (Kieselgel, Methylenchlorid/Methanol/konz. wässriges Ammoniak = 90:10:1)
   Massenspektrum (ESI⁺): m/z = 501, 503 [M+H]⁺
(123) 1-(2-{2-[(Methoxycarbonyl)amino]-phenyl}-2-oxo-ethyl)-3-methyl-7-(3-methyl-2-buten-1-yl)-8-(3-amino-piperidin-1-yl)-xanthin
   R_{f}-Wert: 0.42 (Reversed Phase DC-Fertigplatte (E. Merck), Acetonitril/Wasser/ Trifluoressigsäure = 50:50:0.1)
   Massenspektrum (ESI⁺): m/z = 524 [M+H]⁺
(124) 1-(2-Phenyl-2-oxo-ethyl)-3-methyl-7-[(furan-2-yl)methyl]-8-(3-amino-piperidin-1-yl)-xanthin
   R_{f}-Wert: 0.23 (Kieselgel, Methylenchlorid/Methanol/konz. wässriges Ammoniak = 90:10:1)
   Massenspektrum (ESI⁺): m/z = 463 [M+H]⁺
(125) 1-(2-Phenyl-2-oxo-ethyl)-3-methyl-7-(2-chlor-allyl)-8-(3-amino-piperidin-1-yl)-xanthin
   R_{f}-Wert: 0.18 (Kieselgel, Methylenchlorid/Methanol/konz. wässriges Ammoniak = 90:10:1)
(126) 1-{2-[2-(1-Methoxycarbonyl-ethoxy)-phenyl]-2-oxo-ethyl}-3-methyl-7-(2-butin-1-yl)-8-(3-amino-piperidin-1-yl)-xanthin
   Massenspektrum (ESI⁺): m/z = 537 [M+H]⁺
(127) 1-{2-[2-(1-Aminocarbonyl-ethoxy)-phenyl]-2-oxo-ethyl}-3-methyl-7-(2-butin-1-yl)-8-(3-amino-piperidin-1-yl)-xanthin
   Massenspektrum (ESI⁺): m/z = 522 [M+H]⁺
(128) 1-(2-Phenyl-2-oxo-ethyl)-3-methyl-7-(2-butin-1-yl)-8-((*S*)-3-amino-piperidin-1-yl)-xanthin
   Massenspektrum (ESI⁺): m/z = 435 [M+H]⁺
(129) 1-[(3-Methyl-isochinolin-1-yl)methyl]-3-methyl-7-(2-butin-1-yl)-8-((*S*)-3-amino-piperidin-1-yl)-xanthin
   (Durchführung mit isopropanolischer Salzsäure 5-6 M in Methylenchlorid)
   Schmelzpunkt: 155-156.5°C
   Massenspektrum (ESI⁺): m/z = 472 [M+H]⁺
(130) 1-[(3-Methyl-isochinolin-1-yl)methyl]-3-methyl-7-(2-butin-1-yl)-8-((R)-3-amino-piperidin-1-yl)-xanthin
   (Durchführung mit isopropanolischer Salzsäure 5-6 M in Methylenchlorid)
   R_{f}-Wert: 0.52 (Kieselgel, Methylenchlorid/Methanol/konz. wässriges Ammoniak = 90:10:1)
   Massenspektrum (ESI⁺): m/z = 472 [M+H]⁺
(131) 1-[(4-Methyl-isochinolin-1-yl)methyl]-3-methyl-7-(2-butin-1-yl)-8-((*S*)-3-amino-piperidin-1-yl)-xanthin
   (Durchführung mit isopropanolischer Salzsäure 5-6 M in Methylenchlorid)
   R_{f}-Wert: 0.46 (Kieselgel, Methylenchlorid/Methanol/konz. wässriges Ammoniak = 90:10:1)
   Massenspektrum (ESI⁺): m/z = 472 [M+H]⁺
(132) 1-[(4-Methyl-isochinolin-1-yl)methyl]-3-methyl-7-(2-butin-1-yl)-8-((*R*)-3-amino-piperidin-1-yl)-xanthin
   (Durchführung mit isopropanolischer Salzsäure 5-6 M in Methylenchlorid)
   R_{f}-Wert: 0.46 (Kieselgel, Methylenchlorid/Methanol/konz. wässriges Ammoniak = 90:10:1)
   Massenspektrum (ESI⁺): m/z = 472 [M+H]⁺
(133) 1-[(4-Methyl-isochinolin-1-yl)methyl]-3-methyl-7-((E)-2-buten-1-yl)-8-((*S*)-3-amino-piperidin-1-yl)-xanthin
   (Durchführung mit isopropanolischer Salzsäure 5-6 M in Methylenchlorid)
   R_{f}-Wert: 0.48 (Kieselgel, Methylenchlorid/Methanol/konz. wässriges Ammoniak = 90:10:1)
   Massenspektrum (ESI⁺): m/z = 474 [M+H]⁺
(134) 1-[(4-Methyl-isochinolin-1-yl)methyl]-3-methyl-7-((E)-2-buten-1-yl)-8-((*R*)-3-amino-piperidin-1-yl)-xanthin
   (Durchführung mit isopropanolischer Salzsäure 5-6 M in Methylenchlorid)
   Schmelzpunkt: 167.5-172°C
   Massenspektrum (ESI⁺): m/z = 474 [M+H]⁺
(135) 1-[2-(2,3-Dihydro-benzo[1,4]dioxin-5-yl)-2-oxo-ethyl]-3-methyl-7-(2-butin-1-yl)-8-(3-(*R*)-amino-piperidin-1-yl)-xanthin
   (Durchführung mit isopropanolischer Salzsäure 5-6 M in Methylenchlorid)
   R_{f}-Wert: 0.34 (Kieselgel, Methylenchlorid/Methanol/konz. wässriges Ammoniak = 90:10:1)
   Massenspektrum (ESI⁺): m/z = 493 [M+H]⁺
(136) 1-[2-(2,3-Dihydro-benzo[1,4]dioxin-5-yl)-2-oxo-ethyl]-3-methyl-7-(2-butin-1-yl)-8-(3-(*S*)-amino-piperidin-1-yl)-xanthin
   (Durchführung mit isopropanolischer Salzsäure 5-6 M in Methylenchlorid)
   Massenspektrum (ESI⁺): m/z = 493 [M+H]⁺
(137) 1-[(4-Methoxy-naphthalin-1-yl)methyl]-3-methyl-7-(2-butin-1-yl)-8-((*S*)-3-amino-piperidin-1-yl)-xanthin
   (Durchführung mit isopropanolischer Salzsäure 5-6 M in Methylenchlorid)
   R_{f}-Wert: 0.52 (Kieselgel, Methylenchlorid/Methanol/konz. wässriges Ammoniak = 90:10:1)
   Massenspektrum (ESI⁺): m/z = 487 [M+H]⁺
(138) 1-[(4-Methoxy-naphthalin-1-yl)methyl]-3-methyl-7-(2-butin-1-yl)-8-((*R*)-3-amino-piperidin-1-yl)-xanthin
   (Durchführung mit isopropanolischer Salzsäure 5-6 M in Methylenchlorid)
   R_{f}-Wert: 0.52 (Kieselgel, Methylenchlorid/Methanol/konz. wässriges Ammoniak = 90:10:1)
   Massenspektrum (ESI⁺): m/z = 487 [M+H]⁺
(139) 1-[2-(Benzo[1,3]dioxol-4-yl)-2-oxo-ethyl]-3-methyl-7-(2-butin-1-yl)-8-((*R*)-3-am ino-piperidin-1-yl)-xanth in
   (Durchführung mit isopropanolischer Salzsäure 5-6 M in Methylenchlorid)
   R_{f}-Wert: 0.41 (Kieselgel, Methylenchlorid/Methanol/konz. wässriges Ammoniak = 90:10:1)
   Massenspektrum (ESI⁺): m/z = 479 [M+H]⁺
(140) 1-[2-(Benzo[1,3]dioxol-4-yl)-2-oxo-ethyl]-3-methyl-7-(2-butin-1-yl)-8-((S)-3-amino-piperidin-1-yl)-xanthin
   (Durchführung mit isopropanolischer Salzsäure 5-6 M in Methylenchlorid)
   Massenspektrum (ESI⁺): m/z = 479 [M+H]⁺
(141) 1-[(4-Methyl-chinazolin-2-yl)methyl]-3-methyl-7-(2-butin-1-yl)-8-(3-(S)-amino-piperidin-1-yl)-xanthin
   (Durchführung mit isopropanolischer Salzsäure 5-6 M in Methylenchlorid)
   R_{f}-Wert: 0.51 (Kieselgel, Methylenchlorid/Methanol/konz. wässriges Ammoniak = 90:10:1)
   Massenspektrum (ESI⁺): m/z = 473 [M+H]⁺
(142) 1-[(4-Methyl-chinazolin-2-yl)methyl]-3-methyl-7-(2-butin-1-yl)-8-(3-(R)-amino-piperidin-1-yl)-xanthin
   (Durchführung mit isopropanolischer Salzsäure 5-6 M in Methylenchlorid)
   Schmelzpunkt: 198-202°C
   Massenspektrum (ESI⁺): m/z = 473 [M+H]⁺
(143) 1-[2-(3-Methyl-2-oxo-2,3-dihydro-benzooxazol-4-yl)-2-oxo-ethyl]-3-methyl-7-(3-methyl-2-buten-1-yl)-8-(3-amino-piperidin-1-yl)-xanthin
   (Durchführung mit isopropanolischer Salzsäure 5-6 M in Methylenchlorid)
   R_{f}-Wert: 0.53 (Kieselgel, Methylenchlorid/Methanol/konz. wässriges Ammoniak = 90:10:1)
   Massenspektrum (ESI⁺): m/z = 522 [M+H]⁺
(144) 1-(2-{2-[(Ethylaminocarbonyl)methoxy]-phenyl}-2-oxo-ethyl)-3-methyl-7-((E)-2-buten-1-yl)-8-((*S*)-3-amino-piperidin-1-yl)-xanthin
   Massenspektrum (ESI⁺): m/z = 538 [M+H]⁺
(145) 1-(2-{2-[(Isopropylcarbonyl)amino]-phenyl}-2-oxo-ethyl)-3-methyl-7-((E)-2-buten-1-yl)-8-((*S*)-3-amino-piperidin-1-yl)-xanthin
   R_{f}-Wert: 0.49 (Reversed Phase DC-Fertigplatte (E. Merck), Acetonitril/Wasser/ Trifluoressigsäure = 50:50:0.1)
   Massenspektrum (ESI⁺): m/z = 522 [M+H]⁺
(146) 1-(2-{2-[(Ethylcarbonyl)amino]-phenyl}-2-oxo-ethyl)-3-methyl-7-((E)-2-buten-1-yl)-8-((*R*)-3-amino-piperidin-1-yl)-xanthin
   Massenspektrum (ESI⁺): m/z = 508 [M+H]⁺
(147) 1-[2-(2-Acetylamino-phenyl)-2-oxo-ethyl]-3-methyl-7-((E)-2-buten-1-yl)-8-((R)-3-amino-piperidin-1-yl)-xanthin
   Massenspektrum (ESI⁺): m/z = 494 [M+H]⁺
(148) 1-(2-{2-[(Methylaminocarbonyl)methoxy]-phenyl}-2-oxo-ethyl)-3-methyl-7-((E)-2-buten-1-yl)-8-((*R*)-3-amino-piperidin-1-yl)-xanthin
   Massenspektrum (ESI⁺): m/z = 524 [M+H]⁺
(149) 1-[2-(2-Acetylamino-phenyl)-2-oxo-ethyl]-3-methyl-7-((E)-2-buten-1-yl)-8-((*S*)-3-amino-piperidin-1-yl)-xanthin
   R_{f}-Wert: 0.49 (Reversed Phase DC-Fertigplatte (E. Merck), Acetonitril/Wasser/ Trifluoressigsäure = 50:50:0.1)
   Massenspektrum (ESI⁺): m/z = 494 [M+H]⁺
(150) 1-(2-{2-[(Methylaminocarbonyl)methoxy]-phenyl}-2-oxo-ethyl)-3-methyl-7-((E)-2-buten-1-yl)-8-((*S*)-3-amino-piperidin-1-yl)-xanthin
   Massenspektrum (ESI⁺): m/z = 524 [M+H]⁺
(151) 1-(2-{2-[(Isopropylcarbonyl)amino]-phenyl}-2-oxo-ethyl)-3-methyl-7-(2-butin-1-yl)-8-((*R*)-3-amino-piperidin-1-yl)-xanthin
   Massenspektrum (ESI⁺): m/z = 520 [M+H]⁺
(152) 1-(2-{2-[(Isopropylcarbonyl)amino]-phenyl}-2-oxo-ethyl)-3-methyl-7-((E)-2-buten-1-yl)-8-((R)-3-amino-piperidin-1-yl)-xanthin
   Massenspektrum (ESI⁺): m/z = 522 [M+H]⁺
(153) 1-(2-{2-[2-(Morpholin-4-yl)-2-oxo-ethoxy]-phenyl}-2-oxo-ethyl)-3-methyl-7-(2-butin-1-yl)-8-(3-amino-piperidin-1-yl)-xanthin
   Massenspektrum (ESI⁺): m/z = 578 [M+H]⁺
(154) 1-(2-{2-[(Ethylcarbonyl)amino]-phenyl}-2-oxo-ethyl)-3-methyl-7-((E)-2-buten-1-yl)-8-((*S*)-3-amino-piperidin-1-yl)-xanthin
   R_{f}-Wert: 0.50 (Reversed Phase DC-Fertigplatte (E. Merck), Acetonitril/Wasser/ Trifluoressigsäure = 50:50:0.1)
   Massenspektrum (ESI⁺): m/z = 508 [M+H]⁺
(155) 1-(2-{2-[(Ethylcarbonyl)amino]-phenyl}-2-oxo-ethyl)-3-methyl-7-(2-butin-1-yl)-8-((*R*)-3-amino-piperidin-1-yl)-xanthin
   Massenspektrum (ESI⁺): m/z = 506 [M+H]⁺
(156) 1-(2-{2-[(Ethylcarbonyl)amino]-phenyl}-2-oxo-ethyl)-3-methyl-7-(2-butin-1-yl)-8-((S)-3-amino-piperidin-1-yl)-xanthin
   R_{f}-Wert: 0.20 (Kieselgel, Methylenchlorid/Methanol/konz. wässriges Ammoniak = 90:10:1)
   Massenspektrum (ESI⁺): m/z = 506 [M+H]⁺
(157) 1-[2-(2-Acetylamino-phenyl)-2-oxo-ethyl]-3-methyl-7-(2-butin-1-yl)-8-((*R*)-3-amino-piperidin-1-yl)-xanthin
   Massenspektrum (ESI⁺): m/z = 492 [M+H]⁺
(158) 1-[2-(2-Nitro-3-methoxy-phenyl)-2-oxo-ethyl]-3-methyl-7-(3-methyl-2-buten-1-yl)-8-(3-amino-piperidin-1-yl)-xanthin
   (Durchführung mit isopropanolischer Salzsäure 5-6 M in Methylenchlorid)
   R_{f}-Wert: 0.49 (Kieselgel, Methylenchlorid/Methanol/konz. wässriges Ammoniak = 90:10:1)
   Massenspektrum (ESI⁺): m/z = 526 [M+H]⁺
(159) 1-(2-{2-[(Phenylcarbonyl)amino]-phenyl}-2-oxo-ethyl)-3-methyl-7-((E)-2-buten-1-yl)-8-(3-amino-piperidin-1-yl)-xanthin
   R_{f}-Wert: 0.49 (Reversed Phase DC-Fertigplatte (E. Merck), Acetonitril/Wasser/ Trifluoressigsäure = 50:50:0.1)
   Massenspektrum (ESI⁺): m/z = 556 [M+H]⁺
(160) 1-[(2-Acetyl-benzofuran-3-yl)methyl]-3-methyl-7-(2-butin-1-yl)-8-(3-amino-piperidin-1-yl)-xanthin
   (Entsteht als Hauptprodukt bei der Behandlung von 1-{2-[2-(2-Oxo-propoxy)-phenyl]-2-oxo-ethyl}-3-methyl-7-(2-butin-1-yl)-8-[3-(tert.-butyloxycarbonylamino)-piperidin-1-yl]-xanthin mit Trifluoressigsäure in Methylenchlorid)
   Massenspektrum (ESI⁺): m/z = 489 [M+H]⁺
(161) 1-{2-[2-(1-Ethoxycarbonyl-1-methyl-ethoxy)-phenyl]-2-oxo-ethyl}-3-methyl-7-(2-butin-1-yl)-8-(3-amino-piperidin-1-yl)-xanthin
   Massenspektrum (ESI⁺): m/z = 565 [M+H]⁺
(162) 1-[2-(2-Amino-3-methoxy-phenyl)-2-oxo-ethyl]-3-methyl-7-(3-methyl-2-buten-1-yl)-8-(3-amino-piperidin-1-yl)-xanthin
   (Durchführung mit isopropanolischer Salzsäure 5-6 M in Methylenchlorid)
   R_{f}-Wert: 0.38 (Kieselgel, Methylenchlorid/Methanol/konz. wässriges Ammoniak = 90:10:1)
   Massenspektrum (ESI⁺): m/z = 496 [M+H]⁺
(163) 1-[(4-Dimethylamino-chinazolin-2-yl)methyl]-3-methyl-7-(2-butin-1-yl)-8-(3-amino-piperidin-1-yl)-xanthin
   R_{f}-Wert: 0.30 (Kieselgel, Methylenchlorid/Methanol/konz. wässriges Ammoniak = 90:10:0.1)
   Massenspektrum (ESI⁺): m/z = 502 [M+H]⁺
(164) 1-[2-(2-Oxo-2,3-dihydro-benzooxazol-7-yl)-2-oxo-ethyl]-3-methyl-7-(3-methyl-2-buten-1-yl)-8-(3-amino-piperidin-1-yl)-xanthin
   R_{f}-Wert: 0.42 (Kieselgel, Methylenchlorid/Methanol/konz. wässriges Ammoniak = 90:10:1)
   Massenspektrum (ESI⁺): m/z = 508 [M+H]⁺
(165) 1-(2-{2-[(Ethoxycarbonyl)methylamino]-phenyl}-2-oxo-ethyl)-3-methyl-7-((E)-2-buten-1-yl)-8-((R)-3-amino-piperidin-1-yl)-xanthin
   R_{f}-Wert: 0.51 (Reversed Phase DC-Fertigplatte (E. Merck), Acetonitril/Wasser/ Trifluoressigsäure = 50:50:0.1)
   Massenspektrum (ESI⁺): m/z = 538 [M+H]⁺
(166) 1-(2-Phenyl-2-oxo-ethyl)-3-methyl-7-((Z)-2-methyl-2-buten-1-yl)-8-(3-amino-piperidin-1-yl)-xanthin
   R_{f}-Wert: 0.29 (Kieselgel, Methylenchlorid/Methanol/konz. wässriges Ammoniak = 90:10:1)
   Massenspektrum (ESI⁺): m/z = 451 [M+H]⁺
(167) 1-(2-Phenyl-2-oxo-ethyl)-3-methyl-7-((E)-2-methyl-2-buten-1-yl)-8-(3-amino-piperidin-1-yl)-xanthin
   R_{f}-Wert: 0.59 (Kieselgel, Methylenchlorid/Methanol/konz. wässriges Ammoniak = 80:20:1)
   Massenspektrum (ESI⁺): m/z = 451 [M+H]⁺
(168) 1-[(Imidazo[1,2-a]pyridin-2-yl)methyl]-3-methyl-7-(2-butin-1-yl)-8-(3-amino-piperidin-1-yl)-xanthin
   (Durchführung mit isopropanolischer Salzsäure 5-6 M in Methylenchlorid)
   R_{f}-Wert: 0.47 (Kieselgel, Methylenchlorid/Methanol/konz. wässriges Ammoniak = 90:10:1)
   Massenspektrum (ESI⁺): m/z = 447 [M+H]⁺
(169) 1-[(Chinoxalin-2-yl)methyl]-3-methyl-7-(2-butin-1-yl)-8-(3-amino-piperidin-1-yl)-xanthin
   (Durchführung mit isopropanolischer Salzsäure 5-6 M in Methylenchlorid)
   R_{f}-Wert: 0.50 (Kieselgel, Methylenchlorid/Methanol/konz. wässriges Ammoniak = 90:10:1)
   Massenspektrum (ESI⁺): m/z = 459 [M+H]⁺
(170) 1-[2-(1,3-Dimethyl-2-oxo-2,3-dihydro-1H-benzoimidazol-4-yl)-2-oxo-ethyl]-3-methyl-7-(2-butin-1-yl)-8-(3-amino-piperidin-1-yl)-xanthin
   (Durchführung mit isopropanolischer Salzsäure 5-6 M in Methylenchlorid)
   R_{f}-Wert: 0.55 (Kieselgel, Methylenchlorid/Methanol/konz. wässriges Ammoniak = 90:10:1)
   Massenspektrum (ESI⁺): m/z = 519 [M+H]⁺
(171) 1-[(Chinoxalin-6-yl)methyl]-3-methyl-7-(2-butin-1-yl)-8-(3-amino-piperidin-1-yl)-xanthin
   (Durchführung mit isopropanolischer Salzsäure 5-6 M in Methylenchlorid)
   Massenspektrum (ESI⁺): m/z = 459 [M+H]⁺
(172) 1-[(2-Cyano-benzofuran-3-yl)methyl]-3-methyl-7-(2-butin-1-yl)-8-(3-amino-piperidin-1-yl)-xanthin
   Massenspektrum (ESI⁺): m/z = 472 [M+H]⁺
(173) 1-[2-(2-Oxo-2,3-dihydro-benzooxazol-4-yl)-2-oxo-ethyl]-3-methyl-7-(2-butin-1-yl)-8-(3-amino-piperidin-1-yl)-xanthin
   Massenspektrum (ESI⁺): m/z = 492 [M+H]⁺
   R_{f}-Wert: 0.47 (Kieselgel, Methylenchlorid/Methanol/konz. wässriges Ammoniak = 90:10:1)
(174) 1-[(3-Methyl-chinoxalin-2-yl)methyl]-3-methyl-7-(2-butin-1-yl)-8-(3-amino-piperidin-1-yl)-xanthin
   Schmelzpunkt: 188.5-191 °C
   Massenspektrum (ESI⁺): m/z = 473 [M+H]⁺
(175) 1-[(3-Phenyl-isochinolin-1-yl)methyl]-3-methyl-7-(2-butin-1-yl)-8-(3-amino-piperidin-1-yl)-xanthin
   (Durchführung mit isopropanolischer Salzsäure 5-6 M in Methylenchlorid)
   R_{f}-Wert: 0.45 (Reversed Phase DC-Fertigplatte (E. Merck), Acetonitril/Wasser/ Trifluoressigsäure = 50:50:1)
   Massenspektrum (ESI⁺): m/z = 534 [M+H]⁺
(176) 1-(2-{2-[(Methansulfinyl)methoxy]-phenyl}-2-oxo-ethyl)-3-methyl-7-(2-butin-1-yl)-8-(3-amino-piperidin-1-yl)-xanthin x Trifluoressigsäure
   Massenspektrum (ESI⁺): m/z = 527 [M+H]⁺
(177) 1-[(Benzofuran-3-yl)methyl]-3-methyl-7-(2-butin-1-yl)-8-(3-amino-piperidin-1-yl)-xanthin
   (Entsteht bei der Behandlung 1-{[2-(tert.-Butylcarbonyl)-benzofuran-3-yl]methyl}-3-methyl--yl)-8-[3-(tert.-butyloxycarbonylamino)-piperidin-1-yl]-xanthin mit Trifluoressigsäure in Methylenchlorid)
   Massenspektrum (ESI⁺): m/z = 447 [M+H]⁺
(178) 1-[(3,4-Dimethyl-isochinolin-1-yl)methyl]-3-methyl-7-(2-butin-1-yl)-8-(3-amino-piperidin-1-yl)-xanthin-hydrochlorid
   (Durchführung mit isopropanolischer Salzsäure 5-6 M in Methylenchlorid)
   R_{f}-Wert: 0.75 (Aluminiumoxid, Methylenchlorid/Methanol = 10:1)
   Massenspektrum (ESI⁺): m/z = 486 [M+H]⁺
(179) 1-[(Benzofuran-2-yl)methyl]-3-methyl-7-(2-butin-1-yl)-8-((*R*)-3-amino-piperidin-1-yl)-xanthin
   Massenspektrum (ESI⁺): m/z = 447 [M+H]⁺
(180) 1-{[4-(Morpholin-4-yl)-chinazolin-2-yl]methyl}-3-methyl-7-(2-butin-1-yl)-8-(3-amino-piperidin-1-yl)-xanthin
   (Durchführung mit isopropanolischer Salzsäure 5-6 M in Methylenchlorid)
   R_{f}-Wert: 0.45 (Kieselgel, Methylenchlorid/Methanol/konz. wässriges Ammoniak = 90:10:0.1)
   Massenspektrum (ESI⁺): m/z = 544 [M+H]⁺
(181) 1-{[4-(Piperidin-1-yl)-chinazolin-2-yl]methyl}-3-methyl-7-(2-butin-1-yl)-8-(3-amino-piperidin-1-yl)-xanthin
   (Durchführung mit isopropanolischer Salzsäure 5-6 M in Methylenchlorid)
   R_{f}-Wert: 0.55 (Kieselgel, Methylenchlorid/Methanol/konz. wässriges Ammoniak = 90:10:0.1)
   Massenspektrum (ESI⁺): m/z = 542 [M+H]⁺
(182) 1-{[4-(Piperazin-1-yl)-chinazolin-2-yl]methyl}-3-methyl-7-(2-butin-1-yl)-8-(3-amino-piperidin-1-yl)-xanthin
   (Durchführung mit isopropanolischer Salzsäure 5-6 M in Methylenchlorid)
   R_{f}-Wert: 0.23 (Kieselgel, Methylenchlorid/Methanol/konz. wässriges Ammoniak = 90:10:0.1)
   Massenspektrum (ESI⁺): m/z = 543 [M+H]⁺
(183) 1-{[4-(Pyrrolidin-1-yl)-chinazolin-2-yl]methyl}-3-methyl-7-(2-butin-1-yl)-8-(3-amino-piperidin-1-yl)-xanthin
   (Durchführung mit isopropanolischer Salzsäure 5-6 M in Methylenchlorid)
   R_{f}-Wert: 0.50 (Kieselgel, Methylenchlorid/Methanol/konz. wässriges Ammoniak = 90:10:0.1)
   Massenspektrum (ESI⁺): m/z = 528 [M+H]⁺
(184) 1-[2-(3-Methyl-2-oxo-2,3-dihydro-1*H*-benzoimidazol-4-yl)-2-oxo-ethyl]-3-methyl-7-(2-butin-1-yl)-8-(3-amino-piperidin-1-yl)-xanthin
   (Durchführung mit isopropanolischer Salzsäure 5-6 M in Methylenchlorid)
   R_{f}-Wert: 0.43 (Kieselgel, Methylenchlorid/Methanol/konz. wässriges Ammoniak = 90:10:1)
   Massenspektrum (ESI⁺): m/z = 505 [M+H]⁺
(185) 1-[(4-Cyano-naphthalin-1-yl)methyl]-3-methyl-7-(2-butin-1-yl)-8-((R)-3-amino-piperidin-1-yl)-xanthin
   R_{f}-Wert: 0.27 (Kieselgel, Methylenchlorid/Methanol/konz. wässriges Ammoniak = 90:10:1)
   Massenspektrum (ESI⁺): m/z = 482 [M+H]⁺
(186) 1-[(Imidazo[1,2-a]pyridin-3-yl)methyl]-3-methyl-7-(2-butin-1-yl)-8-(3-amino-piperidin-1-yl)-xanthin
   (Durchführung mit isopropanolischer Salzsäure 5-6 M in Methylenchlorid)
   R_{f}-Wert: 0.37 (Kieselgel, Methylenchlorid/Methanol/konz. wässriges Ammoniak = 90:10:1)
   Massenspektrum (ESI⁺): m/z = 447 [M+H]⁺
(187) 1-[(8-Methyl-imidazo[1,2-a]pyridin-2-yl)methyl]-3-methyl-7-(2-butin-1-yl)-8-(3-amino-piperidin-1-yl)-xanthin
   (Durchführung mit isopropanolischer Salzsäure 5-6 M in Methylenchlorid)
   R_{f}-Wert: 0.46 (Kieselgel, Methylenchlorid/Methanol/konz. wässriges Ammoniak = 90:10:1)
   Massenspektrum (ESI⁺): m/z = 461 [M+H]⁺
(188) 1-[(4-Amino-chinazolin-2-yl)methyl]-3-methyl-7-(2-butin-1-yl)-8-(3-amino-piperidin-1-yl)-xanthin
   (Durchführung mit isopropanolischer Salzsäure 5-6 M in Methylenchlorid)
   R_{f}-Wert: 0.40 (Kieselgel, Methylenchlorid/Methanol/konz. wässriges Ammoniak = 90:10:0.1)
   Massenspektrum (ESI⁺): m/z = 474 [M+H]⁺
(189) 1-(2-Phenyl-2-oxo-ethyl)-3-methyl-7-((Z)-2-buten-1-yl)-8-(3-amino-piperidin-1-yl)-xanthin
   Massenspektrum (ESI⁺): m/z = 437 [M+H]⁺
(190) 1-[(8-Methoxy-chinolin-5-yl)methyl]-3-methyl-7-(2-butin-1-yl)-8-(3-amino-piperidin-1-yl)-xanthin x 2 Trifluoressigsäure
   R_{f}-Wert: 0.45 (Kieselgel, Methylenchlorid/Methanol = 5:1)
   Massenspektrum (ESI⁺): m/z = 488 [M+H]⁺
(191) 1-[(5-Methoxy-chinolin-8-yl)methyl]-3-methyl-7-(2-butin-1-yl)-8-(3-amino-piperidin-1-yl)-xanthin x Trifluoressigsäure
   R_{f}-Wert: 0.20 (Kieselgel, Essigester/Methanol = 1:1)
   Massenspektrum (ESI⁺): m/z = 488 [M+H]⁺
(192) 1-[(4-Phenyl-chinazolin-2-yl)methyl]-3-methyl-7-(2-butin-1-yl)-8-(3-amino-piperidin-1-yl)-xanthin
   (Durchführung mit isopropanolischer Salzsäure 5-6 M in Methylenchlorid)
   R_{f}-Wert: 0.60 (Kieselgel, Methylenchlorid/Methanol/konz. wässriges Ammoniak = 90:10:1)
   Massenspektrum (ESI⁺): m/z = 535 [M+H]⁺
(193) 1-[(7-Methyl-imidazo[1,2-a]pyridin-2-yl)methyl]-3-methyl-7-(2-butin-1-yl)-8-(3-amino-piperidin-1-yl)-xanthin
   (Durchführung mit isopropanolischer Salzsäure 5-6 M in Methylenchlorid)
   R_{f}-Wert: 0.48 (Kieselgel, Methylenchlorid/Methanol/konz. wässriges Ammoniak = 90:10:1)
   Massenspektrum (ESI⁺): m/z = 461 [M+H]⁺
(194) 1-[(2-Cyclopropyl-chinazolin-4-yl)methyl]-3-methyl-7-[(1-cyclopenten-1-yl)methyl]-8-(3-amino-piperidin-1-yl)-xanthin
   (Durchführung mit isopropanolischer Salzsäure 5-6 M in Methylenchlorid)
   R_{f}-Wert: 0.55 (Reversed Phase DC-Fertigplatte (E. Merck), Acetonitril/Wasser/ Trifluoressigsäure = 50:50:1)
   Massenspektrum (ESI⁺): m/z = 527 [M+H]⁺
(195) 1-(2-Oxo-4-phenyl-butyl)-3-methyl-7-(2-butin-1-yl)-8-((R)-3-amino-piperidin-1-yl)-xanthin x Trifluoressigsäure
   Massenspektrum (ESI⁺): m/z = 463 [M+H]⁺
(196) 1-(2-{2-[(Methylaminocarbonyl)methylamino]-phenyl}-2-oxo-ethyl)-3-methyl-7-((E)-2-buten-1-yl)-8-((*R*)-3-amino-piperidin-1-yl)-xanthin
   R_{f}-Wert: 0.52 (Reversed Phase DC-Fertigplatte (E. Merck), Acetonitril/Wasser/ Trifluoressigsäure = 50:50:0.1)
   Massenspektrum (ESI⁺): m/z = 523 [M+H]⁺
(197) 1-[2-(2-Oxo-2,3-dihydro-1H-benzoimidazol-4-yl)-2-oxo-ethyl]-3-methyl-7-(2-butin-1-yl)-8-(3-amino-piperidin-1-yl)-xanthin
   R_{f}-Wert: 0.40 (Kieselgel, Methylenchlorid/Methanol/konz. wässriges Ammoniak = 90:10:1)
   Massenspektrum (ESI⁺): m/z = 491 [M+H]⁺
(198) 1-[(3-Difluormethyl-isochinolin-1-yl)methyl]-3-methyl-7-(2-butin-1-yl)-8-(3-amino-piperidin-1-yl)-xanthin x Trifluoressigsäure
   R_{f}-Wert: 0.75 (Kieselgel, Methylenchlorid/Methanol = 10:1)
   Massenspektrum (ESI⁺): m/z = 508 [M+H]⁺
(199) 1-[2-(2,2-Difluor-benzo[1,3]dioxol-4-yl)-2-oxo-ethyl]-3-methyl-7-(2-butin-1-yl)-8-((*R*)-3-amino-piperidin-1-yl)-xanthin
   (Durchführung mit isopropanolischer Salzsäure 5-6 M in Methylenchlorid)
   R_{f}-Wert: 0.80 (Kieselgel, Methylenchlorid/Methanol/konz. wässriges Ammoniak = 96:4:0.5)
   Massenspektrum (ESI⁺): m/z = 515 [M+H]⁺
(200) 1-[(3-Methyl-imidazo[1,2-a]pyridin-2-yl)methyl]-3-methyl-7-(2-butin-1-yl)-8-(3-amino-piperidin-1-yl)-xanthin
   R_{f}-Wet:0.45 (Kieselgel, Methylenchlorid/Methanol/konz. wässriges Ammoniak = 90:10:1)
   Massenspektrum (ESI⁺): m/z = 461 [M+H]⁺
(201) 1-[2-(2,2-Difluor-benzo[1,3]dioxol-4-yl)-2-oxo-ethyl]-3-methyl-7-(2-butin-1-yl)-8-((S)-3-amino-piperidin-1-yl)-xanthin
   (Durchführung mit isopropanolischer Salzsäure 5-6 M in Methylenchlorid)
   Massenspektrum (ESI⁺): m/z = 515 [M+H]⁺
(202) 1-[(5-Methyl-imidazo[1,2-a]pyridin-2-yl)methyl]-3-methyl-7-(2-butin-1-yl)-8-(3-amino-piperidin-1-yl)-xanthin
   (Durchführung mit isopropanolischer Salzsäure 5-6 M in Methylenchlorid)
   R_{f}-Wert: 0.53 (Kieselgel, Methylenchlorid/Methanol/konz. wässriges Ammoniak = 90:10:1)
   Massenspektrum (ESI⁺): m/z = 461 [M+H]⁺
(203) 1-[(6-Methyl-imidazo[1,2-a]pyridin-2-yl)methyl]-3-methyl-7-(2-butin-1-yl)-8-(3-amino-piperidin-1-yl)-xanthin
   (Durchführung mit isopropanolischer Salzsäure 5-6 M in Methylenchlorid)
   Schmelzpunkt: 176.5-178°C
   Massenspektrum (ESI⁺): m/z = 461 [M+H]⁺
(204) 1-[(3-Benzyl-imidazo[1,2-a]pyridin-2-yl)methyl]-3-methyl-7-(2-butin-1-yl)-8-(3-amino-piperidin-1-yl)-xanthin
   (Durchführung mit isopropanolischer Salzsäure 5-6 M in Methylenchlorid)
   Schmelzpunkt: 201-204°C
   Massenspektrum (ESI⁺): m/z = 537 [M+H]⁺
(205) 1-[(4-Isopropyl-chinazolin-2-yl)methyl]-3-methyl-7-(2-butin-1-yl)-8-(3-amino-piperidin-1-yl)-xanthin
   (Durchführung mit isopropanolischer Salzsäure 5-6 M in Methylenchlorid)
   R_{f}-Wert: 0.50 (Kieselgel, Methylenchlorid/Methanol/konz. wässriges Ammoniak = 90:10:0.1)
   Massenspektrum (ESI⁺): m/z = 501 [M+H]⁺
(206) 1-[(2,3-Dihydro-benzo[1,4]dioxin-6-yl)methyl]-3-methyl-7-(2-butin-1-yl)-8-(3-amino-piperidin-1-yl)-xanthin
   (Durchführung mit isopropanolischer Salzsäure 5-6 M in Methylenchlorid)
   R_{f}-Wert: 0.65 (Kieselgel, Methylenchlorid/Methanol/konz. wässriges Ammoniak = 90:10:1)
   Massenspektrum (ESI⁺): m/z = 465 [M+H]⁺
(207) 1-[(1-Methyl-1*H*-indol-2-yl)methyl]-3-methyl-7-(2-butin-1-yl)-8-(3-amino-piperidin-1-yl)-xanthin-hydrochlorid
   (Durchführung mit isopropanolischer Salzsäure 5-6 M in Methylenchlorid)
   R_{f}-Wert: 0.60 (Kieselgel, Methylenchlorid/Methanol/konz. wässriges Ammoniak = 90:10:1)
   Massenspektrum (ESI⁺): m/z = 460 [M+H]⁺
(208) 1-[(Chinolin-3-yl)methyl]-3-methyl-7-(2-butin-1-yl)-8-(3-amino-piperidin-1-yl)-xanthin
   (Durchführung mit isopropanolischer Salzsäure 5-6 M in Methylenchlorid)
   R_{f}-Wert: 0.50 (Kieselgel, Methylenchlorid/Methanol/konz. wässriges Ammoniak = 90:10:0.1)
   Massenspektrum (ESI⁺): m/z = 458 [M+H]⁺
(209) 1-[(3-Phenyl-imidazo[1,2-a]pyridin-2-yl)methyl]-3-methyl-7-(2-butin-1-yl)-8-(3-amino-piperidin-1-yl)-xanthin
   (Durchführung mit isopropanolischer Salzsäure 5-6 M in Methylenchlorid)
   R_{f}-Wert: 0.50 (Kieselgel, Methylenchlorid/Methanol/konz. wässriges Ammoniak = 90:10:1)
   Massenspektrum (ESI⁺): m/z = 523 [M+H]⁺
(210) 1-[(1*H*-Indol-2-yl)methyl]-3-methyl-7-(2-butin-1-yl)-8-(3-amino-piperidin-1-yl)-xanthin-hydrochlorid
   R_{f}-Wert: 0.55 (Kieselgel, Methylenchlorid/Methanol/konz. wässriges Ammoniak = 90:10:1)
   Massenspektrum (ESI⁺): m/z = 446 [M+H]⁺
(211) 1-[2-(Naphthalin-1-yl)-2-oxo-ethyl]-3-methyl-7-(2-butin-1-yl)-8-(3-amino-piperidin-1-yl)-xanthin
   (Durchführung mit isopropanolischer Salzsäure 5-6 M in Methylenchlorid)
   R_{f}-Wert: 0.60 (Kieselgel, Methylenchlorid/Methanol/konz. wässriges Ammoniak = 90:10:1)
   Massenspektrum (ESI⁺): m/z = 485 [M+H]⁺
(212) 1-[(5-Methoxy-isochinolin-8-yl)methyl]-3-methyl-7-(2-butin-1-yl)-8-(3-amino-piperidin-1-yl)-xanthin x Trifluoressigsäure
   R_{f}-Wert: 0.50 (Kieselgel, Methylenchlorid/Methanol = 5:1)
   Massenspektrum (ESI⁺): m/z = 488 [M+H]⁺
(213) 1-{[1-(1-Cyano-1-methyl-ethyl)-isochinolin-3-yl]methyl}-3-methyl-7-(2-butin-1-yl)-8-(3-amino-piperidin-1-yl)-xanthin
   (Durchführung mit isopropanolischer Salzsäure 5-6 M in Methylenchlorid)
   R_{f}-Wert: 0.25 (Reversed Phase DC-Fertigplatte (E. Merck), Acetonitril/Wasser/ Trifluoressigsäure = 50:50:1)
   Massenspektrum (ESI⁺): m/z = 525 [M+H]⁺
(214) 1-(2-Cyanoimino-2-phenyl-ethyl)-3-methyl-7-(2-butin-1-yl)-8-(3-amino-piperidin-1-yl)-xanthin x Trifluoressigsäure (E/Z-Gemisch)
   Massenspektrum (ESI⁺): m/z = 459 [M+H]⁺
(215) 1-[(1*H*-Benzoimidazol-2-yl)methyl]-3-methyl-7-(2-butin-1-yl)-8-(3-amino-piperidin-1-yl)-xanthin x Trifluoressigsäure
   Massenspektrum (ESI⁺): m/z = 447 [M+H]⁺
(216) 1-[(1-Methyl-1*H*-benzoimidazol-2-yl)methyl]-3-methyl-7-(2-butin-1-yl)-8-(3-amino-piperidin-1-yl)-xanthin
   Massenspektrum (ESI⁺): m/z = 461 [M+H]⁺
(217) 1-[(4-Phenyl-chinazolin-2-yl)methyl]-3-methyl-7-(2-butin-1-yl)-8-((R)-3-amino-piperidin-1-yl)-xanthin
   (Durchführung mit isopropanolischer Salzsäure 5-6 M in Methylenchlorid)
   Massenspektrum (ESI⁺): m/z = 535 [M+H]⁺
(218) 1-[(2,3-Dimethyl-chinoxalin-6-yl)methyl]-3-methyl-7-(2-butin-1-yl)-8-(3-amino-piperidin-1-yl)-xanthin
   (Durchführung mit isopropanolischer Salzsäure 5-6 M in Methylenchlorid)
   R_{f}-Wert: 0.50 (Kieselgel, Methylenchlorid/Methanol/konz. wässriges Ammoniak = 90:10:0.1)
   Massenspektrum (ESI⁺): m/z = 487 [M+H]⁺
(219) 1-[(2-Methyl-1*H*-benzoimidazol-5-yl)methyl]-3-methyl-7-yl)-8-(3-amino-piperidin-1-yl)-xanthin
   R_{f}-Wert: 0.35 (Kieselgel, Methylenchlorid/Methanol/konz. wässriges Ammoniak = 90:10:0.1)
   Massenspektrum (ESI⁺): m/z = 461 [M+H]⁺
(220) 1-[(4-Phenyl-chinazolin-2-yl)methyl]-3-methyl-7-(2-butin-1-yl)-8-((S)-3-amino-piperidin-1-yl)-xanthin
   (Durchführung mit isopropanolischer Salzsäure 5-6 M in Methylenchlorid)
   Massenspektrum (ESI⁺): m/z = 535 [M+H]⁺
(221) 1-[2-(Chinolin-8-yl-]-2-oxo-ethyl]-3-methyl-7-(2-butin-1-yl)-8-(3-amino-piperidin-1-yl)-xanthin
   (Durchführung mit isopropanolischer Salzsäure 5-6 M in Methylenchlorid)
   R_{f}-Wert: 0.48 (Kieselgel, Methylenchlorid/Methanol/konz. wässriges Ammoniak = 90:10:1)
   Massenspektrum (ESI⁺): m/z = 486 [M+H]⁺
(222) 1-[(3,4-Dimethyl-6,7-dihydro-5H-[2]pyrindin-1-yl)methyl]-3-methyl-7-(2-butin-1-yl)-8-(3-amino-piperidin-1-yl)-xanthin x Trifluoressigsäure
   R_{f}-Wert: 0.25 (Aluminiumoxid, Methylenchlorid/Methanol = 20:1)
   Massenspektrum (ESI⁺): m/z = 476 [M+H]⁺
(223) 1-[(3,4-Dimethyl-5,6,7,8-tetrahydro-isochinolin-1-yl)methyl]-3-methyl-7-(2-butin-1-yl)-8-(3-amino-piperidin-1-yl)-xanthin x Trifluoressigsäure
   R_{f}-Wert: 0.50 (Aluminiumoxid, Methylenchlorid/Methanol = 20:1)
   Massenspektrum (ESI⁺): m/z = 490 [M+H]⁺
(224) 1-[2-(1*H*-Indol-4-yl)-2-oxo-ethyl]-3-methyl-7-(2-butin-1-yl)-8-(3-amino-piperidin-1-yl)-xanthin
   R_{f}-Wert: 0.52 (Kieselgel, Methylenchlorid/Methanol/konz. wässriges Ammoniak = 90:10:1)
   Massenspektrum (ESI⁺): m/z = 474 [M+H]⁺
(225) 1-[(1*H*-Benzoimidazol-5-yl)methyl]-3-methyl-7-(2-butin-1-yl)-8-(3-amino-piperidin-1-yl)-xanthin
   R_{f}-Wert: 0.50 (Kieselgel, Methylenchlorid/Methanol/konz. wässriges Ammoniak = 90:10:1)
   Massenspektrum (ESI⁺): m/z = 447 [M+H]⁺
(226) 1-[(Pyrazolo[1,5-a]pyridin-2-yl)methyl]-3-methyl-7-(2-butin-1-yl)-8-(3-amino-piperidin-1-yl)-xanthin
   (Durchführung mit isopropanolischer Salzsäure 5-6 M in Methylenchlorid)
   R_{f}-Wert: 0.47 (Kieselgel, Methylenchlorid/Methanol/konz. wässriges Ammoniak = 90:10:1)
   Massenspektrum (ESI⁺): m/z = 447 [M+H]⁺
(227) 1-[(1-Methyl-2-oxo-1,2-dihydro-chinolin-6-yl)methyl]-3-methyl-7-(2-butin-1-yl)-8-(3-amino-piperidin-1-yl)-xanthin
   (Durchführung mit isopropanolischer Salzsäure 5-6 M in Methylenchlorid)
   R_{f}-Wert: 0.40 (Kieselgel, Methylenchlorid/Methanol/konz. wässriges Ammoniak = 90:10:1)
   Massenspektrum (ESI⁺): m/z = 488 [M+H]⁺
(228) 1-[(2-Oxo-1,2-dihydro-chinolin-6-yl)methyl]-3-methyl-7-(2-butin-1-yl)-8-(3-amino-piperidin-1-yl)-xanthin
   R_{f}-Wert: 0.23 (Kieselgel, Methylenchlorid/Methanol/konz. wässriges Ammoniak = 90:10:1)
   Massenspektrum (ESI⁺): m/z = 474 [M+H]⁺
(229) 1-[(2,3,8-Trimethyl-chinoxalin-6-yl)methyl]-3-methyl-7-(2-butin-1-yl)-8-(3-amino-piperidin-1-yl)-xanthin
   (Durchführung mit isopropanolischer Salzsäure 5-6 M in Methylenchlorid)
   R_{f}-Wert: 0.37 (Kieselgel, Methylenchlorid/Methanol/konz. wässriges Ammoniak = 90:10:1)
   Massenspektrum (ESI⁺): m/z = 501 [M+H]⁺
(230) 1-[(8-Methyl-chinoxalin-6-yl)methyl]-3-methyl-7-(2-butin-1-yl)-8-(3-amino-piperidin-1-yl)-xanthin
   (Durchführung mit isopropanolischer Salzsäure 5-6 M in Methylenchlorid)
   R_{f}-Wert: 0.35 (Kieselgel, Methylenchlorid/Methanol/konz. wässriges Ammoniak = 90:10:1)
   Massenspektrum (ESI⁺): m/z = 473 [M+H]⁺
(231) 1-[(4-Methyl-phthalazin-1-yl)methyl]-3-methyl-7-(2-butin-1-yl)-8-(3-amino-piperidin-1-yl)-xanthin
   (Durchführung mit isopropanolischer Salzsäure 5-6 M in Methylenchlorid)
   R_{f}-Wert: 0.55 (Reversed Phase DC-Fertigplatte (E. Merck), Acetonitril/Wasser/ Trifluoressigsäure = 50:50:1)
   Massenspektrum (ESI⁺): m/z = 473 [M+H]⁺
(232) 1-[(4-Brom-3-methoxy-isochinolin-1-yl)methyl]-3-methyl-7-(2-butin-1-yl)-8-(3-amino-piperidin-1-yl)-xanthin
   (Durchführung mit isopropanolischer Salzsäure 5-6 M in Methylenchlorid)
   R_{f}-Wert: 0.40 (Reversed Phase DC-Fertigplatte (E. Merck), Acetonitril/Wasser/ Trifluoressigsäure = 50:50:1)
   Massenspektrum (ESI⁺): m/z = 566, 568 [M+H]⁺
(233) 1-(2-Phenyl-2-oxo-ethyl)-3-methyl-7-((E)-1-buten-1-yl)-8-(3-amino-piperidin-1-yl)-xanthin
   R_{f}-Wert: 0.31 (Kieselgel, Methylenchlorid/Methanol/konz. wässriges Ammoniak = 90:10:1)
   Massenspektrum (ESI⁺): m/z = 437 [M+H]⁺
(234) 1-[(4-Difluormethoxy-naphthalin-1-yl)methyl]-3-methyl-7-(2-butin-1-yl)-8-((R)-3-amino-piperidin-1-yl)-xanthin
   R_{f}-Wert: 0.08 (Kieselgel, Methylenchlorid/Methanol/konz. wässriges Ammoniak = 95:5:1)
   Massenspektrum (ESI⁺): m/z = 523 [M+H]⁺
(235) 1-[2-(1*H*-Indol-7-yl)-2-oxo-ethyl]-3-methyl-7-(2-butin-1-yl)-8-(3-amino-piperidin-1-yl)-xanthin x Trifluoressigsäure
   R_{f}-Wert: 0.46 (Kieselgel, Methylenchlorid/Methanol/konz. wässriges Ammoniak = 90:10:1)
   Massenspektrum (ESI⁺): m/z = 474 [M+H]⁺
(236) 1-[(E)-3-(2-Nitro-phenyl)-2-propen-1-yl]-3-methyl-7-(2-butin-1-yl)-8-(3-amino-piperidin-1-yl)-xanthin
   Massenspektrum (ESI⁺): m/z = 478 [M+H]⁺
(237) 1-((E)-3-Pentafluorphenyl-2-propen-1-yl)-3-methyl-7-(2-butin-1-yl)-8-(3-amino-piperidin-1-yl)-xanthin
   Massenspektrum (ESI⁺): m/z = 523 [M+H]⁺
(238) 1-[(4-Nitro-naphthalin-1-yl)methyl]-3-methyl-7-(2-butin-1-yl)-8-((R)-3-amino-piperidin-1-yl)-xanthin
   R_{f}-Wert: 0.38 (Kieselgel, Methylenchlorid/Methanol/konz. wässriges Ammoniak = 90:10:1)
   Massenspektrum (ESI⁺): m/z = 502 [M+H]⁺
(239) 1-{[1-(2-Cyano-ethyl)-1H-benzoimidazol-2-yl]methyl}-3-methyl-7-(2-butin-1-yl)-8-(3-amino-piperidin-1-yl)-xanthin-hydrochlorid
   (Durchführung mit isopropanolischer Salzsäure 5-6 M in Methylenchlorid)
   R_{f}-Wert: 0.55 (Reversed Phase DC-Fertigplatte (E. Merck), Acetonitril/Wasser/ Trifluoressigsäure = 50:50:0.1)
   Massenspektrum (ESI⁺): m/z = 500 [M+H]⁺
(240) 1-({1-[(Methylaminocarbonyl)methyl]-1H-benzoimidazol-2-yl}methyl)-3-methyl-7-(2-butin-1-yl)-8-(3-amino-piperidin-1-yl)-xanthin x Trifluoressigsäure
   Massenspektrum (ESI⁺): m/z = 518 [M+H]⁺
(241) 1-[(1-Benzyl-1H-benzoimidazol-2-yl)methyl]-3-methyl-7-(2-butin-1-yl)-8-(3-amino-piperidin-1-yl)-xanthin
   R_{f}-Wert: 0.47 (Reversed Phase DC-Fertigplatte (E. Merck), Acetonitril/Wasser/ Trifluoressigsäure = 50:50:0.1)
   Massenspektrum (ESI⁺): m/z = 537 [M+H]⁺
(242) 1-[(Benzooxazol-2-y)methyl]-3-methyl-7-(2-butin-1-yl)-8-(3-amino-piperidin-1-yl)-xanthin x Trifluoressigsäure
   R_{f}-Wert: 0.50 (Reversed Phase DC-Fertigplatte (E. Merck), Acetonitril/Wasser/ Trifluoressigsäure = 50:50:0.1)
   Massenspektrum (ESI⁺): m/z = 448 [M+H]⁺
(243) 1-[(5-Nitro-benzooxazol-2-y)methyl]-3-methyl-7-(2-butin-1-yl)-8-(3-amino-piperidin-1-yl)-xanthin
   R_{f}-Wert: 0.49 (Reversed Phase DC-Fertigplatte (E. Merck), Acetonitril/Wasser/ Trifluoressigsäure = 50:50:0.1)
   Massenspektrum (ESI⁺): m/z = 493 [M+H]⁺
(244) 1-[(3-Methyl-isochinolin-1-yl)methyl]-3-methyl-7-(3-methyl-1-buten-1-yl)-8-((*R*)-3-amino-piperidin-1-yl)-xanthin
   R_{f}-Wert: 0.21 (Kieselgel, Methylenchlorid/Methanol/konz. wässriges Ammoniak = 90:10:1)
   Massenspektrum (ESI⁺): m/z = 488 [M+H]⁺
(245) 1-[(Chinolin-7-yl)methyl]-3-methyl-7-(2-butin-1-yl)-8-(3-amino-piperidin-1-yl)-xanthin
   (Durchführung mit isopropanolischer Salzsäure 5-6 M in Methylenchlorid)
   R_{f}-Wert: 0.55 (Kieselgel, Methylenchlorid/Methanol/konz. wässriges Ammoniak = 90:10:1)
   Massenspektrum (ESI⁺): m/z = 458 [M+H]⁺
(246) 1-[([1,5]Naphthyridin-2-yl)methyl]-3-methyl-7-(2-butin-1-yl)-8-(3-amino-piperidin-1-yl)-xanthin
   (Durchführung mit isopropanolischer Salzsäure 5-6 M in Methylenchlorid)
   R_{f}-Wert: 0.51 (Kieselgel, Methylenchlorid/Methanol/konz. wässriges Ammoniak = 90:10:1)
   Massenspektrum (ESI⁺): m/z = 459 [M+H]⁺
(247) 1-[(8-Methyl-chinoxalin-6-yl)methyl]-3-methyl-7-(2-butin-1-yl)-8-((R)-3-amino-piperidin-1-yl)-xanthin
   (Durchführung mit isopropanolischer Salzsäure 5-6 M in Methylenchlorid)
   R_{f}-Wert: 0.49 (Kieselgel, Methylenchlorid/Methanol/konz. wässriges Ammoniak = 90:10:1)
   Massenspektrum (ESI⁺): m/z = 473 [M+H]⁺
(248) 1-[(2,3,8-Trimethyl-chinoxalin-6-yl)methyl]-3-methyl-7-(2-butin-1-yl)-8-((*R*)-3-amino-piperidin-1-yl)-xanthin
   (Durchführung mit isopropanolischer Salzsäure 5-6 M in Methylenchlorid)
   R_{f}-Wert: 0.46 (Kieselgel, Methylenchlorid/Methanol/konz. wässriges Ammoniak = 90:10:1)
   Massenspektrum (ESI⁺): m/z = 501 [M+H]⁺
(249) 1-[([1,6]Naphthyridin-5-yl)methyl]-3-methyl-7-(2-butin-1-yl)-8-(3-amino-piperidin-1-yl)-xanthin
   (Durchführung mit isopropanolischer Salzsäure 5-6 M in Methylenchlorid)
   R_{f}-Wert: 0.50 (Kieselgel, Methylenchlorid/Methanol/konz. wässriges Ammoniak = 90:10:1)
   Massenspektrum (ESI⁺): m/z = 459 [M+H]⁺
(250) 1-[([1,8]Naphthyridin-2-yl)methyl]-3-methyl-7-(2-butin-1-yl)-8-(3-amino-piperidin-1-yl)-xanthin
   (Durchführung mit isopropanolischer Salzsäure 5-6 M in Methylenchlorid)
   R_{f}-Wert: 0.45 (Kieselgel, Methylenchlorid/Methanol/konz. wässriges Ammoniak = 90:10:1)
   Massenspektrum (ESI⁺): m/z = 459 [M+H]⁺
(251) 1-[(4-Fluor-naphthalin-1-yl)methyl]-3-methyl-7-(2-butin-1-yl)-8-((R)-3-amino-piperidin-1-yl)-xanthin
   (Durchführung mit isopropanolischer Salzsäure 5-6 M in Methylenchlorid)
   R_{f}-Wert: 0.50 (Kieselgel, Methylenchlorid/Methanol/konz. wässriges Ammoniak = 90:10:1)
   Massenspektrum (ESI⁺): m/z = 475 [M+H]⁺
(252) 1-[([1,5]Naphthyridin-2-yl)methyl]-3-methyl-7-(2-butin-1-yl)-8-((*R*)-3-amino-piperidin-1-yl)-xanthin
   (Durchführung mit isopropanolischer Salzsäure 5-6 M in Methylenchlorid)
   Massenspektrum (ESI⁺): m/z = 459 [M+H]⁺
(253) 1-[2-(3-Methyl-2-oxo-2,3-dihydro-benzooxazol-4-yl)-2-oxo-ethyl]-3-methyl-7-(2-butin-1-yl)-8-((*R*)-3-amino-piperidin-1-yl)-xanthin
   (Durchführung mit isopropanolischer Salzsäure 5-6 M in Methylenchlorid)
   Schmelzpunkt: 187-189°C
   Massenspektrum (ESI⁺): m/z = 506 [M+H]⁺
(254) 1-[(8-Phenyl-chinoxalin-6-yl)methyl]-3-methyl-7-(2-butin-1-yl)-8-((R)-3-amino-piperidin-1-yl)-xanthin
   (Durchführung mit isopropanolischer Salzsäure 5-6 M in Methylenchlorid)
   R_{f}-Wert: 0.50 (Kieselgel, Methylenchlorid/Methanol/konz. wässriges Ammoniak = 90:10:1)
   Massenspektrum (ESI⁺): m/z = 535 [M+H]⁺
(255) 1-[([1,5]Naphthyridin-4-yl)methyl]-3-methyl-7-(2-butin-1-yl)-8-((*R*)-3-amino-piperidin-1-yl)-xanthin
   (Durchführung mit isopropanolischer Salzsäure 5-6 M in Methylenchlorid)
   R_{f}-Wert: 0.52 (Kieselgel, Methylenchlorid/Methanol/konz. wässriges Ammoniak = 90:10:1)
   Massenspektrum (ESI⁺): m/z = 459 [M+H]⁺
(256) 1-((E)-3-Pentafluorphenyl-allyl)-3-methyl-7-(2-butin-1-yl)-8-((*R*)-3-amino-piperidin-1-yl)-xanthin
   Massenspektrum (ESI⁺): m/z = 523 [M+H]⁺
(257) 1-[(E)-3-(2-Trifluormethyl-phenyl)-allyl]-3-methyl-7-(2-butin-1-yl)-8-((*R*)-3-am ino-piperidin-1-yl)-xanth in
   Massenspektrum (ESI⁺): m/z = 501 [M+H]⁺
(258) 1-[(E)-3-(3-Trifluormethyl-phenyl)-allyl]-3-methyl-7-(2-butin-1-yl)-8-((*R*)-3-amino-piperidin-1-yl)-xanthin
   Massenspektrum (ESI⁺): m/z = 501 [M+H]⁺
(259) 1-[(E)-3-(4-Trifluormethyl-phenyl)-allyl]-3-methyl-7-(2-butin-1-yl)-8-((*R*)-3-amino-piperidin-1-yl)-xanthin
   Massenspektrum (ESI⁺): m/z = 501 [M+H]⁺
(260) 1-[(3-Trifluormethyl-isochinolin-1-yl)methyl]-3-methyl-7-(2-butin-1-yl)-8-((R)-3-amino-piperidin-1-yl)-xanthin x Trifluoressigsäure
   Massenspektrum (ESI⁺): m/z = 526 [M+H]⁺
(261) 1-[(3-Methyl-isochinolin-1-yl)methyl]-3-isopropyl-7-(2-butin-1-yl)-8-((*R*)-3-amino-piperidin-1-yl)-xanthin
(262) 1-[(3-Methyl-isochinolin-1-yl)methyl]-3-(4-fluorphenyl)-7-(2-butin-1-yl)-8-((*R*)-3-amino-piperidin-1-yl)-xanthin
(263) 1-[(3-Methyl-isochinolin-1-yl)methyl]-3-methyl-7-(2-cyano-benzyl)-8-(3-amino-piperidin-1-yl)-xanthin
   (Durchführung mit isopropanolischer Salzsäure 5-6 M in Methylenchlorid)
   R_{f}-Wert: 0.51 (Reversed Phase DC-Fertigplatte (E. Merck), Acetonitril/Wasser/ Trifluoressigsäure = 50:50:1)
   Massenspektrum (ESI⁺): m/z = 535 [M+H]⁺
(264) 1-[(3-Difluormethyl-isochinolin-1-yl)methyl]-3-methyl-7-(2-butin-1-yl)-8-((R)-3-amino-piperidin-1-yl)-xanthin
   Massenspektrum (ESI⁺): m/z = 508 [M+H]⁺
(265) 1-[(4-Chlor-3-methoxy-isochinolin-1-yl)methyl]-3-methyl-7-(2-butin-1-yl)-8-(3-amino-piperidin-1-yl)-xanthin
   (Durchführung mit isopropanolischer Salzsäure 5-6 M in Methylenchlorid)
   Massenspektrum (ESI⁺): m/z = 522, 524 [M+H]⁺
   R_{f}-Wert: 0.40 (Reversed Phase DC-Fertigplatte (E. Merck), Acetonitril/Wasser/ Trifluoressigsäure = 50:50:1)
(266) 1-[(4-Ethoxy-chinazolin-2-yl)methyl]-3-methyl-7-(2-butin-1-yl)-8-(3-amino-piperidin-1-yl)-xanthin
   (Durchführung mit 1 M etherischer Salzsäure)
   R_{f}-Wert: 0.60 (Reversed Phase DC-Fertigplatte (E. Merck), Acetonitril/Wasser/ Trifluoressigsäure = 50:50:1)
   Massenspektrum (ESI⁺): m/z = 503 [M+H]⁺
(267) 1-[(4-Isopropyloxy-chinazolin-2-yl)methyl]-3-methyl-7-(2-butin-1-yl)-8-(3-amino-piperidin-1-yl)-xanthin
   (Durchführung mit isopropanolischer Salzsäure 5-6 M in Methylenchlorid)
   R_{f}-Wert: 0.55 (Reversed Phase DC-Fertigplatte (E. Merck), Acetonitril/Wasser/ Trifluoressigsäure = 50:50:1)
   Massenspektrum (ESI⁺): m/z = 517 [M+H]⁺
(268) 1-[(2-Methyl-benzothiazol-6-yl)methyl]-3-methyl-7-(2-butin-1-yl)-8-(3-amino-piperidin-1-yl)-xanthin
   (Durchführung mit isopropanolischer Salzsäure 5-6 M in Methylenchlorid)
   Schmelzpunkt: 167°C
   Massenspektrum (ESI⁺): m/z = 478 [M+H]⁺
(269) 1-[(3-Phenyl-isochinolin-1-yl)methyl]-3-methyl-7-(2-butin-1-yl)-8-((*R*)-3-amino-piperidin-1-yl)-xanthin
   (Durchführung mit isopropanolischer Salzsäure 5-6 M in Methylenchlorid)
   R_{f}-Wert: 0.45 (Reversed Phase DC-Fertigplatte (E. Merck), Acetonitril/Wasser/ Trifluoressigsäure = 50:50:1)
   Massenspektrum (ESI⁺): m/z = 534 [M+H]⁺
(270) 1-[(4-Phenyloxy-chinazolin-2-yl)methyl]-3-methyl-7-(2-butin-1-yl)-8-(3-amino-piperidin-1-yl)-xanthin
   (Durchführung mit isopropanolischer Salzsäure 5-6 M in Methylenchlorid)
   R_{f}-Wert: 0.60 (Reversed Phase DC-Fertigplatte (E. Merck), Acetonitril/Wasser/ Trifluoressigsäure = 50:50:1)
   Massenspektrum (ESI⁺): m/z = 551 [M+H]⁺
(271) 1-[(4-Phenyl-chinazolin-2-yl)methyl]-3-cyclopropyl-7-(2-butin-1-yl)-8-(3-amino-piperidin-1-yl)-xanthin
   (Durchführung mit isopropanolischer Salzsäure 5-6 M in Methylenchlorid)
   R_{f}-Wert: 0.45 (Reversed Phase DC-Fertigplatte (E. Merck), Acetonitril/Wasser/ Trifluoressigsäure = 50:50:1)
   Massenspektrum (ESI⁺): m/z = 561 [M+H]⁺
(272) 1-[(3-Methyl-isochinolin-1-yl)methyl]-3-cyclopropyl-7-(2-butin-1-yl)-8-(3-amino-piperidin-1-yl)-xanthin
   (Durchführung mit isopropanolischer Salzsäure 5-6 M in Methylenchlorid)
   R_{f}-Wert: 0.55 (Reversed Phase DC-Fertigplatte (E. Merck), Acetonitril/Wasser/ Trifluoressigsäure = 50:50:1)
   Massenspektrum (ESI⁺): m/z = 498 [M+H]⁺
(273) 1-[(2-Phenyl-chinazolin-4-yl)methyl]-3-methyl-7-(2-butin-1-yl)-8-(3-amino-piperidin-1-yl)-xanthin
   (Durchführung mit isopropanolischer Salzsäure 5-6 M in Methylenchlorid)
   R_{f}-Wert: 0.50 (Reversed Phase DC-Fertigplatte (E. Merck), Acetonitril/Wasser/ Trifluoressigsäure = 50:50:1)
   Massenspektrum (ESI⁺): m/z = 535 [M+H]⁺
(274) 1-[2-(2-Methoxy-phenyl)-2-oxo-ethyl]-3-methyl-7-(2-butin-1-yl)-8-(3-amino-piperidin-1-yl)-xanthin
   (Durchführung mit isopropanolischer Salzsäure 5-6 M in Methylenchlorid)
   R_{f}-Wert: 0.29 (Kieselgel, Methylenchlorid/Methanol/konz. wässriges Ammoniak = 90:10:1)
   Massenspektrum (ESI⁺): m/z = 465 [M+H]⁺
(275) 1-[2-(3-Methoxy-phenyl)-2-oxo-ethyl]-3-methyl-7-(2-butin-1-yl)-8-(3-amino-piperidin-1-yl)-xanthin
   (Durchführung mit isopropanolischer Salzsäure 5-6 M in Methylenchlorid)
   R_{f}-Wert: 0.27 (Kieselgel, Methylenchlorid/Methanol/konz. wässriges Ammoniak = 90:10:1)
   Massenspektrum (ESI⁺): m/z = 465 [M+H]⁺
(276) 1-[2-(3-Trifluormethoxy-phenyl)-2-oxo-ethyl]-3-methyl-7-(2-butin-1-yl)-8-((*R*)-3-amino-piperidin-1-yl)-xanthin
   (Durchführung mit isopropanolischer Salzsäure 5-6 M in Methylenchlorid)
   R_{f}-Wert: 0.29 (Kieselgel, Methylenchlorid/Methanol/konz. wässriges Ammoniak = 90:10:1)
   Massenspektrum (ESI⁺): m/z = 519 [M+H]⁺
(277) 1-[2-(Biphenyl-2-yl)-2-oxo-ethyl]-3-methyl-7-(2-butin-1-yl)-8-((*R*)-3-amino-piperidin-1-yl)-xanthin
   (Durchführung mit isopropanolischer Salzsäure 5-6 M in Methylenchlorid)
   R_{f}-Wert: 0.35 (Reversed Phase DC-Fertigplatte (E. Merck), Acetonitril/Wasser/ Trifluoressigsäure = 50:50:1)
   Massenspektrum (ESI⁺): m/z = 511 [M+H]⁺
(278) 1-[2-(Biphenyl-3-yl)-2-oxo-ethyl]-3-methyl-7-(2-butin-1-yl)-8-((*R*)-3-amino-piperidin-1-yl)-xanthin
   (Durchführung mit isopropanolischer Salzsäure 5-6 M in Methylenchlorid)
   R_{f}-Wert: 0.35 (Reversed Phase DC-Fertigplatte (E. Merck), Acetonitril/Wasser/ Trifluoressigsäure = 50:50:1)
   Massenspektrum (ESI⁺): m/z = 511 [M+H]⁺
(279) 1-[2-(3-Isopropyloxy-phenyl)-2-oxo-ethyl]-3-methyl-7-(2-butin-1-yl)-8-((*R*)-3-amino-piperidin-1-yl)-xanthin
   (Durchführung mit isopropanolischer Salzsäure 5-6 M in Methylenchlorid)
   R_{f}-Wert: 0.20 (Kieselgel, Methylenchlorid/Methanol/konz. wässriges Ammoniak = 90:10:1)
   Massenspektrum (ESI⁺): m/z = 493 [M+H]⁺
(280) 1-[(3-Methyl-isochinolin-1-yl)methyl]-3-cyclopropyl-7-(2-butin-1-yl)-8-((*R*)-3-amino-piperidin-1-yl)-xanthin
   (Durchführung mit isopropanolischer Salzsäure 5-6 M in Methylenchlorid)
   R_{f}-Wert: 0.50 (Reversed Phase DC-Fertigplatte (E. Merck), Acetonitril/Wasser/ Trifluoressigsäure = 50:50:1)
   Massenspektrum (ESI⁺): m/z = 498 [M+H]⁺
(281) 1-[(4-Phenyl-chinazolin-2-yl)methyl]-3-cyclopropyl-7-(2-butin-1-yl)-8-((*R*)-3-amino-piperidin-1-yl)-xanthin
   (Durchführung mit isopropanolischer Salzsäure 5-6 M in Methylenchlorid)
   R_{f}-Wert: 0.45 (Reversed Phase DC-Fertigplatte (E. Merck), Acetonitril/Wasser/ Trifluoressigsäure = 50:50:1)
   Massenspektrum (ESI⁺): m/z = 561 [M+H]⁺
(282) 1-[(4-Cyano-naphthalin-1-yl)methyl]-3-cyclopropyl-7-(2-butin-1-yl)-8-((*R*)-3-amino-piperidin-1-yl)-xanthin
   (Durchführung mit isopropanolischer Salzsäure 5-6 M in Methylenchlorid)
   Schmelzpunkt: 191°C
   Massenspektrum (ESI⁺): m/z = 508 [M+H]⁺
(283) 1-[2-(2-Phenyloxy-phenyl)-2-oxo-ethyl]-3-methyl-7-(2-butin-1-yl)-8-((R)-3-amino-piperidin-1-yl)-xanthin
   (Durchführung mit isopropanolischer Salzsäure 5-6 M in Methylenchlorid)
   R_{f}-Wert: 0.40 (Reversed Phase DC-Fertigplatte (E. Merck), Acetonitril/Wasser/ Trifluoressigsäure = 50:50:1)
   Massenspektrum (ESI⁺): m/z = 527 [M+H]⁺
(284) 1-[2-(3-Ethoxy-phenyl)-2-oxo-ethyl]-3-methyl-7-(2-butin-1-yl)-8-((R)-3-amino-piperidin-1-yl)-xanthin
   (Durchführung mit isopropanolischer Salzsäure 5-6 M in Methylenchlorid)
   R_{f}-Wert: 0.29 (Kieselgel, Methylenchlorid/Methanol/konz. wässriges Ammoniak = 90:10:1)
   Massenspektrum (ESI⁺): m/z = 479 [M+H]⁺
(285) 1-[2-(3-Methoxy-phenyl)-2-oxo-ethyl]-3-methyl-7-(2-butin-1-yl)-8-((*R*)-3-amino-piperidin-1-yl)-xanthin
   (Durchführung mit isopropanolischer Salzsäure 5-6 M in Methylenchlorid)
   R_{f}-Wert: 0.28 (Kieselgel, Methylenchlorid/Methanol/konz. wässriges Ammoniak = 90:10:1)
   Massenspektrum (ESI⁺): m/z = 465 [M+H]⁺
(286) 1-[2-(2-Methoxy-phenyl)-2-oxo-ethyl]-3-methyl-7-(2-butin-1-yl)-8-((*R*)-3-amino-piperidin-1-yl)-xanthin
   (Durchführung mit isopropanolischer Salzsäure 5-6 M in Methylenchlorid)
   R_{f}-Wert: 0.34 (Kieselgel, Methylenchlorid/Methanol/konz. wässriges Ammoniak = 90:10:1)
   Massenspektrum (ESI⁺): m/z = 465 [M+H]⁺
(287) 1-[(3-Methyl-isochinolin-1-yl)methyl]-3-methyl-7-(2-chlor-benzyl)-8-((*R*)-3-amino-piperidin-1-yl)-xanthin
   Massenspektrum (ESI⁺): m/z = 544, 546 [M+H]⁺
(288) 1-[(3-Methyl-isochinolin-1-yl)methyl]-3-methyl-7-(2-brom-benzyl)-8-((*R*)-3-amino-piperidin-1-yl)-xanthin
   Massenspektrum (ESI⁺): m/z = 588, 590 [M+H]⁺
(289) 1-[(1,2,3,4-Tetrahydro-phenanthridin-6-yl)methyl]-3-methyl-7-(2-butin-1-yl)-8-(3-amino-piperidin-1-yl)-xanthin x Trifluoressigsäure
   R_{f}-Wert: 0.75 (Aluminiumoxid, Methylenchlorid/Methanol = 10:1)
   Massenspektrum (ESI⁺): m/z = 512 [M+H]⁺
(290) 1-[2-(3-Difluormethoxy-phenyl)-2-oxo-ethyl]-3-methyl-7-(2-butin-1-yl)-8-((*R*)-3-amino-piperidin-1-yl)-xanthin
   (Durchführung mit isopropanolischer Salzsäure 5-6 M in Methylenchlorid)
   R_{f}-Wert: 0.28 (Kieselgel, Methylenchlorid/Methanol/konz. wässriges Ammoniak = 90:10:1)
   Massenspektrum (ESI⁺): m/z = 501 [M+H]⁺
(291) 1-[(3-Methyl-isochinolin-1-yl)methyl]-3-methyl-7-(2-ethinyl-benzyl)-8-((*R*)-3-amino-piperidin-1-yl)-xanthin
(292) 1-[(3-Methyl-isochinolin-1-yl)methyl]-3-phenyl-7-(2-butin-1-yl)-8-((*R*)-3-amino-piperidin-1-yl)-xanthin
(293) 1-[(Phenanthren-9-yl)methyl]-3-methyl-7-(2-butin-1-yl)-8-((*R*)-3-amino-piperidin-1-yl)-xanthin
(294) 1-[(4-Methyl-chinazolin-2-yl)methyl]-3-methyl-7-(2-chlor-benzyl)-8-((*R*)-3-amino-piperidin-1-yl)-xanthin
   R_{f}-Wert: 0.35 (Kieselgel, Methylenchlorid/Methanol/Triethylamin = 90:10:1)
   Massenspektrum (ESI⁺):m/z = 545, 547 [M+H]⁺
(295) 1-[(4-Phenyl-chinazolin-2-yl)methyl]-3-methyl-7-(2-chlor-benzyl)-8-((*R*)-3-amino-piperidin-1-yl)-xanthin
   R_{f}-Wert: 0.40 (Kieselgel, Methylenchlorid/Methanol/Triethylamin = 90:10:1)
   Massenspektrum (ESI⁺): m/z = 607, 609 [M+H]⁺

### Beispiel 3

### 1-[2-(3-Carboxymethoxy-phenyl)-2-oxo-ethyl]-3-methyl-7-(3-methyl-2-buten-1-yl)-8-(3-amino-piperidin-1-yl)-xanthin

hergestellt durch Verseifung von 70 mg 1-(2-{3-[(Methoxycarbonyl)methoxy]-phenyl}-2-oxo-ethyl)-3-methyl-7-(3-methyl-2-buten-1-yl)-8-(3-amino-piperidin-1-yl)-xanthin mit 0.10 ml 4 M Kalilauge in einem Gemisch aus 1 ml Tetrahydrofuran und 0.5 ml Methanol bei Raumtemperatur.
Ausbeute: 57 mg (84 % der Theorie)
R_{f}-Wert: 0.55 (Reversed Phase DC-Fertigplatte (E. Merck), Acetonitril/Wasser/ Trifluoressigsäure = 50:50:0.1)
Massenspektrum (ESI⁺): m/z = 525 [M+H]⁺

### Analog Beispiel 3 werden folgende Verbindungen erhalten:

(1) 1-[2-(2-Carboxymethoxy-phenyl)-2-oxo-ethyl]-3-methyl-7-(3-methyl-2-buten-1-yl)-8-(3-amino-piperidin-1-yl)-xanthin
   (Durchführung mit Natronlauge)
   Massenspektrum (ESI⁻): m/z = 523 [M-H]⁻

### Beispiel 4

### Dragées mit 75 mg Wirksubstanz

### 1 Drageekern enthält:

| | |
|---|---|
| Wirksubstanz | 75,0 mg |
| Calciumphosphat | 93,0 mg |
| Maisstärke | 35,5 mg |
| Polyvinylpyrrolidon | 10,0 mg |
| Hydroxypropylmethylcellulose | 15,0 mg |
| Magnesiumstearat | 1,5 mg |
| | 230,0 mg |

### Herstellung:

Die Wirksubstanz wird mit Calciumphosphat, Maisstärke, Polyvinylpyrrolidon, Hydroxypropylmethylcellulose und der Hälfte der angegebenen Menge Magnesiumstearat gemischt. Auf einer Tablettiermaschine werden Preßlinge mit einem Durchmesser von ca. 13 mm hergestellt, diese werden auf einer geeigneten Maschine durch ein Sieb mit 1,5 mm-Maschenweite gerieben und mit der restlichen Menge Magnesiumstearat vermischt. Dieses Granulat wird auf einer Tablettiermaschine zu Tabletten mit der gewünschten Form gepreßt.

| | |
|---|---|
| Kerngewicht: | 230 mg |
| Stempel: | 9 mm, gewölbt |

Die so hergestellten Drageekerne werden mit einem Film überzogen, der im wesentlichen aus Hydroxypropylmethylcellulose besteht. Die fertigen Filmdragees werden mit Bienenwachs geglänzt.
Drageegewicht: 245 mg.

### Beispiel 5

### Tabletten mit 100 mg Wirksubstanz

### Zusammensetzung:

### 1 Tablette enthält:

| | |
|---|---|
| Wirksubstanz | 100,0 mg |
| Milchzucker | 80,0 mg |
| Maisstärke | 34,0 mg |
| Polyvinylpyrrolidon | 4,0 mg |
| Magnesiumstearat | 2,0 mg |
| | 220,0 mg |

### Herstellungverfahren:

Wirkstoff, Milchzucker und Stärke werden gemischt und mit einer wäßrigen Lösung des Polyvinylpyrrolidons gleichmäßig befeuchtet. Nach Siebung der feuchten Masse (2,0 mm-Maschenweite) und Trocknen im Hordentrockenschrank bei 50°C wird erneut gesiebt (1,5 mm-Maschenweite) und das Schmiermittel zugemischt. Die preßfertige Mischung wird zu Tabletten verarbeitet.

| | |
|---|---|
| Tablettengewicht: | 220 mg |
| Durchmesser: | 10 mm, biplan mit beidseitiger Facette und einseitiger Teilkerbe. |

### Beispiel 6

### Tabletten mit 150 mg Wirksubstanz

### Zusammensetzung:

### 1 Tablette enthält:

| | |
|---|---|
| Wirksubstanz | 150,0 mg |
| Milchzucker pulv. | 89,0 mg |
| Maisstärke | 40,0 mg |
| Kolloide Kieselgelsäure | 10,0 mg |
| Polyvinylpyrrolidon | 10,0 mg |
| Magnesiumstearat | 1,0 mg |
| | 300,0 mg |

### Herstellung:

Die mit Milchzucker, Maisstärke und Kieselsäure gemischte Wirksubstanz wird mit einer 20%igen wäßrigen Polyvinylpyrrolidonlösung befeuchtet und durch ein Sieb mit 1,5 mm-Maschenweite geschlagen.

Das bei 45°C getrocknete Granulat wird nochmals durch dasselbe Sieb gerieben und mit der angegebenen Menge Magnesiumstearat gemischt. Aus der Mischung werden Tabletten gepreßt.

| | |
|---|---|
| Tablettengewicht: | 300 mg |
| Stempel: | 10 mm, flach |

### Beispiel 7

### Hartgelatine-Kapseln mit 150 mg Wirksubstanz

### 1 Kapsel enthält:

| | |
|---|---|
| Wirkstoff | 150,0 mg |
| Maisstärke getr. | ca. 180,0 mg |
| Milchzucker pulv. | ca. 87,0 mg |
| Magnesiumstearat | 3,0 mg |
| | ca. 420,0 mg |

### Herstellung:

Der Wirkstoff wird mit den Hilfsstoffen vermengt, durch ein Sieb von
0,75 mm-Maschenweite gegeben und in einem geeigneten Gerät homogen gemischt.

Die Endmischung wird in Hartgelatine-Kapseln der Größe 1 abgefüllt.

| | |
|---|---|
| Kapselfüllung: | ca. 320 mg |
| Kapselhülle: | Hartgelatine-Kapsel Größe 1 |

### Beispiel 8

### Suppositorien mit 150 mg Wirksubstanz

### 1 Zäpfchen enthält:

| | |
|---|---|
| Wirkstoff | 150,0 mg |
| Polyethylenglykol 1500 | 550,0 mg |
| Polyethylenglykol 6000 | 460,0 mg |
| Polyoxyethylensorbitanmonostearat | 840,0 mg |
| | 2000,0 mg |

### Herstellung:

Nach dem Aufschmelzen der Suppositorienmasse wird der Wirkstoff darin homogen verteilt und die Schmelze in vorgekühlte Formen gegossen.

### Beispiel 9

### Suspension mit 50 mg Wirksubstanz

### 100 ml Suspension enthalten:

| | |
|---|---|
| Wirkstoff | 1,00 g |
| Carboxymethylcellulose-Na-Salz | 0,10 g |
| p-Hydroxybenzoesäuremethylester | 0,05 g |
| p-Hydroxybenzoesäurepropylester | 0,01 g |
| Rohrzucker | 10,00 g |
| Glycerin | 5,00 g |
| Sorbitlösung 70%ig | 20,00 g |
| Aroma | 0,30 g |
| Wasser dest. | ad 100 ml |

### Herstellung:

Dest. Wasser wird auf 70°C erhitzt. Hierin wird unter Rühren p-Hydroxybenzoesäuremethylester und -propylester sowie Glycerin und Carboxymethylcellulose-Natriumsalz gelöst. Es wird auf Raumtemperatur abgekühlt und unter Rühren der Wirkstoff zugegeben und homogen dispergiert. Nach Zugabe und Lösen des Zuckers, der Sorbitlösung und des Aromas wird die Suspension zur Entlüftung unter Rühren evakuiert.

5 ml Suspension enthalten 50 mg Wirkstoff.

### Beispiel 10

### Ampullen mit 10 mg Wirksubstanz

### Zusammensetzung:

| | |
|---|---|
| Wirkstoff | 10,0 mg |
| 0,01 n Salzsäure s.q. | |
| Aqua bidest | ad 2,0 ml |

### Herstellung:

Die Wirksubstanz wird in der erforderlichen Menge 0,01 n HCl gelöst, mit Kochsalz isotonisch gestellt, sterilfiltriert und in 2 ml Ampullen abgefüllt.

### Beispiel 11

### Ampullen mit 50 mg Wirksubstanz

### Zusammensetzung:

| | |
|---|---|
| Wirkstoff | 50,0 mg |
| 0,01 n Salzsäure s.q. | |
| Aqua bidest | ad 10,0 ml |

### Herstellung:

Die Wirksubstanz wird in der erforderlichen Menge 0,01 n HCl gelöst, mit Kochsalz isotonisch gestellt, sterilfiltriert und in 10 ml Ampullen abgefüllt.

## Patentansprüche

1. Verbindungen der allgemeinen Formel 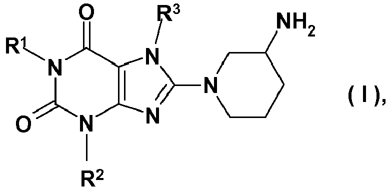 in der
R¹ eine Methylgruppe, die durch eine Dimethylaminocarbonyl-, Pyrrolidin-1-yl-carbonyl-, Piperidin-1-ylcarbonyl-, tert.-Butylcarbonyl- oder eine CyclohexylcarbonylGruppe substituiert ist,
eine Methylgruppe, die durch eine Naphthyl-, Methylnaphthyl-, Methoxynaphthyl-, Nitronaphthyl- oder (Dimethylamino)-naphthyl-Gruppe substituiert ist,
eine Methylgruppe, die durch eine 2-Phenylethenyl- oder eine Biphenylyl-Gruppe substituiert ist,
eine Methylgruppe, die durch eine Phenyl-oxadiazolyl-, 5-Methyl-3-phenyl-isoxazolyl-, Phenyl-pyridinyl-, Indolyl-, Benzothiophenyl-, Chinolinyl-, Isochinolinyl-, Methylisochinolinyl-, (Methoxycarbonylmethylamino)-isochinolinyl-, Cinnolinyl-, Chinazolinyl-, Methylchinazolinyl-, 1,2-Dihydro-1-methyl-2-oxo-chinolinyl-, 1,2-Dihydro-2-methyl-1-oxo-isochinolinyl-, 3,4-Dihydro-4-oxo-phthalazinyl-, 3,4-Dihydro-3-methyl-4-oxo-phthalazinyl-, 3,4-Dihydro-4-oxo-chinazolinyl-, 3,4-Dihydro-3-methyl-4-oxo-chinazolinyl- oder eine 2-Oxo-2H-chromenyl-Gruppe substituiert ist,
eine 2-Methoxyethyl-, 2-Phenyloxyethyl- oder 2-Cyanethyl-Gruppe,
eine Phenylcarbonylmethyl- oder eine 1-(Phenylcarbonyl)-ethyl-Gruppe,
eine Phenylcarbonylmethylgruppe, in der der Phenylteil durch eine Amino-, Cyanmethylamino-, Methylcarbonylamino-, Ethylcarbonylamino-, Isopropylcarbonylamino-, Methoxycarbonylamino-, (Ethyloxycarbonylamino)-carbonylamino- oder eine 2-Oxo-imidazolidin-1-yl-Gruppe substituiert ist,
eine Phenylcarbonylmethylgruppe, in der der Phenylteil durch eine Carboxy-, Methoxycarbonyl-, Ethyloxycarbonyl-, Aminocarbonyl-, Methylaminocarbonyl-, Dimethylaminocarbonyl- oder Morpholin-4-ylcarbonylgruppe substituiert ist,
eine Phenylcarbonylmethylgruppe, in der der Phenylteil durch eine Methylsulfanyl-, Methylsulfinyl- oder Methylsulfonylgruppe substituiert ist,
eine Phenylcarbonylmethylgruppe, in der der Phenylteil durch eine Carboxymethoxy-, Ethyloxycarbonylmethoxy-, Isopropyloxycarbonylmethoxy-, Aminocarbonylmethoxy-, Methylaminocarbonylmethoxy-, Ethylaminocarbonylmethoxy-, Isopropylaminocarbonylmethoxy-, Dimethylaminocarbonylmethoxy-, Pyrrolidin-1-ylcarbonylmethoxy- oder Morpholin-4-ylcarbonylmethoxy-Gruppe substituiert ist,
eine Phenylcarbonylmethylgruppe, in der der Phenylteil durch eine 1-(Methoxycarbonyl)-ethyloxy- oder eine 1-(Aminocarbonyl)-ethyloxy-Gruppe substituiert ist,
eine Phenylcarbonylmethylgruppe, in der der Phenylteil durch eine Methylsulfinylmethoxy-Gruppe substituiert ist,
eine Phenylcarbonylmethylgruppe, in der der Phenylteil durch zwei Methoxygruppen substituiert ist oder
eine Phenylcarbonylmethylgruppe, in der im Phenylteil zwei benachbarte Wasserstoffatome durch eine -O-CH₂-O-, -O-CH₂-CH₂-O- oder eine -N(CH₃)-CO-O-Gruppe ersetzt sind,
R² eine Methyl-, Isopropyl- oder Phenylgruppe
und
R³ eine 2-Methyl-2-propen-1-yl-, 2-Chlor-2-propen-1-yl- oder 3-Brom-2-propen-1-yl-Gruppe
eine 2-Buten-1-yl- oder 2,3-Dimethyl-2-buten-1-yl-Gruppe,
eine 2-Butin-1-yl-Gruppe,
eine 1-Cyclopenten-1-ylmethyl-Gruppe oder
eine 2-Furanylmethyl-Gruppe bedeuten,
sowie die Verbindungen
1-(2-Cyano-ethyl)-3-methyl-7-(2-cyano-benzyl)-8-(3-amino-piperidin-1-yl)-xanthin,
1-(2-{2-[(Ethoxycarbonyl)methoxy]-phenyl}-2-oxo-ethyl)-3-methyl-7-(3-methyl-2-buten-1-yl)-8-(3-amino-piperidin-1-yl)-xanthin,
1-(2-{2-[(Aminocarbonyl)methoxy]-phenyl}-2-oxo-ethyl)-3-methyl-7-(3-methyl-2-buten-1-yl)-8-(3-amino-piperidin-1-yl)-xanthin,
1-(2-{3-[(Methansulfinyl)methoxy]-phenyl}-2-oxo-ethyl)-3-methyl-7-(3-methyl-2-buten-1-yl)-8-(3-amino-piperidin-1-yl)-xanthin,
1-(1-Methyl-2-oxo-2-phenyl-ethyl)-3-methyl-7-(3-methyl-2-buten-1-yl)-8-(3-amino-piperidin-1-yl)-xanthin,
1-(2-Phenoxy-ethyl)-3-methyl-7-(2-cyano-benzyl)-8-(3-amino-piperidin-1-yl)-xanthin,
1-(2-Phenyl-2-oxo-ethyl)-7-(3-methyl-2-buten-1-yl)-8-(3-amino-piperidin-1-yl)-xanthin,
1-(2-{3-[(Ethoxycarbonyl)methoxy]-phenyl}-2-oxo-ethyl)-3-methyl-7-(3-methyl-2-buten-1-yl)-8-(3-amino-piperidin-1-yl)-xanthin,
1-(2-{2-[(Methylaminocarbonyl)methoxy]-phenyl}-2-oxo-ethyl)-3-methyl-7-(3-methyl-2-buten-1-yl)-8-(3-amino-piperidin-1-yl)-xanthin,
1-(2-{2-[(Dimethylaminocarbonyl)methoxy]-phenyl}-2-oxo-ethyl)-3-methyl-7-(3-methyl-2-buten-1-yl)-8-(3-amino-piperidin-1-yl)-xanthin,
1-(2-Methoxy-ethyl)-3-methyl-7-(2-cyano-benzyl)-8-(3-amino-piperidin-1-yl)-xanthin,
1-Methyl-3-[(methoxycarbonyl)methyl]-7-(2-cyano-benzyl)-8-(3-amino-piperidin-1-yl)-xanthin,
1-Methyl-3-cyanomethyl-7-(2-cyano-benzyl)-8-(3-amino-piperidin-1-yl)-xanthin,
1-Methyl-3-(2-propin-1-yl)-7-(2-cyano-benzyl)-8-(3-amino-piperidin-1-yl)-xanthin,
1-{2-[3-(2-Oxo-imidazolidin-1-yl)-phenyl]-2-oxo-ethyl}-3-methyl-7-(3-methyl-2-buten-1-yl)-8-(3-amino-piperidin-1-yl)-xanthin,
1-Methyl-3-(2-propen-1-yl)-7-(2-cyano-benzyl)-8-(3-amino-piperidin-1-yl)-xanthin,
1-(2-{2-[(Ethylcarbonyl)amino]-phenyl}-2-oxo-ethyl)-3-methyl-7-(3-methyl-2-buten-1-yl)-8-(3-amino-piperidin-1-yl)-xanthin,
1-Methyl-3-phenyl-7-(2-cyano-benzyl)-8-(3-amino-piperidin-1-yl)-xanth in,
1-[2-(2-Amino-phenyl)-2-oxo-ethyl]-3-methyl-7-(3-methyl-2-buten-1-yl)-8-((*S*)-3-am ino-piperidin-1-yl)-xanthin,
1-(2-Phenyl-2-oxo-ethyl)-3-cyanomethyl-7-(3-methyl-2-buten-1-yl)-8-(3-amino-piperidin-1-yl)-xanthin,
1-[(Chinolin-2-yl)methyl]-3-methyl-7-(3-methyl-2-buten-1-yl)-8-(3-amino-piperidin-1-yl)-xanthin,
1-[(2-Oxo-2*H*-chromen-4-yl)methyl]-3-methyl-7-(3-methyl-2-buten-1-yl)-8-(3-amino-piperidin-1-yl)-xanthin,
1-[(Cinnolin-4-yl)methyl]-3-methyl-7-(3-methyl-2-buten-1-yl)-8-(3-amino-piperidin-1-yl)-xanthin,
1-[(1-Methyl-2-oxo-1,2-dihydro-chinolin-4-yl)methyl]-3-methyl-7-(3-methyl-2-buten-1-yl)-8-(3-amino-piperidin-1-yl)-xanthin,
1-[(4-Oxo-3,4-dihydro-phthalazin-1-yl)methyl]-3-methyl-7-(3-methyl-2-buten-1-yl)-8-(3-amino-piperidin-1-yl)-xanthin,
1-[(Chinazolin-4-yl)methyl]-3-methyl-7-(3-methyl-2-buten-1-yl)-8-(3-amino-piperidin-1-yl)-xanthin,
1-[(5-Methyl-3-phenyl-isoxazol-4-yl)methyl]-3-methyl-7-(3-methyl-2-buten-1-yl)-8-(3-am ino-piperidin-1-yl)-xanthin,
1-[(Isochinolin-3-yl)methyl]-3-methyl-7-(3-methyl-2-buten-1-yl)-8-(3-amino-piperidin-1-yl)-xanthin,
1-[(3-Phenyl-[1,2,4]oxadiazol-5-yl)methyl]-3-methyl-7-(3-methyl-2-buten-1-yl)-8-(3-amino-piperidin-1-yl)-xanthin,
1-[(4-Phenyl-pyridin-2-yl)methyl]-3-methyl-7-(3-methyl-2-buten-1-yl)-8-(3-amino-piperidin-1-yl)-xanthin,
1-[(5-Phenyl-pyridin-2-yl)methyl]-3-methyl-7-(3-methyl-2-buten-1-yl)-8-(3-amino-piperidin-1-yl)-xanthin,
1-[(3-Methyl-4-oxo-3,4-dihydro-phthalazin-1-yl)methyl]-3-methyl-7-(3-methyl-2-buten-1-yl)-8-(3-amino-piperidin-1-yl)-xanthin,
1-[2-(3-Methylsulfanyl-phenyl)-2-oxo-ethyl]-3-methyl-7-(3-methyl-2-buten-1-yl)-8-(3-am ino-piperidin-1-yl)-xanthin,
1-[2-(3-Methansulfinyl-phenyl)-2-oxo-ethyl]-3-methyl-7-(3-methyl-2-buten-1-yl)-8-(3-am ino-piperidin-1-yl)-xanthin,
1-[2-(3-Methansulfonyl-phenyl)-2-oxo-ethyl]-3-methyl-7-(3-methyl-2-buten-1-yl)-8-(3-am ino-piperidin-1-yl)-xanthin,
1-[2-(3-Carboxy-phenyl)-2-oxo-ethyl]-3-methyl-7-(3-methyl-2-buten-1-yl)-8-(3-aminopiperidin-1-yl)-xanthin,
1-[2-(3-Methoxycarbonyl-phenyl)-2-oxo-ethyl]-3-methyl-7-(3-methyl-2-buten-1-yl)-8-(3-amino-piperidin-1-yl)-xanthin,
1-{2-[3-(Methylaminocarbonyl)-phenyl]-2-oxo-ethyl}-3-methyl-7-(3-methyl-2-buten-1-yl)-8-(3-amino-piperidin-1-yl)-xanthin,
1-{2-[3-(Dimethylaminocarbonyl)-phenyl]-2-oxo-ethyl}-3-methyl-7-(3-methyl-2-buten-1-yl)-8-(3-amino-piperidin-1-yl)-xanthin,
1-{2-[3-(Morpholin-4-yl-carbonyl)-phenyl]-2-oxo-ethyl}-3-methyl-7-(3-methyl-2-buten-1-yl)-8-(3-amino-piperidin-1-yl)-xanthin,
1-[2-(2-Carboxy-phenyl)-2-oxo-ethyl]-3-methyl-7-(3-methyl-2-buten-1-yl)-8-(3-amino-piperidin-1-yl)-xanthin,
1-[2-(2-Ethoxycarbonyl-phenyl)-2-oxo-ethyl]-3-methyl-7-(3-methyl-2-buten-1-yl)-8-(3-amino-piperidin-1-yl)-xanthin,
1-{2-[2-(Dimethylaminocarbonyl)-phenyl]-2-oxo-ethyl}-3-methyl-7-(3-methyl-2-buten-1-yl)-8-(3-amino-piperidin-1-yl)-xanthin,
1-{2-[2-(Morpholin-4-yl-carbonyl)-phenyl]-2-oxo-ethyl}-3-methyl-7-(3-methyl-2-buten-1-yl)-8-(3-amino-piperidin-1-yl)-xanthin,
1-[2-(2,6-Dimethoxy-phenyl)-2-oxo-ethyl]-3-methyl-7-(3-methyl-2-buten-1-yl)-8-(3-amino-piperidin-1-yl)-xanthin,
1-((E)-3-Phenyl-allyl)-3-methyl-7-(3-methyl-2-buten-1-yl)-8-(3-amino-piperidin-1-yl)-xanthin,
1-[(Benzo[*b*]thiophen-3-yl)methyl]-3-methyl-7-(3-methyl-2-buten-1-yl)-8-(3-amino-piperidin-1-yl)-xanthin,
1-[(1*H*-Indol-3-yl)methyl]-3-methyl-7-(3-methyl-2-buten-1-yl)-8-(3-amino-piperidin-1-yl)-xanthin,
1-[(Biphenyl-4-yl)methyl]-3-methyl-7-(3-methyl-2-buten-1-yl)-8-(3-amino-piperidin-1-yl)-xanthin,
1-(2-Cyclohexyl-2-oxo-ethyl)-3-methyl-7-(3-methyl-2-buten-1-yl)-8-(3-amino-piperidin-1-yl)-xanthin,
1-(3,3-Dimethyl-2-oxo-butyl)-3-methyl-7-(3-methyl-2-buten-1-yl)-8-(3-amino-piperidin-1-yl)-xanthin,
1-({5-[(Methoxycarbonyl)methylamino]-isochinolin-1-yl}methyl)-3-methyl-7-(3-methyl-2-buten-1-yl)-8-(3-amino-piperidin-1-yl)-xanthin,
1-(2-Dimethylamino-2-oxo-ethyl)-3-methyl-7-(3-methyl-2-buten-1-yl)-8-(3-amino-piperidin-1-yl)-xanthin,
1-[2-(Piperidin-1-yl)-2-oxo-ethyl]-3-methyl-7-(3-methyl-2-buten-1-yl)-8-(3-amino-piperidin-1-yl)-xanthin,
1-[(2-Methyl-1-oxo-1,2-dihydro-isochinolin-4-yl)methyl]-7-(3-methyl-2-buten-1-yl)-8-(3-amino-piperidin-1-yl)-xanthin,
1-[2-(2,3-Dimethoxy-phenyl)-2-oxo-ethyl]-3-methyl-7-(3-methyl-2-buten-1-yl)-8-(3-amino-piperidin-1-yl)-xanthin,
1-[2-(Pyrrolidin-1-yl)-2-oxo-ethyl]-3-methyl-7-(3-methyl-2-buten-1-yl)-8-(3-amino-piperidin-1-yl)-xanthin,
1-[2-(2,3-Dihydro-benzo[1,4]dioxin-5-yl)-2-oxo-ethyl]-3-methyl-7-(3-methyl-2-buten-1-yl)-8-(3-amino-piperidin-1-yl)-xanthin,
1-[2-(3-Methyl-2-oxo-2,3-dihydro-benzooxazol-7-yl)-2-oxo-ethyl]-3-methyl-7-(3-methyl-2-buten-1-yl)-8-(3-amino-piperidin-1-yl)-xanthin,
1-[2-(Benzo[1,3]dioxo1-4-yl)-2-oxo-ethyl]-3-methyl-7-(3-methyl-2-buten-1-yl)-8-(3-amino-piperidin-1-yl)-xanthin,
1-Methyl-3-isopropyl-7-(2-cyano-benzyl)-8-(3-amino-piperidin-1-yl)-xanthin,
1-[2-(2-Cyanomethylamino-phenyl)-2-oxo-ethyl]-3-methyl-7-(3-methyl-2-buten-1-yl)-8-(3-amino-piperidin-1-yl)-xanthin,
1-[(Isochinolin-1-yl)methyl]-3-[(methoxycarbonyl)methyl]-7-(3-methyl-2-buten-1-yl)-8-(3-amino-piperidin-1-yl)-xanthin,
1-(2-{2-[(Isopropyloxycarbonyl)methoxy]-phenyl}-2-oxo-ethyl)-3-methyl-7-(3-methyl-2-buten-1-yl)-8-(3-amino-piperidin-1-yl)-xanthin,
1-[2-(2-{[(Ethoxycarbonylamino)carbonyl]amino}-phenyl)-2-oxo-ethyl]-3-methyl-7-(3-methyl-2-buten-1-yl)-8-(3-amino-piperidin-1-yl)-xanthin,
1-[2-(2-Acetylamino-phenyl)-2-oxo-ethyl]-3-methyl-7-(3-methyl-2-buten-1-yl)-8-((*S*)-3-am ino-piperidin-1-yl)-xanthin,
1-(2-{2-[(Methylaminocarbonyl)methoxy]-phenyl}-2-oxo-ethyl)-3-methyl-7-(3-methyl-2-buten-1-yl)-8-((*S*)-3-amino-piperidin-1-yl)-xanthin,
1-Methyl-7-(2-cyano-benzyl)-8-(3-amino-piperidin-1-yl)-xanthin,
1-(2-{2-[(Isopropylcarbonyl)amino]-phenyl}-2-oxo-ethyl)-3-methyl-7-(3-methyl-2-buten-1-yl)-8-(3-amino-piperidin-1-yl)-xanthin,
1-(2-{2-[(Methoxycarbonyl)amino]-phenyl}-2-oxo-ethyl)-3-methyl-7-(3-methyl-2-buten-1-yl)-8-(3-amino-piperidin-1-yl)-xanthin,
1-[2-(3-Methyl-2-oxo-2,3-dihydro-benzooxazol-4-yl)-2-oxo-ethyl]-3-methyl-7-(3-methyl-2-buten-1-yl)-8-(3-amino-piperidin-1-yl)-xanthin,
1-[2-(2-Nitro-3-methoxy-phenyl)-2-oxo-ethyl]-3-methyl-7-(3-methyl-2-buten-1-yl)-8-(3-amino-piperidin-1-yl)-xanthin,
1-[2-(2-Amino-3-methoxy-phenyl)-2-oxo-ethyl]-3-methyl-7-(3-methyl-2-buten-1-yl)-8-(3-amino-piperidin-1-yl)-xanthin,
1-[2-(2-Oxo-2,3-dihydro-benzooxazol-7-yl)-2-oxo-ethyl]-3-methyl-7-(3-methyl-2-buten-1-yl)-8-(3-amino-piperidin-1-yl)-xanthin,
1-[(3-Methyl-isochinolin-1-yl)methyl]-3-methyl-7-(3-methyl-1-buten-1-yl)-8-((*R*)-3-am ino-piperidin-1-yl)-xanthin,
1-[(3-Methyl-isochinolin-1-yl)methyl]-3-methyl-7-(2-cyano-benzyl)-8-(3-amino-piperidin-1-yl)-xanthin,
1-[2-(3-Carboxymethoxy-phenyl)-2-oxo-ethyl]-3-methyl-7-(3-methyl-2-buten-1-yl)-8-(3-amino-piperidin-1-yl)-xanthin_und
1-[2-(2-Carboxymethoxy-phenyl)-2-oxo-ethyl]-3-methyl-7-(3-methyl-2-buten-1-yl)-8-(3-amino-piperidin-1-yl)-xanthin,
deren Tautomere, Enantiomere, Diastereomere, deren Gemische, deren Prodrugs und deren Salze.

2. Verbindungen der allgemeinen Formel I gemäß Anspruch 1, in denen
R¹ eine 4-Methoxy-1-naphthylmethyl-Gruppe,
eine 2-Chinolinylmethyl-, 4-Chinolinylmethyl- oder eine 6-Chinolinylmethyl-Gruppe,
eine 1-Isochinolinylmethyl-, 3-Methyl-1-isochinolinylmethyl-, 4-Methyl-1-isochinolinylmethyl- oder eine 3-Isochinolinylmethyl-Gruppe oder
eine 2-Chinazolinylmethyl-, 4-Methyl-2-chinazolinylmethyl- oder eine 4-Chinazolinylmethyl-Gruppe,
R² eine Methylgruppe und
R³ eine 2-Buten-1-yl- oder eine 2-Butin-1-yl-Gruppe bedeuten,
deren Tautomere, Enantiomere, Diastereomere, deren Gemische und deren Salze.

3. Verbindungen der allgemeinen Formel I gemäß Anspruch 1, in denen
R¹ eine [2-(Methylcarbonylamino)-phenyl]-carbonylmethyl-Gruppe,
eine [2-(Ethylcarbonylamino)-phenyl]-carbonylmethyl-Gruppe oder
eine [2-(Isopropylcarbonylamino)-phenyl]-carbonylmethyl-Gruppe,
R² eine Methylgruppe und
R³ eine 2-Buten-1-yl- oder eine 2-Butin-1-yl-Gruppe bedeuten,
deren Tautomere, Enantiomere, Diastereomere, deren Gemische und deren Salze.

4. Verbindungen der allgemeinen Formel I gemäß Anspruch 1, in denen
R¹ eine [2-(Aminocarbonylmethoxy)-phenyl]-carbonylmethyl-Gruppe,
[2-(Methylaminocarbonylmethoxy)-phenyl]-carbonylmethyl-Gruppe,
eine [2-(Ethylaminocarbonylmethoxy)-phenyl]-carbonylmethyl-Gruppe oder
eine [2-(Isopropylaminocarbonylmethoxy)-phenyl]-carbonylmethyl-Gruppe,
R² eine Methylgruppe und
R³ eine 2-Buten-1-yl-Gruppe,
eine 2-Butin-1-yl-Gruppe oder
eine 1-Cyclopenten-1-ylmethyl-Gruppe bedeuten,
deren Tautomere, Enantiomere, Diastereomere, deren Gemische und deren Salze.

5. Verbindungen der allgemeinen Formel I, in denen
R¹ eine Methylgruppe, die durch eine Naphthyl-, Fluornaphthyl-, Methylnaphthyl-, Methoxynaphthyl-, (Difluormethoxy)-naphthyl-, Cyanonaphthyl-, Nitronaphthyl- oder (Dimethylamino)-naphthyl-Gruppe substituiert ist,
eine Methylgruppe, die durch eine Phenanthrenylgruppe substituiert ist,
eine Methylgruppe, die durch eine 2-Phenylethenyl-, 2-[(Trifluormethyl)-phenyl]-ethenyl-, 2-(Nitrophenyl)ethenyl-, 2-(Pentafluorphenyl)ethenyl- oder eine Biphenylyl-Gruppe substituiert ist,
eine Methylgruppe, die durch eine Phenyloxadiazolyl-, Phenylpyridinyl-, Indolyl-, Methylindolyl-, Dimethyl-6,7-dihydro-5H-[2]pyrindinyl-, Benzimidazolyl-, Methylbenzimidazolyl-, (Cyanoethyl)-benzimidazolyl-, (Methylaminocarbonylmethyl)benzimidazolyl-, Benzylbenzimidazolyl-, Benzofuranyl-, Acetylbenzofuranyl-, Cyanobenzofuranyl-, Benzoxazolyl-, Nitrobenzoxazolyl-, Benzothiophenyl-, Methylbenzothiazolyl-, Chinolinyl-, Methoxychinolinyl-, Isochinolinyl-, Methylisochinolinyl-, (Difluormethyl)-isochinolinyl-, (Trifluormethyl)-isochinolinyl-, Dimethylisochinolinyl-, (1-Cyano-1-methyl-ethyl)isochinolinyl-, Phenylisochinolinyl-, Methoxyisochinolinyl-, Methoxy-chlor-isochinolinyl-, Methoxy-brom-isochinolinyl-, (Methoxycarbonyl-methylamino)-isochinolinyl-, Dimethyl-5,6,7,8-tetrahydroisochinolinyl-, 1,2,3,4-Tetrahydrophenanthridinyl-, Cinnolinyl-, Chinazolinyl-, Methylchinazolinyl-, Isopropylchinazolinyl-, Cyclopropylchinazolinyl-, Phenylchinazolinyl-, Aminochinazolinyl-, (Dimethylamino)-chinazolinyl-, Pyrrolidin-1-ylchinazolinyl-, Piperidin-1-ylchinazolinyl-, Piperazin-1-ylchinazolinyl-, Morpholin-4-ylchinazolinyl-, Ethoxychinazolinyl-, Isopropyloxychinazolinyl-, Phenyloxychinazolinyl-, Imidazo[1,2-a]pyridinyl-, Methyl-imidazo[1,2-a]pyridinyl-, Phenylimidazo[1,2-a]pyridinyl-, Benzylimidazo[1,2-a]pyridinyl-, Pyrazolo[1,5-a]pyridinyl-, Chinoxalinyl-, Methylchinoxalinyl-, Dimethylchinoxalinyl-, Trimethylchinoxalinyl-, Phenylchinoxalinyl-, Methylphthalazinyl-, Naph-thyridinyl-, 2,3-Dihydro-benzo[1,4]dioxinyl-, 1,2-Dihydro-2-oxo-chinolinyl-, 1,2-Di-hydro-1-methyl-2-oxo-chinolinyl-, 1,2-Dihydro-2-methyl-1-oxo-isochinolinyl-, 3,4-Dihydro-4-oxo-phthalazinyl-, 3,4-Dihydro-3-methyl-4-oxo-phthalazinyl-, 3,4-Dihydro-4-oxo-chinazolinyl-, 3,4-Dihydro-3-methyl-4-oxo-chinazolinyl- oder eine 2-Oxo-2H-chromenyl-Gruppe substituiert ist,
eine Phenylcarbonylmethyl-Gruppe,
eine Phenylcarbonylmethylgruppe, in der der Phenylteil durch eine Amino-, Cyanmethylamino-, (Ethyloxycarbonylmethyl)amino-, (Methylaminocarbonyl)methylamino-, Methylcarbonylamino-, Ethylcarbonylamino-, Isopropylcarbonylamino-, Phenylcarbonylamino-, Methoxycarbonylamino-, (Ethyloxycarbonylamino)-carbonylamino- oder eine 2-Oxo-imidazolidin-1-yl-Gruppe substituiert ist,
eine Phenylcarbonylmethylgruppe, in der der Phenylteil durch eine Phenylgruppe substituiert ist,
eine Phenylcarbonylmethylgruppe, in der der Phenylteil durch eine Carboxy-, Methoxycarbonyl-, Ethyloxycarbonyl-, Aminocarbonyl-, Methylaminocarbonyl-, Dimethylaminocarbonyl- oder Morpholin-4-ylcarbonylgruppe substituiert ist,
eine Phenylcarbonylmethylgruppe, in der der Phenylteil durch eine Methylsulfanyl-, Methylsulfinyl- oder Methylsulfonylgruppe substituiert ist,
eine Phenylcarbonylmethylgruppe, in der der Phenylteil durch eine Methoxy-, Difluormethoxy-, Trifluormethoxy-, Ethyloxy-, Isopropyloxy- oder Phenyloxygruppe substituiert ist,
eine Phenylcarbonylmethylgruppe, in der der Phenylteil durch eine Methylsulfinylmethoxy-, Carboxymethoxy-, Ethyloxycarbonylmethoxy-, Isopropyloxycarbonylmethoxy-, Aminocarbonylmethoxy-, Methylaminocarbonylmethoxy-, Ethylaminocarbonylmethoxy-, Isopropylaminocarbonylmethoxy-, Dimethylaminocarbonylmethoxy-, Pyrrolidin-1-ylcarbonylmethoxy- oder Morpholin-4-ylcarbonylmethoxy-Gruppe substituiert ist,
eine Phenylcarbonylmethylgruppe, in der der Phenylteil durch eine 1-(Ethyloxycarbonyl)-1-methyl-ethyloxy-, 1-(Methoxycarbonyl)-ethyloxy- oder eine 1-(Aminocarbonyl)-ethyloxy-Gruppe substituiert ist,
eine Phenylcarbonylmethylgruppe, in der der Phenylteil durch zwei Methoxygruppen substituiert ist,
eine Phenylcarbonylmethylgruppe, in der der Phenylteil durch eine Methoxygruppe und eine Nitrogruppe substituiert ist,
eine Phenylcarbonylmethylgruppe, in der der Phenylteil durch eine Methoxygruppe und eine Aminogruppe substituiert ist,
eine Phenylcarbonylmethylgruppe, in der im Phenylteil zwei benachbarte Wasserstoffatome durch eine -O-CH₂-O-, -O-CF₂-O-, -O-CH₂-CH₂-O-, -NH-CO-NH-, -N(CH₃)-CO-NH-, -N(CH₃)-CO-N(CH₃)-, -NH-CO-O- oder eine -N(CH₃)-CO-O-Gruppe ersetzt sind,
eine (2-Phenylethyl)carbonylmethylgruppe,
eine Naphthylcarbonylmethyl-, Indolylcarbonylmethyl- oder Chinolinylcarbonylmethylgruppe oder
eine 2-Cyanimino-2-phenyl-ethylgruppe,
R² eine Methyl-, Isopropyl-, Cyclopropyl-, Phenyl- oder Fluorphenylgruppe und
R³ eine 2-Methyl-2-propen-1-yl-, 2-Chlor-2-propen-1-yl- oder 3-Brom-2-propen-1-yl-Gruppe,
eine 1-Buten-1-yl-, 3-Methyl-1-buten-1-yl-, 2-Buten-1-yl-, 2-Methyl-2-buten-1-yl- oder 2,3-Dimethyl-2-buten-1-yl-Gruppe,
eine 2-Butin-1-yl-Gruppe,
eine 1-Cyclopenten-1-ylmethyl-Gruppe oder
eine 2-Furanylmethyl-Gruppe bedeuten,
deren Tautomere, Enantiomere, Diastereomere, deren Gemische, deren Prodrugs und deren Salze.

6. Verbindungen der allgemeinen Formel I gemäß Anspruch 5, in denen
R¹ und R² wie in Anspruch 5 erwähnt definiert sind und
R³ eine 1-Buten-1-yl-, 2-Buten-1-yl- oder 2-Butin-1-yl-Gruppe bedeutet,
deren Tautomere, Enantiomere, Diastereomere, deren Gemische und deren Salze.

7. Verbindungen der allgemeinen Formel I gemäß Anspruch 6, in denen
R¹ eine Methylgruppe, die durch eine Naphthyl-, Fluornaphthyl-, Methylnaphthyl-, Methoxynaphthyl-, (Difluormethoxy)-naphthyl-, Cyanonaphthyl- oder Nitronaphthyl-Gruppe substituiert ist,
eine Methylgruppe, die durch eine 2-(Pentafluorphenyl)ethenyl-Gruppe substituiert ist,
eine Methylgruppe, die durch eine Benzofuranyl-, Methylbenzothiazolyl-, Chinolinyl-, Methoxychinolinyl-, Isochinolinyl-, Methylisochinolinyl-, (Difluormethyl)-isochinolinyl-, (Trifluormethyl)-isochinolinyl-, Dimethylisochinolinyl-, (1-Cyano-1-methyl-ethyl)isochinolinyl-, Phenylisochinolinyl-, Methoxyisochinolinyl-, 1,2,3,4-Tetrahydrophenanthridinyl-, Chinazolinyl-, Methylchinazolinyl-, Isopropylchinazolinyl-, Cyclopropylchinazolinyl-, Phenylchinazolinyl-, Aminochinazolinyl-, (Dimethylamino)-chinazolinyl-, Pyrrolidin-1-ylchinazolinyl-, Piperidin-1-ylchinazolinyl-, Piperazin-1-ylchinazolinyl-, Morpholin-4-ylchinazolinyl-, Ethoxychinazolinyl-, Isopropyloxychinazolinyl-, Chinoxalinyl-, Methylchinoxalinyl-, Dimethylchinoxalinyl-, Trimethylchinoxalinyl-, Phenylchinoxalinyl-, [1,5]Naphthyridinyl-, [1,6]Naphthyridinyl-, [1,8]Naphthyridinyl- oder eine 1,2-Dihydro-1-methyl-2-oxo-chinolinyl-Gruppe substituiert ist,
eine Phenylcarbonylmethyl-Gruppe,
eine Phenylcarbonylmethylgruppe, in der der Phenylteil durch eine Phenylgruppe substituiert ist,
eine Phenylcarbonylmethylgruppe, in der der Phenylteil durch eine Methoxy-, Difluormethoxy-, Trifluormethoxy-, Ethyloxy-, Isopropyloxy- oder Phenyloxygruppe substituiert ist,
eine Phenylcarbonylmethylgruppe, in der im Phenylteil zwei benachbarte Wasserstoffatome durch eine -O-CH₂-O-, -O-CF₂-O-, -O-CH₂-CH₂-O-, -N(CH₃)-CO-N(CH₃)- oder eine -N(CH₃)-CO-O-Gruppe ersetzt sind,
eine Naphthylcarbonylmethyl-, Indolylcarbonylmethyl- oder Chinolinylcarbonylmethylgruppe oder
eine 2-Cyanimino-2-phenyl-ethylgruppe,
R² eine Methyl-, Isopropyl-, Cyclopropyl-, Phenyl- oder 4-Fluorphenylgruppe und
R³ eine 1-Buten-1-yl-, 2-Buten-1-yl- oder eine 2-Butin-1-yl-Gruppe bedeuten,
deren Tautomere, Enantiomere, Diastereomere, deren Gemische und deren Salze.

8. Verbindungen der allgemeinen Formel I gemäß Anspruch 5, in denen R¹ und R² wie in Anspruch 7 erwähnt definiert sind und R³ eine 1-Buten-1-yl-Gruppe bedeutet, deren Tautomere, Enantiomere, Diastereomere, deren Gemisch und deren Salze.

9. Verbindungen der allgemeinen Formel I gemäß Anspruch 5, in denen R¹ und R² wie in Anspruch 7 erwähnt definiert sind und R³ eine 2-Buten-1-yl-Gruppe bedeutet, deren Tautomere, Enantiomere, Diastereomere, deren Gemische und deren Salze.

10. Verbindungen der allgemeinen Formel I gemäß Anspruch 5, in denen R¹ und R² wie in Anspruch 7 erwähnt definiert sind und R³ eine 2-Butin-1-yl-Gruppe bedeutet, deren Tautomere, Enantiomere, Diastereomere, deren Gemische und deren Salze.

11. Verbindungen der allgemeinen Formel I, in denen
R¹ eine Methylgruppe, die durch eine Naphthyl-, Fluornaphthyl-, Methylnaphthyl-, Methoxynaphthyl-, (Difluormethoxy)-naphthyl-, Cyanonaphthyl- oder Nitronaphthyl-Gruppe substituiert ist,
eine Methylgruppe, die durch eine 2-(Pentafluorphenyl)ethenyl-Gruppe substituiert ist, oder
eine Methylgruppe, die durch eine Benzofuranyl-, Methylbenzothiazolyl-, Chinolinyl-, Methoxychinolinyl-, Isochinolinyl-, Methylisochinolinyl-, (Difluormethyl)-isochinolinyl-, (Trifluormethyl)-isochinolinyl-, Dimethylisochinolinyl-, (1-Cyano-1-methyl-ethyl)isochinolinyl-, Phenylisochinolinyl-, Methoxyisochinolinyl-, 1,2,3,4-Tetrahydrophenanthridinyl-, Chinazolinyl-, Methylchinazolinyl-, Isopropylchinazolinyl-, Cyclopropylchinazolinyl-, Phenylchinazolinyl-, Aminochinazolinyl-, (Dimethylamino)-chinazolinyl-, Pyrrolidin-1-ylchinazolinyl-, Piperidin-1-ylchinazolinyl-, Piperazin-1-ylchinazolinyl-, Morpholin-4-ylchinazolinyl-, Ethoxychinazolinyl-, Isopropyloxychinazolinyl-, Chinoxalinyl-, Methylchinoxalinyl-, Dimethylchinoxalinyl-, Trimethylchinoxalinyl-, Phenylchinoxalinyl-, [1,5]Naphthyridinyl-, [1,6]Naphthyridinyl-, [1,8]Naphthyridinyl- oder eine 1,2-Dihydro-1-methyl-2-oxo-chinolinyl-Gruppe substituiert ist,
R² eine Methyl-, Isopropyl-, Cyclopropyl- oder Phenylgruppe und
R³ eine 2-Chlorbenzyl-, 2-Brombenzyl-, 2-Ethinylbenzyl- oder 2-Cyanbenzyl-Gruppe bedeuten,
deren Tautomere, Enantiomere, Diastereomere, deren Gemische und deren Salze.

12. Verbindungen der allgemeinen Formel I gemäß Anspruch 11, in denen
R¹ eine (3-Methyl-isochinolin-1-yl)methylgruppe,
R² eine Methylgruppe und
R³ eine 2-Chlorbenzyl-, 2-Brombenzyl-, 2-Ethinylbenzyl- oder 2-Cyanbenzyl-Gruppe bedeuten,
deren Tautomere, Enantiomere, Diastereomere, deren Gemische und deren Salze.

13. Verbindungen der allgemeinen Formel I gemäß Anspruch 11, in denen R¹ und R² wie in Anspruch 11 erwähnt definiert sind und R³ eine 2-Chlorbenzyl-Gruppe bedeutet, deren Tautomere, Enantiomere, Diastereomere, deren Gemische und deren Salze.

14. Verbindungen der allgemeinen Formel I gemäß Anspruch 11, in denen R¹ und R² wie in Anspruch 11 erwähnt definiert sind und R³ eine 2-Brombenzyl-Gruppe bedeutet, deren Tautomere, Enantiomere, Diastereomere, deren Gemische und deren Salze.

15. Verbindungen der allgemeinen Formel I gemäß Anspruch 11, in denen R¹ und R² wie in Anspruch 11 erwähnt definiert sind und R³ eine 2-Ethinylbenzyl-Gruppe bedeutet, deren Tautomere, Enantiomere, Diastereomere, deren Gemische und deren Salze.

16. Verbindungen der allgemeinen Formel I gemäß Anspruch 11, in denen R¹ und R² wie in Anspruch 11 erwähnt definiert sind und R³ eine 2-Cyanbenzyl-Gruppe bedeutet, deren Tautomere, Enantiomere, Diastereomere, deren Gemische und deren Salze.

17. Folgende Verbindungen der allgemeinen Formel I gemäß den Ansprüchen 1, 5 und 11:
(1) 1-[(Chinazolin-2-yl)methyl]-3-methyl-7-(2-butin-1-yl)-8-((*R*)-3-amino-piperidin-1-yl)-xanthin,
(2) 1-(2-{2-[(Ethylaminocarbonyl)methoxy]-phenyl}-2-oxo-ethyl)-3-methyl-7-(2-butin-1-yl)-8-((*R*)-3-amino-piperidin-1-yl)-xanthin,
(3) 1-(2-{2-[(Methylaminocarbonyl)methoxy]-phenyl}-2-oxo-ethyl)-3-methyl-7-(2-butin-1-yl)-8-((*R*)-3-amino-piperidin-1-yl)-xanthin,
(4) 1-(2-Phenyl-2-oxo-ethyl)-3-methyl-7-((E)-2-buten-1-yl)-8-((*R*)-3-amino-piperidin-1-yl)-xanthin,
(5) 1-[(3-Methyl-isochinolin-1-yl)methyl]-3-methyl-7-(2-butin-1-yl)-8-((S)-3-amino-piperidin-1-yl)-xanthin,
(6) 1-[(3-Methyl-isochinolin-1-yl)methyl]-3-methyl-7-(2-butin-1-yl)-8-((R)-3-amino-piperidin-1-yl)-xanthin,
(7) 1-[(4-Methyl-isochinolin-1-yl)methyl]-3-methyl-7-(2-butin-1-yl)-8-((S)-3-amino-piperidin-1-yl)-xanthin,
(8) 1-[(4-Methyl-isochinolin-1-yl)methyl]-3-methyl-7-(2-butin-1-yl)-8-((R)-3-amino-piperidin-1-yl)-xanthin,
(9) 1-[2-(2,3-Dihydro-benzo[1,4]dioxin-5-yl)-2-oxo-ethyl]-3-methyl-7-(2-butin-1-yl)-8-(3-(*R*)-amino-piperidin-1-yl)-xanthin,
(10) 1-[(4-Methoxy-naphthalin-1-yl)methyl]-3-methyl-7-(2-butin-1-yl)-8-((S)-3-amino-piperidin-1-yl)-xanthin,
(11) 1-[(4-Methoxy-naphthalin-1-yl)methyl]-3-methyl-7-(2-butin-1-yl)-8-((R)-3-amino-piperidin-1-yl)-xanthin,
(12) 1-[2-(Benzo[1,3]dioxol-4-yl)-2-oxo-ethyl]-3-methyl-7-(2-butin-1-yl)-8-((*R*)-3-amino-piperidin-1-yl)-xanthin,
(13) 1-[(4-Methyl-chinazolin-2-yl)methyl]-3-methyl-7-(2-butin-1-yl)-8-(3-(*R*)-amino-piperidin-1-yl)-xanthin,
(14) 1-(2-{2-[(Isopropylcarbonyl)amino]-phenyl}-2-oxo-ethyl)-3-methyl-7-((E)-2-buten-1-yl)-8-((*S*)-3-amino-piperidin-1-yl)-xanthin,
(15) 1-(2-{2-[(Methylaminocarbonyl)methoxy]-phenyl}-2-oxo-ethyl)-3-methyl-7-((E)-2-buten-1-yl)-8-((R)-3-amino-piperidin-1-yl)-xanthin,
(16) 1-(2-{2-[(Methylaminocarbonyl)methoxy]-phenyl}-2-oxo-ethyl)-3-methyl-7-((E)-2-buten-1-yl)-8-((*S*)-3-amino-piperidin-1-yl)-xanthin,
(17) 1-(2-{2-[(Isopropylcarbonyl)amino]-phenyl}-2-oxo-ethyl)-3-methyl-7-(2-butin-1-yl)-8-((*R*)-3-amino-piperidin-1-yl)-xanthin,
(18) 1-(2-{2-[(Isopropylcarbonyl)amino]-phenyl}-2-oxo-ethyl)-3-methyl-7-((E)-2-buten-1-yl)-8-((*R*)-3-amino-piperidin-1-yl)-xanthin,
(19) 1-[(4-Cyano-naphthalin-1-yl)methyl]-3-methyl-7-(2-butin-1-yl)-8-((R)-3-amino-piperidin-1-yl)-xanthin,
(20) 1-[(4-Phenyl-chinazolin-2-yl)methyl]-3-methyl-7-(2-butin-1-yl)-8-((R)-3-amino-piperidin-1-yl)-xanthin,
(21) 1-[(8-Methyl-chinoxalin-6-yl)methyl]-3-methyl-7-(2-butin-1-yl)-8-((*R*)-3-amino-piperidin-1-yl)-xanthin,
(22) 1-[(4-Fluor-naphthalin-1-yl)methyl]-3-methyl-7-(2-butin-1-yl)-8-((*R*)-3-amino-piperidin-1-yl)-xanthin,
(23) 1-((E)-3-Pentafluorphenyl-allyl)-3-methyl-7-(2-butin-1-yl)-8-((*R*)-3-amino-piperidin-1-yl)-xanthin ,
(24) 1-[(3-Trifluormethyl-isochinolin-1-yl)methyl]-3-methyl-7-(2-butin-1-yl)-8-((R)-3-amino-piperidin-1-yl)-xanthin,
(25) 1-[(3-Difluormethyl-isochinolin-1-yl)methyl]-3-methyl-7-(2-butin-1-yl)-8-((*R*)-3-amino-piperidin-1-yl)-xanthin,
(26) 1-[2-(Biphenyl-2-yl)-2-oxo-ethyl]-3-methyl-7-(2-butin-1-yl)-8-((*R*)-3-amino-piperidin-1-yl)-xanthin,
(27) 1-[(3-Methyl-isochinolin-1-yl)methyl]-3-cyclopropyl-7-(2-butin-1-yl)-8-((R)-3-amino-piperidin-1-yl)-xanthin,
(28) 1-[2-(3-Methoxy-phenyl)-2-oxo-ethyl]-3-methyl-7-(2-butin-1-yl)-8-((*R*)-3-amino-piperidin-1-yl)-xanthin,
(29) 1-[(3-Methyl-isochinolin-1-yl)methyl]-3-methyl-7-(2-chlor-benzyl)-8-((*R*)-3-amino-piperidin-1-yl)-xanthin und
(30) 1-[(3-Methyl-isochinolin-1-yl)methyl]-3-methyl-7-(2-brom-benzyl)-8-((*R*)-3-amino-piperidin-1-yl)-xanthin
sowie deren Tautomere, Enantiomere, Diastereomere, deren Gemische und deren Salze.

18. Physiologisch verträgliche Salze der Verbindungen nach mindestens einem der Ansprüche 1 bis 17 mit anorganischen oder organischen Säuren oder Basen.

19. Arzneimittel, enthaltend eine Verbindung nach mindestens einem der Ansprüche 1 bis 17 oder ein physiologisch verträgliches Salz gemäß Anspruch 18 neben gegebenenfalls einem oder mehreren inerten Trägerstoffen und/oder Verdünnungsmitteln.

20. Verwendung einer Verbindung nach mindestens einem der Ansprüche 1 bis 18 zur Herstellung eines Arzneimittels, das zur Behandlung von Diabetes mellitus Typ I und Typ II, Arthritis, Adipositas, Allograft Transplantation und durch Calcitonin verursachte Osteoporose geeignet ist.

21. Verfahren zur Herstellung eines Arzneimittels gemäß Anspruch 19, **dadurch gekennzeichnet, daß** auf nichtchemischen Weg eine Verbindung nach mindestens einem der Ansprüche 1 bis 18 in einen oder mehrere inerte Trägerstoffe und/oder Verdünnungsmittel eingearbeitet wird.

22. Verfahren zur Herstellung der Verbindungen der allgemeinen Formel I gemäß den Ansprüchen 1 bis 18, **dadurch gekennzeichnet, daß**
a) eine Verbindung der allgemeinen Formel 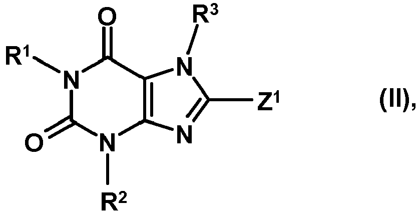 in der
R¹ bis R³ wie in den Ansprüchen 1 bis 16 erwähnt definiert sind und
Z¹ eine Austrittsgruppe wie ein Halogenatom, eine substituierte Hydroxy-, Mercapto-, Sulfinyl-, Sulfonyl- oder Sulfonyloxygruppe darstellt,
mit 3-Aminopiperidin, dessen Enantiomeren oder dessen Salzen umgesetzt wird, oder
b) eine Verbindung der allgemeinen Formel 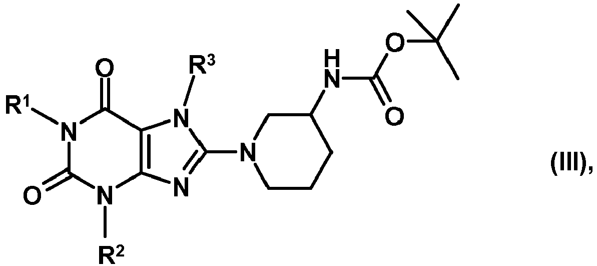 in der R¹, R² und R³ wie in den Ansprüchen 1 bis 16 erwähnt definiert sind, entschützt wird, oder
c) zur Herstellung einer Verbindung der allgemeinen Formel I, in der R¹ gemäß der in Anspruch 1 erwähnten Definition eine Carboxygruppe enthält:
eine Verbindung der allgemeinen Formel 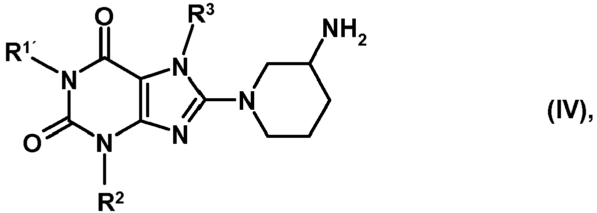
in der R² und R³ wie in den Ansprüchen 1 bis 16 erwähnt definiert sind und R^{1'} eine durch eine C₁₋₄-Alkylgruppe geschützte Carboxygruppe enthält, entschützt wird und/oder
anschließend gegegebenenfalls während der Umsetzung verwendete Schutzgruppen abgespalten werden und/oder
die so erhaltenen Verbindungen der allgemeinen Formel I in ihre Enantiomeren und/oder Diastereomeren aufgetrennt werden und/oder
die erhaltenen Verbindungen der Formel I in ihre Salze, insbesondere für die pharmazeutische Anwendung in ihre physiologisch verträglichen Salze mit anorganischen oder organischen Säuren oder Basen, übergeführt werden.
